# EUROPEAN PATENT APPLICATION

(11) **EP 3 034 620 A1**
(43) Date of publication of application: **22.06.2016**
(21) Application number: 14198610.9
(22) Date of filing: 17.12.2014
(51) Int. Cl.: C12Q 1/68

(54) **Compositions and methods for diagnosing thyroid cancer**

(71) Applicant: Diaxonhit, 75013 Paris (FR)
(72) Inventor: Beurdeley, Pascale, 94120 Fontenay sous Bois (FR); Brenner, Michael, Damascus, MD 20872 (US); Jarrige, Anne-Charlotte, 75014 Paris (FR); Pallaris, Diego, 75014 Paris (FR)
(74) Representative: Becker, Philippe

(57) **Abstract**

The present application relates to methods and compositions for detecting thyroid cancer in mammals, in particular in humans. The invention provides novel biomarkers which, in combination, allow effective, specific and sensitive detection of thyroid cancer, particularly to assist in the diagnosis of thyroid lesions in human subjects. The invention further relates to tools and/or kits (such as reagents, probes, primers, chips or arrays) suitable for implementing said methods, and the preparation and uses thereof.

## Description

The present application relates to methods and compositions for detecting thyroid cancer in mammals, in particular in human beings. The invention provides novel biomarkers which allow effective, specific and sensitive detection of thyroid cancer, particularly to assist in the diagnosis of thyroid lesions in human subjects. The invention further relates to tools and/or kits (such as reagents, probes, primers, chips or arrays) suitable for implementing said methods, and the preparation and uses thereof.

Thyroid cancer is the most common malignancy of the endocrine system (Curado, Edwards et al. 2007). Over 60 000 new cases are diagnosed each year in the US; despite its low malignancy potential, it is estimated that 1,890 patients will die of this disease in 2014 (http://seer.cancer.gov/statfacts/html/thyro.html accessed October 15, 2014).

Three different types of thyroid cancer have been defined according to their histological features: differentiated thyroid cancer (DTC) deriving from epithelial cells from the thyroid follicles; medullary thyroid cancer (MTC); and anaplastic thyroid cancer (ATC). Approximately 90% of diagnosed thyroid cancers correspond to DTC, with papillary (PTC) histology being the most frequent (75%), followed by follicular (FTC) (10%), Hürthle cells (5%), and poorly differentiated carcinomas (1-6%). Overall, MTC accounts for approximately 10% of all thyroid tumors and ATC barely 1% [2]. Although the papillary thyroid cancer (PTC) is the most frequent, the follicular thyroid cancer (FTC) is less common but more aggressive. Hürthle cell thyroid cancer (HCC) is an uncommon but distinct and generally more aggressive type of thyroid cancer, which is now classified by the World Health Organization (WHO) as a subtype of follicular cancer (Harris and Bible 2011). Most DTC patients have a very good prognosis if diagnosed at early stages, with a 91 % survival rate at 20 years when the classical treatment with surgery followed by suppression of thyroid stimulating hormone (TSH) is employed.

Thyroid nodules are a frequent clinical finding during thyroid examination with a prevalence ranging between 4 and 7% in the general population. This prevalence is even higher in case of ultrasonography of the cervical area and can reach 50% of patients over the age of 65. The most accurate tool for evaluating thyroid nodules is fine needle aspiration (FNA), with an accuracy > 95%. There are several classification systems for thyroid cytology, the most recognized and the most widely used is the Bethesda classification (Cibas and Ali, 2009) composed of 6 categories for which the recommended patient management is different:
1. Bethesda category I: Non diagnostic (frequency: about 10%): the cytological analysis is not possible because, for instance, insufficient cells have been collected, the repeat of the FNA is recommended.
2. Cat II: Benign (frequency: about 70%): the malignancy rate is below 3%, the patients are referred to clinical follow up.
3. Cat III: Follicular lesion or neoplasm of unknown significance: the malignancy rate is of 5% to 15%, the patients are referred to clinical follow up and repeated FNA.
4. Cat IV: Follicular lesion or neoplasm: the malignancy rate is in the range of 15% to 30%. The recommended approach can include partial or complete thyroidectomy. The frequency of follicular neoplasms and atypia/follicular lesions of undetermined significance is about 15%.
5. Cat V: Suspicious of malignancy: the malignancy rate is of 60% to 75% and complete removal of the thyroid is recommended.
6. Cat VI: Malignant: the malignancy rate is 97% to 99%, the patient's thyroid gland should be resected.

The frequency of category V and category VI (suspicion for malignancy and malignancy) is ≤5%.

Because FNA is unable to provide a definitive diagnosis for the nodules in categories 3, 4 and 5, most patients with indeterminate cytology undergo diagnostic surgery (i.e., partial thyroidectomy) to establish a histopathological diagnosis. If malignancy is identified, the patients require a complete thyroid resection. In contrast, if the pathological exam reveals a benign nodule, partial thyroidectomy has proven to be unnecessary (frequency: 60 to 80%). This situation of indeterminate cytology is encountered in up to 25% of the patients with a thyroid nodule undergoing FNA and shows the need of more powerful molecular tools to decrease the number of unnecessary surgeries.

Recent work has investigated the potential benefit of combining microscopic and mutation analyses (Nikiforov et al, 2011). There are several molecular tests are available such as Thyroseq (Nikiforova et al, 2013) or Afirma (Alexander et al, 2012). These tests, however, have either low specificity or low sensitivity. Epigenetic and peripheral blood markers have also been studied but they have limited sensitivity and fail to detect 30% to 40% of cancers (Hu et al, 2006).

There is a need for alternative methods of detecting thyroid cancer that are specific and sensitive, therefore increasing the number of definitive diagnoses and reducing unnecessary diagnostic surgeries.

### Summary of the Invention

The present invention provides novel biomarkers which allow very specific and sensitive diagnosis of thyroid cancer. Starting from approximately 20 000 genes and 35 000 alternative splicing events, the inventors have been able to identify target genes as highly relevant biomarkers of thyroid cancer. By combining expression markers from these genes and/or domains of these genes, it is possible to determine with a high level of certainty, the malignant *vs* benign stage of thyroid lesions in human samples. The invention specifically improves the ability to distinguish malignant versus benign profiles within a population of FNA samples being considered as "indeterminate" (especially in categories III and IV from the Bethesda classification) during a cytological exam. By providing a determinant diagnosis to subjects initially identified as "indeterminate" the invention improves the clinical management of patients with thyroid nodules in order to avoid unnecessary thyroid surgeries.

An object of the invention more particularly relates to a method for aiding in the diagnosis of thyroid cancer in mammals, comprising measuring a set of target biomarkers in a biological sample from the mammal and diagnosing said thyroid cancer by comparing the measured value to a reference value, wherein the set of target biomarkers comprises an expression product of at least two genes selected among CITED1, TENM1, CCM2, SCLY and IGSF10 or at least two distinct domains of at least one expression product of a gene selected among CITED1, TENM1, CCM2, SCLY and IGSF10.

A further object of the invention relates to a method as defined above for determining whether the mammal has or is likely to have a malignant thyroid lesion.

Another object of the invention relates to a method for diagnosing thyroid cancer in mammals, comprising measuring an expression product of at least two genes selected among CITED1, TENM1, CCM2, SCLY and IGSF10, or at least two distinct domains of at least one expression product of a gene selected among CITED1, TENM1, CCM2, SCLY and IGSF10, in a biological sample from the mammal, and diagnosing said thyroid cancer by comparing the measured value to a reference value.

The invention further relates to the use of a set of target biomarkers comprising an expression product of at least two genes selected among CITED1, TENM1, CCM2, SCLY and IGSF10, or at least two distinct domains of at least one expression product of a gene selected among CITED 1, TENM1, CCM2, SCLY and IGSF10, for the diagnosis in vitro or ex vivo of thyroid cancer.

In a preferred embodiment, the method comprises measuring in a biological sample from the subject an expression product of CITED1 and an expression product of TENM1. In a further preferred embodiment, the method comprises measuring in a biological sample from the subject an expression product of CITED1, TENM1, CCM2, SCLY and IGSF10 genes.

The above biomarkers may be further combined with additional biomarkers. In this regard, in a particular embodiment, the set of biomarkers further comprises an expression product of the FRMD3 gene.

In further particular embodiments of the invention, the set of biomarkers further comprises at least one protein or RNA encoded by a gene selected from Table A, preferably each gene listed in Table A.

In further particular embodiments of the invention, the set of biomarkers further comprises at least one protein or RNA encoded by a gene selected from Table B, preferably each gene listed in Table B.

In a particular embodiment of the invention, the set of biomarkers comprises one expression product of CITED1, TENM1, CCM2, SCLY, IGSF10 and FRMD3 and of each gene listed in Table A and in Table B.

In a specific mode of carrying out the invention, the method comprises:
a. providing a biological sample (comprising thyroid cells) from the subject having a thyroid condition or suspected to have a thyroid condition; and
b. measuring in said biological sample the expression of at least two genes selected among CITED1, TENM1, CCM2, SCLY and IGSF10, or at least two distinct domains of at least one expression product of a gene selected among CITED1, TENM1, CCM2, SCLY and IGSF10.

A further object of the invention resides in a kit comprising a solid support comprising at least two capture agents attached thereto, wherein each capture agent binds to an expression product of a distinct gene selected among CITED1, TENM1, CCM2, SCLY and IGSF10, or to at least two distinct domains of at least one expression product of a gene selected among CITED1, TENM1, CCM2, SCLY and IGSF10.

Another object of the invention relates to a method of treating thyroid cancer in a subject, the method comprising (i) measuring the expression product of at least 2 genes selected among CITED1, TENM1, CCM2, SCLY and IGSF10 in a sample from the subject, said measure allowing the diagnosis of thyroid cancer, and (ii) resecting the cancer when the subject is diagnosed to have thyroid cancer in step (i).

The invention may be used on any mammal, in particular any human subject. It is particularly effective to discriminate between malignant and benign thyroid lesions or nodules in a subject, and therefore to determine whether a thyroid lesion in a subject is malignant. The invention may be used on cellular samples, tissue biopsy or fine thyroid needle aspirate.

### Legend to the figures

Figure 1: ROC curves generated using a p-value cut-off of 10⁻³. **A**. Correlation with Malignant profile, **B**. Correlation with Benign profile and **C**. Difference between Correlation with Benign and Malignant profiles.
Figure 2: Correlation of each specimen with the average benign and malignant profiles (cut-off 10⁻³).
Figure 3: ROC curves generated using a p-value cut-off of 10⁻⁵. **A**. Correlation with Malignant profile, **B.** Correlation with Benign profile and **C.** Difference between Correlation with Benign and Malignant profiles.
Figure 4: Correlation of each specimen with the average benign and malignant profiles (cut-off 10⁻⁵).
Figure 5: ROC curves generated using a p-value cut-off of 10⁻⁷. **A**. Correlation with Malignant profile, **B**. Correlation with Benign profile and **C**. Difference between Correlation with Benign and Malignant profiles.
Figure 6: Correlation of each specimen with the average benign and malignant profiles (cut-off 10⁻⁷).

### Detailed Description of the invention

The present invention provides novel methods for diagnosing thyroid cancer based on the analysis of new biomarkers. Upon comprehensive analysis of all of the human genes in biological samples from patients with malignant or benign nodules, the inventors identified genes which represent strong diagnostic biomarkers of thyroid cancer and may be used to determine, with high level of certainty, the malignant *vs* benign status of thyroid lesions in human subject samples. Expression of these gene products is strongly correlated to the disease and allows discrimination between malignant and benign nodules of the thyroid gland based on only two genes or on only two distinct domains of one of said genes.

An object of the invention more particularly relates to a method for (aiding in) the diagnosis of thyroid cancer in mammals, comprising the measurement of an expression product of at least two genes selected among CITED1, TENM1, CCM2, SCLY and IGSF10 or of two distinct domains of one of said genes, in a sample from the mammal and the diagnosis of said thyroid cancer by comparing the measured value to a reference value.

Another object of the invention relates to a method for (aiding in) the diagnosis of thyroid cancer in mammals, comprising the measurement of the expression of at least two distinct domains of at least one, preferably at least 2 genes selected among CITED1, TENM1, CCM2, SCLY and IGSF10 in a sample from the mammal and the diagnosis of said thyroid cancer by comparing the measured value to a reference value.

In a particular embodiment, the method comprises:
a) providing a biological sample from a subject having a thyroid condition (e.g. a thyroid lesion or nodule) or suspected to have a thyroid condition; and
b) measuring in said biological sample the expression product of genes as defined above.

In a further particular embodiment, the invention is conducted after cytological exam and allows the diagnosis of patient samples remaining "indeterminate" after said cytological exam. In this regard, a particular method of the invention comprises:
a) the provision (e.g., the collection) of a biological sample from the subject having a thyroid condition or suspected to have a thyroid condition;
b) a cytological exam of the sample to classify the thyroid condition (in a category comprised between 1 and 6) according to the Bethesda classification, and
c) for any sample of indeterminate cytology, the measurement of the expression product of target genes as described above.

### Target genes

The invention discloses the identification of genes whose expression products allow efficient diagnosis of thyroid cancer. Said genes have been selected from a set of approximately 20,000 human genes and demonstrate a greaterthan 97% specificity in a thyroid cancer diagnostic, which is remarkable. These genes, in various combinations, are sufficient to produce strong diagnostic results of thyroid cancer, which is very advantageous when compared with current proposed tests comprised of more than 100 genes.

Preferred target genes for use in the invention are CITED1, TENM1, CCM2, SCLY and IGSF10. As disclosed in the examples, detecting a gene product of at least 2 of these genes, or at least two domains of a gene product of one of these genes, is sufficient to provide a reliable diagnosis of thyroid cancer.

The full length nucleic acid sequences of these genes are publicly available and may be found in the NCBI Entrez gene database. The identifications for each of these genes is provided below as well as in Table C:

| Entrez Gene ID | Gene name | Gene description |
|---|---|---|
| 4435 | CITED 1 | Cbp/p300-interacting transactivator, with Glu/Asp-rich carboxy-terminal domain, 1 |
| 10178 | TENM1 | odz, odd Oz/ten-m homolog 1(Drosophila) |
| 51540 | SCLY | selenocysteine lyase |
| 83605 | CCM2 | cerebral cavernous malformation 2 |
| 257019 | FRMD3 | FERM domain containing 3 |
| 285313 | IGSF10 | immunoglobulin superfamily, member 10 |

Within the context of the present invention, the term "CITED1 gene" designates preferably a human CITED1 gene, particularly a nucleic acid molecule or sequence comprising (i) a sequence of gene# 4435 or a sequence complementary thereto; or (ii) a natural variant of a sequence of (i) such as a polymorphism; or (iii) a sequence having at least 90% identity, preferably at least 95, 96, 97, 98 or 99%, to a sequence of (i) or (ii); or (iv) a sequence hybridizing under stringent conditions to a sequence of (i) or (ii).

The same definition applies to the TENM1, CCM2, SCLY and IGSF10 genes by reference to gene# 10178, 83605, 51540 and 285313, respectively, as well as to any other gene quoted in the present application, by reference to the corresponding reference sequence (see Tables A to C).

The present invention shows that the above genes or alternative isoforms of those genes are deregulated in thyroid cancer subjects so that any domain or sequence of an expression product encoded by said genes may be measured. For improved detection, however, especially when the method is based on a combined measure of the expression product of only 1 to 3 of said genes, the invention shows that particular domains or sequences ("target sequences") may be targeted in the expression products of these genes for improved thyroid cancer diagnosis.

In this regard, in relation to CITED1 gene, the method preferably comprises measuring (e.g., determining the presence, absence or amount of) at least one sequence encoded by exon4, exon3, or exon(-2) in an expression product of the CITED1 gene. As shown in the experimental section, by designing reagents (e.g., probes or primers) that detect such preferred domains of CITED1 gene products, a more specific and sensitive detection of thyroid cancer is obtained. In a more preferred embodiment, the method comprises measuring (e.g., determining the presence, absence or amount of) a target sequence of CITED1 comprised in any of the sequences listed as SEQ ID NOs: 106-119.

With regard to TENM1, in a particular embodiment, the method comprises measuring (e.g., determining the presence, absence or amount of) at least one sequence encoded by exon1, 3, 13, 15, 17, 18, 19, 25 or 32 in an expression product of the TENM1 gene. As shown in the experimental section, by designing reagents that detect such preferred domains of TENM1 gene products, a more specific and sensitive detection of thyroid cancer is obtained. In a more preferred embodiment, the method comprises measuring (e.g., determining the presence, absence or amount of) a target sequence of TENM1 comprised in any of the sequences listed as SEQ ID NOs: 817-838.

With regard to CCM2, in a particular embodiment, the method comprises measuring a target sequence comprised in SEQ ID NO: 83.

With regard to SCLY, in a particular embodiment, the method comprises measuring a target sequence comprised in SEQ ID NO: 749.

With regard to IGSF10, in a particular embodiment, the method comprises measuring a target sequence comprised in SEQ ID NO: 335.

Moreover, in a further particular embodiment, the invention comprises measuring at least two distinct target sequence of an expression product the above target genes. The invention indeed shows that by monitoring two or more distinct domains in e.g., CITED1 or TENM1 gene products, an efficient and reliable diagnosis of thyroid cancer is made.

In this regard, in a particular embodiment, the method comprises measuring at least one sequence encoded by exon4 of the CITED1 gene and at least one sequence encoded by exon 3 and/or exon(-2) of the CITED1 gene.

In another particular embodiment, the method comprises measuring at least one sequence encoded by exon17, 18, 19, 25, or 32 of the TENM1 gene and at least one sequence encoded by exon1, 3, 13 or 15 of the TENM1 gene.

The results shown in the experimental section demonstrate that, by detecting distinct target sequences or domains of a single target gene as defined above, a very reliable detection of thyroid cancer is obtained. For instance, by detecting 3 domains of CITED1, a sensitivity and specificity of 75% and 90%, respectively, can be achieved, and by detecting several distinct target sequences as defined above of CITED 1 and TENM1, a specificity of 92.5% can be obtained, which is remarkable and suitable to design a commercial test.

In a preferred embodiment, the invention comprises measuring a gene product of at least the CITED 1 and TENM1 genes.

In another embodiment, the invention comprises measuring a gene product of at least the CITED 1 and CCM2 genes.

In another embodiment, the invention comprises measuring a gene product of at least the CITED 1 and SCLY genes.

In another embodiment, the invention comprises measuring a gene product of at least the CITED 1, TENM1, CCM2, SCLY and IGSF10 genes.

Furthermore, while the above 5 target genes already provide highly reliable test, it is possible to further improve the method by combining said genes with further marker genes identified by the inventors.

In this regard, in a particular and preferred embodiment, the method further comprises the determination of at least one gene product of the FRMD3 gene (gene#257019). In a particular embodiment, for measuring FRMD3 gene expression, the method comprises measuring a target sequence comprised in any of the sequences listed as SEQ ID NOs: 249 to 269.

In a preferred embodiment, the invention comprises measuring a gene product of at least the CITED 1 and FRMD3 genes.

In another embodiment, the invention comprises measuring a gene product of at least the CITED 1 and TENM1 and FRMD3 genes.

In another embodiment, the invention comprises measuring a gene product of at least the CITED 1, TENM1, CCM2, SCLY, IGSF10, and FRMD3 genes.

Moreover, to design more universal tests, the invention combines additional marker(s) identified by the inventors, selected from the expression products of the genes listed in table A and/or in Table B below:

**Table A**

| **Entrez Gene ID** | **Gene name** | **Gene description** |
|---|---|---|
| 607 | BCL9 | B-cell CLL/lymphoma 9 |
| 954 | ENTPD2 | ectonucleoside triphosphate diphosphohydrolase 2 |
| 956 | ENTPD3 | ectonucleoside triphosphate diphosphohydrolase 3 |
| 1000 | CDH2 | cadherin 2, type 1, N-cadherin (neuronal) |
| 1756 | DMD | dystrophin |
| 2059 | EPS8 | epidermal growth factor receptor pathway substrate 8 |
| 2563 | GABRD | gamma-aminobutyric acid (GABA) A receptor, delta |
| 3489 | IGFBP6 | insulin-like growth factor binding protein 6 |
| 4038 | LRP4 | low density lipoprotein receptor-related protein 4 |
| 4133 | MAP2 | microtubule-associated protein 2 |
| 5090 | PBX3 | pre-B-cell leukemia homeobox 3 |
| 6258 | RXRG | retinoid X receptor, gamma |
| 6271 | S100A1 | S100 calcium binding protein A1 |
| 6447 | SCG5 | secretogranin V (7B2 protein) |
| 6585 | SLIT1 | slit homolog 1 (Drosophila) |
| 6715 | SRD5A1 | steroid-5-alpha-reductase, alpha polypeptide 1 (3-oxo-5 alpha-steroid delta 4-dehydrogenase alpha 1) |
| 6920 | TCEA3 | transcription elongation factor A (SII), 3 |
| 7024 | TFCP2 | transcription factor CP2 |
| 7991 | TUSC3 | tumor suppressor candidate 3 |
| 10921 | RNPS1 | RNA binding protein S 1, serine-rich domain |
| 22807 | IKZF2 | IKAROS family zinc finger 2 (Helios) |
| 23043 | TNIK | TRAF2 and NCK interacting kinase |
| 23248 | RPRD2 | regulation of nuclear pre-mRNA domain containing 2 |
| 23362 | PSD3 | pleckstrin and Sec7 domain containing 3 |
| 25963 | TMEM87A | transmembrane protein 87A |
| 55164 | SHQ1 | SHQ1 homolog (S. cerevisiae) |
| 93035 | PKHD1L1 | polycystic kidney and hepatic disease 1 (autosomal recessive)-like 1 |
| 120892 | LRRK2 | leucine-rich repeat kinase 2 |
| 134548 | SOWAHA | ankyrin repeat domain 43 |
| 143872 | ARHGAP42 | Rho GTPase activating protein 42 |
| 153090 | DAB2IP | DAB2 interacting protein |
| 164832 | LONRF2 | LON peptidase N-terminal domain and ring finger 2 |
| 165215 | FAM171B | family with sequence similarity 171, member B |
| 200879 | LIPH | lipase, member H |

**Table B**

| **Entrez Gene ID** | **Gene name** | **Gene description** |
|---|---|---|
| 35 | ACADS | acyl-CoA dehydrogenase, C-2 to C-3 short chain |
| 132 | ADK | adenosine kinase |
| 134 | ADORA1 | adenosine A1 receptor |
| 165 | AEBP1 | AE binding protein 1 |
| 334 | APLP2 | amyloid beta (A4) precursor-like protein 2 |
| 430 | ASCL2 | achaete-scute complex homolog 2 (Drosophila) |
| 627 | BDNF | brain-derived neurotrophic factor |
| 720 | C4A | complement component 4A (Rodgers blood group) |
| 721 | C4B | complement component 4B (Chido blood group) |
| 770 | CA11 | carbonic anhydrase XI |
| 825 | CAPN3 | calpain 3, (p94) |
| 835 | CASP2 | caspase 2, apoptosis-related cysteine peptidase |
| 836 | CASP3 | caspase 3, apoptosis-related cysteine peptidase |
| 894 | CCND2 | cyclin D2 |
| 953 | ENTPD1 | ectonucleoside triphosphate diphosphohydrolase 1 |
| 961 | CD47 | CD47 molecule |
| 1001 | CDH3 | cadherin 3, type 1, P-cadherin (placental) |
| 1014 | CDH16 | cadherin 16, KSP-cadherin |
| 1188 | CLCNKB | chloride channel Kb |
| 1271 | CNTFR | ciliary neurotrophic factor receptor |
| 1281 | COL3A1 | collagen, type III, alpha 1 |
| 1290 | COL5A2 | collagen, type V, alpha 2 |
| 1465 | CSRP1 | cysteine and glycine-rich protein 1 |
| 1474 | CST6 | cystatin E/M |
| 1499 | CTNNB1 | catenin (cadherin-associated protein), beta 1, 88kDa |
| 1508 | CTSB | cathepsin B |
| 1611 | DAP | death-associated protein |
| 1717 | DHCR7 | 7-dehydrocholesterol reductase |
| 1728 | NQO1 | NAD(P)H dehydrogenase, quinone 1 |
| 1730 | DIAPH2 | diaphanous homolog 2 (Drosophila) |
| 1809 | DPYSL3 | dihydropyrimidinase-like 3 |
| 1837 | DTNA | dystrobrevin, alpha |
| 1846 | DUSP4 | dual specificity phosphatase 4 |
| 1877 | E4F1 | E4F transcription factor 1 |
| 1889 | ECE1 | endothelin converting enzyme 1 |
| 1893 | ECM1 | extracellular matrix protein 1 |
| 1958 | EGR1 | early growth response 1 |
| 1967 | EIF2B1 | eukaryotic translation initiation factor 2B, subunit 1 alpha, 26kDa |
| 2065 | ERBB3 | v-erb-b2 erythroblastic leukemia viral oncogene homolog 3 (avian) |
| 2115 | ETV1 | ets variant 1 |
| 2119 | ETV5 | ets variant 5 |
| 2167 | FABP4 | fatty acid binding protein 4, adipocyte |
| 2202 | EFEMP1 | EGF containing fibulin-like extracellular matrix protein 1 |
| 2542 | SLC37A4 | solute carrier family 37 (glucose-6-phosphate transporter), member 4 |
| 2560 | GABRB1 | gamma-aminobutyric acid (GABA) A receptor, beta 1 |
| 2561 | GABRB2 | gamma-aminobutyric acid (GABA) A receptor, beta 2 |
| 2569 | GABRR1 | gamma-aminobutyric acid (GABA) receptor, rho 1 |
| 2581 | GALC | galactosylceramidase |
| 2595 | GANC | glucosidase, alpha; neutral C |
| 2635 | GBP3 | guanylate binding protein 3 |
| 2859 | GPR35 | G protein-coupled receptor 35 |
| 2869 | GRK5 | G protein-coupled receptor kinase 5 |
| 3014 | H2AFX | H2A histone family, member X |
| 3098 | HK1 | hexokinase 1 |
| 3249 | HPN | hepsin |
| 3476 | IGBP1 | immunoglobulin (CD79A) binding protein 1 |
| 3679 | ITGA7 | integrin, alpha 7 |
| 3682 | ITGAE | integrin, alpha E (antigen CD103, human mucosal lymphocyte antigen 1; alpha polypeptide) |
| 3685 | ITGAV | integrin, alpha V (vitronectin receptor, alpha polypeptide, antigen CD51) |
| 3710 | ITPR3 | inositol 1,4,5-triphosphate receptor, type 3 |
| 3751 | KCND2 | potassium voltage-gated channel, Shal-related subfamily, member 2 |
| 3977 | LIFR | leukemia inhibitory factor receptor alpha |
| 4000 | LMNA | lamin A/C |
| 4013 | VWA5A | von Willebrand factor A domain containing 5A |
| 4050 | LTB | lymphotoxin beta (TNF superfamily, member 3) |
| 4124 | MAN2A1 | mannosidase, alpha, class 2A, member 1 |
| 4128 | MAOA | monoamine oxidase A |
| 4233 | MET | met proto-oncogene (hepatocyte growth factor receptor) |
| 4240 | MFGE8 | milk fat globule-EGF factor 8 protein |
| 4257 | MGST1 | microsomal glutathione S-transferase 1 |
| 4281 | MID1 | midline 1 (Opitz/BBB syndrome) |
| 4301 | MLLT4 | myeloid/lymphoid or mixed-lineage leukemia (trithorax homolog, Drosophila); translocated to, 4 |
| 4524 | MTHFR | methylenetetrahydrofolate reductase (NAD(P)H) |
| 4628 | MYH10 | myosin, heavy chain 10, non-muscle |
| 4646 | MYO6 | myosin VI |
| 4664 | NAB1 | NGFI-A binding protein 1 (EGR1 binding protein 1) |
| 4665 | NAB2 | NGFI-A binding protein 2 (EGR1 binding protein 2) |
| 4753 | NELL2 | NEL-like 2 (chicken) |
| 4781 | NFIB | nuclear factor I/B |
| 4897 | NRCAM | neuronal cell adhesion molecule |
| 4907 | NT5E | 5'-nucleotidase, ecto (CD73) |
| 4915 | NTRK2 | neurotrophic tyrosine kinase, receptor, type 2 |
| 4926 | NUMA1 | nuclear mitotic apparatus protein 1 |
| 4967 | OGDH | oxoglutarate (alpha-ketoglutarate) dehydrogenase (lipoamide) |
| 5053 | PAH | phenylalanine hydroxylase |
| 5066 | PAM | peptidylglycine alpha-amidating monooxygenase |
| 5087 | PBX1 | pre-B-cell leukemia homeobox 1 |
| 5125 | PCSK5 | proprotein convertase subtilisin/kexin type 5 |
| 5162 | PDHB | pyruvate dehydrogenase (lipoamide) beta |
| 5164 | PDK2 | pyruvate dehydrogenase kinase, isozyme 2 |
| 5166 | PDK4 | pyruvate dehydrogenase kinase, isozyme 4 |
| 5255 | PHKA1 | phosphorylase kinase, alpha 1 (muscle) |
| 5270 | SERPINE2 | serpin peptidase inhibitor, clade E (nexin, plasminogen activator inhibitor type 1), member 2 |
| 5315 | PKM | pyruvate kinase, muscle |
| 5362 | PLXNA2 | plexin A2 |
| 5365 | PLXNB3 | plexin B3 |
| 5371 | PML | promyelocytic leukemia |
| 5504 | PPP1R2 | protein phosphatase 1, regulatory (inhibitor) subunit 2 |
| 5567 | PRKACB | protein kinase, cAMP-dependent, catalytic, beta |
| 5650 | KLK7 | kallikrein-related peptidase 7 |
| 5655 | KLK10 | kallikrein-related peptidase 10 |
| 5725 | PTBP1 | polypyrimidine tract binding protein 1 |
| 5747 | PTK2 | PTK2 protein tyrosine kinase 2 |
| 5786 | PTPRA | protein tyrosine phosphatase, receptor type, A |
| 5793 | PTPRG | protein tyrosine phosphatase, receptor type, G |
| 5873 | RAB27A | RAB27A, member RAS oncogene family |
| 5887 | RAD23B | RAD23 homolog B (S. cerevisiae) |
| 5921 | RASA1 | RAS p21 protein activator (GTPase activating protein) 1 |
| 5981 | RFC1 | replication factor C (activator 1) 1, 145kDa |
| 6196 | RPS6KA2 | ribosomal protein S6 kinase, 90kDa, polypeptide 2 |
| 6284 | S100A13 | S100 calcium binding protein A13 |
| 6297 | SALL2 | sal-like 2 (Drosophila) |
| 6299 | SALL1 | sal-like 1 (Drosophila) |
| 6385 | SDC4 | syndecan 4 |
| 6415 | SEPW1 | selenoprotein W, 1 |
| 6441 | SFTPD | surfactant protein D |
| 6449 | SGTA | small glutamine-rich tetratricopeptide repeat (TPR)-containing, alpha |
| 6569 | SLC34A1 | solute carrier family 34 (sodium phosphate), member 1 |
| 6695 | SPOCK1 | sparc/osteonectin, cwcv and kazal-like domains proteoglycan (testican) 1 |
| 6709 | SPTAN1 | spectrin, alpha, non-erythrocytic 1 (alpha-fodrin) |
| 6812 | STXBP1 | syntaxin binding protein 1 |
| 6843 | VAMP1 | vesicle-associated membrane protein 1 (synaptobrevin 1) |
| 7033 | TFF3 | trefoil factor 3 (intestinal) |
| 7039 | TGFA | transforming growth factor, alpha |
| 7048 | TGFBR2 | transforming growth factor, beta receptor II (70/80kDa) |
| 7050 | TGIF1 | TGFB-induced factor homeobox 1 |
| 7064 | THOP1 | thimet oligopeptidase 1 |
| 7069 | THRSP | thyroid hormone responsive |
| 7088 | TLE1 | transducin-like enhancer of split 1 (E(sp1) homolog, Drosophila) |
| 7159 | TP53BP2 | tumor protein p53 binding protein, 2 |
| 7164 | TPD52L1 | tumor protein D52-like 1 |
| 7224 | TRPC5 | transient receptor potential cation channel, subfamily C, member 5 |
| 7262 | PHLDA2 | pleckstrin homology-like domain, family A, member 2 |
| 7462 | LAT2 | linker for activation of T cells family, member 2 |
| 8170 | SLC14A2 | solute carrier family 14 (urea transporter), member 2 |
| 8202 | NCOA3 | nuclear receptor coactivator 3 |
| 8321 | FZD1 | frizzled homolog 1 (Drosophila) |
| 8506 | CNTNAP1 | contactin associated protein 1 |
| 8557 | TCAP | titin-cap (telethonin) |
| 8645 | KCNK5 | potassium channel, subfamily K, member 5 |
| 8654 | PDE5A | phosphodiesterase 5A, cGMP-specific |
| 8714 | ABCC3 | ATP-binding cassette, sub-family C (CFTR/MRP), member 3 |
| 8848 | TSC22D1 | TSC22 domain family, member 1 |
| 8869 | ST3GAL5 | ST3 beta-galactoside alpha-2,3-sialyltransferase 5 |
| 8909 | ENDOU | endonuclease, polyU-specific |
| 9076 | CLDN1 | claudin 1 |
| 9122 | SLC16A4 | solute carrier family 16, member 4 (monocarboxylic acid transporter 5) |
| 9194 | SLC16A7 | solute carrier family 16, member 7 (monocarboxylic acid transporter 2) |
| 9203 | ZMYM3 | zinc finger, MYM-type 3 |
| 9213 | XPR1 | xenotropic and polytropic retrovirus receptor 1 |
| 9223 | MAGI1 | membrane associated guanylate kinase, WW and PDZ domain containing 1 |
| 9249 | DHRS3 | dehydrogenase/reductase (SDR family) member 3 |
| 9353 | SLIT2 | slit homolog 2 (Drosophila) |
| 9414 | TJP2 | tight junction protein 2 (zona occludens 2) |
| 9455 | HOMER2 | homer homolog 2 (Drosophila) |
| 9617 | MTRF1 | mitochondrial translational release factor 1 |
| 9688 | NUP93 | nucleoporin 93kDa |
| 9697 | TRAM2 | translocation associated membrane protein 2 |
| 9882 | TBC1D4 | TBC 1 domain family, member 4 |
| 9902 | MRC2 | mannose receptor, C type 2 |
| 9931 | HELZ | helicase with zinc finger |
| 9969 | MED 13 | mediator complex subunit 13 |
| 10205 | MPZL2 | myelin protein zero-like 2 |
| 10241 | CALCOCO2 | calcium binding and coiled-coil domain 2 |
| 10351 | ABCA8 | ATP-binding cassette, sub-family A (ABC1), member 8 |
| 10454 | TAB1 | TGF-beta activated kinase 1/MAP3K7 binding protein 1 |
| 10551 | AGR2 | anterior gradient homolog 2 (Xenopus laevis) |
| 10577 | NPC2 | Niemann-Pick disease, type C2 |
| 10585 | POMT1 | protein-O-mannosyltransferase 1 |
| 10810 | WASF3 | WAS protein family, member 3 |
| 10962 | MLLT11 | myeloid/lymphoid or mixed-lineage leukemia (trithorax homolog, Drosophila); translocated to, 11 |
| 11098 | PRSS23 | protease, serine, 23 |
| 11123 | RCAN3 | RCAN family member 3 |
| 11216 | AKAP10 | A kinase (PRKA) anchor protein 10 |
| 22925 | PLA2R1 | phospholipase A2 receptor 1, 180kDa |
| 23015 | GOLGA8A | golgin A8 family, member A |
| 23052 | ENDOD1 | endonuclease domain containing 1 |
| 23077 | MYCBP2 | MYC binding protein 2 |
| 23081 | KDM4C | lysine (K)-specific demethylase 4C |
| 23140 | ZZEF1 | zinc finger, ZZ-type with EF-hand domain 1 |
| 23154 | NCDN | neurochondrin |
| 23211 | ZC3H4 | zinc finger CCCH-type containing 4 |
| 23220 | DTX4 | deltex homolog 4 (Drosophila) |
| 23250 | ATP11A | ATPase, class VI, type 11A |
| 23345 | SYNE1 | spectrin repeat containing, nuclear envelope 1 |
| 23348 | DOCK9 | dedicator of cytokinesis 9 |
| 23355 | VPS8 | vacuolar protein sorting 8 homolog (S. cerevisiae) |
| 23493 | HEY2 | hairy/enhancer-of-split related with YRPW motif 2 |
| 23499 | MACF1 | microtubule-actin crosslinking factor 1 |
| 23514 | KIAA0146 | KIAA0146 |
| 23541 | SEC14L2 | SEC14-like 2 (S. cerevisiae) |
| 23635 | SSBP2 | single-stranded DNA binding protein 2 |
| 25797 | QPCT | glutaminyl-peptide cyclotransferase |
| 25825 | BACE2 | beta-site APP-cleaving enzyme 2 |
| 25833 | POU2F3 | POU class 2 homeobox 3 |
| 25915 | NDUFAF3 | NADH dehydrogenase (ubiquinone) 1 alpha subcomplex, assembly factor 3 |
| 25976 | TIPARP | TCDD-inducible poly(ADP-ribose) polymerase |
| 25987 | TSKU | tsukushi small leucine rich proteoglycan homolog (Xenopus laevis) |
| 26022 | TMEM98 | transmembrane protein 98 |
| 26034 | IPCEF1 | interaction protein for cytohesin exchange factors 1 |
| 26224 | FBXL3 | F-box and leucine-rich repeat protein 3 |
| 26234 | FBXL5 | F-box and leucine-rich repeat protein 5 |
| 26297 | SERGEF | secretion regulating guanine nucleotide exchange factor |
| 26470 | SEZ6L2 | seizure related 6 homolog (mouse)-like 2 |
| 26953 | RANBP6 | RAN binding protein 6 |
| 26960 | NBEA | neurobeachin |
| 27112 | FAM155B | family with sequence similarity 155, member B |
| 27241 | BBS9 | Bardet-Biedl syndrome 9 |
| 27344 | PCSK1N | proprotein convertase subtilisin/kexin type 1 inhibitor |
| 28965 | SLC27A6 | solute carrier family 27 (fatty acid transporter), member 6 |
| 29087 | THYN1 | thymocyte nuclear protein 1 |
| 29781 | NCAPH2 | non-SMC condensin II complex, subunit H2 |
| 29798 | C2ORF27A | chromosome 2 open reading frame 27A |
| 29842 | TFCP2L1 | transcription factor CP2-like 1 |
| 50509 | COL5A3 | collagen, type V, alpha 3 |
| 51115 | FAM82B | family with sequence similarity 82, member B |
| 51195 | RAPGEFL1 | Rap guanine nucleotide exchange factor (GEF)-like 1 |
| 51310 | SLC22A17 | solute carrier family 22, member 17 |
| 51330 | TNFRSF12A | tumor necrosis factor receptor superfamily, member 12A |
| 51364 | ZMYND10 | zinc finger, MYND-type containing 10 |
| 51377 | UCHL5 | ubiquitin carboxyl-terminal hydrolase L5 |
| 51479 | ANKFY1 | ankyrin repeat and FYVE domain containing 1 |
| 51566 | ARMCX3 | armadillo repeat containing, X-linked 3 |
| 51704 | GPRC5B | G protein-coupled receptor, family C, group 5, member B |
| 51809 | GALNT7 | UDP-N-acetyl-alpha-D-galactosamine:polypeptide N-acetylgalactosaminyltransferase 7 (GalNAc-T7) |
| 53405 | CLIC5 | chloride intracellular channel 5 |
| 53905 | DUOX1 | dual oxidase 1 |
| 54103 | PION | pigeon homolog (Drosophila) |
| 54344 | DPM3 | dolichyl-phosphate mannosyltransferase polypeptide 3 |
| 54470 | ARMCX6 | armadillo repeat containing, X-linked 6 |
| 54471 | SMCR7L | Smith-Magenis syndrome chromosome region, candidate 7-like |
| 54532 | USP53 | ubiquitin specific peptidase 53 |
| 54587 | MXRA8 | matrix-remodelling associated 8 |
| 55129 | ANO10 | anoctamin 10 |
| 55220 | KLHDC8A | kelch domain containing 8A |
| 55294 | FBXW7 | F-box and WD repeat domain containing 7 |
| 55339 | WDR33 | WD repeat domain 33 |
| 55450 | CAMK2N1 | calcium/calmodulin-dependent protein kinase II inhibitor 1 |
| 55521 | TRIM36 | tripartite motif containing 36 |
| 55558 | PLXNA3 | plexin A3 |
| 55650 | PIGV | phosphatidylinositol glycan anchor biosynthesis, class V |
| 55652 | SLC48A1 | solute carrier family 48 (heme transporter), member 1 |
| 55740 | ENAH | enabled homolog (Drosophila) |
| 55755 | CDK5RAP2 | CDK5 regulatory subunit associated protein 2 |
| 55813 | UTP6 | UTP6, small subunit (SSU) processome component, homolog (yeast) |
| 56171 | DNAH7 | dynein, axonemal, heavy chain 7 |
| 56654 | NPDC1 | neural proliferation, differentiation and control, 1 |
| 56848 | SPHK2 | sphingosine kinase 2 |
| 56928 | SPPL2B | signal peptide peptidase-like 2B |
| 56999 | ADAMTS9 | ADAM metallopeptidase with thrombospondin type 1 motif, 9 |
| 57121 | LPAR5 | lysophosphatidic acid receptor 5 |
| 57154 | SMURF1 | SMAD specific E3 ubiquitin protein ligase 1 |
| 57184 | FAM219B | chromosome 15 open reading frame 17 |
| 57185 | NIPAL3 | NIPA-like domain containing 3 |
| 57214 | KIAA1199 | KIAA1199 |
| 57474 | ZNF490 | zinc finger protein 490 |
| 57477 | SHROOM4 | shroom family member 4 |
| 57493 | HEG1 | HEG homolog 1 (zebrafish) |
| 57639 | CCDC146 | coiled-coil domain containing 146 |
| 57664 | PLEKHA4 | pleckstrin homology domain containing, family A (phosphoinositide binding specific) member 4 |
| 58480 | RHOU | ras homolog gene family, member U |
| 60314 | C120RF10 | chromosome 12 open reading frame 10 |
| 63891 | RNF123 | ring finger protein 123 |
| 64091 | POPDC2 | popeye domain containing 2 |
| 64478 | CSMD1 | CUB and Sushi multiple domains 1 |
| 64769 | MEAF6 | MYST/Esa1-associated factor 6 |
| 65986 | ZBTB10 | zinc finger and BTB domain containing 10 |
| 79143 | MBOAT7 | membrane bound O-acyltransferase domain containing 7 |
| 79159 | NOL12 | nucleolar protein 12 |
| 79365 | BHLHE41 | basic helix-loop-helix family, member e41 |
| 79745 | CLIP4 | CAP-GLY domain containing linker protein family, member 4 |
| 79852 | EPHX3 | epoxide hydrolase 3 |
| 79856 | SNX22 | sorting nexin 22 |
| 79875 | THSD4 | thrombospondin, type I, domain containing 4 |
| 79897 | RPP21 | ribonuclease P/MRP 21kDa subunit |
| 79968 | WDR76 | WD repeat domain 76 |
| 80003 | PCNXL2 | pecanex-like 2 (Drosophila) |
| 80714 | PBX4 | pre-B-cell leukemia homeobox 4 |
| 81847 | RNF146 | ring finger protein 146 |
| 81849 | ST6GALNAC5 | ST6 (alpha-N-acetyl-neuraminyl-2,3-beta-galactosyl-1,3)-N-acetylgalactosaminide alpha-2,6-sialyltransferase 5 |
| 83660 | TLN2 | talin 2 |
| 83891 | SNX25 | sorting nexin 25 |
| 84220 | RGPD5 | RANBP2-like and GRIP domain containing 5 |
| 84239 | ATP13A4 | ATPase type 13A4 |
| 84417 | C2ORF40 | chromosome 2 open reading frame 40 |
| 84527 | ZNF559 | zinc finger protein 559 |
| 84561 | SLC12A8 | solute carrier family 12 (potassium/chloride transporters), member 8 |
| 84705 | GTPBP3 | GTP binding protein 3 (mitochondrial) |
| 84954 | MPND | MPN domain containing |
| 89797 | NAV2 | neuron navigator 2 |
| 89866 | SEC16B | SEC16 homolog B (S. cerevisiae) |
| 89927 | C16ORF45 | chromosome 16 open reading frame 45 |
| 90161 | HS6ST2 | heparan sulfate 6-O-sulfotransferase 2 |
| 90231 | KIAA2013 | KIAA2013 |
| 90627 | STARD13 | StAR-related lipid transfer (START) domain containing 13 |
| 91775 | NXPE3 | family with sequence similarity 55, member C |
| 92014 | SLC25A51 | mitochondrial carrier triple repeat 1 |
| 93611 | FBXO44 | F-box protein 44 |
| 113201 | CASC4 | cancer susceptibility candidate 4 |
| 113444 | C1ORF212 | chromosome 1 open reading frame 212 |
| 114800 | CCDC85A | coiled-coil domain containing 85A |
| 115353 | LRRC42 | leucine rich repeat containing 42 |
| 122786 | FRMD6 | FERM domain containing 6 |
| 124056 | NOXO1 | NADPH oxidase organizer 1 |
| 125206 | SLC5A10 | solute carrier family 5 (sodium/glucose cotransporter), member 10 |
| 126133 | ALDH16A1 | aldehyde dehydrogenase 16 family, member A1 |
| 129285 | PPP1R21 | KLRAQ motif containing 1 |
| 140469 | MYO3B | myosin IIIB |
| 140688 | C20ORF112 | chromosome 20 open reading frame 112 |
| 146439 | CCDC64B | coiled-coil domain containing 64B |
| 149175 | MANEAL | mannosidase, endo-alpha-like |
| 149461 | CLDN19 | claudin 19 |
| 154467 | CCDC167 | chromosome 6 open reading frame 129 |
| 155054 | ZNF425 | zinc finger protein 425 |
| 158158 | RASEF | RAS and EF-hand domain containing |
| 163702 | IL28RA | interleukin 28 receptor, alpha (interferon, lambda receptor) |
| 166929 | SGMS2 | sphingomyelin synthase 2 |
| 168391 | GALNTL5 | UDP-N-acetyl-alpha-D-galactosamine:polypeptide N-acetylgalactosaminyltransferase-like 5 |
| 196410 | METTL7B | methyltransferase like 7B |
| 202374 | STK32A | serine/threonine kinase 32A |
| 222171 | PRR15 | proline rich 15 |
| 253970 | SFTA3 | surfactant associated 3 |
| 283208 | P4HA3 | prolyl 4-hydroxylase, alpha polypeptide III |
| 283316 | CD163L1 | CD163 molecule-like 1 |
| 284418 | FAM71E2 | family with sequence similarity 71, member E2 |
| 285180 | RUFY4 | RUN and FYVE domain containing 4 |
| 285533 | RNF175 | ring finger protein 175 |
| 286144 | TRIQK | chromosome 8 open reading frame 83 |
| 286256 | LCN12 | lipocalin 12 |
| 340595 | ZCCHC16 | zinc finger, CCHC domain containing 16 |
| 342184 | FMN1 | formin 1 |
| 373156 | GSTK1 | glutathione S-transferase kappa 1 |
| 386653 | IL31 | interleukin 31 |
| 400746 | NCMAP | chromosome 1 open reading frame 130 |
| 401474 | SAMD12 | sterile alpha motif domain containing 12 |
| 440270 | GOLGA8B | golgin A8 family, member B |
| 440335 | LOC440335 | hypothetical LOC440335 |
| 645121 | CCNI2 | cyclin I family, member 2 |
| 727936 | GXYLT2 | glucoside xylosyltransferase 2 |
| 728492 | SERF1B | small EDRK-rich factor 1B (centromeric) |
| 100131187 | TSTD1 | thiosulfate sulfurtransferase (rhodanese)-like domain containing 1 |
| 100293534 | LOC100293534 | complement C4-B-like |
| 100509091 | LOC100509091 | extracellular matrix protein 2-like |

For each gene included in Table A and in Table B, a most preferred sequence (or domain) to be targeted for measuring gene expression according to the invention is provided in Table C. Accordingly, measuring BCL9 preferably comprises measuring a target sequence comprised in anyone of the sequences listed as SEQ ID NOs: 52 to 54 in Table C; measuring ENTPD2 preferably comprises measuring a target sequence comprised in SEQ ID NO: 205 as listed in Table C, etc.

Specific combinations of target gene products are provided in the following table D, which combinations can provide strong specificity and sensitivity for detection of thyroid cancer:

**Table D: Combinations of marker genes**

| Signature# | Expression Product(s) |
|---|---|
| 1 | CITED 1 (several exons) |
| 2 | CITED 1 and TENM 1 |
| 3 | CITED and SCLY |
| 4 | CITED 1 and CCM2 |
| 5 | CITED and FRMD3 |
| 6 | TENM1, CITED1, CCM2, SCLY |
| 7 | CITED1, TENM1, CCM2, SCLY and IGSF10 |
| F | TENM1, CITED1, CCM2, SCLY, FRMD3, IGSF10 |
| D | TENM1, CITED1, CCM2, SCLY, FRMD3, IGSF10 + all genes of table A |
| B | TENM1, CITED1, CCM2, SCLY, FRMD3, IGSF10 + all genes of table A + all gene of Table B |

### Methods for measuring gene expression products

Within the context of the invention, the "measure" of a gene or gene expression product designates a measure or determination of the presence, absence, amount or alteration of such gene product. More preferably, the term "measure" or "measuring" designates a determination of the presence or amount of a gene product or domain, or of the level of expression of a gene. The gene product may be a RNA (e.g., a messenger RNA) or a polypeptide (or protein).

In a first preferred embodiment, the gene product is an RNA (which may be converted during the method into a cDNA). Various techniques for detecting a nucleic acid (e.g., RNA) in a sample can be used in the present invention, such as for example northern blot, selective hybridisation, the use of supports covered with oligonucleotide probes, nucleic acid amplification such as for example RT-PCR, quantitative PCR or ligation-PCR, etc. These methods can include the use of a nucleic probe (for example an oligonucleotide) capable of detecting selectively or specifically the target nucleic acid in the sample. Amplification can be performed according to various methods known to the person skilled in the art, such as PCR, LCR, transcription mediated amplification (TMA), strand displacement amplification (SDA), NASBA, the use of allele specific oligonucleotides (ASO), allele specific amplification, RNAseq. Detection can also be made using, e.g., Southern blot, single-strand conformation analysis (SSCA), *in situ* hybridisation (e.g., FISH), gel migration, heteroduplex analysis, NextGen sequencing, etc.

According to a preferred embodiment, the method comprises determining the presence or absence or (relative) amount of an RNA encoded by a target gene as defined above, preferably by selective hybridisation or selective amplification.

### Measure by selective hybridization

Selective hybridisation is typically performed using nucleic probes, preferably immobilised on a support, such as a solid or semi-solid support having at least one surface, flat or not, for immobilising nucleic probes. Such supports are, for example, a slide, bead, membrane, filter, column, plate, etc. They can be made out of any compatible material, such as in particular glass, silica, plastic, fiber, metal, polymer, etc. The nucleic probes can be any nucleic acid (DNA, RNA, PNA, etc.), preferably single-stranded, with a sequence specific to a target RNA or DNA molecule. The probes typically comprise from 5 to 400 bases, preferably from 8 to 200, more preferentially less than 100, and even more preferentially less than 75, 60, 50, 40 or even 30 bases. The probes can be synthetic oligonucleotides, produced on the basis of the sequences of the target genes, according to standard synthesis techniques. Such oligonucleotides typically comprise from 10 to 50 bases, preferably from 20 to 40, for example approximately 25 bases. In a particularly advantageous embodiment, so as to improve the detection of signal, several different oligonucleotides (or probes) are used to detect the same target molecule (transcribed from the amplification of the patient's RNA to produce either cRNA or cDNA) during hybridisation. This may include specific oligonucleotides of various regions of the same target sequence, or centred differently on a given region. Advantageously, use is made of probe sets comprising 1-3 probes, which can be overlapping or not, completely or partially, and which are specific of the same target molecule. Use can also be made of probe pairs, in which one member is paired perfectly with the target sequence, and the other presents a mismatch, thus making it possible to estimate background signal. Probes can be designed to hybridise with a region of an exon or an intron, or with an exon-exon or exon-intron junction region. Thus, the probes make it possible to reveal and to distinguish various alternative spliced isoforms.

In a preferred embodiment, use is made of probes whose sequence comprises all or part of a nucleic acid sequence selected from anyone of SEQ ID NOs: 1-918 or of a sequence complementary thereto. In a preferred mode, use is made of nucleic acid probes having a length between 15 and 50 bases, more preferentially between 15 and 40 bases, and whose sequence is identical to a fragment of a sequence selected from anyone of SEQ ID NOs: 1-918 or of a sequence complementary thereto. The probe can also be designed in the opposite orientation. Such probes constitute another object of the present application, as well as the use thereof for diagnosing thyroid cancer in a subject.

Specific examples of probes are provided in Table E. These probes have been designed to bind target sequences as disclosed in Table C. Further probes can be designed using information provided in the invention, directed specifically to each target sequence of Table C. Each probe as disclosed in Table E represents a specific object of the invention.

**Table E**

| Probe Set ID | Probe ID | Probe Sequence | SEQ ID # |
|---|---|---|---|
| HSX2_01064533 | HSX2_01064533-39 | CACCGCTGAGCTGCCGAGAGGAAG | 919 |
| | HSX2_01064533-104 | GCAGTTCTCGGTGAGGAGGAAGAG | 920 |
| | | | |
| HSX2_01064539 | HSX2_01064539-42 | TCCAACTTCTGCCAAGGCTCTGAA | 921 |
| | HSX2_01064539-77 | GTCGAGGCCTGCACTTGATGTCAA | 922 |
| | | | |
| HSX2_01064543 | HSX2_01064543-27 | GGCCTACTCCAACCTTGCGGTGAA | 923 |
| | HSX2_01064543-46 | GTGAAAGATCGCAAAGCAGTGGCC | 924 |
| | | | |
| HSX2_01064546 | HSX2_01064546-23 | CCTCGGGATCTCCAATAGGCTCTC | 925 |
| | HSX2_01064546-24 | CTCGGGATCTCCAATAGGCTCTCC | 926 |
| HSX2_01064547 | HSX2_01064547-31 | CACTTGCTGGCTAGTATGCACCTG | 927 |
| | HSX2_01064547-166 | TCACTCTCTCCTTCTGCTGGTGCC | 928 |
| | | | |
| HSX2_01064548 | HSX2_01064548-32 | ATGAGGAAGTGCTGATGTCGCTGG | 929 |
| | HSX2_01064548-127 | ACTGCGGACTTTCCATCTAGCTGC | 930 |
| | | | |
| HSX2_01477556 | HSX2_01477556-78 | GCAAACCCTACCAGCCTCTACCAA | 931 |
| | HSX2_01477556-189 | GGGAGACCCTGCACGAGTATAACC | 932 |
| | | | |
| HSX2_01477563 | HSX2_01477563-15 | CCTGTTTGTTGTGACATGGAGGCT | 933 |
| | HSX2_01477563-23 | TTGTGACATGGAGGCTCAAGCTGG | 934 |
| | | | |
| HSX2_01477568 | HSX2_01477568-13 | GCCCACACAACCAGTTCACCTTCA | 935 |
| | HSX2_01477568-174 | AGCACGCAGGATTCAGTCCATCTG | 936 |
| | | | |
| HSX2_01477573 | HSX2_01477573-25 | TGGTTCCTCTGCGATCTTCAGTGC | 937 |
| | HSX2_01477573-189 | GCGCCACTGCAATCACAGTGACTT | 938 |
| | | | |
| HSX2_01477615 | HSX2_01477615-1 | CTCACTACTTAGATGCTGTCCGAG | 939 |
| | HSX2_01477615-1 | CTCACTACTTAGATGCTGTCCGAG | 940 |
| | | | |
| HSX2_01477625 | HSX2_01477625-72 | GGCTCACCAGATCCTCTTGACCTC | 941 |
| | HSX2_01477625-106 | GCCAAACTCTCTTCTCTCAGCACA | 942 |
| | | | |
| HSX2_01477635 | HSX2_01477635-73 | GAGAACACTCTGGTTGCCTTGGAA | 943 |
| | HSX2_01477635-192 | TGCTTCCTTCACCGCTCACATCAT | 944 |
| | | | |
| HSX2_01477640 | HSX2_01477640-81 | CCTGCTACGGATCCTTCTGACACA | 945 |
| | HSX2_01477640-162 | ACTCACACAGAAGTGGTTTCCCGC | 946 |
| | | | |
| HSX2_01477645 | HSX2_01477645-41 | TCTCTGGGAGCAAAGGACAGTCGT | 947 |
| | HSX2_01477645-87 | GCTTCTAACCTAGGAGGCTGGTCT | 948 |
| | | | |
| SX2_01477665 | HSX2_01477665-35 | TGTGGATGGGACTATGATTCGCAA | 949 |
| | HSX2_01477665-106 | TGACTTCCACACAACCACTGAGCT | 950 |
| | | | |
| HSX2_01477675 | HSX2_01477675-104 | TCGTACCATCAGCAGGAACCAAGC | 951 |
| | HSX2_01477675-288 | ACCAGCAGCAATGGCAATTCAGTG | 952 |
| | | | |
| HSX2_01477695 | HSX2_01477695-17 | TACACAGCCAGCGGCGTTACATCT | 953 |
| | HSX2_01477695-242 | ACAAGTACACCAAGCAAGCAAGGC | 954 |
| | | | |
| HSX2_01477710 | HSX2_01477710-333 | CCTGGTGCTCATCGGTAACACTGG | 955 |
| | HSX2_01477710-515 | ACGTGTTGGAGATTGCCAGACAGC | 956 |
| HSX2_01477711 | HSX2_01477711-469 | CAGCCACCATCTCCTAGAGTGAGG | 957 |
| | HSX2_01477711-4356 | ACAGGTTCATGTTCTCTGGCTGCA | 958 |
| | | | |
| HSX2_01771392 | HSX2_01771392-460 | AGAACCGTCCTCCAGTGGTGAAGC | 959 |
| | HSX2_01771392-673 | TCCAGAGTGGTCTCTTCTCAGGCC | 960 |
| | | | |
| HSX2_02073060 | HSX2_02073060-1 | CCACATCCAGTTCCACCACCAATG | 961 |
| | HSX2_02073060-141 | CAGGACTCAGCATGATGGACAGTG | 962 |
| | | | |
| HSX2_02319619 | HSX2_02319619-4 | CCTGAGGTGCACAGAGCCAACATT | 963 |
| | HSX2_02319619-6 | TGAGGTGCACAGAGCCAACATTAC | 964 |
| | | | |
| HSX2_02487762 | HSX2_02487762-163 | CCAGTTGCCTAGTGTCCACCTCCT | 965 |
| | HSX2_02487762-422 | ATGCAGGCTCACTTTTCCACATGT | 966 |
| | | | |
| HSX2_02509166 | HSX2_02509166-208 | CACTTGCTGGCTAGTATGCACCTG | 967 |
| | HSX2_02509166-401 | ATGAGGAAGTGCTGATGTCGCTGG | 968 |

An object of the invention thus relates to a nucleic acid having anyone of the nucleotide sequences as described in Table E, as well as to the use of these nucleic acids for diagnosing thyroid cancer.

The probes can be synthesised in advance and then deposited on the support, or synthesised directly *in situ*, on the support, according to methods known to the person skilled in the art. The probes can also be manufactured by genomic or molecular techniques, for example by amplification, recombination, ligation, etc.

The probes can by hybridized with nucleic acids, preferably RNAs contained in test samples under standard, conventional conditions allowing specific hybridization to occur. Hybridisation can be performed under standard conditions, known to and adjustable by the person skilled in the art (see Sambrook, Fritsch, Maniatis (1989) Molecular Cloning, Cold Spring Harbor Laboratory Press). Most notably, hybridisation can be performed under conditions of high, medium or low stringency, according to the level of sensitivity needed, the quantity of material available, etc. For example, suitable hybridisation conditions include a temperature between 55°C and 63°C for 2 hours to 18 hours. Other hybridisation conditions, adapted to high density supports, are for example a hybridisation temperature between 45°C and 55°C. After hybridisation, various washes can be performed to eliminate the non-hybridised molecules, typically in SSC buffers including SDS, such as a buffer comprising 0.1X to 10X SSC and 0.5% to 0.01% SDS. Other wash buffers containing SSPE, MES, NaCl or EDTA can also be used.

In a typical embodiment, the nucleic acids (or arrays or supports) are pre-hybridised in hybridisation buffer (Rapid Hybrid Buffer, Amersham) typically containing 100 µg/ml of salmon sperm DNA at 65°C for 30 min. The nucleic acids of the sample are then placed in contact with the probes (typically applied to the support or the array) at 65°C for 2 hours to 18 hours. Preferably, the nucleic acids of the sample are marked beforehand by any known marker (biotin, radioactive, enzymatic, fluorescent, luminescent, etc.). The supports are then washed in 5X SSC, 0.1% SDS buffer at 65°C for 30 min., then in a 0.2X SSC, 0.1% SDS buffer. The expression profile is analysed according to standard techniques, such as for example by measuring labelling on the support by means of a suitable instrument (for example InstantImager, Packard Instruments). Hybridisation conditions naturally can be adjusted by the person skilled in the art, for example by modifying hybridisation temperature and/or the salt concentration of the buffer as well as by adding auxiliary substances such as formamide or single-strand DNA.

A particular object of the invention thus resides in a method to diagnose thyroid cancer in mammals comprising contacting, under conditions allowing hybridisation between complementary sequences to occur, (i) nucleic acids from a sample from the subject and (ii) a set of probes specific of target gene expression products as defined above; and diagnosing thyroid cancer by measuring hybridization to determine expression values and comparing said values to a reference value.

In a preferred embodiment, the method uses:
- from 1 to 5 probes, preferably from 1 to 3, typically 2, overlapping or not, each comprising all or part of the sequence of CITED1 gene, preferably of anyone of the sequences listed as SEQ ID NOs: 106-119, or of a sequence complementary thereto; and
- from 1 to 5 probes, preferably from 1 to 3, typically 2, overlapping or not, each comprising all or part of the sequence of TENM1 gene, preferably of anyone of the sequences listed as SEQ ID NOs: 817-838, or of a sequence complementary thereto,
wherein the term "part" advantageously indicates a region of 15 to 50 consecutive nucleotides, preferably of 15 to 30 nucleotides, even more preferably of 20-30 nucleotides.

In a more preferred embodiment, the method uses:
- from 1 to 5 probes, preferably from 1 to 3, typically 2, overlapping or not, each comprising all or part of the sequence of CITED1 gene or of a sequence complementary thereto, preferably of anyone of the sequences listed as SEQ ID NOs: 106-119, or of a sequence complementary thereto;
- from 1 to 5 probes, preferably from 1 to 3, typically 2, overlapping or not, each comprising all or part of the sequence of TENM1 gene or of a sequence complementary thereto, preferably of anyone of the sequences listed as SEQ ID NOs: 817-838, or of a sequence complementary thereto; and
- from 1 to 5 probes, preferably from 1 to 3, typically 2, overlapping or not, each comprising all or part of the sequence of SCLY gene or of a sequence complementary thereto, preferably of SEQ ID NO: 749, or of a sequence complementary thereto;
wherein the term "part" advantageously indicates a region as defined above.

In a particularly preferred embodiment, the method uses:
- from 1 to 5 probes, preferably from 1 to 3, typically 2, overlapping or not, each comprising all or part of the sequence of CITED 1 gene or of a sequence complementary thereto, preferably of anyone of the sequences listed as SEQ ID NOs: 106-119, or of a sequence complementary thereto;
- from 1 to 5 probes, preferably from 1 to 3, typically 2, overlapping or not, each comprising all or part of the sequence of TENM1 gene or of a sequence complementary thereto, preferably of anyone of the sequences listed as SEQ ID NOs: 817-838, or of a sequence complementary thereto;
- from 1 to 5 probes, preferably from 1 to 3, typically 2, overlapping or not, each comprising all or part of the sequence of SCLY gene or of a sequence complementary thereto, preferably of SEQ ID NO: 749, or of a sequence complementary thereto;
- from 1 to 5 probes, preferably from 1 to 3, typically 2, overlapping or not, each comprising all or part of the sequence of CCM2 gene or of a sequence complementary thereto, preferably of SEQ ID NO: 83, or of a sequence complementary thereto;
- from 1 to 5 probes, preferably from 1 to 3, typically 2, overlapping or not, each comprising all or part of the sequence of IGSF10 gene or of a sequence complementary thereto, preferably of SEQ ID NO: 335, or of a sequence complementary thereto; and
- from 1 to 5 probes, preferably from 1 to 3, typically 2, overlapping or not, each comprising all or part of the sequence of FRMD3 gene or of a sequence complementary thereto, preferably of anyone of the sequences listed as SEQ ID NOs: 249 to 269, or of a sequence complementary thereto;
wherein the term "part" advantageously indicates a region as defined above.

In another preferred embodiment, the method uses:
- at least 1 probe that specifically hybridizes with exon 4 of the sequence of CITED 1 gene or of a sequence complementary thereto; and
- at least 1 probe that specifically hybridizes with exon 3 of the sequence of CITED 1 gene or of a sequence complementary thereto and, ,
- at least 1 probe that specifically hybridizes with exon(-2) of the sequence of CITED 1 gene or of a sequence complementary thereto.

In another preferred embodiment, the method uses:
- at least 1 probe that specifically hybridizes with exon1, 3, 13, or 15, of the sequence of TENM1 gene or of a sequence complementary thereto; and
- at least 1 probe that specifically hybridizes with exon17, 18, 19, 25, or 32 of the sequence of TENM1 gene or of a sequence complementary thereto.

The invention may utilize additional probes specific for any group or combination of target genes of the invention, as preferably listed in the above table.

### Measure by selective amplification

Selective amplification is preferably performed using a primer or a pair of primers to amplify all or part of one of the target nucleic acids in the sample, when the target nucleic acid is present. The primer can be specific for a target sequence according to anyone of SEQ ID NOs: 1 to 918, or for a region flanking the target sequences in a nucleic acid of the sample. The primer typically comprises a single-strand nucleic acid, with a length advantageously between 5 and 50 bases, preferably between 5 and 30 bases. Such a primer constitutes another object of the present application, as well as the use thereof (primarily *in vitro*) for diagnosing thyroid cancer in a subject. The primers can be designed to hybridise with a region of an exon or an intron, or with an exon-exon or exon-intron junction region. Thus, the primers reveal and distinguish various forms of gene splicing.

In this respect, another object of the invention resides in the use of a set of nucleotide primers comprising at least 2 pairs of primers, each pair of primers comprising a sense and/or antisense nucleic acid primers complementary to and specific of a target nucleic acid selected from anyone of the sequences listed as SEQ ID NOs: 1-918, or the complementary strand thereof, for *in vitro* or *ex vivo* diagnosis of thyroid cancer in a subject.

Another specific object of the invention resides in a method to diagnose thyroid cancer in mammals, comprising contacting, under conditions allowing amplification, nucleic acids from a sample from mammals and a set of primers comprising at least 2 pairs of primers, each pair of primers comprising nucleic acid primers complementary to and specific of a target nucleic acid selected from anyone of the sequences listed as SEQ ID NOs: 1-918 or the complementary strand thereof to obtain an amplification profile, and comparing said amplification profile to a reference value to diagnose thyroid cancer.

The invention also relates to a composition or kit comprising a set of primers comprising at least 2 pairs of primers, each pair of primers comprising a sense and/or antisense nucleic acid primers complementary to and specific of a target nucleic acid selected from anyone of the sequences listed as SEQ ID NOs: 1-918 or the complementary strand thereof.

### Measuring polypeptide

In another embodiment, the method comprises the measurement of a polypeptide encoded by a target gene such as defined previously. Measuring or assaying a polypeptide in a sample can be performed by any known technique, most notably by means of a specific ligand, for example an antibody or an antibody fragment or derivative. Preferably, the ligand is a specific antibody of the polypeptide, or a fragment of such an antibody (for example Fab, Fab', CDR, etc.), or a derivative of such an antibody (for example a single-chain variable-fragment antibody, scFv). The ligand is typically immobilised on a support, such as a slide, bead, column, plate, etc. The presence or quantity of target polypeptide in the sample can be detected by revealing a complex between the target and the ligand, for example by using a labelled ligand or by using a second labelled indicator ligand, etc. Immunological techniques that can be used and are well known are ELISA, IHC and RIA techniques, etc. If necessary, the quantity of polypeptide detected can be compared to a reference value, for example a median or mean value observed among patients who do or do not have thyroid cancer, or with a value measured in parallel in a control sample. Thus, it is possible to demonstrate variation in expression levels.

Specific antibodies of target polypeptides can be produced by conventional techniques, most notably by immunization of a non-human mammal with an immunogen comprising the polypeptide (or an immunogenic fragment thereof), and recovery of the antibodies (polyclonal) or cells producing monoclonal antibodies. Production techniques for polyclonal or monoclonal antibodies, single-chain variable-fragment antibodies and human or humanised antibodies are described for example in Harlow et al., A Laboratory Manual, CSH Press, 1988; Ward et al., Nature 341 (1989) 544; Bird et al., Science 242 (1988) 423; WO 94/02602; US 5,223,409; US 5,877,293 and WO 93/01288. The immunogen can be produced by synthesis, or by expression, in a suitable host, of a nucleic acid target such as defined previously. Such antibodies, monoclonal or polyclonal, as well as derivatives with the same antigenic specificity, constitute further objects of the present application, as well as the use thereof to diagnose thyroid cancer.

Changes in protein expression and/or structure can also be detected by means of techniques, known by the person skilled in the art, involving mass spectroscopy, more generally grouped under the name proteome analysis.

### Implementation of the method

The method of the invention is applicable to any biological sample from the tested mammal, most notably any nature of sample that includes nucleic acids or polypeptides from a thyroid cell, most preferably from a thyroid lesion or nodule. Specific examples include any tissue, organ or biological fluid that includes nucleic acids or polypeptides from a thyroid cell, most preferably from a thyroid lesion or nodule. Most preferred samples are a tissue biopsy from thyroid, or a fine needle thyroid aspirate.

In one preferred and particularly advantageous embodiment, the sample is a sample collected by the physician for cytological analysis of the subject's thyroid lesion or nodules. Upon completion of such cytological analysis, if the status of the sample is indeterminate, then this sample may be used to perform the current test.

The sample can be obtained by any known technique, for example by drawing, by non-invasive techniques, or from sample collections or biobanks, etc. Further, the sample can be pre-treated to facilitate access to the target molecules, for example by lysis (mechanical, chemical, enzymatic, etc.), purification, centrifugation, separation, etc. The sample can also be labelled to facilitate the determination of the presence of target molecules (biotin, fluorescent, radioactive, luminescent, chemical or enzymatic labelling, etc.). The nucleic acids of the sample can in addition be separated, treated, enriched, purified, reverse transcribed, amplified, fragmented, etc. In a particular embodiment, the nucleic acids of the sample are RNAs, most notably mRNA of the sample. In a very specific embodiment, the nucleic acids are the product of amplification of RNA, most notably of mRNA, or cDNA prepared from RNA, most notably mRNA of the sample.

In a particular embodiment, the method comprises:
a) providing a biological sample from a subject having a thyroid condition (e.g. a thyroid lesion or nodule) or suspected to have a thyroid condition; and
b) measuring in said biological sample the expression product of genes as defined above.

In a further particular embodiment, the invention is conducted after a cytological exam and allows the diagnosis of patient samples remaining "indeterminate" after said cytological exam. In this regard, a particular method of the invention comprises:
a) the provision (e.g., the collection) of a biological sample from the subject having a thyroid condition or suspected to have a thyroid condition;
b) a cytological exam of the sample to classify the thyroid condition (e.g., in a category comprised between 1 and 6) according to the Bethesda classification, and
c) for any sample of indeterminate cytology, the measurement of the expression product of target genes as described above.

In a typical embodiment, the method measures a gene expression product and compares such expression signal to a reference value in order to diagnose thyroid cancer. In this regard, the reference value may be any control value, or any mean value in healthy subjects, or 2 reference values characteristic of a diseased and a non-diseased subject, respectively. In this regard, in a preferred embodiment of the invention, the measured value is compared to a reference value of a control benign thyroid condition and to a reference value of a control malignant thyroid condition, wherein, when the measured value is closer to the reference value of the control benign thyroid condition, then the tested thyroid condition is benign, and when the measured value is closer to the reference value of the control malignant thyroid condition, then the tested thyroid condition is malignant.

The invention is applicable to any mammal, preferably human beings. The data provided in the examples show that the invention can detect the presence of malignant or benign lesions when the cytology was unable to provide a diagnostic result. 60 samples (40 benign and 20 malignant lesions) were used to validate the relevance of the invention, which addresses a clinical unmet need and allows reduction or avoidance of unnecessary surgeries.

Another object of the present application relates to a product comprising a support on which are immobilised nucleic acids comprising a sequence complementary to and/or specific for at least 2 genes selected from CITED1, TENM1, SCXLY, CCM2 and IGSF10 or the complementary strand thereof, more preferably at least 3, 4 or 5; or for at least 2 distinct domains of an expression product of at least one gene selected from CITED 1, TENM1, SCXLY, CCM2 and IGSF10, more preferably at least 3, 4 or 5.

A particular object of the invention relates to a product comprising a support on which are immobilised:
- at least 1 probe that specifically hybridizes with an RNA encoded by a CITED 1 gene; and
- at least 1 probe that specifically hybridizes with an RNA encoded by a TENM1 gene.

A particular object of the invention relates to a product comprising a support on which are immobilised:
- at least 1 probe that specifically hybridizes with exon 4 of the sequence of CITED 1 gene or of a sequence complementary thereto; and/or
- at least 1 probe that specifically hybridizes with exon 3 of the sequence of CITED 1 gene or of a sequence complementary thereto, and/or
- at least 1 probe that specifically hybridizes with exon (-2) of the sequence of CITED 1 gene or of a sequence complementary thereto.

Another particular object of the invention relates to a product comprising a support on which are immobilised:
- at least 1 probe that specifically hybridizes with exon1, 3, 13, or 15, of the sequence of TENM1 gene or of a sequence complementary thereto; and
- at least 1 probe that specifically hybridizes with exon17, 18, 19, 25, or 32 of the sequence of TENM1 gene or of a sequence complementary thereto.

Another particular object of the invention relates to a product comprising a support on which is immobilised:
- from 1 to 5 probes, overlapping or not, each comprising all or part of the sequence of CITED 1 gene or of a sequence complementary thereto; and
- from 1 to 5 probes, overlapping or not, each comprising all or part of the sequence of TENM1 gene or of a sequence complementary thereto,
wherein, the term "part" is as defined above.

Another particular object of the invention relates to a product comprising a support on which is immobilised:
- from 1 to 5 probes, preferably from 1 to 3, typically 2, overlapping or not, each comprising all or part of the sequence of CITED 1 gene or of a sequence complementary thereto, preferably all or part of anyone of the sequences listed as SEQ ID NOs: 106-119, or of a sequence complementary thereto;
- from 1 to 5 probes, preferably from 1 to 3, typically 2, overlapping or not, each comprising all or part of the sequence of TENM1 gene or of a sequence complementary thereto, preferably all or part of anyone of the sequences listed as SEQ ID NOs: 817-838, or of a sequence complementary thereto;
- from 1 to 5 probes, preferably from 1 to 3, typically 2, overlapping or not, each comprising all or part of the sequence of SCLY gene or of a sequence complementary thereto, preferably of SEQ ID NO: 749, or of a sequence complementary thereto;
- from 1 to 5 probes, preferably from 1 to 3, typically 2, overlapping or not, each comprising all or part of the sequence of CCM2 gene or of a sequence complementary thereto, preferably of SEQ ID NO: 83, or of a sequence complementary thereto;
- from 1 to 5 probes, preferably from 1 to 3, typically 2, overlapping or not, each comprising all or part of the sequence of IGSF10 gene or of a sequence complementary thereto, preferably of SEQ ID NO: 335, or of a sequence complementary thereto; and
- from 1 to 5 probes, preferably from 1 to 3, typically 2, overlapping or not, each comprising all or part of the sequence of FRMD3 gene or of a sequence complementary thereto, preferably all or part of anyone of the sequences listed as SEQ ID NOs: 249 to 269, or of a sequence complementary thereto;
wherein the term "part" advantageously indicates a region as defined above.

The invention also relates to a kit comprising a product such as defined previously and reagents for a hybridisation reaction.

The invention further relates to the use of a product or kit defined above for *in vitro* or *ex vivo* diagnosis of thyroid cancer in a subject.

Another object of the present application relates to a product comprising a support on which is immobilised at least two ligands that specifically bind a polypeptide coded by at least 2 genes selected from CITED1, TENM1, CCM2, SCLY, IGSF10 and FRMD3.

The support can be any solid or semi-solid support having at least one surface, flat or not (*i.e.,* in two or three dimensions), allowing the immobilisation of nucleic acids or polypeptides. Such supports are for example a slide, bead, membrane, filter, column, plate, etc. They can be made of any compatible material, such as most notably glass, silica, plastic, fibre, metal, polymer, polystyrene, Teflon, etc. The reagents can be immobilised on the surface of the support by known techniques, or, in the case of nucleic acids, synthesised directly *in situ* on the support. Immobilisation techniques include passive adsorption (Inouye et al., J. Clin. Microbiol. 28 (1990) 1469) and covalent bonding. Techniques are described for example in WO 90/03382 and WO 99/46403. The reagents immobilised on the support can be placed in a predetermined order, to facilitate detection and identification of the complexes formed, and according to a variable and adaptable density.

In one embodiment, the inventive product comprises a multiplicity of synthetic oligonucleotides, of length between 5 and 100 bases, and specific for one or more genes or RNAs such as previously defined.

The products of the invention typically comprise control molecules, which are used to calibrate and/or standardise the results.

Another object of the present application relates to a kit comprising a compartment or container comprising at least one, preferably several, nucleic acids comprising a complementary and/or specific sequence of one or more genes or RNAs such as previously defined and/or one, preferably several ligands of one or more polypeptides such as previously defined. Preferably, the product comprises at least 5 distinct nucleic acids and/or ligands selected from the nucleic acids and ligands mentioned above.

Another object of the invention relates to the use of a product or kit such as defined above for the diagnosis of thyroid cancer in a mammalian subject, preferably a human subject.

It is understood that any equivalent technique can be used within the scope of the present application to determine the presence of a target molecule.

Further aspects and advantages of the present invention will appear upon consideration of the following examples, which must be regarded as illustrative and non-restrictive.

### Examples

### A. Material and Methods

### 1. Biological samples :

Candidate signatures have been identified using retrospective cohorts (DeCanThyr, Hospices Civils de Lyon and FNAC, Centre Baclesse - Caen) on a total of 60 subjects, 20 patients had malignant nodules and 40 patients had benign lesions. All samples were previously classified as "indeterminate" according to the Bethesda classification and were associated with the histopathological analysis results of the surgical specimen. Table 1 below provides details about the distribution of cancerous and benign thyroid lesions in each Bethesda indeterminate category.

**Table 1**

| **Samples** | **Bethesda classification** | **Cytology based** | **Histology benign** | **Histology malignant** |
|---|---|---|---|---|
| | III | 12 | 11 | 1 |
| 60 patients | IV | 29 | 22 | 7 |
| | V | 19 | 7 | 12 |

### 2. Total RNA extraction or storage

Dedicated fine needle aspirations were collected in 350 µl of RLT buffer (RNeasy Micro kit, Qiagen) and stored at -80°C. Total RNA was extracted using the manufacturer's recommended procedure and stored at -80°C.

The acceptance criteria were the following:
- Absorbance measure (A260/A280) greater then 1.7
- Ribosomal ratio (28S / 18S) greater or equal to 0.4
- RIN value (Agilent Bioanalyzer 2100) greater than 4

### 3. HsGWSA2.0.2.Dx POC 1M Agilent array

The Hs GWSA 2.0.2.Dx POC 1M Microarray on the Agilent platform is a tool created to detect and quantify gene expression and alternative expression of transcripts. To achieve this, gene transcripts are identified that represent reference and alternative expression patterns and are processed to resolve the discriminative regions in these expression pattern. The identified alternative event patterns may include: alternative exons, novel splice sites, elimination of splice sites, skipping of normally constitutive exons or some combination thereof.

Analysis was performed on publically available sequence data from a set of human genes identified in the NCBI release 189 and using the NCBI Human genome release 37.3. These sequences were analyzed with a proprietary software system that processed two overlapping datasets: modeling the exon structure of the annotated reference forms of the genes and generating models to identify alternative events identified by these reference forms within the genes.

The reference form analysis for the genes involved identification of expression regions that represented every exon of the reference exemplar sequence data. The alternative event analysis was performed by modeling expression patterns of the identified alternative transcripts and deriving the discriminative expression regions.

After the expression regions were identified a second analysis was performed to identify a minimally redundant expression path through the gene for all selected expression features (reference form expression and included discriminative expression regions). A series of probes were designed from this path: probes that target the expressed exons of the reference forms, junctions to constitutive exons of the reference forms and probes designed against the identified discriminative regions and the junctions (explicit and implicit) indicated by the evidence of the alternative transcripts. This combination of exon and junction probes enables the platform to specifically monitor the expression of the individual components of the alternatively spliced mRNA populations.

19,391 human genes were submitted to the analysis process. 34056 alternative events were identified that matched the selection and scoring criteria of the analysis and selection algorithms. Probes were designed to the sense strand of the sequence as a 24 nucleotide sequence with a 35 nucleotide poly-T linker and the microarrays were produced by Agilent Technologies.

### 4. RNA amplification

15 to 50 ng of total RNA were used as matrix for target synthesis using the Ovation Pico WTA System v2 kit (NuGen, San Carlos, CA), as recommended by the manufacturer. The quality and quantity of cDNA generated were evaluated using Agilent's 2100 Bioanalyser (Agilent Technologies) and the NanoDrop 1000 Spectrophotometer (Labtech International, UK). 2 µg of cDNA molecules were labeled using Agilent SureTag DNA labeling kit, following manufacturer's recommended protocol. The quality of the Cy3-labelled cDNA is assessed by measuring the yield of cDNA and the specific activity, proportional to the degree of labeling, by measuring the absorbance of the purified product at 260nm and 550 nm.

### 5. Hybridization with GWSA slide

10 µg of cDNA amplified and labeled with Cy3 were used for the hybridization on the HsGWSA2.0.0.Dx POC 1M Agilent array following manufacturer's recommendation, at 55°C for 17 hours. Non-specific hybridization signals were removed by a 2-step wash of the arrays, following manufacturer's protocol. Hybridization signals were measured using the G5761A SureScan Dx Microarray Scanner System (Agilent Technologies, USA).

### 6. Data extraction and normalization

Intensity signal measurements were extracted with Feature Extraction 11.0.1.1 software (Agilent Technologies). The files (.Tiff) processed with Feature Extraction were quantile normalized using GeneSpring Gx software (Agilent Technologies) prior to statistical analysis. Expression values below 20 were all floored at 20. Probe sets were filtered out if the ratio of the maximum to minimum intensity across the training set was lower or equal to 2.5 or if the difference between the maximum and minimum intensity was lower or equal to 50. 207 043 probe sets from the 448 863 probe sets on the chips remained for further analysis.

### 7. Statistical analysis and signature identification

The point biserial correlation coefficient was calculated for probe sets between probe set expression and a binary indicator, the value of which was defined by the malignancy status of the patient (0 for cancer free and 1 for patients with Cancer) (van 't Veer, et al. 2002). The probe sets with the highest absolute correlation are those most correlated with the occurrence of thyroid cancer. T-tests to evaluate whether point biserial correlation values were significantly different from 0 were used.

To develop a prediction rule to classify samples from new patients with unknown status, we chose the nearest centroid prediction rule (Michiels et al, 2005). This prediction rule classifies new patients according to the correlation between the expression of their molecular classifier probe sets (or genes or events) and the average profiles in the two categories (the average Benign and Malignant profiles) are defined as the vector of the average expression values of the molecular classifier probe sets in benign and malignant samples in the training set, the predicted category being the one with the highest correlation. The molecular classifier probe sets were defined as those for which the p-value of the point biserial correlation coefficient with the benign or malignant binary status variable was below a particular threshold.

Leave-one-out cross-validation (LOOCV) was used to estimate the prediction accuracy of the rule determined on the training set. One sample is left out, and the remaining samples are used to build the prediction rule, which is then used to classify the left-out sample. The entire model-building process was repeated for each leave-one-out training set to provide unbiased estimates of the prediction accuracy of the prediction rule determined on the entire training set: correlation coefficients were recalculated, sorted according to their absolute value, the molecular classifier probe sets defined, and the nearest centroid prediction rule constructed. We predicted the outcome of the sample that was left out in the first place, on the basis of the highest correlation coefficient it had with the average benign and malignant profiles from the remaining other samples. At the end of the leave-one-out cross-validation procedure, we counted in how many cases the predictions were correct and in how many cases the predictions were incorrect to estimate the misclassification rate of the prediction rule determined on the entire training set. The results obtained are listed in the table below. ROC-curves are constructed to determine optimal cut-offs based on the correlation values with the cancer and benign profiles, and area under the ROC curve (AUC) has been calculated for estimating predictive accuracy.

### Classifier size reduction

- Starting from the list of probe sets (PS) included in the classifier developed using the nearest centroid prediction (NCP) rule and the 25 PS with p-value below 1e⁻⁷, the correlation between each PS expression level was estimated using Pearson correlation coefficients and associated p-values.

Based on these correlations coefficients (Approach C), 3 different selection methods were used in order to reduce the size of the signature:
1. For a given gene: selection of the PS the most correlated with the other PS of a given gene and the PS the least correlated with the other PS of this gene.
2. For a given gene: selection of the PS the least correlated with the other PS of this gene and the PS the most correlated with the other PS of this gene but slightly correlated with the PS of the other genes.
3. For a given gene : selection of the PS the most correlated with the other PS of this gene, the PS the least correlated with the other PS of this gene and the PS the most correlated with the other PS of this gene but slightly correlated with the PS of the other genes.

Two other approaches were used to reduce the size of the classifiers:
- by steps of 5 PS from 5 to 25 PS contained in the 25PS classifier (Approach A)
- by selecting the PS for which the correlation with all the patient samples is the most significant (Approach B).

### B. Results

Expression values below 20 were all floored at 20. Probe sets (PS) were filtered out if the ratio of the maximum to minimum intensity across the training set was lower or equal to 2.5 or if the difference between the maximum and minimum intensity was lower or equal to 50. 207 043 probe sets out of the 448 863 probe sets per chip remained for further analysis.

The probe sets associated to the highest fold changes and the most statistically significant of a difference between malignant and benign nodules were identified and are listed in Table 2.

**Table 2: Summary of PS Identification**

| Cut-off | Number of significant PS | Signature |
|---|---|---|
| 1e⁻² | 4196 | A |
| 1e⁻³ | 918 | B |
| 1e⁻⁴ | 278 | C |
| 1e⁻⁵ | 109 | D |
| 1e⁻⁶ | 51 | E |
| 1e⁻⁷ | 25 | F |
| 1e⁻⁸ | 12 | G |

Molecular signatures have been developed using the p-value thresholds described above. Table 3 summarizes the leave one out cross-validation results. In this table, the signatures error rates, sensitivities and specificities obtained with the nearest centroid prediction rules are denoted as Error Rate (diff), Sensitivity (diff) and Specificity (diff) because based on the difference in the correlation with the Malignant and Benign profiles.

The accuracy of the signatures is measured by the area under the ROC [Receiver Operating Characteristics] curve. The ROC curve is created by plotting the true positive rate (sensitivity) against the false positive rate (1-specificity) at various threshold settings. The point with coordinate (0,1) (also called perfect classification) in the ROC space is associated to a sensitivity of 100% and a specificity of 100%. A test with perfect discrimination has a ROC curve that passes through the upper left corner (100% sensitivity, 100% specificity). Therefore the closer the ROC curve is to the upper left corner, the higher the overall accuracy of the test (Zweig & Campbell, 1993).

The **A**rea **U**nder the ROC **C**urve (AUC) based on the difference between the correlation values (Malignant and/or Benign profiles) are labeled as AUC (diff), AUC (M) or AUC (B).

**Table 3: Summary of Leave-One-Out Cross Validation.**

| **Signature** | **Cut-off** | **Error Rate (diff)** | **Sensitivity (diff)** | **Specificity (diff)** | **AUC (diff)** | **AUC (B)** | **AUC (M)** |
|---|---|---|---|---|---|---|---|
| A | 1e⁻² | 18.3% | 60.0% | 92.5% | 70.4% | 76.5% | 62.9% |
| B | 1e⁻³ | 13.3% | 70.0% | 95.0% | 81.9% | 87.4% | 69.8% |
| C | 1e⁻⁴ | 13.3% | 75.0% | 92.5% | 81.9% | 86.5% | 78.0% |
| D | 1e⁻⁵ | 13.3% | 75.0% | 92.5% | 83.0% | 86.1% | 79.3% |
| E | 1e⁻⁶ | 16.7% | 70.0% | 90.0% | 81.8% | 79.5% | 77.1% |
| F | 1e⁻⁷ | 18.3% | 65.0% | 90.0% | 86.0% | 67.5% | 85.1% |
| G | 1e⁻⁸ | 23.3% | 60.0% | 85.0% | 77.0% | 52.1% | 77.0% |

### Performance of Signature B (918PS)

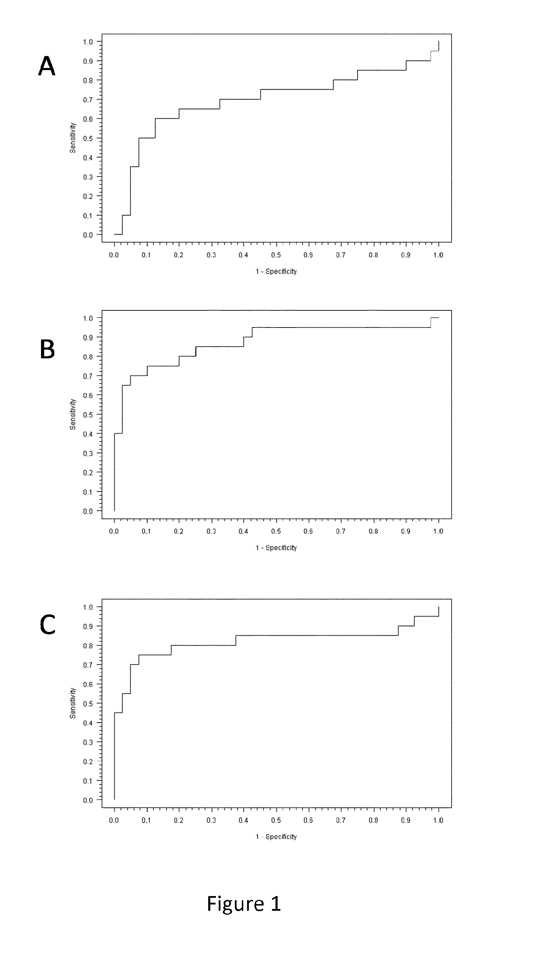

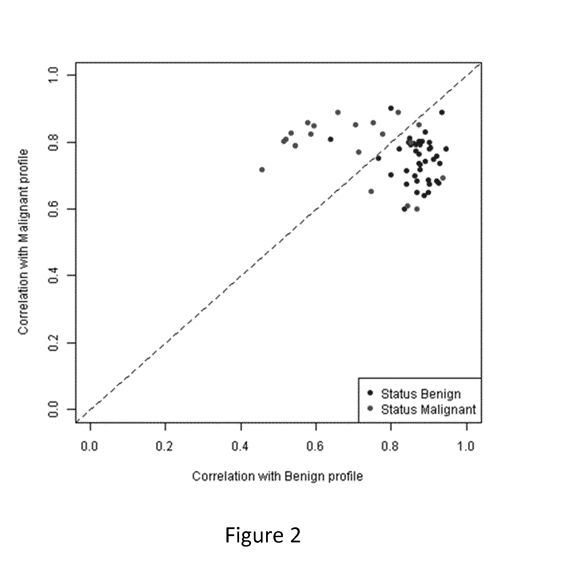

Signature B, based on the combination of 918 probe sets with p-values below 1e⁻³, produces the lowest overall error rate (13.3%). For this signature, the leave-one-out cross validated estimations of sensitivity and specificity are 70% and 95%, respectively. The ROC curves associated to the 918PS signature are presented in Figure 1. The correlation of each FNA sample with the average malignant and the benign profiles using LOOCV with a p-value cut-off of 10⁻³ is shown in Figure 2.

Signature B contains a plurality of probes that allows specific detection of at least one expression product of CITED1, TENM1, CCM2, SCLY, IGSF10 and FRMD3 and of each gene listed in Table A and in Table B.

### Performance of Signature D (109PS)

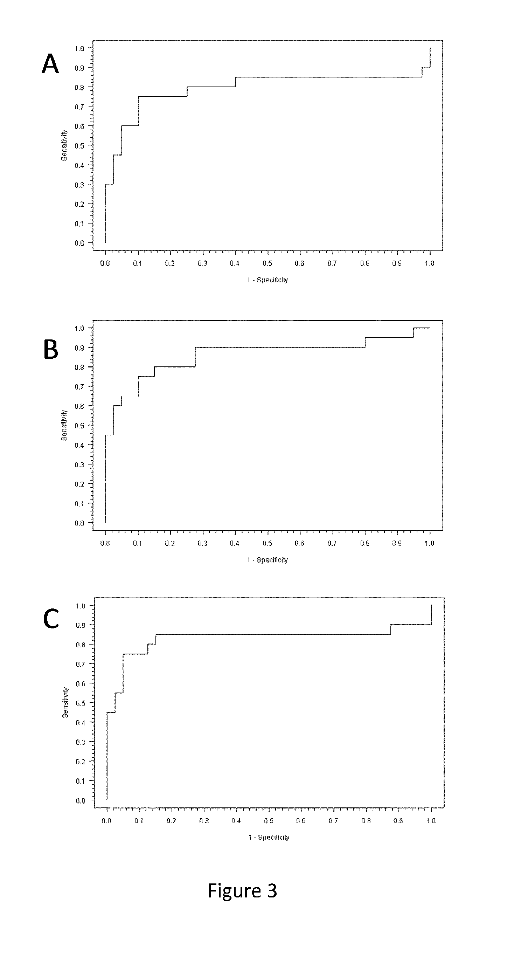

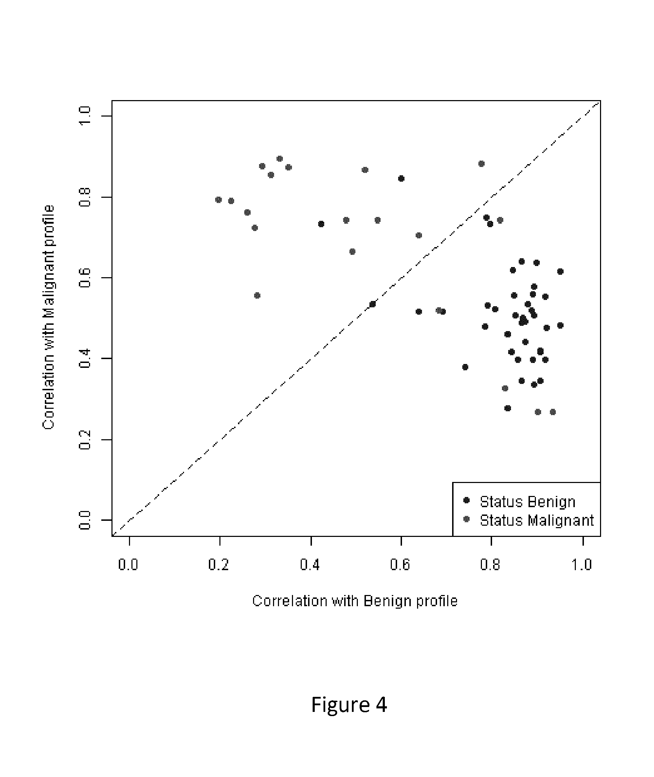

Signature D, based on the combination of 109 probe sets with p-values below 1e⁻⁵, produces also the lowest overall error rate (13.3%). For this signature, the leave-one-out cross validated estimations of sensitivity and specificity are 75% and 92.5%, respectively. The ROC curves associated to the 109PS signature are presented in Figure 3. The correlation of each FNA sample with the average malignant and the benign profiles using LOOCV with a p-value cut-off of 10⁻⁵ is shown in Figure 4.

Signature D contains a plurality of probes that allows specific detection of at least one expression product of CITED1, TENM1, CCM2, SCLY, IGSF10 and FRMD3 and of each gene listed in Table A.

### Performance of Signature F (25PS)

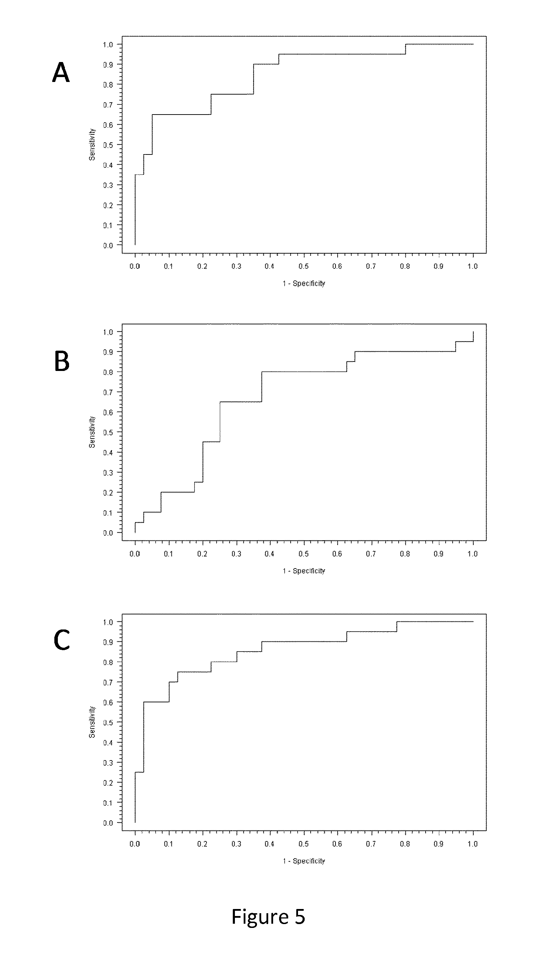

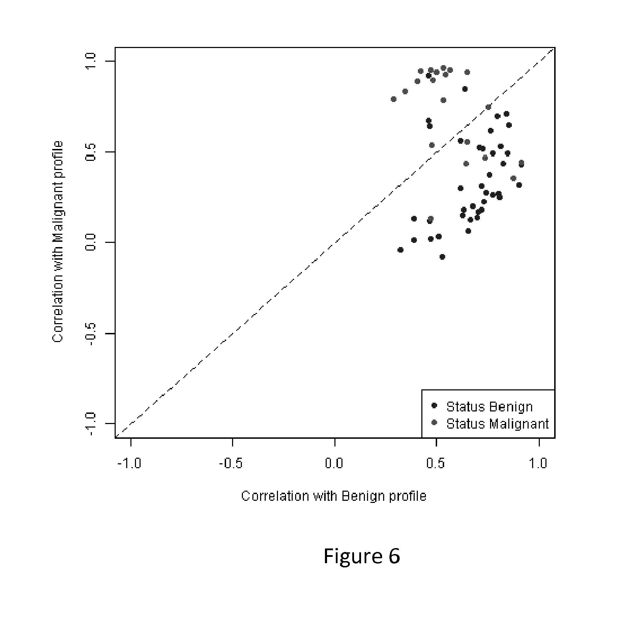

Signature F, based on the combination of 25 probe sets with p-values below 1e⁻⁷, produces an error rate of 18.3%. For this signature, the leave-one-out cross validated estimations of sensitivity and specificity are 65% and 90%, respectively. The ROC curves associated to the 25PS signature are presented in Figure 5. The correlation of each FNA sample with the average malignant and the benign profiles using LOOCV with a p-value cut-off of 10⁻⁷ is shown in Figure 6.

Each of these signatures may be used to effectively determine the malignant status of a thyroid lesion with very high specificity and a good sensitivity.

### Analysis of the content of Signature F (25PS)

In order to identify the smallest signature composition able to classify patient with high accuracy, 3 approaches (see Material and Methods) were selected and applied on the composition of the 25PS signature (90% specificity and 65% sensitivity). The results show that Signature F actually targets 6 distinct marker genes, namely CITED1, TENM1, SCLY, CCM2, IGSF10 and FRMD3. The composition and associated fold changes of the 25 PS are listed in Table 4. These target genes represent valuable markers which can be used to analyse thyroid cancer in patients.

**Table 4: 25 PS signature composition and associated fold changes**

| **Column ID** | **Fold-Change (M/B)** | **Ranking** | **Gene** |
|---|---|---|---|
| HSX2_01477710-333,HSX2_01477710-515\|HSX2_01477710 | 3,52 | 1 | TENM1 |
| HSX2_01477640-162,HSX2_01477640-81\|HSX2_01477640 | 4,88 | 2 | TENM1 |
| HSX2_01477635-192,HSX2_01477635-73\|HSX2_01477635 | 3,57 | 3 | TENM1 |
| HSX2_01477563-23,HSX2_01477563-15\|HSX2_01477563 | 6,53 | 4 | TENM1 |
| HSX2_01477645-87,HSX2_01477645-41\|HSX2_01477645 | 5,67 | 5 | TENM1 |
| HSX2_01477615-1\|HSX2_01477615 | 5,73 | 6 | TENM1 |
| HSX2_01477675-104,HSX2_01477675-288\|HSX2_01477675 | 4,40 | 7 | TENM1 |
| HSX2_01064548-127,HSX2_01064548-32\|HSX2_01064548 | 14,39 | 8 | CITED1 |
| HSX2_01064539-42,HSX2_01064539-77\|HSX2_01064539 | 11,43 | 9 | CITED1 |
| HSX2_02073060-1,HSX2_02073060-141\|HSX2_02073060 | 2,08 | 10 | CCM2 |
| HSX2_02509166-208,HSX2_02509166-401\|HSX2_02509166 | 8,21 | 11 | CITED1 |
| HSX2_01771392-673,HSX2_01771392-460\|HSX2_01771392 | 2,99 | 12 | SCLY |
| HSX2_01064533-104,HSX2_01064533-39\|HSX2_01064533 | 1,85 | 13 | CITED1 |
| HSX2_01064546-23,HSX2_01064546-24\|HSX2_01064546 | 12,74 | 14 | CITED1 |
| HSX2_01477625-72,HSX2_01477625-106\|HSX2_01477625 | 5,46 | 15 | TENM1 |
| HSX2_01064543-27,HSX2_01064543-46\|HSX2_01064543 | 10,95 | 16 | CITED1 |
| HSX2_01477568-13,HSX2_01477568-174\|HSX2_01477568 | 3,14 | 17 | TENM1 |
| HSX2_01477711-4356,HSX2_01477711-469\|HSX2_01477711 | 3,45 | 18 | TENM1 |
| HSX2_01477695-242,HSX2_01477695-17\|HSX2_01477695 | 3,17 | 19 | TENM1 |
| HSX2_02487762-422,HSX2_02487762-163\|HSX2_02487762 | 1,79 | 20 | IGSF10 |
| HSX2_01477665-106,HSX2_01477665-35\|HSX2_01477665 | 3,40 | 21 | TENM1 |
| HSX2_01477573-25,HSX2_01477573-189\|HSX2_01477573 | 3,40 | 22 | TENM1 |
| HSX2_02319619-6,HSX2_02319619-4\|HSX2_02319619 | 5,56 | 23 | FRMD3 |
| HSX2_01477556-78,HSX2_01477556-189\|HSX2_01477556 | 1,76 | 24 | TENM1 |
| HSX2_01064547-31,HSX2_01064547-166\|HSX2_01064547 | 3,44 | 25 | CITED1 |

Starting from the list of probe sets (PS) included in the 25PS signature, the correlation between each PS expression level was estimated using Pearson correlation coefficients and associated p-values. Based on these correlation coefficients, 3 different selection methods (Approach C methods 1, 2 and 3 as described in Material and Methods) as well as the following two approaches were used to reduce the size of the signatures:
- by steps of 5 PS from 5 to 25 PS contained in the 25PS signature (Approach A)
- by selecting the PS for which the correlation with all the patient samples is the most significant (Approach B)

Signatures associated to error rates below or equal to 20% were identified for the 3 approaches used and are listed in Table 5.

**Table 5: optimal signatures identified during signature size reduction analysis.**

| **# PS** | **Sensitivity** | **Specificity** | **Target Genes** |
|---|---|---|---|
| 15 | 65% | 87,50% | TENM1, CITED1, CCM2, SCLY |
| 8 | 75% | 82,50% | TENM1, CITED1, CCM2, SCLY, FRMD3, IGSF10 |
| 8 | 70% | 87,50% | TENM1, CITED1, CCM2, SCLY, FRMD3, IGSF10 |
| 10 | 70% | 90,00% | TENM1, CITED1, CCM2, SCLY, FRMD3, IGSF10 |
| 4 | 65% | 92,50% | TENM1, CITED1 |

### PS analysis focusing on CITED1 and TENM1

Various probe sets are designed that target particular regions of the CITED1 or TENM1 gene products. Tables 6 and 7 identify the exons targeted by these probes for TENM1 and CITED1, respectively, and the upregulations of these exons measured in the malignant samples of cytological indeterminate thyroid lesions.

**Table 6: Upregulation and exons identified with the PS composing the 25 PS signature and covering TENM1 gene.**

| **ProbeSetID** | **Targeted exon** | **Fold-Change(M vs**. **B)** |
|---|---|---|
| HSX2_01477556 | 1 | 1,76 |
| HSX2_01477568 | 4 | 3,14 |
| HSX2_01477695 | 29 | 3,17 |
| HSX2_01477573 | 5 | 3,40 |
| HSX2_01477665 | 23 | 3,40 |
| HSX2_01477711 | UTR | 3,45 |
| HSX2_01477710 | 32 | 3,52 |
| HSX2_01477635 | 17 | 3,57 |
| HSX2_01477675 | 25 | 4,40 |
| HSX2_01477640 | 18 | 4,88 |
| HSX2_01477625 | 15 | 5,46 |
| HSX2_01477645 | 19 | 5,67 |
| HSX2_01477615 | 13 | 5,73 |
| HSX2_01477563 | 3 | 6,53 |

**Table 7: Upregulation and exons identified with the PS composing the 25 PS signature and covering CITED 1 gene.**

| **ProbeSetID** | **Targeted exon** | **Fold-Change(M vs. B)** |
|---|---|---|
| HSX2_01064533 | (-2) | 1,85 |
| HSX2_01064547 | 4 | 3,44 |
| HSX2_02509166 | 4 | 8,21 |
| HSX2_01064543 | 4 | 10,95 |
| HSX2_01064539 | 3 | 11,43 |
| HSX2_01064546 | 4 | 12,74 |
| HSX2_01064548 | 4 | 14,39 |

Various combinations of such probes were assessed for relevance. The invention shows that when a limited number of target genes are monitored according to the invention, it is highly preferred to use two distinct probes specific for distinct regions of the expression product of said gene. In this regard, monitoring exon4 and/or exon3 and/or exon-2 of CITED1 and targeting exons 32, 25, 19, 18, 17, and/or 1, for TENM1 is particularly advantageous.

The following particular signatures have been tested for sensitivity:

Signature G1: one target gene (CITED 1), 3 different targeted domains (exon3, exon4, exon-2) of the expression product:
ProbeSets:
   HSX2_01064548
   HSX2_01064539
   HSX2_02509166
   HSX2_01064533
Sensitivity 73% and Specificity 83%

Signature G2: one target gene (CITED1), 3 different targeted domains (exon3, exon4, exon-2) of the expression product:
ProbeSets:
   HSX2_01064548
   HSX2_01064539
   HSX2_02509166
   HSX2_01064533
   HSX2_01064546
   HSX2_01064543
   HSX2_01064547
Sensitivity 75% and Specificity 90%

These results show that an effective and reliable detection of thyroid cancer can be implemented by monitoring 2 or 3 distinct domains of CITED1 expression product.

As discussed below, by further combining such marker with TENM1, a further specific and sensitive detection is obtained.

### Signature H: CITED1 & TENM1

In this experiment, two probeSets specific for CITED1 and two probeSets specific for TENM1 were combined. The specific probeSets used are listed below.
TENM1:
   HSX2_01477675
   HSX2_01477556
CITED1:
   HSX2_01064533
   HSX2_01064546
Sensitivity 65% and Specificity 92.5%

Further exon analysis results linked to the performances listed in table 5 are reported in Table 8, focusing only on CITED 1 and TENM1.

**Table 8: targeted exons for TENM1 and CITED1 in optimal signatures**

| **#PS** | **Targeted exons TENM1** | **Targeted exons CITED1** | **Sensitivity** | **Specificity** |
|---|---|---|---|---|
| 15 | 32, 18, 17, 3, 19, 13, 25, 15 | 3, 4, (-2) | 65% | 87,50% |
| 8 | 32, 18 | 3, 4 | 75% | 82,50% |
| 8 | 32, 18, 17 | 3, 4, (-2) | 65% | 87,50% |
| 8 | 25, 1 | 4, (-2) | 70% | 87,50% |
| 10 | 25, 19, 1 | 4, (-2) | 70% | 90,00% |
| 4 | 1,25 | 4, (-2) | 65% | 92,50% |

These results show that by detecting exon1, 19 and/or 25 of TENM1 and exon 4 and/or (-2) of CITED1, highly relevant diagnosis can be made.

### References

Curado, M. P., B. Edwards, et al. (2007). "Cancer Incidence in Five Continents." IARC Scientific Publications 9(160): 1-10.
Cibas, E. S. and S. Z. Ali (2009). "The Bethesda System for Reporting Thyroid Cytopathology." Thyroid 19(11): 1159-1165.
Nikiforov, Y. E., N. P. Ohori, et al. (2011). "Impact of mutational testing on the diagnosis and management of patients with cytologically indeterminate thyroid nodules: a prospective analysis of 1056 FNA samples." J Clin Endocrinol Metab 96(11): 3390-3397.
Alexander EK, Kennedy GC, Baloch ZW, Cibas ES, Chudova D, Diggans J, Friedman L, Kloos RT, LiVolsi VA, Mandel SJ, Raab SS, Rosai J, Steward DL, Walsh PS, Wilde JI, Zeiger MA, Lanman RB, Haugen BR. Preoperative diagnosis of benign thyroid nodules with indeterminate cytology. N Engl J Med. 2012 Aug 23;367(8):705-15. doi: 10.1056/NEJMoa1203208. Epub 2012 Jun 25
Hu S, Ewertz M, Tufano RP, Brait M, Carvalho AL, Liu D, Tufaro AP, Basaria S, Cooper DS, Sidransky D, Ladenson PW, Xing M. Detection of serum deoxyribonucleic acid methylation markers: a novel diagnostic tool for thyroid cancer. J Clin Endocrinol Metab. 2006 Jan;91(1):98-104. Epub 2005 Nov 1.
Nikiforova MN, Wald AI, Roy S, Durso MB, Nikiforov YE. Targeted next-generation sequencing panel (ThyroSeq) for detection of mutations in thyroid cancer. J Clin Endocrinol Metab. 2013 Nov;98(11):E1852-60.
Zweig MH1, Campbell G. Receiver-operating characteristic (ROC) plots: a fundamental evaluation tool in clinical medicine. Clin Chem. 1993 Apr;39(4):561-77.
Van 't Veer, L. J., H. Dai, et al. (2002). "Gene expression profiling predicts clinical outcome of breast cancer." Nature 415(6871): 530-536.
Michiels S, Koscielny S, Hill C. Prediction of cancer outcome with microarrays: a multiple random validation strategy. Lancet 2005; 365:488-92.

| **Entrez ID** | **Entrez Name** | **ProbeSetID** | **SEQ ID** # | **Target Sequence** |
|---|---|---|---|---|
| 51115 | 2410011022RIK | HSX2_01747164 | 1 | GCCTTAGGCTACTTTCACAGGGCAGAACAAG |
| 285533 | 335F15R | HSX2_02344199 | 2 | CTTTCATGAATTCTGCATCCGAGGTTGGTGTATCGTTGGGAAAAAGCAGACTTGCC CTTACTGCAAAGAGAAAGTTGATTTGAAGAGGATGATCAGTAATCC |
| 10921 | 4930402H05RIK | HSX2_02538806 | 3 | GGCGGCGGCGGGAAGATGATGCTGCTCTTTGGCG |
| 79897 | AA408606 | HSX2_02030256 | 4 | ATTCCAAACCACTACAACCCTTGCCAAACACAGCCCACTCCATTTCAGACCGCCTTCC TGAGGAGAAAATGCAGACTCAGGGTTCCAGTAACCAGTGATGGATTCACCCCATCT CCCAAATAAAGTTTACTTGTTTTACATTC |
| 10351 | ABCA8 | HSX2_01491300 | 5 | GCTATTAATTCTAATGGCTGGATTTTGCCCTCTTCTTGTGGAGTATACCATGGTGAA AATATATCAAAACAGTTACACCTGGGAACTTTCTCCTCATTTGTATTTCCTTGCTCCT GGACAACAACCACATGACCCTCTCACTCAACTACTGATCATCAATAAAACAG |
| 8714 | ABCC3 | HSX2_01355653 | 6 | GTTCTGGCCCCTGTCATCCTCATGCTGCTCAATTCCTTCTTCAACGCCATCTCCACTC TTGTGGTCATCATGGCCAGCACGCCGCTCTTCACTGTGGTCATCCTGCCCCTGGCTG TGCTCTACACCTTAGTGCAG |
| 35 | ACADS | HSX2_00754990 | 7 | ACTACCGCGACGCCCGCATCACTGAGATCTACGAGGGCACCAGCGAAATCCAGCG GCTGGTGATCGCCGGGCATCTGCTCAGGAGCTACCGGAGCTGAGCCCGCGGCGGA CTGCCCCAGGACTGCGGGAAGGCGCGGGAGCCAGGGGCCTCCACCCCAACCCCGG CTCAGAGACTGGGCGGCCCGGCGGGGGCTCCCTGGGGACCCCAGATGGGCTCAGT GCTGCCACCCAGATCAGATCACATGGGAATGAGGCCCTCCGACCATTGGCAGCTCC GCCTCTGGGCCTTTCCGCCTCCTCACCACTGTGCCTCAAGTTCCTCATCTAAGTGGC CCTGGCCTCCTGGGGGCGGGGTTGTGGGGGGCTGAGCGACACTCAGGGACACCT CAGTTGTCCTCCCGCGGGCCCTGGTGCCCTGGCATGAAGGCCCAGTGCGACAGGCC CTTGGTGGGGTCTGTCTTTTCCTTGAGGTCAGAGGTCAGGAGCAGGGCTGGGGTC AGGATGACGAGGCCTGGGGTCCTGGTGTTGGGCAGGTGGTGGGGCTGGGCCATG GAGCTGGCCCAGAGGCCCCTCAGCCCTTTGTAAAGTCTGATGAAGGCAGGGGTGG TGATTCATGCTGTGTGACTGACTGTGGGTAATAAACACACCTGTCCCCCA |
| 79968 | ACVR2B | HSX2_02034207 | 8 | GTAAATGAATATAAAGAAAATCAAAACATCGCTTATGTGTCTCTGAGACCAGCACA GACTACAGTTTTAATAAAAACAGCTAAGGTCTATCTTGCCCCCTTTTCACTCAG |
| 835 | ADAMDEC1 | HSX2_00819195 | 9 | CG |
| 56999 | ADAMTS9 | HSX2_01905394 | 10 | GGAGGGAGGGGTGGAAGCACCATGCAGTTTGTATCCTGGGCCACACTGCTAACG CTCCTGGTGCGGGACCTGGCCGAGATGGGGAGCCCAGACGCCGCGGCGGCCGTG CGCAAGGACAGGCTGCACCCGAGGCAAG |
| 56999 | ADAMTS9 | HSX2_01905410 | 11 | CTGGGCACATTCCGGTCTCATGATGGGGATTATTTATTGAACCACTACAGTCTATG GATGAACAAGAAGATGAAGAGGAACAAAACAAACCCCACATCATTTATAGGCGCA GCGCCCCCCAGAGAGAGCCCTCAACAGGAAGGCATGCATGTGACACCTCAG |
| 56999 | ADAMTS9 | HSX2_01905487 | 12 | GTTTAACATGCCTCATGATGACAACAACAAATGTAAAGAAGAAGGAGTTAAGAGTC CCCAGCATGTCATGGCTCCAACACTGAACTTCTACACCAACCCCTGGATGTGGTCAA AGTGTAGTCGAAAATATATCACTGAGTTTTTAGA |
| 56999 | ADAMTS9 | HSX2_01905592 | 13 | TGTTCAAAAAGCTGTGACGGTGGGACCCAGAGGAGAAGGGCTATTTGTGTCAATA CCCGAAATGATGTACTGGATGACAGCAAATGCACACATCAAGAGAAAGTTACCATT CAGAGGTGCAGTGAGTTCCCTTGTCCACAGTGGAAATCTGGAGACTGGTCAGAG |
| 56999 | ADAMTS9 | HSX2_01905598 | 14 | TGCTTGGTCACCTGTGGAAAAGGGCATAAGCACCGCCAGGTCTGGTGTCAGTTTGG TGAAGATCGATTAAATGATAGAATGTGTGACCCTGAGACCAAGCCAACATCTATGC AGACTTGTCAGCAGCCGGAATGTGCATCCTGGCAGGCGGGTCCCTGGGGACAG |
| 56999 | ADAMTS9 | HSX2_01905636 | 15 | TGCTCTGTGACCTGTGGGCAAGGTAGGGCAACCCGGCAAGTGATGTGTGTCAACT ACAGTGACCACGTGATCGATCGGAGTGAGTGTGACCAGGATTATATCCCAGAAACT GACCAGGACTGTTCCATGTCACCATGCCCTCAAAGGACCCCAGACAGTGGCTTAGC TCAGCACCCCTTCCAAAATGAGGACTATCGTCCCCGGAGCGCCAGCCCCAGCCGCA CCCATGTGCTCGGTGGAAACCAGTGGAGAACTGGCCCCTGGGGAGCA |
| 56999 | ADAMTS9 | HSX2_01905650 | 16 | CCAACGGTGAACGGTTTCCAGATTTGAGCTGTGAAATTCTTGATAAACCTCCCGATC GTGAGCAGTGTAACACACATGCTTGTCCACACGACGCTGCATGGAGTACTGGCCCT TGGAGC |
| 56999 | ADAMTS9 | HSX2_01905665 | 17 | GAACACACAAAATAGCCAGAGAGACCGAGTGCAACCCATACACCAGACCGGAGTC GGAACGCGACTGCCAAGGCCCACGGTGTCCCCTCTACACTTGGAGGGCAGAGGAA TGGCAAGAA |
| 56999 | ADAMTS9 | HSX2_01905746 | 18 | GATGGTACCCGAGTCGTAGGGAAATGCGGTGGTTACTGTGGAAAATGCACTCCAT CCTCTGGTACTGGCCTGGAGGTGCGAGTTTTATAGCTAAG |
| 56999 | ADAMTS9 | HSX2_01905758 | 19 | AGACCAAAGAGATTAGGAAAAGCCTGGCAGTATTCTCCAACTCCAAACAAGCTCTA GAGTGCTCCAGGAAAAGTTATATTCAGTATATGAATAAGTGTTATTCTCCATTATTA ATGTGTTCTGAAAATATATTATGAATAAATACATCACCACACCCAA |
| 56999 | ADAMTS9 | HSX2_02560668 | 20 | CCAACGGTGAACGGTTTCCAGATTTGAGCTGTGAAATTCTTGATAAACCTCCCGATC GTGAGCAGTGTAACACACATGCTTGTCCACACGACGCTGCATGGAGTACTGGCCCT TGGAGCTCG |
| 132 | ADK | HSX2_00763371 | 21 | CTTACAGTTTCCCCAGAGTCAGTATTAAAGGTGGATGTTGATATATGAAAGTTATGC CTTCTCACCATGCTTCTGAAAACAACAGGATTTTCACTTTGAATCTATCTGCACCGTT TATTAGCCAGTTCTACAAGGAATCATTG |
| 132 | ADK | HSX2_00763381 | 22 | GAAGCTGCCACTTTTGCTAGAGAGCAAGGCTTTGAG |
| 132 | ADK | HSX2_00763387 | 23 | CATTAAAGAGATAGCCAAAAAGACACAAGCCCTGCCAAAGATGAACTCAAAGAGG CAGCGAATCGTGATCTTCACCCAAGGGAGAGATGACACTATAATGGCT |
| 132 | ADK | HSX2_02491081 | 24 | AGAAAATATTCTCTTTGGAATGGGAAATCCTCTGCTTGACATCTCTGCTGTAGTGGA CAAAGATTTCCTTGATAA |
| 132 | ADK | HSX2_02491083 | 25 | GGCTTTTTCTTACAGTTTCCCCAGAGTCAGTATTAAAGGTGGCTCACCATGCTTCT GAAAACAACAGGATTTTCACTTTGAATCTATCTGCACCGTTTATTAGCCAGTTCTAC AAGGAATCATTGATGAAAGTTATGCCTTATGTTGATATACTTTTTGGAAATGAGACA |
| 132 | ADK | HSX2_02491084 | 26 | AAAGAGGCAGCGAATCGTGATCTTCACCCAAGGGAGAGATGACACTATAATGGCT ACAG |
| 134 | ADORA1 | HSX2_00763516 | 27 | GGAACGTGCTGGTGATCTGGGCGGTGAAGGTGAACCAGGCGCTGCGGGATGCCA CCTTCTGCTTCATCGTGTCGCTGGCGGTGGCTGATGTGGCCGTGGGTGCCCTGGTC ATCCCCCTCGCC |
| 165 | AEBP1 | HSX2_02491233 | 28 | AGTGTCCCCCCATTGGGATGGAGTCACACCGTATTGAGGACAACCAGATCCGAGCC TCCTCCATGCTGCGCCACGGCCTGGGGGCACAGCGCGGCCGGCTCAACATGCAG |
| 10551 | AGR2 | HSX2_01509381 | 29 | TGGAGAAAATTCCAGTGTCAGCATTCTTGCTCCTTGTGGCCCTCTCCTACACTCTGG CCAGAGATACCACAGTCAAACCTGGAGCCAAAAAGGACACAAAGGACTCTCGACC CAAACTGCCCCAGACCCTCTCCA |
| 126133 | ALDH16A1 | HSX2_02196227 | 30 | GTGTTCCAGGCTGGTGATGTGCCTTCGGAACGCCCATTCTATCCCCCAACCTTGGTC TCCAACCTGCCCCCAGCCTCCCCATGTGCCCAGGTGGAG |
| 23541 | ALMS1 | HSX2_02544485 | 31 | ATGTCCTGCGGTTTGACAACACCTACAGCTTCATTCAT |
| 51479 | ANKFY1 | HSX2_01767855 | 32 | CTTCTTGCGGGAGCCAAAGTGAACGAATTAACCAAGCATCGCCAGACTGCCCTCCA TCTTGCTGCCCAGCAGGACCTGCCCACCATCTGCTCAGTCCTCCTAGAGAATGGCGT GGACTTTGCTGCCGTGGATGAGAATGGAAACAATG |
| 60314 | ANKRD20A7P | HSX2_02562966 | 33 | ATGTATGAGAACTTTGTGGAGGAGGTGGATGCTGTGGACAATGGGATCTCCCAGT GGGCAGAGGGGGAGCCTCGATATGCACTGACCACTACCCTGAGTGCACGAGTTGC TCGACTTAATCCTACCTGGAACCACCCCGACCAAGACACTGAG |
| 6415 | ANKZF1 | HSX2_01213456 | 34 | TTTGAAGTGATGGTAGCCGGGAAGTTGATTCACTCTAAG |
| 6415 | ANKZF1 | HSX2_02518360 | 35 | TGGCGCTTGAGGCTACAAGTCCAAG |
| 55129 | ANO10 | HSX2_01833062 | 36 | GCCAGGCACTGTCATAGGTCCTGGGGTCTAGTCATGGCCCTGCTCTCTGGAGACCA CATACCAG |
| 334 | APLP2 | HSX2_00781384 | 37 | GTAAGAATAAAGTGGATGAAAACATG |
| 143872 | ARHGAP42 | HSX2_02226497 | 38 | TCTTCTCATTCCTCTGAACAAAATAGCACTACAAAGTCAGCTTCCTGCCAGCCCAGG GAGAAATCTGGAGGGATTCCTTGGATTGCAACCCCATCATCTTCCAATGGACAGAA AAGCCTTGGTCTGTGGACAACTAGTCCTGAATCAAGTTCCAGAGAAGATGCAACCA AGACAGATGCAGAATCAGACTGCCAGAGTGTTGCTTCAGTCACTAGCCCAGGAGAC GTTTCCCCACCCATAGACCTAGTCAAGAAAGAGCCTTATGGGCTTTCAGGACTGAA AAGAGCTTCTGCTTCTTCTCTCAGATCCATCTCTGCAGCTGAAG |
| 143872 | ARHGAP42 | HSX2_02226509 | 39 | GAAACAAGAGCTACAGTGGATCTATTCAAAGCTTAACTTCTGTAGGTTCCAAGGAG ACACCCAAAGCTTCACCAAACCCAGACCTGCCTCCGAAAATGTGCAGGAGATTAAG ACTAGACACTGCCTCAAGCAATGGCTATCAGCGGCCTGGCTCAGT |
| 7024 | ARMC3 | HSX2_01262294 | 40 | AGATGATGTGATCCAAATCTGTGGCCCTGCAGATGGAATCAGACTTTTTAATGCATT AAAAGGCCG |
| 7024 | ARMC3 | HSX2_02521603 | 41 | GGGCAGATTTATTGAAATTAACTAGAGATGATGTGATCCAAATCTGTGGCCCTGCA GATGGAATCAGACTTTTTAATGCATTAAAAGGCCG |
| 51566 | ARMCX3 | HSX2_01772667 | 42 | TGGGTCCAAGCCTCTCCCGGGTGGC |
| 51566 | ARMCX3 | HSX2_02552621 | 43 | TGGGTCCAAGCCTCTCCCGGGTGGCCAG |
| 54470 | ARMCX6 | HSX2_02553938 | 44 | GTTTGTAGGTCTGTTGGCCTGTAGGTTTCGGCACAAGTTTCAGCGAGAGAAGGAG AAAACTGCCTTGGTTGGAACCTTGCAG |
| 430 | ASCL2 | HSX2_00787901 | 45 | CAGGTGGACCGGCCCGCGCTCCGCCCCCAGCGAGCCGGGGACCCACCCACCACCC CCCGCACCGCCGACGCCGCCTCGTTCGTCCGGCCCAGCCTGACCAATGCCGCGGTG GAAACGGGCTTGGAGCTGGCCCCATAAGGGCTGGCGGCTTCCTCCGACGCCGCCC CTCCCCACAGCTTCTCGACTGCAGTGGGGCGGGGGGCACCAACACTTGGAGATTTT TCCGGAGGGGAGAGGATTTTCTAAGGGCACAGAGAATCCATTTTCTACACATTAAC TTGAGCTGCTGGAGGGACAC |
| 23250 | ATP11A | HSX2_01615000 | 46 | GTGTGGCTATTTACACGGGAATGGAAACCAAGATGGCATTAAATTATCAATCAAAA TCTCAGAAGCGATCTGCCGTGGAAAA |
| 23250 | ATP11A | HSX2_01615045 | 47 | TTCCTCAAGGCATTCACGGACTTCCTGGCCTTCATGGTCCTCTTTAACTACATCATCC CTGTGTCCATGTACGTCACGGTCGAGATGCAGAAGTTCCTCGGCTCTTACTTCATCA CCTGGGACGAAGACATGTTTGACGAGGAGACTGGCGAGGGGCCTCTGGTGAACAC GTCGGACCTCAATGAAGAGCTGGGACAG |
| 84239 | ATP13A4 | HSX2_02091652 | 48 | CTGGAGTTCAAGAGCTGGAATCACGCGTCCTGGTGCCTGGAGATTTATTAATTTTG ACAGGGAACAAAGTGCTAATGCCATGTGATGCCGTTCTGATTGAAGGCAGCTGTGT GGTGGATGAAGGCATGCTGACAG |
| 84239 | ATP13A4 | HSX2_02091695 | 49 | GAAATGGCTTTTTCTGGGGACGATTTCCACATCAAGGGAGTGCCGGCACATGCCAT GGTAGTTAAGCCCTGCAGAACAGCCAGCCAG |
| 51310 | AU018682 | HSX2_02551672 | 50 | CTTCATTGCCCATGCCATTCGCCACTGCTACCAGCCTGTGGGAGGAGGAGGGAGCC CATCGGACTTCTACCTGTGCTCTCTGCTGGCCAGCGGCACCGCAGCCCTGGCCTGT GTCTTCCTGGGGGTCACCGTGGACCGATTTGGCCGCCGGGGCATCCTTCTTCTCTCC ATGACCCTTACCGGCATTGCTTCCCTGGTCCTGCTGGGCCTGTGGGATT |
| 27241 | BBS9 | HSX2_02548207 | 51 | GTGGTTGTACTACAATCCCAGAGTCAGACCTAGAAGAAAGATCAGTAGAACAAGAC TCTACAGAACTGTTTACCAACCACAGACATCTCACTGCAGAGACACCCAGGCCTG |
| 607 | BCL9 | HSX2_00799653 | 52 | TAGCCCTAAGTCAAAGCAGGAGGTGATGGTCCGTCCCCCTACAGTGATGTCCCCAT CTGGAAACCCCCAGCTGGATTCCAAATTCTCCAATCAGG |
| 607 | BCL9 | HSX2_00799660 | 53 | TGACCCCATCAAATGCTACAGCCCCCAGGTCTTCTACCCCCTCCCATGGCCAAACTA CTGCCACAGAGCCCACACCTGCTCAGAAGACTCCAGCCAAAGTGGTGTACGTGTTT TCTACTGAGATGGCCAATAA |
| 607 | BCL9 | HSX2_02493341 | 54 | CTGGAAACCCCCAGCTGGATTCCAAATTCTCCAATCAGGTAAACAGGGGGGCTCA GCCAGCCAATCCCAGCCATCCCCCTGTGACTCCAAGAGTGGGGGCCATACCCCTAA AGCACTCCCTGGCCCAGGTGGGAGCATGGGGCTGAAGAATGGGGCTGGAAATGG TGCCAAGGGCAAGGGGAAAAGGGAGCGAAGTATTTCCGCCGACTCCTTTGATCAG AGAGATCCTGGGACTCCAAACGATGACTCTGACATTAAAG |
| 6585 | BCOR | HSX2_01227688 | 55 | AATGCTGCGGAACAACCGCATCAGCTGCATCCACAACGACAGCTTCACGGGCCTGC GCAACGTCCGGCTCCTCTCGCTCTACGACAACCAGATCACCACCGTATCCCCAGGA GCCTTCGACACCCTCCAGTCCCTCTCCACACT |
| 6585 | BCOR | HSX2_01227758 | 56 | GTCTCCACGGCAGAGGACAATGGGATCCTTCTGTACAACGGGGACAACGACCACAT TGCAGTTGAGCTGTACCAGGGCCATGTGCGTGTCAGCTACGACCCAGGCAGCTACC CCAGCTCTGCCATCTACAG |
| 627 | BDNF | HSX2_00800274 | 57 | CCATTCACCGCGGAGAGGGCTGCGCTGCCG |
| 79365 | BHLHE41 | HSX2_02006257 | 58 | ACTCTGGGACATCTGGAGAAAGCTGTAGTCTTGGAATTAACTTTGAAACACTTAAA AGCTTTAACCGCCTTAACCGAGCAACAGCATCAGAAGATAATTGCTTTACAGAATG |
| 440335 | C030011L09RIK | HSX2_02404970 | 59 | GAAAGACCCAAGGGAGTGGATAACTTGGCCCTGGAACCCTGACCCTGTGTCTCCTG CCCGGTGGCAGTAACAAAGCCTTCTGTCTGCCCAGA |
| 89927 | C16ORF45 | HSX2_02130811 | 60 | CCACAAACTGGTGCAGAAGAGAGACTTCCTGGTGGACGATGCGGAGGTCGAGCGG TTAAG |
| 29798 | C2ORF27A | HSX2_01722550 | 61 | GCTTTTAGAGCACTGTGATGTAACATGTCAAGCAGAAATAGGGAGCATGTTTACAG CCATTCTATGAAAAAGTGTTCGGAATGTACAGACTAGCACAGAAGCTGGACTAATT GAACAAGTATTGCTGAAAATGAGTGCTGTAGATGACATGATAGCAG |
| 84417 | C2ORF40 | 84417.001.1-T | 62 | GCCGTTGATGAGAATAAAGCCAAAGAATTCCTTGGCAGCCTGAAGCGCCAGAAGC GGCAGCTGTGGGACCGGACTCGGCCCGAGGTGCAGCAGTGGTACCAGCAGTTTCT CTACATGGGCTTTGACGAAGCGAAATTTGAAGATGACATCACCTATTGGCTTAACA GAGATCGAAATGGACATGAATACTATGGCGATTACTACCAACGTCACTATGATGAA GACTCTGCAATTGGTCCCCGGAGCCCCTACGGCTTTAGGCATGGAGCCAGCGTCAA CTACGATGACTACTAACCATGACTTGCCACACGCTGTACAAGAAGCAAATAGCGAT TCTCTTCATGTATCTCCTAATGCCTTACACTACTTGGTTTCTGATTTGCTCTATTTCAG CAGATCTTTCTACCTACTTTGGTGATCAAAAAAGAAGAGTTAAAACAACAC |
| 84417 | C2ORF40 | 84417.001.2-T | 63 | GCCGTTGATGAGAATAAAGCCAAAGAATTCCTTGGCAGCCTGAAGCGCCAGAAGC GGCAGCTGTGGGACCGGACTCGGCCCGAGGTGCAGCAGTGGTACCAGCAGTTTCT CTACATGGGCTTTGACGAAGCGAAATTTGAAGATGACATCACCTATTGGCTTAACA GAGATCGAAATGGACATGAATACTATGGCGATTACTACCAACGTCACTATGATGAA GACTCTGCAATTGGTCCCCGGAGCCCCTACGGCTTTAGGCATGGAGCCAGCGTCAA CTACGATGACTACTAACCATGACTTGCCACACGCTGTACAAGAAGCAAATAGCGAT TCTCTTCATGTATCTCCTAATGCCTTACACTACTTGGTTTCTGATTTGCTCTATTTCAG CAGATCTTTCTACCTACTTTGGTGATCAAAAAAGAAGAGTTAAAACAACAC |
| 84417 | C2ORF40 | HSX2_02098213 | 64 | CTATGGCGATTACTACCAACGTCACTATGATGAAGACTCTGCAATTGGTCCCCGGA GCCCCTACGGCTTTAGGCATGGAGCCAGCGTCAACTACGATGACTACTAACCATGA CTTGCCACACGCTGTACAAGAAGCAAATAGCGATTCTCTTCATGTATCTCCTAATGC CTTACACTACTTGGTTTCTGATTTGCTCTATTTCAGCAGATCTTTTCTACCTACTTTGT GTGATCAAAAAGAAGAGTTAAAACAACACATGTAAATGCCTTTTGATATTTCATG GGAATGCCTCTCATTTAAAAATAGAAATAAAGCATTTTGTTAAAAAGAA |
| 720 | C4A | HSX2_00807218 | 65 | GCCAGGGACCTGGAAGATCTCAGCCCGATTCTCAGATGGCCTGGAATCCAACAGCA GCACCCAGTTTGAGGTGAAGAAATATG |
| 721 | C4B | HSX2_00807494 | 66 | GCCAGGGACCTGGAAGATCTCAGCCCGATTCTCAGATGGCCTGGAATCCAACAGCA GCACCCAGTTTGAGGTGAAGAAATATG |
| 721 | C4B | HSX2_00807696 | 67 | GCCAGGGACCTGGAAGATCTCAGCCCGATTCTCAGATGGCCTGGAATCCAACAGCA GCACCCAGTTTGAGGTGAAGAAATATG |
| 1E+08 | C4B_2 | HSX2_02455999 | 68 | GCCAGGGACCTGGAAGATCTCAGCCCGATTCTCAGATGGCCTGGAATCCAACAGCA GCACCCAGTTTGAGGTGAAGAAATATG |
| 1E+08 | C4B_2 | HSX2_02456123 | 69 | TGGATAAAAACCTGACTGTGAGCGTCCACGTGTC |
| 770 | CA11 | HSX2_00811047 | 70 | CCTAAGCCTCCCCACAAGGCGAGGGGAGTTACCCCTAAAACAAAGCTATTAAAGGG ACAGAATACTT |
| 10241 | CALCOCO2 | HSX2_01482492 | 71 | CTTTTGAAGAGGGAGAACAGCAGATTGCTCAGTTACATGGGTCTGGATTTTAATTC TTTGCCGTATCAAGTACCTACTTCAGATGAAGGAGGCGCAAGACAAAATCCAGGAC TTGCCTATGGAAACCCATATTCTG |
| 55450 | CAMK2N1 | HSX2_01852736 | 72 | CCTCCTGGTGTCTAACTCCCCCAAAGACAATGAGTTAAGGGAGAGAATAAGAACGG CGGTAACAGTTATTGGCAAAAAGCATGAAAAGAGAAAGCACTTTGAAATTTATTAC TAGCTTGCTACCCACGATGAAATCAACAACCTGTATCTGGTATCAGGCCGGGAGAC AGATGAGGCGAGAGGAGGAGGAGGAGGAGGAGAAGGCTCTGGGCTCCTCTGCAA AAATAAAAATAAAAAAATAAATAAAATTTTAAAAATAATAAAAATTCACTATATACA CATATAAAGAAATAAAAAGAAGTCTCAGTTGCAGCTATTTGTCAAAATTAATATCCA TTTCTTTTTATATACGGTGAATATTGCGCAATTATAGATCTGGATTTTGAACCACTTA ATGAAGCGGCAACACCAGGTGTTTTGAGGTGTTGGCATTCTTCGCTGATTTGGCTG TTCCCAATGTTTACATTATTTAATCTTGCAAAAATGGTTCTGTGCACTTGGATGTGA AATGCTGTCCAGTTTTATTTTTTTTATGTTGTTATCCTTGGATGTACAAAAAATTCAG AAAATGATCTCTGTAGATATTCTGTTTTATTTTGGTCATCTTTAGAAGTTATCAGGAA TGTGTTTAAAACAAGAAGAGAACTTTTCTAAGGAATGATACATAGAAAAGATTTTA TTTTAAAATGAGTTGTAAAGCTTGTGTTTCTTTGTTGCTGCAAGCTATCTGCCCAAG TTAATGCAAATGGACACATTTTTTATGTCAGAAAAACACACACACACACACACACAC ACACACACACACACGAAAAACAAAGAAAAAAATGCTTGAGCTTTTCTAACTTCCCC TTGCAGTCTGTTGTGTGAGCAGCCTGTTTATTTCTCTAATATTATGTCAGTTTATTCT CTTTAATGGACTGTAAAAAAATGTAATCACAAGAGTGCCAAATATCTTGAAATGCC AAAAGGCATTTTAGTTTCTTTTCTCTGTGCTCTGAGTCCACGTACAGGAATGCTTGG AGTGTCTTTTCTGTTATTTATAGGGATTCTCTTAAGGCACACCAGCTGCCTGTTTTGC ATGGTATTTGCAAAAATGCCTCTTGCGTGAGGAAATCTTTTACCATTTTTTGTTTGCA ACTTTGGACCTCAAGAGGTTTCCCTTCCCTTCCCCGTTCCCTCTTTTCTTAATTCAATA |
| 825 | CAPN3 | HSX2_00817525 | 73 | ATGGTAAAGATCAGCCTGTGGCTTTTACGTATTGTGCCAAAACATCCATCCTGAGCC TGGAGAAGCTCTCACTCAACATTGCCACTGACTGGGAG |
| 825 | CAPN3 | HSX2_00817595 | 74 | GGAAGTTGAAAATACCATCTCCGTGGATCGGCCAGTG |
| 113201 | CASC4 | HSX2_02162192 | 75 | AGCAACTTGCTGAGCTTCGTCAGGAATTTCTTCGACAAGAAGACCAGCTTCAGGAC TATAGGAAGAACAATACTTACCTTGTGAAGAGGTTAGAATATGA |
| 836 | CASP3 | HSX2_00819290 | 76 | GTGGGTGTGCCCTGCACCTGCCTCTTCCCCCATTCTCA |
| 836 | CASP3 | HSX2_02027519 | 77 | GACCCAGGACTTAATTACCCAGTGCCCAGTTGTG |
| 57639 | CCDC146 | HSX2_01937063 | 78 | CAAGCTTTTAAAGGAGCAAGAAAACATGAAAGAGCTAGTAGTCAACCTTCTCCGCA TGACTCAAATCAAAATTGATGAAAAGGAACAAAAGTCCAAGGATTTCCTGAAAGCT CAG |
| 57639 | CCDC146 | HSX2_01937168 | 79 | TGCTGAGCTGTCCATGAAACAAGCCCTAACCATTGAACTCCAAAAGGAAGTCAGGG AGAAAGAAGACTTCATCTTCACTTGCAATTCCAGGATAGAAAAAGGTCTGCCACTC AATAAGGAAATTGAGAAAGAATGGTTGAAAGTCCTTCGAGATGAAGAAATGCACG CCTTGGCCATCGCTGAAAAGTCTCAG |
| 154467 | CCDC167 | HSX2_02255236 | 80 | ATCGATGGGCTAGAGGAGAAGCTGTCCCAGTGTCGGAGAGACCTGGAGGCCGTGA ACTCCAGACTCCACAGCCGGGAGCTGAGCCCAGAGGCCAG |
| 146439 | CCDC64B | HSX2_02233673 | 81 | CTCACTTTCCTCTTCAGGCCAATGGAGCGACAGGGCCCAGATTGTCTTCCAAAAGA AGCTGATGCCCTCCCCGCCCCAGGATGCTGGGCAAGGGCTCATCGGGAGCAGGGG CTCATCCGGAGCAGGGGCCAGCTTTGTGGGGCAGGAGCTCCAGGTGGGTGGCAG GGGCAGGTGAACAGAGCTATTTTCCGAATTAATATA |
| 114800 | CCDC85A | HSX2_02167533 | 82 | CATATTCATTCAGTGTATCCATTGGTGTTAATATTAACAATTTCAACTAAAAACAACA AACATAATTTTTCTAAGTGTTTCGAGCTGTGAGATGTCAATTTCTACAATTTAGTTTC TAGACATGCTGTATATTTAATAAACTTTATGTAAACTTTAAAATATTGCACTGCATTT ATGAAAAAAGCTTTCTTTGCCTACTGCAGCTATCTAACATTTTATGAAACTGACGCA TGTCCTTCATTATTGAGGCTAAAAGCTCTTGTTCAGATTGCTTGAGGTTTGAAAAAG AGGTGTGCTAGCTTTGCTTAGTTTATTTCTTATTTTTGGTATACATATACATATATAT AACATTAGAATGTGTGTACTGGAAATGCTATGATAGGTATTTTGTGTTAATCCAAAT GCAATGATAGTTTCTTGTATGAATGTGCAAGAGGCCTGTGACCGAATGCTACGGTT GTGTGGAGGAAACTTGGAGATGCTGCAGGTTCGTGTCCTGGAATTAGGCAACATTT GTCAGGAAACCAGTACAAAGGACCAATGTGAGATGCACTCTTTTTCAAACAGGAGA TCACCACTGCCAGAAAGTGATAGAAGACAAGAAGAAAAAGGAAAGAGTGGGTTTC CACAAACCTGGACTCATGGAATAACATGGAGTGATTGTACATTGCACATATCTCCCC TCTAAAACCTGTAGTACAACTTCTCCCCTCAAGCTGATATTCTGTGTCTCTCACCTCA ATGTCCACAACAGTCAATCTCAAACAAGGTAGCTTTGAACATCTCAAACAGAGTAG CTTTGAAAATCAACATTTTGGTACCAGTGTTTTCATTAGAAATAAGTGTTTTTAACTC CCCAGATATAATATTGTAATTGGGAAACCTGAATAGTTTTAAGAGTCCAGTGGAGC CTCAGTGTTTTGAATAGAGATGACTCATTACACATAATGGTAGGAGTTTTCTACCAT ATCATATTGAAGATATGAAGAGGTATTTTCCAGCTTCCCAAGTTTGAGAGTTGGGC ATTTACCTCTTACACTTCAGTCAACAAACAAACTAGTAATTCCTAATGCATAATTAAT TTGGATGCAAGATAAGATTAAGTTTCTGGAGTTTTGTTTTGACTTCTGAGGTGCTAA AAAATACCATGTAAATTCTCCCTACCAGCTAGAAGGGTAGCAAGACGTTGTAGAGT |
| 83605 | CCM2 | HSX2_02073060 | 83 | TCGGCACCCTCAGAGGGGGATGAGTGGACCGCATGATCTCGGACATCAGCAGCG ACATTGAGGCGCTGGGCTGCAGCATGGACCAGGACTCAGCATGATGGACAGTGGA TGGGGGGGCACCCACACCTTCCGCGCAGTCGTCATAGGCCTTCCCAGAAGGAGCT GCC |
| 894 | CCND2 | HSX2_00822288 | 84 | GAGTGGGAACTGGTGGTGCTGGGGAAGTTGAAGTGGAACCTGGCAGCTGTCACTC CTCATGACTTCATTGAGCACATCTTGCGCAAGCTGCCCCAGCAGCGGGAGAAGCTG TCTCTGATCCGCAAGCATGCTCAGACCTTCATTGCTCTGTGTGCCACCG |
| 645121 | CCNI2 | HSX2_02420442 | 85 | GTCTTCAGTCATCCTCCTGCACAGACAACTTTGTGTCACCTGCCAACTAGCCCCTCT GCCTCCACCCCGGGGCTTTCAGAGCATAGTGTGAAACCTCCTTGCTTGGACTACCAT GAGTTCTTTGGCTTGTTATGAATCCTGTAAAAAGGGAAGGTGGCTCTGGAAGAGCA ACTGAGAAAAAGTTCCCAACTGAGCCCTTGGAAAAAAAATAAAGGGGAGAGGGGA AAGGCAGGCTGAGGTCAGTAGAGGTCAGGGTGGTCTGATAGACTATGACCCTGTC TTCCCTTTTTCTCCTCTGGGCCTGAAGCCAGGGAGTATGAATGAATGTTCAAATGGC AGCTTGTTTTACCTTCCTTCTCCAGCAAGGGTTGGCATTGGCCCCTGGGAGCGCTG GAATATGAAACCAAGAGGCAGGCCTGGCTCAGGGCTGAAGGGCTGGGGCTAGCTC TGACCAGTTCTTGCTGCTCTACCTGCCAGAGCTGGCCAGGTCCTTCCAACTTCCCAG GCTTATCCCAAACACATATGTGGTGCTTCCAGTCCCAACTACCCTGCC |
| 283316 | CD163L1 | HSX2_02326932 | 86 | CTTCCAGGGCTCACCAGATCAACCTCTAAATATC |
| 961 | CD47 | HSX2_00827007 | 87 | AAAGCTGTAGAGGAACCCCTTAATG |
| 961 | CD47 | HSX2_00827010 | 88 | CTATACAACCTCCTAGGAAAGCTGTAGAGGAACC |
| 1014 | CDH16 | HSX2_00832581 | 89 | CGGTCCCTACAGCTTCACCCTTGGTCCCAACCCCACGGTGCAACGGGATTGGCGCC TCCAGACTCTCAATG |
| 1001 | CDH3 | HSX2_00831293 | 90 | GGCCCCTTTTGTGCTGAAGCTCCCAACCTCCACAGCCACCATAGTGGTCCACGTGG AGGATGTGAATGAGGCACCTGTGTTTGTCCCACCCTCCAAAGTCGTTGAGGTCCAG GAGGGCATCCCCACTGGGGAGCCTGTGTGTGTCTACACTGCAGAAGACCCTGACA AGGAGAATCAAAAGATCAG |
| 1001 | CDH3 | HSX2_00831298 | 91 | CTACCGCATCCTGAGAGACCCAGCAGGGTGGCTAGCCATGGACCCAGACAGTGGG CAGGTCACAGCTGTGGGCACCCTCGACCGTGAGGATGAGCAGTTTGTGAGGAACA ACATCTATGAAGTCATGGTCTTGGCCATGGACAATG |
| 1001 | CDH3 | HSX2_00831310 | 92 | GTGACACAGTGGTCTTGTCCCTGAAGAAGTTCCTGAAGCAGGATACATATGACGTG CACCTTTCTCTGTCTGACCATGGCAACAAAGAGCAGCTGACGGTGATCAGGGCCAC TGTGTGCGACTGCCATGGCCATGTCGAAACCTGCCCTGGACCCTGGAAGGGAGGTT TCATCCTCCCTGTGCTGGGGGCTGTCCTGGCTCTGCTGT |
| 1001 | CDH3 | HSX2_00831320 | 93 | GACTATGACATCACCCAGCTCCACCGAGGTCTGGAGGCCAGGCCGGAGGTGGTTC TCCGCAATGACGTGGCACCAACCATCATCCCGACACCCATGTACCGTCCTCGGCCA GCCAACCCAGATGAAATCGGCAACTTTA |
| 1001 | CDH3 | HSX2_00831328 | 94 | CTGCTGTGACTGACCTACAGTGGACTTTCTCTCTGGAATGGAACCTTCTTAGGCCTC CTGGTGCAACTTAATTTTTTTTTTTAATGCTATCTTCAAAACGTTAGAGAAAGTTCTT CAAAAGTGCAGCCCAGAGCTGCTGGGCCCACTGGCCGTCCTGCATTTCTGGTTTCC AGACCCCAATGCCTCCCATTCGGATGGATCTCTGCGTTTTTATACTGAGTGTGCCTA GGTTGCCCCTTATTTTTTATTTTCCCTGTTGCGTTGCTATAGATGAAGGGTGAGGAC AATCGTGTATATGTACTAGAACTTTTTTATTAAAGAAACTTTTCCCA |
| 1001 | CDH3 | HSX2_00831335 | 95 | GCAACTTTATAATTGAGAACCTGAAGGCGGCTAA |
| 1001 | CDH3 | HSX2_02495403 | 96 | GACTATGACATCACCCAGCTCCACCGAGGTCTGGAGGCCAGGCCGGAGGTGGTTC TCCGCAATGACGTGGCACCAACCATCATCCCGACACCCATGTACCGTCCTCGGCCA GCCAACCCAGATGAAATCGGCAACTTTATAATTGAG |
| 1001 | CDH3 | HSX2_02495404 | 97 | GTTCGACTATGAGGGCAGCGGCTCCGACGCCGCGTCCCTGAGCTCCCTCACCTCCT CCGCCTCCGACCAAGACCAAGATTACGATTATCTGAACGAGTGGGGCAGCCGCTTC AAGAAGCTGGCAGACATGTACGGTGGCGGGGAGGACGACTAGGCGGCCTGCCTG CAGGGCTGGGGACCAAACGTCAGGCCACAGAGCATCTCCAAGGGGTCTCAGTTCC CCCTTCAGCTGAGGACTTCGGAGCTTGTCAGGAAGTGGCCGTAGCAACTTGGCGG AGACAGGCTATGAGTCTGACGTTAGAGTGGTGGCTTCCTTAGCCTTTCAGGATGGA GGAATGTGGGCAGTTTGACTTCAGCACTGAAAACCTCTCCACCTGGGCCAGGGTTG CCTCAGAGGCCAAGTTTCCAGAAGCCTCTTACCTGCCGTAAAATGCTCAACCCTGTG TCCTGGGCCTGGGCCTGCTGTGACTGACCTACAGTGGACTTTCTCTCTGGAATGGA ACCTTCTTAGGCCTCCTGGTGCAACTTAATTTTTTTTTTTAATGCTATCTTCAAAACG TTAGAGAAAGTTCTTCAAAAGTGCAGCCCAGAGCTGCTGGGCCCACTGGCCGTCCT GCATTTCTGGTTTCCAGACCCCAATGCCTCCCATTCGGATGGATCTCTGCGTTTTTAT ACTGAGTGTGCCTAGGTTGCCCCTTATTTTTTATTTTCCCTGTTGCGTTGCTATAGAT GAAGGGTGAGGACAATCGTGTATATGTACTAGAACTTTTTTATTAAAGAAACTTTTC |
| 55755 | CDK5RAP2 | HSX2_01874332 | 98 | CCTGAGGTGCCCCTTCAGCCTGACAAACACG |
| 55755 | CDK5RAP2 | HSX2_01874383 | 99 | TTTCAGAGACTCCTCCACTCTCTGGGAATGACACGGACTCCCTCTCCTGCGACAGTG GCAGTTCGGCAACTAGCACTCCGTGTGTGTCCCGCCTGGTCACTGGCCACCACCTG TGGGCCAGCAAGAATGGCCGCCATGTCCTGGGCCTGATTGAGGACTATGAGGCCC TGCTCAAACAGATCAGCCAGGGACAGAGGCTCCTTGCTGAAATGGACATTCAAACC CAAGAGGCTCCCAGCTCCACAAGTCAAGAGCTGGGAACAAAG |
| 55755 | CDK5RAP2 | HSX2_01874417 | 100 | AAAAGAAGTTGCAAAACACCATGAAGCTTTTGCAGCTGAGCAAGCGCCAGGAAAA AGTCATCTTTGATC |
| 57214 | CEMIP | HSX2_01916997 | 101 | GCTGTTGTTCCTGAAGCTGAAAGCTCAGAACGAGAGAGAGAAGTTTGCTTTCTGCT CCATGAAAGGCTGTGAGAGGATAAAGATTAAAGCTCTGATTCCAAAGAACGCAGG CGTCAGTGACTGCACAGCCACAGCTTACCCCAAGTTCACCGAGAGGGCTGTCGTAG ACGTGCCGATGCCCAAGAAGCTCTTTGGTTCTCAGCTG |
| 8869 | CENPP | HSX2_01367269 | 102 | CAAGACATCAATGGCGCTGTTATTTGAGCACAGGTATAGCGTGGACTTACTCCCTTT TGTGCAG |
| 8869 | CENPP | HSX2_01367270 | 103 | AAGGCCCCCAAAGACAGTGAAGCTGAGTCCAAGTACGATCCTCCTTTTGGGTTCCG GAAGTTCTCCAGTAAAGTCCAGACCCTCTTGGAACTCTTGCCAGAGCACGACCTCCC TGAACACTTGAAAGCCAAGACCTGTCGGCGCTGTGTGGTTATTGGAAGCGGAGGA ATACTGCACGGATTAGAACTGGGCCACACCCTGAACCAGTTCGATGTTGTGATAAG |
| 8869 | CENPP | HSX2_01367276 | 104 | TAAGGATGACTTATCCAGAGGGCGCACCACTGTCTGACCTTGAATATTATTCCAATG ACTTATTTGTTGCTGTTTTATTTAAGAGTGTTGATTTCAACTGGCTTCAAGCAATGGT AAAAAAGGAAACCCTG |
| 8869 | CENPP | HSX2_01367281 | 105 | CCATTCTGGGTACGACTCTTCTTTTGGAAGCAGGTGGCAGAAAAAATCCCACTGCA GCCAAAACATTTCAGGATTTTGAATCCAGTTATCATCAAAGAGACTGCCTTTGACAT CCTTCAGTACTCAGAGCCTCAGTCAAGGTTCTGGGGCCGAGATAAG |
| 4435 | CITED1 | HSX2_01064533 | 106 | CTATAAAAATGTGCCCGGCGGCAAAGCTGCAGAAGGGGCACCGCTGAGCTGCCGA GAGGAAGCTCGCTCTGCCCGGCTGCCCTCTTGTAGTCCGCCGGCGAGGGGCAGTTC TCGGTGAGGAGGAAGAGAGC |
| 4435 | CITED1 | HSX2_01064535 | 107 | GCACAGCACCCGCGCGGGCCCTCC |
| 4435 | CITED1 | HSX2_01064539 | 108 | GCTCTGAAATGCCAACAACGTCGAGGCCTGCACTTGATGTCAAGGGTGGCACCTCA CCTGCGAAGGAG |
| 4435 | CITED1 | HSX2_01064543 | 109 | GATGCCAACCAAGAGATGAGCTCCGTGGCCTACTCCAACCTTGCGGTGAAAGATCG CAAAGCAGTGGCCATTCTGCA |
| 4435 | CITED1 | HSX2_01064546 | 110 | GCCAGTGGGGCTCCCACTAGTTCCTCGGGATCTCCAATAGGCTCTCCTACAACCACC CCTCCCACTAAA |
| 4435 | CITED1 | HSX2_01064547 | 111 | AAACTTAATAGCCAGTATCAGGGGATGGCTGCTGCCACTCCAGGCCAACCCGGGG AGGCAGGACCCCTGCAAAACTGGGACTTTGGGGCCCAGGCGGGAGGGGCAGAAT CACTCTCTCCTTCTGCTGGTGCCCAG |
| 4435 | CITED1 | HSX2_01064548 | 112 | GGTGGAACTGGGGTTGGACCGAGCCAATGAGCTTCCGGAGCTGTGGCTGGGGCA GAATGAGTTTGACTTCACTGCGGACTTTCCATCTAGCTGCTAATGCCAAGTGTCCCT AAAGATGGAGGAATAAAGCCACCAATTCTGTTGTAAATAAAAATAAAGTTACTTAC AA |
| 4435 | CITED1 | HSX2_01064567 | 113 | GAGTCCCTGCTTCGCAGAGCACCGGACTTGGAGTCAGAGAGACCTGGCTGCGAAT CCCGGCTCCGCCACTTTCTAGCGCTGTGACCTTGG |
| 4435 | CITED1 | HSX2_01064569 | 114 | TGCTGTTTGGATTTTTAAATTTCTCCTTCTGAGTGAGAAAATAGCAATCGAGATGGA ACCATCCG |
| 4435 | CITED1 | HSX2_01064572 | 115 | TAGCGCTGTGACCTTGGTTTTCAGGTGGCGAAAC |
| 4435 | CITED1 | HSX2_01064573 | 116 | CGAGATGGAACCATCCGCACAACAGCTCCAGCTG |
| 4435 | CITED1 | HSX2_01064574 | 117 | TAGCGCTGTGACCTTGGCACAACAGCTCCAGCTG |
| 4435 | CITED1 | HSX2_02509162 | 118 | CTATAAAAATGTGCCCGGCGGCAAAGCTGCAGAAGGGGCACCGCTGAGCTGCCGA GAGGAAGCTCGCTCTGCCCGGCTGCCCTCTTGTAGTCCGCCGGCGAGGGGCAGTTC TCGGTGAGGAGGAAGAGAGCAGCGGACGGCACAGCACCCGCGCGGGCCCTCC |
| 4435 | CITED1 | HSX2_02509166 | 119 | CAAAGCAGTGGCCATTCTGCACTACCCTGGGGTAGCCTCAAATGGAACCAAGGCCA GTGGGGCTCCCACTAGTTCCTCGGGATCTCCAATAGGCTCTCCTACAACCACCCCTC CCACTAAACCCCCATCCTTCAACCTGCACCCCGCCCCTCACTTGCTGGCTAGTATGC ACCTGCAGAAACTTAATAGCCAGTATCAGGGGATGGCTGCTGCCACTCCAGGCCAA CCCGGGGAGGCAGGACCCCTGCAAAACTGGGACTTTGGGGCCCAGGCGGGAGGG GCAGAATCACTCTCTCCTTCTGCTGGTGCCCAGAGCCCTGCTATCATCGATTCGGAC CCAGTGGATGAGGAAGTGCTGATGTCGCTGGTGGTGGAACTGGGGTTGGACCGA GCCAATGAGCTTCCGGAGCTGTGGCTGGGGCAGAATGAGTTTGACTTCACTGCGG ACTTTCCATCTAGCTGCTAATGCCAAGTGTCCCTAAAGATGGAGGAATAAAGCCAC CAATTCTGTTGTAAATAAAAATAAAGTTACTTACAA |
| 1188 | CLCNKB | HSX2_00842841 | 120 | ATCAGTGTCGCCCCCAGGCGTCCTGTTCAGCATC |
| 9076 | CLDN1 | HSX2_01381993 | 121 | GCACATTGCAAGCAACCCGTGCCTTGATGGTGGTTGGCATCCTCCTGGGAGTGATA GCAATCTTTGTGGCCACCGTTGGCATGAAGTGTATGAAGTGCTTGGAAGACGATGA GGTGCAGAAGATGAGGATGGCTGTCATTGGGGGTGCGATATTTCTTCTTGCAG |
| 9076 | CLDN1 | HSX2_01381997 | 122 | GTCTGGCTATTTTAGTTGCCACAGCATGGTATGGCAATAGAATCGTTCAAGAATTCT ATGACCCTATGACCCCAGTCAATGCCAG |
| 9076 | CLDN1 | HSX2 01382002 | 123 | GGGAGGTGCCCTACTTTGCTGTTCCTGTCCCCGAAAAACAACCTCTTACCCAACACC AAGGCCCTATCCAAAACCTGCACCTTCCAGCGGGAAAGACTACGTGTGACACAGAG GCAAAAGGAGAAAATCATGTTGAAACAAACCGAAAATGGACATTGAGATACTATC ATTAACATTAGGACCTTAGAATTTTGGGTATTGTAATCTGAAGTATGGTATTACAAA ACAAACAAACAAACAAAAAACCCATGTGTTAAAATACTCAGTGCTAAACATGGCTT AATCTTATTTTATCTTCTTTCCTCAATATAGGAGGGAAGATTTTTCCATTTGTATTAC TGCTTCCCATTGAGTAATCATACTCAACTGGGGGAAGGGGTGCTCCTTAAATATAT ATAGATATGTATATATACATGTTTTTCTATTAAAAATAGACAGTAAAATACTATTCTC ATTATGTTGATACTAGCATACTTAAAATATCTCTAAAATAGGTAAATGTATTTAATTC CATATTGATGAAGATGTT |
| 149461 | CLDN19 | HSX2_02242186 | 124 | ACACCTGTCAATGCCAGGTATGAATTTGGCCCAG |
| 53405 | CLIC5 | HSX2_01785484 | 125 | AACACCGGGAATCCAACACAGCGGGCATCGACAT |
| 79745 | CLIP4 | HSX2_02019683 | 126 | CTTTCTTTGATCCTAATGATGCATCATGCCAGGAAATTCTTTTTGATCCCAAAACTTC AGTTTCAGAATTATTTGCCATTTTGAGACAGTGGGTTCCTCAGGTCCAACAAAACAT TGACATTATTGGAAATGAG |
| 79745 | CLIP4 | HSX2_02019711 | 127 | ATTGACAGATATGACTCTTTTACATTATACCTGCAAATCTGGAGCTCATGGTAT |
| 79745 | CLIP4 | HSX2_02019770 | 128 | ACAACTTGTGTGCAGGTGCTGTGAAGTGCCTCTTGGAGCAGGGAGCAAATCCTGCA TTTAGG |
| 1271 | CNTFR | HSX2_02496357 | 129 | GCATGGCTCCAAAATTATGGTCTGTGAGAAGGACCCAGCCCTCAAGAACCGCTGCC ACATTCGCTACATGCACCTGTTCTCCACCATCAAGTACAAGGTCTCCATAAGTGTCA GCAATGCCCTGGGCCACAATGCCACAGCTATCACCTTTGACGAGTTCACCATTG |
| 8506 | CNTNAP1 | HSX2_01339423 | 130 | ACAGAGCGCTTGGGGGCGAAAGGAGAGAGGGAGGGAAGGGTGGGTAAGGAGGA GAGAGCGGTCTGCTGCAAACCCCAGGAGGAGAGCTTGGAGCCCAAGCCAGAACTC GAGCCCTAGCCGGAGCCGTTCACAGGGAGGCGGCTGCCGGGACCGTCAGCCCTGC ATGATGCATCTCCGGCTCTTCTGCATCCTGCTCGCCGCGGTCTCAGGAGCCGAGGG CTGGGGCTACT |
| 1281 | COL3A1 | HSX2_00847965 | 131 | GGTAACCCAGGACCCCCAGGTCCCAGCGGTTCTCCAGGCAAGGATGGGCCCCCAG GTCCTGCGGGTAACACTGGTGCTCCTGGCAGCCCTGGAGTGTCTGGACCAAAAGGT GATGCTGGCCAACCAGGAGAGAAGGGATCGCCTGGTGCCCAGGGCCCACCA |
| 1290 | COL5A2 | HSX2_00850255 | 132 | GCCCAAAAGGGGAACCAGGGCCACATGGTATTCA |
| 50509 | COL5A3 | HSX2_01735527 | 133 | TGCAACTTCACGGCGGGAGGAGAGACCTGCCTCTATCCCGACAAGAAGTTTGAGAT C |
| 6271 | COLGALT1 | HSX2_01205429 | 134 | CAACC |
| 6271 | COLGALT1 | HSX2_01205433 | 135 | GTGCACTGCTGCAATGGGCTCTGAGCTGGAGACGGCGATGGAGACCCTCATCAAC GTGTTCCACGCCCACTCGGGCAAAGAGGGGGACAAGTACAAGCTGAGCAAGAAGG AGCTGAAAGAGCTGCTGCAGACGGAGCTCTCTGGCTTCCTGGAT |
| 6271 | COLGALT1 | HSX2_01205437 | 136 | GGAGACGGGGAGGTGGACTTCCAGGAGTATGTGGTGCTTGTGGCTGCTCTCACAG TGGCCTGTAACAATTTCTTCTGGGAGAACAGTTGAGCAGACAGCCACATTGGGCAG CGCCCTTCCTCTCCACCCTCCCAGACCTGCCTCTTCCCCCTGCTTCCACCTCACCCCAC TTATCCCTCTCCATAACCCCACCCTTGCCCACCCCACCCCCACCCCCACCAAGGGCGC AAGAGTAGCGGTCCAAGCCTGCAACTCATCTTTCATTAAAGGCTTCTCTCTCACCAG C |
| 64478 | CSMD1 | HSX2_01977629 | 137 | TATCCAGCTCCGTATGACAACAACCTCCACTGCACCTGGATTATAGAGGCAGACCC AGGAAAGACCATTAG |
| 1465 | CSRP1 | HSX2_00864524 | 138 | TGCAAGAAGAATCTGGACAGTACCACTGTGGCCGTGCATGGTGAGGAG |
| 1474 | CST6 | HSX2_00864973 | 139 | GAGGGCTCCGACGGCACTGACGGCCATGGCGCGTTCGAACCTCCCGCTGGCGCTG GGCCTGGCCCTGGTCGCATTCTGCCTCCTGGCGCTGCCACGCGACGCCCGGGCCCG GCCGCAGGAGCGCATGGTCGGAGAACTCCGGGACCTGTCGCCCGACGACCCGCAG GTGCAGAAGGCGGCGCAGGCGGCCGTGGCCAGCTACAACATGGGCAGCAACAGC ATCTACTACTTCCGAGACACGCACATCATCAAGGCGCAGAGCCAG |
| 1499 | CTNNB1 | HSX2_00866738 | 140 | CTGATTTGATGGAGTTGGACATGGCCATGGAACCAGACAGAAAAGCGGCTGTTAG TCACTGGCAGCAACAGTCTTACCTGGACTCTGGAATCCATTCTGGTGCCACTACCAC AGCTCCTTCTCTGAGTGGTAAAGGCAATCCTGAGGAAGAGGATGTGGATACCTCCC AAGTCCTGTATGAGTGGGAACAGGGATTTTCTCAGTCCTTCA |
| 1499 | CTNNB1 | HSX2_00866791 | 141 | CGCCTTCACTATGGACTACCAGTTGTGGTTAAGCTCTTACACCCACCATCCCACTGG CCTCTGATAAAG |
| 1499 | CTNNB1 | HSX2_00866821 | 142 | GGATG |
| 1499 | CTNNB1 | HSX2_02497461 | 143 | CTCATCATACTGGCTAGTGGTGGACCCCAAGCTTTAGTAAATATAATGAGGACCTAT ACTTACGAAAAACTACTGTGGACCACAAGCAGAGTGCTGAAGGTGCTATCTGTCTG CTCTAGTAATAAGCCGGCTATTGTAGAAGCTG |
| 1508 | CTSB | HSX2_00867649 | 144 | AGAATCACGTCCTGGAACTGCATGTTCTTGCGACTCTTGGGACTTCATCTTAACTTC TCGCTGCCCCAGCCATGTTTTCAACCATGGCATCCCTCCCCCAATTAGTTCCCTGT |
| 7050 | D430041D05RIK | HSX2_02521755 | 145 | GGATGGCAAAGATCCAAATCAGTTCACAATTTCCCGCCGTGGGGCCAAGATTTCTG AAACGAGCTCTGTGGAGTCCGTGATGGGCATCAAAAACTTCATGCCAGCTCTAGAG GAGACCCCATTTCATTCCTGTACAGCTGGGCCAAACCCAACCCTAGGGAGGCCACT GTCTCCTAAGCCGTCATCCCCGGGATCAGTTTTGGCTCGTCCATCAGTGATCTGCCA TACCACTGTGACTGCATTGAAAGATGTCCCTTTCTCTCTCTGCCAGTCGGTCGGTGT GGGACAAAACACAGATATACAGCAGATAGCGGCCAAAAACTTCACAGACACCTCTC TCATGTACCCAGAGGACACTTGTAAATCTGGACCAAGTACGAATACACAGAGTGGT CTTTTCAACACTCCTCCCCCTACTCCACCGGACCTCAACCAGGACTTCAGTGGATTTC AGCTTCTAGTGGATGTTGCACTCAAACGGGCTGCAGAGATGGAGCTTCAGGCAAA ACTTACAGCTTAACCCATTTTCAAGCAAAACAGTTCTCAGAAATGTCATGATTGCCG GGGTGAAGGCAAGAGATGAATTGCATTATTTATATATTTTTTATTAATATTTGCAC ATGGGATTGCTAAAACAGCTTCCTGTTACTGAGATGTCTTCAATGGAATACAGTCAT TCCAAGAACTATAAACTTAAAGCTACTGTAGAAACAAAGGGTTTTCTTTTTTAAATG TTTCTTGGTAGATTATTCATAATGTGAGATGGTTCCCAATATCATGTGATTTTTTTTT CCTCCCCTTCCCTTTTTTTGTTATTTTTTCAGACTGTGCAATACTTAGAGAACCTATA GCATCTTCTCATTCCCATGTGGAACAGGATGCCCACATACTGTCTAATTAATAAATT |
| 29087 | D9MIT260 | HSX2_01718539 | 146 | ATCTCCGTTGATTCCAGAATCGTCCGCACTAAAG |
| 153090 | DAB2IP | HSX2_02252381 | 147 | GTGAGTTCATCAAAGCGCTGTATGAGTCAGATGAGAACTGCGAAGTGGATCCCAG CAAGTGCTCGGCCGCTGACCTCCCAGAGCACCAGGGCAACCTCAAGATGTGCTGCG AGCTGGCCTTCTGCAAGATCATCAACTCCTACTG |
| 153090 | DAB2IP | HSX2_02252398 | 148 | AGTCGCGGCCGCCCGGACATCAGTGAGCGGCTCATCAGCGCCTCCCTCTTCCTGCG CTTCCTCTGCCCAGCCATCATGTCGCCCTCACTCTTCAACCTGCTGCAGGAGTACCCT GATGACCGCACTGCCCGCACCCTCACCCTCATCGCCAAGGTCACCCAGAACCTGGC CAACTTTGCCAA |
| 153090 | DAB2IP | HSX2_02252492 | 149 | GAGAAGCGCATTGCCTCGTTGGATGCCGCCAATGCCCGCCTCATGAGTGCCCTGAC CCAGCTGAAAGAGAG |
| 1611 | DAP | HSX2_00873858 | 150 | TGAAAGCTGGTGGAATGCGAATTGTGCAGAAACACCCACATACAG |
| 1611 | DAP | HSX2_00873859 | 151 | GAGACACCAAAGAAGAGAAAGACAAGGATGACCAGGAATGGGAAAGCCCCAG |
| 1717 | DHCR7 | HSX2_00880610 | 152 | GTGGACTGGTTTTCACTGGCGAGCGTCATCTTCCTACTGCTGTTCGCCCCCTT |
| 9249 | DHRS3 | HSX2_01394249 | 153 | CGTGCTGGCACTGTCTGCCATCCCCGGTGCCATCGACTACTGCACATCCAAAGCGTC AGCCTTCGCCTTCATGGAGAGCCTGACCCTGGGGCTGCTGGACTGTCCGGGAGTCA GCGCCACCACAGTGCTGCCCTTCCACACCAGCACCGAGATGTTCCAGGGCATGAGA GTCAG |
| 9249 | DHRS3 | HSX2_02529585 | 154 | CCTCAACTCCGTGCTGGCACTGTCTGCCATCCCCGGTGCCATCGACTACTGCACATC CAAAGCGTCAGCCTTCGCCTTCATGGAGAGCCTGACCCTGGGGCTGCTGGACTGTC CGGGAGTCAGCGCCACCACAGTGCTGCCCTTCCACACCAGCACCGAGATGTTCCAG GGCATGAGAGTCAG |
| 1730 | DIAPH2 | HSX2_00881579 | 155 | TCTAGATAAACTTTTAGGGGCTATAACAACAGCAGCAGAAAGAAATAACAGGGAA CGATTTTCACCAATTGTGGAAGGTTTAGAAAATCAGGAAGCCTTGCAATTACAG |
| 1756 | DMD | HSX2_00884014 | 156 | GTGAGGAAGATCTTCTCAGTCCTCCCCAGGACACAAGCACAGGGTTAGAGGAGGT GATGGAGCAACTCAACAACTCCTTCCCTAGTTCAAGAG |
| 56171 | DNAH7 | HSX2_01892342 | 157 | CACAAAAGCTACATCGAATACACAAAGACTCTGCCACTGACCCCAGCACCAGAAAT CTTTGGGATGAATGCCAATGCAGATATCACTAAGGATCAGTCAGAAACTCAGCTGC TATTTGATAACATTCTTCTTACACAG |
| 23348 | DOCK9 | HSX2_01626871 | 158 | CTACAGGCAAGACAGCAATAAGCTATCCAATGATGACATGCTCAAGTTACTTGCAG ACTTTCGGAA |
| 23348 | DOCK9 | HSX2_01627334 | 159 | ATGTCCATTTCAACGAGGATGTGCTGATGGAGCT |
| 23348 | DOCK9 | HSX2_01627375 | 160 | GTCAATGCTGGCCCACTAGCATATGCGCGAGCTTTCTTAGATGATACAAACACAAA GCGATATCCTGACAATAAAGTGAAGCTGCTTAAGGAAGTTTTCAG |
| 23348 | DOCK9 | HSX2_01627409 | 161 | AACGCCATCAGTGGGACTCCAACAAGCACAATGG |
| 54344 | DPM3 | HSX2_02553775 | 162 | AGTTCGGACCTCTGGGATCGGCGATTCCCCTCTGGCCAGGGCTGGTTTTAATGCTC TCCGTGGGCGGGCTTCGGTTGAGTTTGGTCCGCTTTTCCTTTCTGCTCCTCAGGGGA GCATTGCTTCCTTCTCTCGCA |
| 1809 | DPYSL3 | HSX2_00890973 | 163 | TCTCTACGTCACAAAGGTCATGAGCAAGAGTGCAGCTGACCTCATCTCACAAGCCA GGAAAAAAG |
| 1837 | DTNA | HSX2_00892824 | 164 | AGGATGAGATCATAGTTTCAGGATCCTCAGGAAACCCTGGTACTGGCAGCAGCCAG CCTCTGCTGTGCCCACATGACCCACAACTCTGGC |
| 23220 | DTX4 | HSX2_01610552 | 165 | GAATCACTGGGATCCTCATGAGTGCAGCGGGGCTGCCTGTGTGTCTCACCAGGCCA CCAAAGCTGGTCCTACACCCACCCCCCGTCAGCAAGAGTGAAATAAAATCCATCCC AGGGGTTTCCAACACAAGCCGCAAGACCACCAAAAAACAAGCCAAGAAAG |
| 53905 | DUOX1 | HSX2_01787484 | 166 | GGTGCGGGAGGCCCTGACCTGTGAGCTGAGCAGGGCCGAGTTTGCCGAGTCCCTG GGCCTCAAGCCCCAGGACATGTTTGTGGAGTCCATGTTCTCTCTGGCTGACAAGGA TGGCAATGGCTACCTGTCCTTCCGAGAGTTCCTGGACATCCTGGTGGTCTTCATGAA AG |
| 1846 | DUSP4 | HSX2_00893757 | 167 | AAAAAAAGAAAAGAAAAAAAAAGACAGCATCTCACTATGTTGCCAAGGCTCATCTC AAGCTCTTGGGCTCAAGAGATCCTCCCACCTCGGCCTCCTGAGTAGCTGGGACTGC AGGTGTGTGTCATCATGACCAATGTGAATTGCTTTTGAAGCTGGTTCATGGGCATG TAGGCCACCGAAGCAATTTTAGACCACAGTAAGTCAAGCTTTTTCCCTCCGATGAT CACTGGGTGGTTGCAGCATTTTTTGCATAAACCTGCCTAAGACTTGTCTATCGTCTG TGATCAATATGCCATATTACACTAAGGTGCTCCTGGAAAATTGGGTGCAGTTCAAAT TTTCCTACAGCAAATCATTTGGCAAGGCCAGCCATTGGGGAAACCAGACAACTAGA GATAACCCTGAAATGAATCCTTTTGTAAATTGAAGCACCATCTTTTCTTTTTTTGCAT AAATTGGAGGTTTTAATTTTAGGGCAGTTACCTGAAGTGAAATATACCAACAATTTC TTGTGTTCTTTAAATTCCTAGTTAGGTGAATATTTTTGAAGGTCCTCTTTTGAATAAA GAGGGGAATGGACACCACATTTCAGGTCTTCTCGAAGTGTGGAAGGGCAAGAGAG CATCAGTGAGCTGATGGTGGATTGCTTACATCGGATTCCATTGGTATGAATTTCCCA AACTGGAAATCAAAGCGCCAGGGTGGGGTTGGGGCTGACTGCTGGTGAGGGGGC TGGCCGCTGGCTCCCGTGACGTGCGTCATGGGCACGCAGGCGCCATTTTGAATCTA TCGTCGGCACGTGGGTGCCATTTTGAATCCTTAGTTGGGCCTTTCTAAATGGAGAA TGGCTTTGGAGGGAGACACGTTTTCTGTGGGGAGGGTTTGGGGGGGAGGGAGGA GGGAACAAGCTACATGCTATTTTGTTTGTAGTATTGTGGAACAGTCTTGTTATGGA GTGCCAGCTTAGAGGTTGTTGCAAACTTGTCTAGAAGTGAGAGCATGGTTTTTTTT AGCCCTTTGAGAGTCTACATCTAATGAACATTCTTGCTCACCCATAAATAACGTCAA GCCTCAATGTCACCGTCACGTTGGGATACTCTTTCTCATCTGGCATCCTAGACAGGA CAAGGTTGGTTACCTTTCCTTCCATGAACCATGAACCTGTGACGGCATCATTCATCC TGACTTCACCAAGCTCCGCCTGTGGGTGAGGCCAGAGCTCCCACTGGCAATTTTTA GAAGAGCCAGAGGCTCCCTGCTTCCTCTAGAAATAACAGTTCAGGGTGAAGCATGG AGGGTTTCAGTTCCCAGACAATGGAACCATTTAGAGACAACACAGTTGGACATTTC CACTTTTTCCTTGATTCCTGGAAGTCCAGTGGGTTCTGCAGCTGAAAAAGCCCTGGG TCCCAGCAGCAGAGAGACAGGACAGAGGGGATGCTTGGGCGGGGAGGGACGGTA ACCTGCAGAACAGATTCCATTTTTATAGAACGAGTACACGTTTGCTAAAACAGTCCT GCTTTCCCAGACTGGATTCCCACCACAGGGACAGTCGGAACTCAGGACTAGCTCCA GCGACATCTTTCCTCCGAATTCAAGCCTTCTATCACAATGTCAAAACAGCTATTTATA AAGCCATTTTCATTGTACTTGATAACAGCACGAGTCCCAAAACTTTTAGAAATAAAA TAGGACATTGGCTTGATTGAAAAGAGGGACTTTTTAAAAATTGTTCTTTCGTCAGAA GCCTTTTGGATGACTTACAATAGCTCTGATGAAGATACCACCCCAGCGTCAGTCCAA TAGGTCAGTGAGTTTCAACAGGCATCCATCCCTCCCATGAAGGGATTCTGGTGATG GGAAGTTTCTGTAATGACAGGAAAGCATTGACCCTCATTGATTGTCAACTTTGGTAT TAGCCATGAAAGACAGGATGCTCATTGGGTGTTCTGTAGAGTGAGGAATGCTGCCT |
| 1877 | E4F1 | HSX2_00894999 | 168 | GCACCAG |
| 1889 | ECE1 | HSX2_00895217 | 169 | ACCCCAACGGCCTGCAGGTGAACTTCCACAGCCC |
| 1889 | ECE1 | HSX2_00895219 | 170 | GATCCCCCTCTGTGTGCCTGAGCGAAGCTTGTGTCTCAGTGACCAGCTCCATCTTGA GCTCCATGGACCCCACAGTGGACCCCTGCCATGACTTCTTCAGCTACGCCTGTGGG GGCTGGATCAAGGCCAACCCAGTCCCTGATGGCCACTCACGCTGGGGGACCTTCAG CAACCTCTGGGAACACAACCAAGCAATCATCAAGCACCTCCTCG |
| 1889 | ECE1 | HSX2_00895231 | 171 | CTCGGGGGCTGGAACATCACAGGTCCCTGGGCCAAGGACAACTTCCAGGACACCCT GCAGGTGGTCACCGCCCACTACCGCACCTCACCCTTCTTCTCTGTCTATGTCAGTGC CGATTCCAAGAACTCCAACAGCAACGTGATCCAG |
| 1889 | ECE1 | HSX2_00895252 | 172 | ACAACTACATGATCTGGAACCTGGTGCGGAAAACAAGCTCCTTCCTTGACCAGCGC TTTCAGGACGCCGATGAGAAGTTCATGGAAGTCATGTACGGGACCAAGAAG |
| 1889 | ECE1 | HSX2_00895278 | 173 | GTGGAGCATGACCCCGCCCATGGTGAACGCCTACTACTCGCCCACCAAGAATGAGA TTGTGTTTCCGGCCGGGATCCTGCAGGCACCATTCTACACACGCTCCTCACCCAA |
| 1889 | ECE1 | HSX2_00895288 | 174 | GACGGGAGTATGACAAGGACGGGAACCTCCGGCCATGGTGGAAGAACTCATCCGT GGAGGCCTTCAAGCGTCAGACCGAGTGCATGGTAGAGCAGTACAGCAACTACAGC GTGAACGGGGAGCCGGTGAACGGGCGGCACACCCTGGGGGAGAACATCGCCGAC AACGGGGGTCTCAAGGCGGCCTATCGG |
| 1893 | ECM1 | HSX2_00895741 | 175 | TGGGAGGATACCCTTGACAAATACTGTGACCGGGAGTATGCTGTGAAGACCCACCA CCACTTGTGTTGCCGCCACCCTCCCAGCCCTACTCGGGATGAGTGCTTTGCCCGTCG GGCTCCTTACCCCAACTATGACCGGGACATCTTGACCATTGACATCGGTCGAGTCAC CCCCAACCTCATGGGCCACCTCTGTGGAAACCAAAGAGTTCTCACCAAGCA |
| 2202 | EFEMP1 | HSX2_00921723 | 176 | ACAGCCCAGATTATTGTCAATAATGAACAGCCTCAG |
| 1958 | EGR1 | HSX2_00900683 | 177 | CCCA |
| 1958 | EGR1 | HSX2_00900684 | 178 | GCCAAACCACTCGACTGCCCCCCATCACCTATACTGGCCGCTTTTCCCTGGAGCCTG CACCCAACA |
| 1958 | EGR1 | HSX2_00900686 | 179 | GCCTCCGCCTCCCAGAGCCCACCCCTGAGCTGCGCAGTGCCATCCAACGACAGCAG TCCCATTTACTCAG |
| 1958 | EGR1 | HSX2_00900687 | 180 | CGGCACCCACCTTCCCCACGCCGAACACTGACATTTTC |
| 1958 | EGR1 | HSX2_00900688 | 181 | GCCTCCTGCCTACCCTGCCGCCAAGGGTGGCTTCCAGGTTCCCATGATCCCCGACTA CCTGTTTCCACAGCAGCAGGGGGATCTGGGCCTGGGCACCCCAGACCAGAAGCCC TTCCAGGGCCTGGAGAGCCGCACCCAGCAGCCTTCGCTAACCCCTCTGTCTACTATT AAGGCCTTTGCCACTCAG |
| 1958 | EGR1 | HSX2_00900689 | 182 | ACCCAGCCGCATGCGCAAGTACCCCAACCGGCCCAGCAAGACGCCCCCCCACGAAC GCCCTTACGCTTGCCCAGTGGAGTCCTGTGATCGCCGCTTCTCCCGCTCCGACGAGC TCACCCGCCACATCCGCATCCACACAGGCCAGAAGCCCTTCCAGTGCCGCATCTGCA TGCGCAACTTCAGCCGCAGCGACCACCTCACCACCCACATCCGCACCCACACAGGC GAAAAGCCCTTCGCCTGCGACATCTGTGGAAGAAAGTTTGCCAGGAGCGATGAAC GCAAGAGGCATACCAAGATCCACTTGCGGCAGAAGGACAAGAAAGCAGACAAAAG TGTTGTGGCCTCTTCGGCCACCTCCTCTCTCTCTTCCTACCCGTCCCCGGTTGCTACC TCTTACCCGTCCCCGGTTACTACCTCTTATCCATCCCCGGCCACCACCTCATACCCAT CCCCTGTGCCCACCTCCTTCTCCTCTCCCGGCTCCTCGACCTACCCATCCCCTGTGCA |
| 1958 | EGR1 | HSX2_00900692 | 183 | TGTGACAATAAGTTTGAACCTTTTTTTTGAAACAGCAGTCCCAGTATTCTCAGAGC ATGTGTCAGAGTGTTGTTCCGTTAACCTTTTGTAAATACTGCTTGACCGTACTCTCA CATGTGGCAAAATATGGTTTGGTTTTCTTTTTTTTTTTTTTGAAAGTGTTTTTCTT CGTCCTTTTGGTTTAAAAAGTTTCACGTCTTGGTGCCTTTTGTGTGATGCGCCTTGCT GATGGCTTGACATGTGCAATTGTGAGGGACATGCTCACCTCTAGCCTTAAGGGGGG CAGGGAGTGATGATTTGGGGGAGGCTTTGGGAGCAAAATAAGGAAGAGGGCTGA GCTGAGCTTCGGTTCTCCAGAATGTAAGAAAACAAAATCTAAAACAAAATCTGAAC TCTCAAAAGTCTATTTTTTTAACTGAAAATGTAAATTTATAAATATATTCAGGAGTTG GAATGTTGTAGTTACCTACTGAGTAGGCGGCGATTTTTGTATGTTATGAACATGCA GTTCATTATTTTGTGGTTCTATTTTACTTTGTACTTGTGTTTGCTTAAACAAAGTGAC TGTTTGGCTTATAAACACATTGAATGCGCTTTATTGCCCATGGGATATGTGGTGTAT ATCCTTCCAAAAAATTAAAACGAAAATAAAGTAGCTGCGATTGGG |
| 1967 | EIF2B1 | HSX2_00901053 | 184 | ATGCTGCT |
| 55740 | ENAH | HSX2_01871605 | 185 | AGAAGTGGGTGCCAGCTGGTGGCTCAACTGGATTCAGCAGAGTTCATATCTATCAC CATACAGGCAAC |
| 55740 | ENAH | HSX2_01871707 | 186 | AGATAGGACTTTAATCTGGAGGAAAAATATCCTACAAACAACAACTGTTCACAACA GCAAACCCCTACATTTATGAGCTGTAAGAAGAAAATGGAGACAAACAGAAGGAGG GAAAAACCAACCTACTCTGAAAGCCTTCAGACATTATGACTCTGGTGATAAGCTCTT TCCCTCTCCGTTTGCTGCTTTTTTCTGGCCTTTACAACAGAATGGAAGAGAATCATTT AAGAGTTCCTGTAACAGTTATGCAGAAAATACTAAAACCCATCAGGCAAGATCACC ACGCATTGAAATATTTTCATATCAAGATAAAGTCGCACATTTTCCACAATACATTGC TAAAATAAAGAGGAGAAAGGCTTAGGAAGTTTTTTTGCAGAGAGTGCTGGTAAAG AATTGAGCAAGTTTGCTATTGTATTGTAATGTTTCTCTCAGGTTTGTTCTTCCTATCA TGTTTGATATTCCATGAATAATTGAGATCAGCCCTATGTAAGTTAAGATCATAATAT GTGGAACAAATGGAATTGTAAGTGCTTTCAAAGGGTAATATTTATAAGAAAGTGTC CGAAAAATGTTTCTTCAGCTTGAGAAATTTTAGAATGATAGGAAGTTTCTCGAGTTA GCCTTCATGCAATTTTGTAGATTAAAACATAAAATTTGTCCAGAACTTAA |
| 55740 | ENAH | HSX2_01871807 | 187 | TATGATTCATTACACAGACCAAAATCCACACCCT |
| 55740 | ENAH | HSX2_01871851 | 188 | TCACCTGTTATCTCCAGACCAAAATCCACACCCT |
| 23052 | ENDOD1 | HSX2_01590519 | 189 | CCTCTGTGAACAGCCTGGGAAGCAAGCAAGCCTTGAATACAGATTACCTTGATTCT GATTACCAAAGAGGACAGCTTTACCCATTCTCCCTTAGCAGTGATGTCCAGGTGGC CACATTTACTCTCACAAATTCAGCCCCAATGACTCAGTCCTTCCAGGAACGGTGGTA TGTGAATCTCCACAGCCTAATGGACCGGGCTTTGACCCCACAGTGTGGCAGTGGGG AAGACCTATATATCCTCACAGGCACAGTGCCCTCAGACTACAGAGTTAAAGACAAA GTGGCAGTCCCTGAGTTTGTTTGGCTGGCAGCCTGTTGTGCTGTCCCTGGAGGAGG CTGGGCCATGGGCTTTGTCAAGCACACCCGGGACAGTGACATCATAGAAGATGTG ATGGTAAAAGATCTTCAGAAACTGCTTCCATTTAACCCTCAGCTGTTTCAGAACAAC TGTGGTGAAACTGAGCAAGACACAGAGAAAATGAAAAAAATCCTGGAAGTGGTTA ACCAAATCCAGGATGAAGAACGAATGGTACAATCTCAAAAGAGTTCTAGTCCCCTT TCTAGCACCAGGAGCAAGAGGTCTACTCTGTTGCCTCCAGAGGCATCTGAGGGAA GTAGTAGCTTTTTGGGAAAACTCATGGGCTTCATTGCTACCCCATTCATCAAGCTTT TTCAATTAATTTATTACCTTGTGGTAGCAATCCTGAAGAACATTGTCTATTTCCTGTG GTGTGTTACCAAGCAGGTGATTAATGGCATAGAAAGTTGCCTTTACCGCCTGGGCT CAGCCACCATCTCATACTTCATGGCCATTGGGGAAGAGTTGGTGAGCATTCCCTGG AAGGTGCTCAAGGTCGTGGCCAAAGTCATCAGGGCTCTCCTCCGGATCCTTTGTTG TCTGCTGAAGGCCATTTGCCGAGTTCTGAGCATCCCTGTCCGTGTCCTTGTGGATGT GGCCACTTTCCCTGTGTACACCATGGGCGCTATTCCAATTGTTTGCAAGGACATTGC ACTGGGCCTTGGTGGCACTGTCTCACTGCTCTTTGACACTGCTTTTGGTACCCTGGG TGGCCTATTTCAGGTGGTTTTTAGTGTCTGCAAGCGGATTGGCTACAAGGTTACTTT TGACAATTCTGGGGAGTTATAAACTCAAAAAACTAATAGTATCCAGTCACAGTGAA TTTGAAAGCTGGAATAGTTTGTCTTTACAATGGGTTTCTGTTCACTGTCAGTTATCA TTATATTTTGGCCTTTGGTGGGGATGTCTGCTTGTTTTTGCAAAAGAAGATGGCAG AATTTAGACTTGACAGAGGAGAAATGCTCAGGGTGAGATTAGGTGTAGTAATCTG CTGTTTACCTCCAGTTATATGTGCAAACTCCCAAGCCACTAATAACTTCAGTTATGCA CTCTAACACAGACGACCACCTGAAATGCACTGGTATTTATTTCTGATAATTAAAAAT |
| 953 | ENTPD1 | HSX2_00825916 | 190 | TTTGCTCCAAGAATATCCTAGCCATCCTTGGCTTCTCCTCTATCATAGCTGTGATAGC TTTGCTTGCTGTGGGGTTGACCCAGAACAAAGCATTGCCAGAAAACGTTAAG |
| 953 | ENTPD1 | HSX2_00825924 | 191 | TGCCAGAAAACGTTAAGTATGGGATTGTGCTGGA |
| 953 | ENTPD1 | HSX2_00825926 | 192 | GTCCTGGAATCTCAAAATTTGTTCAGAAAGTAAATGAAATAGGCATTTACCTGACTG ATTGCATGGAAAGAGCTAGGGAAGTGATTCCAAGGTCCCAGCACCAAGAGACACC CGTTTACCTGGGAGCCACGGCAGGCATGCGGTTG |
| 953 | ENTPD1 | HSX2_00825931 | 193 | AGAATGCAGGGTTAAAGGTCCTGGAATCTCAAAA |
| 953 | ENTPD1 | HSX2_00825934 | 194 | GGTGCCAGGATCATTACTGGCCAAGAGGAAGGTGCCTATGGCTGGATTACTATCAA CTATCTGCTGGGCAAATTCAGTCAG |
| 953 | ENTPD1 | HSX2_00825945 | 195 | AGTGAACGTAAGTGACCTTTACAAGACCCCCTGCACCAAGAGATTTGAGATGACTC TTCCATTCCAG |
| 953 | ENTPD1 | HSX2_00825947 | 196 | TTTGAAATCCAGGGTATTGGAAACTATCAACAATGCCATCAAAGCATCCTGGAGCT CTTCAACACCAGTTACTGCCCTTACTCCCAGTGTGCCTTCAATGGGATTTTCTTGCCA CCACTCCAGGGGGATTTTGGG |
| 953 | ENTPD1 | HSX2_00825952 | 197 | TGGCCAAGGACATTCAGGTTGCAAGTAATGAAAT |
| 953 | ENTPD1 | HSX2_00825954 | 198 | CTCAGGAAAAGGTGACTGAGATGATGAAAAAGTTCTGTGCTCAGCCTTGGGAGGA G |
| 953 | ENTPD1 | HSX2_00825959 | 199 | ATAAAAACATCTTACGCTGGAGTAAAGGAGAAGTACCTGAGTGAATACTGCTTTTC TGGTACCTACATTCTCTCCCTCCTTCTGCAAGGCTATCATTTCACAGCTGATTCCTGG GAGCACATCCATTTCATTGGCAAG |
| 953 | ENTPD1 | HSX2_00825964 | 200 | GATCCCAGCTGAGCAACCATTGTCCACACCTCTCTCCCACTCCACCTATGTCTTCCTC ATGGTTCTATTCTCCCTGGTCCTTTTCACAGTGGCCATCATAGGCTTGCTTATCTTTC ACAAGCCTTCATATTTCTGGAAAGATATGGTATAGCAAAAGCAGCTGAAATATGCT GGCTGGAGTGAGGAAAAAAATCGTCCAGGGAGCATTTTCCTCCATCGCAGTGTTCA AGGCCATCCTTCCCTGTCTGCCAGGGCCAGTCTTGACGAGTGTGAAGCTTCCTTGG CTTTTACTGAAGCCTTTCTTTTGGAGGTATTCAATATCCTTTGCCTCAAGGACTTCGG CAGATACTGTCTCTTTCATGAGTTTTTCCCAGCTACACCTTTCTCCTTTGTACTTTGTG CTTGTATAGGTTTTAAAGACCTGACACCTTTCATAATCTTTGCTTTATAAAAGAACAA |
| 953 | ENTPD1 | HSX2_00825979 | 201 | TATGGAAGATACAAAGGAGTCTAACGTGAAGACA |
| 953 | ENTPD1 | HSX2_02495027 | 202 | CTATCATAGCTGTGATAGCTTTGCTTGCTGTGGGGTTGACCCAGAACAAAGCATTG CCAGAAAACGTTAAG |
| 953 | ENTPD1 | HSX2_02495030 | 203 | GTCCTGGAATCTCAAAATTTGTTCAGAAAGTAAATGAAATAGGCATTTACCTGACTG ATTGCATGGAAAGAGCTAGGGAAGTGATTCCAAGGTCCCAGCACCAAGAGACACC CGTTTACCTGGGAGCCACGGCAGGCATGCGGTTGCTCAG |
| 953 | ENTPD1 | HSX2_02495034 | 204 | CAGTTTGAAATCCAGGGTATTGGAAACTATCAACAATGCCATCAAAGCATCCTGGA GCTCTTCAACACCAGTTACTGCCCTTACTCCCAGTGTGCCTTCAATGGGATTTTCTTG CCACCACTCCAGGGGGATTTTGGG |
| 954 | ENTPD2 | HSX2_00826105 | 205 | TGATCCCCGCCGACCCGCCGGGGCTGCGCAAGGGCACAGACTTCAGCTCCTGGGTC GTCCTCCTGCTGCTCTTCGCCTCCGCGCTCCTGGCTGCGCTTGTCCTGCTGCTGCGT CAGGTGCACTCCGCCAAGCTGCCAAGCACCATTTAGGGGCCGACGGGGGCAGCTG CCCCATCCCTCCCCCAACCCCTGTATCCCCACCCCGTACTCCCACCCCTCCCACAACC CCTGTACCTCCCACCCCTGTATCCCCACCCCTCCACCCACCCCTCTCCCAACCTCTCTC CCCGCCCCTGTATCCTGCATTCCTCCACCCACCCTCTATCCCCCACCGCTCCACCCCA CCACTGTCTTCTCCATCCTTCCACCCCACCCTCAGCGTCTCTGCCCCTAAGGCAGCCC AGGAAATAGGAACTGAGACTCTGGTACCCACAGGAGCCTGGGTGGGCAAAGAGC GCTCAATCCAGCTCCTTGAACCCCTCCAGCCCGCTTCAGCCTGGGCATCACTGCAGG CCCCGTGCTCCTCCTCCTCCTCAGGGCTGGGTCTCCAGAGAGTGGGGCCTTGG TCCTGAGAATCAGCCCTTAGAGGCTCCTTCTGTGTAGTCTGGGTCTGTACTGGGGA GGGTCACAGCCCACGGGCTGGCAGCCAGCCCAGCACCTACTTGTAAAAATTTTGTA |
| 956 | ENTPD3 | HSX2_00826288 | 206 | TGGACTTAGGTGGTGCCTCCACCCAAATATCCTTCGTGGCAGGAGAGAAGATGGAT CTGAACACCAGCGACATCATGCAGGTGTCCCTGTATGGCTACGTATACACGCTCTAC ACACACAGCTTCCAGTGCTATGGCCGGAATGAGGCTGAGAAGAAGTTTCTGGCAAT GCTCCTGCAG |
| 956 | ENTPD3 | HSX2_00826298 | 207 | GCTTTTGCAGGATTCTACTACACAGCCAGTGCTTTAAATCTTTCAGGTAGCTTTTCCC TGGACACCTTCAACTCCAGCACCTGGAATTTCTGCTCACAGAATTGGAGTCAG |
| 79852 | EPHX3 | HSX2_02027236 | 208 | AGCTCGGGCCTGCGTCTGCACTATGTCTCGGCTGGACGAGGTAACGGACCCCTCAT GCTGTTTCTGCACGGCTTCCCTGAGAACTG |
| 2059 | EPS8 | HSX2_00910337 | 209 | TGGCTACGGATCATCACCTACCTTTTCCCAGACGGACAGAGAACATGGTTCAAAAA CAAGTGCAAAGGCCCTTTATG |
| 2059 | EPS8 | HSX2_00910352 | 210 | TTGACTACCTTTGTCCTGGATCGGAAAGATGCTATGATCACTGTTGATGATGGAATA AGGAAATTGAAATTGCTTGATGCCAAGGGCAAAGTGTGGACTCAAGATATGATTCT TCAAGTGGATGACAGAGCTGTGAGCCTGATTGATTTAGAATCAAAG |
| 2059 | EPS8 | HSX2_00910358 | 211 | AATGAACTGGAGAATTTTCCTTTAAACACAATCCAGCACTGCCAAGCTGTGATGCAT TCATGCAGCTATGATTCAGTTCTTGCACTGGTGTGCAAAGAGCCAACCCAGAACAA GCCAGATCTTCATCTCTTCCAGTGTGATGAGGTTAAG |
| 2059 | EPS8 | HSX2_00910368 | 212 | GATGATTTCCAATGCAGACCCTAGTATACCGCCTCCACCCAGAGCTCCTGCCCCTGC GCCCCCTGGGACCGTCACCCAGGTGGATGTTAGAAGTCGAGTGGCAGCCTGGTCT GCATGGGCAGCCGACCAAGGGGACT |
| 2059 | EPS8 | HSX2_00910373 | 213 | TTGAGAAACCAAGGCAGTATCATGAGCAGGAAGAAACACCTGAGATGATGGCAGC CCGCATTGACAGAGATGTG |
| 2059 | EPS8 | HSX2_00910388 | 214 | GCCAAACTGAAGTCTCATATTCAGAATCCTAGTGCTGCAGATTTGGTTCACTTTTTG TTTACTCCATTAAATATG |
| 2059 | EPS8 | HSX2_00910393 | 215 | GTGGTGCAGGCAACAGGAGGTCCTGAACTAGCCAGTTCAGTACTTAGTCCCCTATT GAATAAGGACACAATTGATTTCTTAAATTATACTGTCAATGGTGATGAACGGCAGC TGTGGATGTCATTGGGAGGAACTTGGATGAAAGCCAG |
| 2059 | EPS8 | HSX2_00910398 | 216 | GCTGGGAGCCCCCAATGCTGAACTTTATGGGAGCCACAATGGAACAAGATCTTTAT CAACTGGCAGAATCTGTGGCAAATGTAGCAGAACATCAGCGCAAACAGGAAATAA AAAGATTATCCACAGAG |
| 2059 | EPS8 | HSX2_00910403 | 217 | CATTCCAGTGTATCAGAGTATCATCCAGCCGATGGCTATGCGTTCAGTAGCAACATT TACACAAGAGGATCCCACCTGGACCAAGGGGAAGCTGCTGTTGCTTTTAAGCCAAC TTCTAATCGCCATATAGATAG |
| 2059 | EPS8 | HSX2_00910408 | 218 | AAATTATGAACCACTCAAAACACAACCCAAGAAATATGCCAAATCCAAGTATGACTT TGTAGCAAGGAACAACAGTGAGCTCTCGGTTCTAAAGGATGATATTTTAGAG |
| 2059 | EPS8 | HSX2_00910413 | 219 | ATACTTGATGATCGGAAGCAATGGTGGAAAGTTCGAAATGCAAGTGGAGACTCTG GATTTGTGCCAAATAACATTTTGGATATTGTGAGACCTCCAGAATCTGGATTGGGG CGTGCTGATCCACCTTATACTCATACTATACAG |
| 2059 | EPS8 | HSX2_00910418 | 220 | AAACAAAGGATGGAGTATGGCCCAAGACCAGCTGATACTCCCCCTGCTCCATCACC TCCTCCAACACCAGCTCCTGTTCCTGTTCCCCTTCCCCCTTCCACTCCAGCACCTGTTC CTGTGTCAAAGGTCCCAGCAAATATAACACGTCAAAACAGCAGCTCCAGTGACAGT GGTGGCAGTATCGTGCGAGACAGCCAGAGACACAAACAACTTCCGGTGGACC |
| 2059 | EPS8 | HSX2_00910423 | 221 | TGGTCGGAGTGCCGCTCAGAAGAAATTCCATGTGCCACGGCAGAACGTGCCAGTT ATCAATATCACTTACGACTCCACACCAGAGGATGTGAAGACGTGGTTACAGTCAAA GGGATTCAACCCTGT |
| 2059 | EPS8 | HSX2_00910433 | 222 | ACTGAAAAAATTTAAACTGAATTTTTTGACCTTGGAAAATATTTTTCTTACTTTACCA AGGTGAAGTTTCCTTAATTAGACTAATTATTTTATCCCCATCCCAGGGTATAAACAG GAATTGTTTTGATAGTGGTGGAGTTATTCACTGCAACAAAGCAACAATGTTGTCCAT GATTCAAAATCTAAGCAGTTTCGATTTTGCCTGTGAATATGGTGTCTGTCATTCAGG GCATAGCTCACTGTAGGCTAGCCTCTGCTTACTTAAGTCTCTTCTCTGACATACTCAA TGGAAGAATATTTAGATTTATTTAAAGTTCTTAATGCCAACAGTTTAAAAAAAAATT AAAACATTTGAATGAACTGTAAAGTACAGCCATACCTTGGACATGCAAATATAAAT CTATGGAGCATTCTCAAGAGAGTTTGTCATGGCTCTGTTGATTGCAACTCCTTGTAT AGCTTGTATTTTGATTTAGTTTATATTCTGCTTATTATGTATACTGTGTTCTTATATAT GAGAAAGCACAAATGCGAAAGAGGTCATGTCTTCTCAAAATCTAGCAAAGGAAGT AGTCTGCATTGGTGTGCATTACAGTATTTTGCTTAATGAAAGCCTCAGTTCTGAATG TTGATATGAGTAGTTAAAAGGAAGTGGGGCCATTTTATGTGTTTATCTGTGTCAAG TATTTCTGGTAATAAGAAGCACTTAATTTACACATATTTTAATCCTGTGAAAGATTCC ACATAGAGAAAAGAAAGATACCTAACCTTCAACAAATGTTATTTTTGGAAACACAA TTTTTGTCATTAAATGTTATATTATTTCACATATATAAAACAGATGTTATGTAAGAAT GTTGTATATTTTAACATAAATCATTTAGAGAAATTATCTAGATTCATTAATTTTCATA GTGCCTTTTTCACATGAGTCAGCTGGAAAGTCTGCAATAAACAGTATTTGCTGTCTG TTAAGATAGCAGTGGCAGCTCCGAGTTACAAGAAATTATGAGAAGACGACAGGAA AAAATCAGTGCTGCCGCTAGTGATTCAGGAGTGGAATCTTTTGATGAAGGAAGCA GTCACTAATTTGTTTGTTTGTATTTAAACTCCATTGTTTTTGGCATTATTCCAACATG |
| 2059 | EPS8 | HSX2_02499999 | 223 | CACTTGACTACCTTTGTCCTGGATCGGAAAGATGCTATGATCACTGTTGATGATGGA ATAAGGAAATTGAAATTGCTTGATGCCAAGGGCAAAGTGTGGACTCAAGATATGAT TCTTCAAGTGGATGACAGAGCTGTGAGCCTGATTGATTTAGAATCAAAG |
| 2065 | ERBB3 | HSX2_00910994 | 224 | AGATACACACCTCAAAGGTACTCCCTCCTCCCGG |
| 7224 | ESRP2 | HSX2_01280361 | 225 | TTTGTTGCTCAGCCCAACTGCCAACAGTTGCTTGCCACCCTGTGGTATGATGGCTTC CCTGGATGGCGGCGGAAACACTGGGTAGTCAAGCTTCTAACCTGCATGACCATTGG GTTCCTGTTTCCCATGCTGTCTATAGCCTACCTGATCTCACCCAGGAGCAACCTTGG GCTGTTCATCAAGAAACCCTTTATCAAGTTTATCTGCCACACAGCATCCTATTTGACC TTCCTCTTTATGCTTCTCCTGGCTTCTCAGCACATTGTCAGGACAGACCTTCATGTAC AGGGGCCTCCCCCAACTGTCGTGGAATGGATGATATTGCCTTGGGTTCTAG |
| 7224 | ESRP2 | HSX2_01280366 | 226 | GTTTCATTTGGGGTGAGATTAAGGAAATGTGGGATGGTGGATTTACTGAATACATC CATGACTGGTGGAACCTGATGGATTTTGCAATGAACTCCCTCTACCTGGCAACTATT TCCCTGAAGATTGTGGCCTATGTCAAG |
| 7224 | ESRP2 | HSX2_01280397 | 227 | AGTCCCCACCAGCTATCAGATAAGGCAAACTACCCTGCCCATCTTTGTTGCCATTCA GTGCTACGCGCTGCCCATGCTGGGTCAATGAACAGCATCAAAGGGAGGCTGAAGA GATTTCAAAGAATTGTCAGCTTGTGGTTTCTGGGTATAAGTGGCTTGTTTCGTTCTT AAATACTACAACTTCTTATTGTGGGGGCTATTGAGTTTTCTTGTTTCTTAAAGATGT GTTAGTTTCCTAATGTGTGCCAATGAAATATTTACCCCATAGTATATGTCATATCTAA TAAGTTAGGCGAATTATTTTTCTGTTTGTGTGTTTCAGTTAACTTACTACATTTGAAA ATTTGATAGTATCAAAATTAGTATTTAGAATTTGTGCTATTTTTTAAAAACATCTACT ACACTCTAACCAATAGTCACTACATCTCATACATTGGCAAGATAAATAGTTGTCATA TTTGTTGGGTCCTATTCTACTGTTGATAGTTTTAATTATCATTGCTATCAAATAATTT CTAAGATAAAAGCCTGAATTTGAAATATTTTTATCAGAAAAATGGCATATTGCTGAA GTGTCAAAATATGAGAAAAGAGCATCTTCATTTTAATTAACTGCTTTATAAAAATGG CAACTTTAGGTATAGATAACCTAATTCAGGTTGCAGTTTTAATATTCATTGGGGGCA GTGGAATTGCATGTGTAAATTATGTTAGATATATATCATAACAACCCTAGTGGATTC ATGGAATTATAAACGTGATCCAAATTCTACTGTCTTCCAAGAAAGCTACATTTATAC ATCCAAACAAATTCTTTTCCACAGCTTAAGAGTACCTCATCAGAATACAGTGGTTCA AGTTAAAAAATAAAGTCCATAATGCATATGGTCATCATTCTAGACCTGATAACCACT GAGAACCTGCTGAGCAAGGACGGCTTTTACCAACATTAGCACAAAGGTTTCCTCAG TTTAGACTCTAACATATGACCTGAAACAACTCTAGGTCTGAGTCCTAGAGATTACAT AAATAGTGTGTAGCCCAGAAAAGTAAAATAGGTGTCACGCTGTTTTTTTAATGTCA ACACCAATAGGAATCCATTTAGATTAATGTATGTAATTTCTGATAAAAGTAAGAGAA ATGAGGAACTTTCGAACTGCAAATATGCCTCCAAACTAGCTTTTGATAAGTGTGGTT TCTGTTATTTGAATGGATTCCCATCTTTGTCTCTAGCCTGGAGAAGAGCAAAATGGA GGGGGAGGGGCCAGAAATAGGAGGAGGTTTTGAAGAATAAAGCCTCATATCCAG GAAAAAGTGATCAAGTGAAAAAGCTGCATCAAGACCCATTTGGCAAGAGGTTGTC CGAAAATAATTCCTTGATCATATCTGAAGTGGTTATTTACTCAAATCAATTTCTCCCC TGCTTCGCTAAGACTGTCATAAAATGGCCTTGACATTTAATCACAGGTTACTTTCCA |
| 2115 | ETV1 | HSX2_00914001 | 228 | AGGGAAGGACGTCCTATGTACCAACGCCAGATGTCTGAGCCAAACATCCCCTTCCC ACCACAAGGCTTTAAGCAGGAGTACCACGACCCAGTGTATGAACACAACACCATGG TTGGCAGTGCGGCCAGCCAAAGCTTTCCCCCTCCTCTGATGATTAAACAGGAACCC AGAGATTTTGCATATGACTCAG |
| 2119 | ETV5 | 2119.005.1-D | 229 | TCAGTCAACTTCAAGAGGCTTGGTTAGC |
| 2119 | ETV5 | HSX2_00914314 | 230 | GGTGCGCGGAGCGGTTCACCGTCTTCGGAGCGGTTCGGCCCAGCCTTTCGCCCAG GCGCCCAGGCCCGCTGCGCGCGTGCGTGAGCGCGCCTGCGCCGCCGGGGCCGCTG CAAGGGGAGGAGAGAGGCCGCCTCAG |
| 2119 | ETV5 | HSX2_00914317 | 231 | GAGACGCTGAAAGCACCATGGACGGGTTTTATGATCAGCAAGTCCCTTTTATGGTC CCAGGG |
| 2119 | ETV5 | HSX2_00914321 | 232 | AAATCTCGATCTGAGGAATGCAGAGGGCGGCCTGTGATTGACAGAAAGAGGAAGT TTTTGGACACAGATCTGGCTCACGATTCTGAAG |
| 2119 | ETV5 | HSX2_00914326 | 233 | AGCTATTTCAGGATCTCAGTCAACTTCAAGAGGCTTGGTTAGCTGAAG |
| 2119 | ETV5 | HSX2_00914331 | 234 | CACAAGTTCCTGATGATGAACAGTTTGTCCCAGATTTTCAGTCTGATAACC |
| 2119 | ETV5 | HSX2_00914336 | 235 | CTGAGCTGTCGTCTTGTAGCCATGAGCAGGCTCTTGGTGCTAACTATGGAGAAAAG TGCCTCTACAACTATTG |
| 2119 | ETV5 | HSX2_00914372 | 236 | TTAGCCAGGGCCGTGCCAACTTATGAAGACACTGCAGCTTGAGAGGGGCTTTGCTG AGGCTTCCCCTTGGCCATGTGAAAGCCCGCCTTGTTGCCTGCTTTGTGCTTTCTGCA CCAGACAACCTGATGGAACATTTGCACCTGAGTTGTACATTTTTGAAGTGTGCAGG GCAGCCTGGACACAAGCTTAGATTCTCTATGTATAGTTCCCCGTGTTCACTAACATG CCCTCTCTGGAAAGCATATGTATATAACATGTGTCATGTCCTTTGGAAACCTGGTCA CCTGGTGAAAACCCTTGGGATTCTTCCCTGGGCATGACTGATGACAATTTCCATTTC ATCAGTTTGTTTTGTTTTCCTTTTTCTTTAAATCTTGGACTTTAAACCCTACCTGTGTG ATTCAGTAGGGTTTGAGACTTACGTGTGATACTGACAGGTAAGCAACAGTGCTAGC ATTCTAGATTCCTGCCTTTTTTTAAAAAGAAATTATTCTCATTGCTGTATTATATTGG AAAAGTTTTAAACAACCAAGCTAAAGCTATGTGAAAGTTGAGCTCAAAGTAGAGGA AAAGTTACTGGTGGTACCTTGCTGCCTGCTCTGCTGGTAGAATTCTGTGCTCCCCGT GACACTTAGTACATTAAGAATGACTACACTGTTCCTCGTATGTGAAGGAGGCAGTG CTGACTCCGTGAGTGTGAGACACGTGCTTTGAACTGCTTTTCTATTCATGGAGCACT CCATAGTCTCAAACTGTCCCCCTTATGACCAACAGCACATTTGTGAAGAGGTTCGCA GGGATAAGGGGTGCACTTTATAGCTATGGAAACATGAGATTCTCCTCTATTGGAAG CTAATTAGCCCACAAAGGTGGTAAACCTGTAGATTGGGCCTTAATTAGCATTGTACT CTAATCAAAGGACTCTTTCTAAACCATATTTATAGCTTTCTTAACCTACACATAGTCT ATACATAGATGCATATTTTACCCCCAGCTGGCTAGAGATTTATTTGTTGTAAATGCT GTATAGATTTGGTTTTCCTTTCTTTACTTACCCTGGTTTGGATTTTTTTTTTTTTTCTTT TGAATGGATTTATGCTGTCTTAGCAATATGACAATAATCCTCTGTAGCTTGAGCTAC CCCTCCCCTGCTGTAACTTACGTGACCTGTGCTGTCACTGGGCATAGGACAGCGGC ATCACGGTTGCATTCCCATTGGACTCATGCACCTCCCGGATGGTTTTTGTTTTTTTCG GGGGTTCTTTGGGGTTTGTTTGTTTGCTTCTTTTCCAGAGTGTGGAAAGTCTACAGT |
| 2167 | FABP4 | HSX2_02500430 | 237 | GAATGCGTCATGAAAGGCGTCACTTCCACGAGAGTTTATGAGAGAGCATAAGCCA AGGGACGTTGACCTGGACTGAAGTTCGCATTGAACTCTACAACATTCTGTGGGATA TATTGTTCAAAAAGATATTGTTGTTTTCCATGATTTAGCAAGCAACTAATTTTCTCCC AAGCTGATTTTATTCAATATGGTTACGTTGGTTAAATAAACTTTTTTTAGATT |
| 25987 | FAM135B | HSX2_02546256 | 238 | CCCCCACCATGCCGTGGCCCCTGCTGCTGCTGGCCGTGAGTGGGGCCCAGACA ACCCGGCCATGCTTCCCCGGGTGCCAATGCGAGGTGGAGACCTTCGGCCTTTTCGA CAGCTTCAGCCTGACTCGGGTGGATTGTAGCGGCCTGGGCCCCCACATCATGCCGG TGCCCATCCCTCTGGACACAGCCCACTTGGACCTGTCCTCCAACCGGCTGGAGATG GTGAATGAGTCGGTGTTGGCGGGGCCGGGCTACACGACGTTGGCTGGCCTGGATC TCAGCCACAACCTGCTCACCAGCATCTCACCCACTGCCTTCTCCCGCCTTCGCTACCT GGAGTCGCTTGACCTCAGCCACAATGGCCTGACAGCCCTGCCAGCCGAGAGCTTCA CCAGCTCACCCCTGAGCGACGTGAACCTTAGCCACAACCAGCTCCGGGAGGTCTCA GTGTCTGCCTTCACGACGCACAGTCAGGGCCGGGCACTACACGTGGACCTCTCCCA CAACCTCATTCACCGCCTCGTGCCCCACCCCACGAGGGCCGGCCTGCCTGCGCCCAC CATTCAGAGCCTGAACCTGGCCTGGAACCGGCTCCATGCCGTGCCCAACCTCCGAG ACTTGCCCCTGCGCTACCTGAGCCTGGATGGGAACCCTCTAGCTGTCATTGGTCCG GGTGCCTTCGCGGGGCTGGGAGGCCTTACACACCTGTCTCTGGCCAGCCTGCAGA GGCTCCCTGAGCTGGCGCCCAGTGGCTTCCGTGAGCTACCGGGCCTGCAGGTCCTG GACCTGTCGGGCAACCCCAAGCTTAACTGGGCAGGAGCTGAGGTGTTTTCAGGCCT GAGCTCCCTGCAGGAGCTGGACCTTTCGGGCACCAACCTGGTGCCCCTGCCTGAGG CGCTGCTCCTCCACCTCCCGGCACTGCAGAGCGTCAGCGTGGGCCAGGATGTGCGG TGCCGGCGCCTGGTGCGGGAGGGCACCTACCCCCGGAGGCCTGGCTCCAGCCCCA AGGTGGCCCTGCACTGCGTAGACACCCGGGATTCTGCTGCCAGGGGCCCCACCATC TTGTGACAAATGGTGTGGCCCAGGGCCACATAACAGACTGCTGTCCTGGGCTGCCT CAGGTCCCGAGTAACTTATGTTCAATGTGCCAACACCAGTGGGGAGCCCGCAGGCC TATGTGGCAGCGTCACCACAGGAGTTGTGGGCCTAGGAGAGGCTTTGGACCTGGG AGCCACACCTAGGAGCAAAGTCTCACCCCTTTGTCTACGTTGCTTCCCCAAACCATG AGCAGAGGGACTTCGATGCCAAACCAGACTCGGGTCCCCTCCTGCTTCCCTTCCCCA CTTATCCCCCAAGTGCCTTCCCTCATGCCTGGGCCGGCCTGACCCGCAATGGGCAG AGGGTGGGTGGGACCCCCTGCTGCAGGGCAGAGTTCAGGTCCACTGGGCTGAGTG TCCCCTTGGGCCCATGGCCCAGTCACTCAGGGGCGAGTTTCTTTTCTAACATAGCCC TTTCTTTGCCATGAGGCCATGAGGCCCGCTTCATCCTTTTCTATTTCCCTAGAACCTT AATGGTAGAAGGAATTGCAAAGAATCAAGTCCACCCTTCTCATGTGACAGATGGGG AAACTGAGGCCTTGAGAAGGAAAAAGGCTAATCTAAGTTCCTGCGGGCAGTGGCA TGACTGGAGCACAGCCTCCTGCCTCCCAGCCCGGACCCAATGCACTTTCTTGTCTCC TCTAATAAGCCCCACCCTCCCCGCCTGGGCTCCCCTTGCTGCCCTTGCCTGTTCCCCA TTAGCACAGGAGTAGCAGCAGCAGGACAGGCAAGAGCCTCACAAGTGGGACTCTG GGCCTCTGACCAGCTGTGCGGCATGGGCTAAGTCACTCTGCCCTTCGGAGCCTCTG GAAGCTTAGGGCACATTGGTTCCAGCCTAGCCAGTTTCTCACCCTGGGTTGGGGTC |
| 27112 | FAM155B | HSX2_01700655 | 239 | CCAGCGCTGGGTGCCCTGCAAGCAATACTGCCTGGAGGTGCAGACCCGGTGCCCCT TTATACTCCCCGACAATGAGGAAATGGTGTACGGAGGGCTCCCTGGCTTTATCTGT ACAG |
| 27112 | FAM155B | HSX2_01700664 | 240 | GGTTGCTGGATACTTCACCAAAGCGTCTGGAAACCAAGTGCTGTGACGTGCAGTGG GTCTCCTGTGAGGCGAAGAAGAAGAAGTTCAAGGAGTCTGAGGCCCCCAAAACCC ACCAGCAGCAATTCCACCACTCCTATTTCCACCACTACCACCAACAGTACCATCACTA CCACCCCCATCATGATCCCCCAGGCCGTGTCAGCAACAAGCCCGCCCTGCTGCCGG TCTCTGGGGGCTCCCGCCTCAGCCCTAGCAGGATCCGGCTCTGCGTCCTTGTTCTCA TGCTCCTCCATACCGTGGTGTCCTTCTCCAGCAACCAGGGTGGTGGGGGATTGGGG CTGGAGACACTGCCTGCCCTAGAGGAGGGCCTGACACGGGAAGAGTGACAGTAG GGAGGGAGGACAGACCTCCACCACACTGACATCAGCTCCAGCTCCCCCAGGTTGG GGGGGAGGGGGCTCCTCCCATGGGAGGTGTAGGATAAGGTGGGGGCGGGGGAA ATGGGGGAATGACACATCCCCCCCAACCTACCCCCACCCCAAAAGCAGCTCCAACA GAATGGCCAGAGGGCCTCAGGGAGCTGCAAAACTCATCCAGGAGAAAAAGGCAG GAGCAGCAAGTGACCCCTCCCAAGCTCCCATCTATGGGGCTTAGCAAAAAAAGGG AGGAAGTGGGGGCTGGATATGCCCACCCCTGCCAAAAGCCCTGACCCCAGGGAAG GAGGCTGCTCACCCAGCCTTGGCCCTGCAGGGAATGTTGGGGGGCACAGAGGGGA GAAGCTCTTTCCCCCACTCCACATTCCTTTTGTTGCCAAGATCCTTAATTCCCTCCTG CCCATATCCCTACAGGCGACGGCAGACAGTGCAATGGCCCTCCTGCCACTTCAGCA CACCTTGCCCCACCTGGGACCATCACACGTGAAGCACCAGGCTGGGAGATGAGGT GCACACAGTTGCAGCTAGGTCGGGGCCCCAGTTAAGCTGTGCCCCACCACCCTAGG CAATGAGGGGCAGGAAAGGGGTACAGAATGAATGGTGAAAGAGAGTGGAGATAC GGAAGGGGGAGAGGAGAGGAGAAATGTGGACAGAGGGCTTGAAGACATGGCCG AATAGGCTACTGCCACCCGGCTTTGGGGAGATGGGCGATAAGTGTTGAGGTAGGC TCGAGAACTGCTCCCCAAATCAGTGAGAATTGCATTAGGAGCCCTCTGGGGAAAGA GCACAGGAACTGAGTTGCTGGGCCTCAAAACAAAAGGCTGCAGGCACATGGTTAG ACTGCCGCTGTCTGAAGGACCATATCTTACAGAAGGTGCACATACTCCCAAGGGCC ACTCTTGCCCTGGAGATCTTGGCCTTGGGGCCAAAGACATTTCCATTTCCGATCTCC ATGGGGAGGAGGGACTTAAGCCCAAGTGGGTCAGGCCTGGCCCAGGTCCTTATAC TCCCCTATCTTGTCCTAGCCCAGGCCTCCATGAAGGAGAACCTGGTGGCATTGCCCC AGAGCTCTCCAGAGATGAGCCTCCCCTATCCCCACCCTGTCCCCGTCCCCAGCCTAC CAAAGAGTATTCACACCTTTCCATCCCTGACTCTATGGGTTACTGTCCCAATTGCGA AACACCCATACTCCTCTTCACCCTGTAGCAGGGTCTTCTGCTCCAGTCACCCCGGCC TCTTGCAAGAGAAAGTCCAGGGACTGGCAAGAAGACCCTTTGACTAAAGATAATGT GCTGTCATATCTTGGCTGAGAAACTGTTGAGTCAGGCTAGAGGACTGGACAAGAG AAGAGGCATTGAGAGCCCCCTTGGGAGCCATTAGCCTTGCTCTGGTCTGCTGTGTG GGGTTGGGGAGTAGGGGGGATCCCACATTGCCTGGGACATCTGAATGCTGATACG AATGGACAGGAAAGGGAGCCAGAGCTGTATACCTGGGACAGGCCAGGGAGCCTTT |
| 165215 | FAM171B | HSX2_02270148 | 241 | TGGACATATGATGCACCACATTTGGGGTACTGGATAGCAGCTCCACTTCCAGGAAC TAGAG |
| 57184 | FAM219B | HSX2_01914824 | 242 | GTGAGCTTTGAATGGCATAGACAGGGTTCCCAGCCCAATAGTCGGGATTGGGGT ATAGGTTCTACATGGGAAAGTATCCAGTCTCTTGCTTTACTCAGTGTCCTGTGACCA CCAGAGGCATGAGAGGGACAACACACTCTTTTCGCTCTACCACAAGGGGTGACTAG AAGATGAGCAAGTCTAATAAAAAGATACGGCCTTA |
| 284418 | FAM71E2 | HSX2_02337017 | 243 | TGGCAGGGCCATGGCGGAGGCCCGAGTCTCCCAGCCTGGGGCATCTCCACGCTCT GTAACGCTGAGCTCCAGGCACCCGTGAAGCCCCACGGGTCAAGGCTGGTGGGCCG GGGCTGGGAGGCCTGCACGCCTGGGTTCTGGGTCCCTAAACCAGTACCCATCCACC ACAGCCACCATGATCTGGCTTCGAAACAGGAGGTGCCTTGAGCCGCTCCAGGGCAC CCCGAAGTGGGTCCCTGTTCTGGGGGAGCTGCAAAAGACCCTCCAGAAGG |
| 26224 | FBXL3 | HSX2_02546986 | 244 | GAGAAATCCAAGAAACTGAGGACTACAAATGAGCATTCTCAGACTTGTGATTGGG GTAATCTCCTTCAGGACATTATTCTCCAAGTATTTAAATATTTGCCTCTTCTTGACCG GGCTCATGCTTCACAAGTTTGCCGCAACTGGAACCAGGTATTTCACATGCCTGACTT GTGGAGATGTTTTGAATTTGAACTGAATCAGCCAGCTACATCTTATTTGAAAGCTAC CCATCCAGAGCTGATCAAACAGATTATTAAAAGACATTCAAACCATCTACAATATGT CAGCTTCAAG |
| 26234 | FBXL5 | HSX2_01687167 | 245 | AATGAATATGAACAGTTAAATTATGCAAAACAACTGAAAGAGAGATTGGAGGCTTT TACAAGAGATTTTCTTCCTCACATGAAAGAGGAAGAGGAG |
| 93611 | FBX044 | HSX2_02575024 | 246 | AATTCCCCAATGACCAGGGTTCGCAGCCAGGCCA |
| 55294 | FBXW7 | HSX2_01846734 | 247 | CCGGACACACGGGGGCACAGAATCACTGAAGGGGAAAAATACAGAAAATATGGGT TTCTACGGCACATTAAAAATGATTTTTTACAAA |
| 342184 | FMN1 | HSX2_02583328 | 248 | AGAAGCCGGACTTGAAGTGGCTCAGTCGATCCTGAGTAAGTTCTCTATGAAATCCC TATTTGGGTTTACGAGTAAATTAGAATCTGTGAATCCAGAAGAGGAAGATGCTGTG CTGAAAGCATTCCATAGTTTAGACGTGAATCCCACATCTCAGCAGGATGATTCCAGC AATGGCTTGGATCCACAGGAAGCAGGGTCTCGGGTTTCACCTGACCTCGGAAATGA TGAGAAAATTGCAAGCGTGGAGACAGAATCAGAGGGAAGTCAGAGAAAAGAAGC AGGGACATCTCTCCTTGCTCAAGAGCTGCTTCCACTGTCTACTTTGAAAGGGACAAA AGACGATGTCATTTGTGTTCGTGGGACCCTGGTGCACACCACCAGTGATTCTGACT CTGATGATGGTGGCCAGGAGCCAGAGGAGGGAAGCAGCACCAATGGCCCAAAGT CCCCTAGTGGTGTTTTATCTGAGCCATCTCAGGAGTCCAAAGAAAACCCAGGAGGT TTCAGGGAAAATACTGTCACTGGGGAAATGAATGGTGCAGAGCTCTGTGCAGAAG ATCCTCAAAGGATTCCACCTGAGATGAGCAGCAAACTGGAAGCTGGCAACGGTGG TCTTCAGACAGAGCGTCGCCCCAGCCAGGACCAGGTGGGAGAAGAAGGGTCCCAA GACCTACCTGCAGTAACAAACCAGAATTCGAGTGTTGGTATCACTGAGAGTGCCTC TTCTAAAAAAGAAGTCTCTGGAGAGAAGTCATTCCAACTTCCAGCTTTTTTCAGTGG GCTTCGTGTGCTGAAGAAGGGGGCTACCGCTGAGGGAGGAGAGACCATCACGGA AATTAAACCAAAGGATGGGGACTTAGCTTTGCTCAAATTGACGCAGCCTGTGCAGA AGTCCTTAGTGCAGGCAGGGCTGCAGACAGTGAAGAGTGAGAAGAAAGCAACTGA TCCGAAGGCCACTCCCACTCTCCTGGAGCAGCTCTCTCTGCTGCTCAACATTGACAT GCCCAAAACTGAACCGAAGGGAGCAGACCCTGAGTCGCCCAGGAGAGAAGAGAT GGGCTGCAATGCTGACCAAGAGAGCCAGAGCGGCCCTGGAGTACCACAAACCCAG GGTGGTGAGGTCAAGCCAAAGTCGCCAGAGACAGCCCTGGAGGCTTTTAAAGCCC TATTCATCCGGCCCCCCAGAAAGGGGACCACAGCTGACACCTCTGAGCTGGAAGCT |
| 257019 | FRMD3 | HSX2_02319439 | 249 | GCGAGCACCGCGCCGGCCCTGAGCCTCCCGCTCGCTCCCCACGGCCGCGGTGCATG TTCGCCTCCTGCCACTGTGTGCCGAGAGGCAGGAGGACCATGAAAATGATCCACTT TCGGAGCTCCAGCGTCAAATCGCTCAGCCAGGAGATGAGATGCACCATCCGGCTGC TGGACGACTCGGAGATCTCCTGCCACATCCAG |
| 257019 | FRMD3 | HSX2_02319452 | 250 | AGGGAAACCAAAGGGCAGTTTCTCATTGACCACATCTGCAACTACTACAGCCTGCT GGAGAAGGACTACTTTGGCATTCGCTATGTGGACCCAGAGAAGCAAAGG |
| 257019 | FRMD3 | HSX2_02319480 | 251 | CTCATCCACCATACACCATGTGCTTTAGAGTGAAATTCTACCCACATGAACCCTTGA AGATTAAAGAAGAGCTCACAAG |
| 257019 | FRMD3 | HSX2_02319488 | 252 | CTTCAAGCAAATGAAAACTCATCCACCATACACC |
| 257019 | FRMD3 | HSX2_02319496 | 253 | ATACCTTTTATACCTTCAGATTAAAAGGGACATTTTTCATGGCCGCCTGCTGTGCTC CTTTTCTGATGCTGCCTACCTGGGTGCCTGTATTGTTCAAG |
| 257019 | FRMD3 | HSX2_02319512 | 254 | GAGATTTTCCCCAAGCAGTCACAGAAGCTGGAAAGAAAAATAGTGGAAATTCATAA AAATGAACTCA |
| 257019 | FRMD3 | HSX2_02319534 | 255 | GGGGCAGAGCCCACCAGTTGCTGAATTTAACTTGCTCCTGAAAGCTCACACTTTGG AAACCTACGGGGTGGATCCTCACCCATGCAAG |
| 257019 | FRMD3 | HSX2_02319546 | 256 | GATTCAACAGGCACAACAACATTTTTAGGATTCACAGCTGCAGGCTTTGTGGTCTTT CAGGGAAATAAGAGAATCCATTTGATAAAATG |
| 257019 | FRMD3 | HSX2_02319551 | 257 | ATCCTCACCCATGCAAGGATTCAACAGGCACAAC |
| 257019 | FRMD3 | HSX2_02319566 | 258 | AAAAAAGCCATGTTGGCATTCCATACTTCAACACCAGCTGCCTGCAAACATCTTTGG AAGTGTGGAGTGGAAAACCAGGCCTTTTATAA |
| 257019 | FRMD3 | HSX2_02319579 | 259 | GTATGCAAAATCCAGTCAGATCAAGACTGTATCAAGCAGCAAGATATTTTTTAAAG GAAGTAGATTTCGATATAG |
| 257019 | FRMD3 | HSX2_02319588 | 260 | AACCAGGCCTTTTATAAGTATGCAAAATCCAGTC |
| 257019 | FRMD3 | HSX2_02319595 | 261 | TGGGAAAGTTGCCAAAGAGGTGGTGGAGGCCAGTTCCAAGATCCAGAGGGAGCCT CCTGAGGTGCACAG |
| 257019 | FRMD3 | HSX2_02319604 | 262 | AGTAGATTTCGATATAGTGGGAAAGTTGCCAAAG |
| 257019 | FRMD3 | HSX2_02319610 | 263 | CATGGAACCCCTGCAGCCCCTGCTTCCTTCCCCCAGCGAGCAAGAAGAAGAACTTC CTCTGGGTGAGG |
| 257019 | FRMD3 | HSX2_02319619 | 264 | CCTCCTGAGGTGCACAGAGCCAACATTACTCAGA |
| 257019 | FRMD3 | HSX2_02319626 | 265 | TGAAGGCAGCCCGGGAGTATGAAGATCCCCCTAGTGAAGAGGAAGATAAAATAAA AGAAGAACCTTTAACCATCTCTGAACTAGTGTACAACCCAAGTGCCAGCCTGCTCCC CACCCCTGTGGATGACGATGAGATTGACATGCTCTTTGACTGTCCTTCTAGGCTTGA GTTGGAAAGAGAAGACACAGATTCATTTGAGGATCTGGAAGCAGATGAAAACGCC TTTTTGATTGCTGAAGAAGAGGAGCTGAAGGAGGCTCGCCGTGCTTTGTCGTGGA GCTATGACATTCTGACTGGCCATATTCGGGTGAACCCACTGGTCAAGAGTTTTTCCA GGCTCCTTGTGGTGGGCCTGGGACTGCTGCTCTTTGTATTTCCCCTGCTCCTCCTCC TTTTGGAGTCAG |
| 257019 | FRMD3 | HSX2_02319629 | 266 | ACTATGAATACTACTGTCCCCTCAAGGAGTGGGTGGCTGGGAAAGTCCACCTCATC CTCTACATGCTGGGTTGCTCATGAAGTTAATCTCTCACGTGACTAAGGGCTATATTC AATGCTAGTGATTTCTTTTTTTCAGCAAATGCCTGGTTCTGAAGGGTCACGGGGCTG TCAACAGGTGTTCCTTACTCATAATTGATTATTCAAACCTTTAAGTTAGCTTTCCATA ATTCACTGCACTTAAATAAGTTTAAATCAAATACAGTTATTTTAGTTACAGGTTAGG AAGATGGTCTTTAAATAACCAAAAATATGTTTATTTTTTATTATAGTGTAGACATACC CTTCATCTATTATATCATAATACATGTTACATTGGACTGAATTAGATTTTCCCATTTC TAATAGTTGGCACCATTATAAGCTATAAGGTTCAGAATCAGAATTTTAGTAACAACT CAAGAGAAAGTTGTTGAATATAATCCTTAGTGAAAACAGTGTCCTCTAACCAATGC CTATACAACTAAATTTATGCTGGGTTTTTGGTTCTGTTTTTTTAAAAATATTTTTATG TGTTCAAACTATTTTGGTAAATTTTTAGCAA |
| 257019 | FRMD3 | HSX2_02319637 | 267 | CCTCCTTTTGGAGTCAGGTATTGATCTCTCCTTC |
| 257019 | FRMD3 | HSX2_02319641 | 268 | ACTTCCTCTGGGTGAGGGTGTTCCATTGCCTAAA |
| 257019 | FRMD3 | HSX2_02581974 | 269 | GAGATTTTCCCCAAGCAGTCACAGAAGCTGGAAAGAAAAATAGTGGAAATTCATAA AAATGAACTCAG |
| 122786 | FRMD6 | HSX2_02188584 | 270 | CATCAGACTTTACCAGAAAGACTTCCTGCGCATTGCAGGTCTGTGTCAGGACACTG CTCAGAGTTACACCTTTGGATGTGGCCATGAACTGGATGAGGAAGGCCTCTATTGC AACAGTTGCTTGGCCCAGCAGTGCATCAACATCCAAGATGCTTTTCCAGTCAAAAG AACCAGCAAATACTTTTCTCTGGATCTCACTCATGATGAAGTTCCAGAGTTTGTTGT GTAAAGTCCGTCTGTGTGCAGCTGTACAGGCAGCTTACTGTTTGCTAGAGGATGCG AAAGTCATAAGTTCTTTACATATTACTTGTGCCATATCTTCTTCACCCTAAACATAGC TCTTTCTTTATAATATTTGTGATGATGGAAACAAAAGCCTTGGAACAATTGCACTTT AAGTATTACACAGAAGTAAAAGAACTACAGAAAATGTACAGCAAGACAAGTGCCC GGAAGTTCACTGATCCTTCAGAAGGAAATGCGCTTTACTGATTGCAAAGCCTTCAG AATATTGGAGTGTGGTGTGTTTGCTCATCTGATGCTTTTTAGTTCAGTTACATGTAA CATCACATTTTTTTATCACGTGAAAGATGTTAGATTTGTTTGCTTATAAATTTTTTAC CACTCCCACATAAAATGCTCATAGTTTGGGAGAGGAAAGAGGGAAGATTCTCTCTT CTTTTAACAGAGAGATGATTGCTCTGTATACCCATTGCTTCCTCCCTGAGGCTGTCC CAAAGTGAACACTGATGGAGTGGTCAAAATCATAAGGTTGTAGCAAGCCAAAGAT ACGTATGTGACAGAAGCACATAAGCAATAAGCAGAAAACCAGAAGTGCATGCTGT GATGCCTGTGACTCCTTCATCCCGCTCAGTGCCATGTCCTCTTTTGTGATCTTCCAGA |
| 8321 | FZD1 | HSX2_01329270 | 271 | GGCCACTAGCGCGGCGCCGCCAGCCGGGAGCCAGCGAGCCGAGGGCCAGGAAGG CGGGACACGACCCCGGCGCGCCCTAGCCACCCGGGTTCTCCCCGCCGCCCGCGCTT CATGAATCGCAAGTTTCCGCGGCGGCGGCGGCTGCGGTACGCAGAACAGGAGCCG GGGGAGCGGGCCGAAAGCGGCTTGGGCTCGACGGAGGGCACCCGCGCAGAGGTC TCCCTGGCCGCAGGGGGAGCCGCCGCCGGCCGTGCCCCTGGCAGCCCCAGCGGAG CGGCGCCAAGAGAGGAGCCGAGAAAGTATGGCTGAGGAGGAGGCGCCTAAGAAG TCCCGGGCCGCCGGCGGTGGCGCGAGCTGGGAACTTTGTGCCGGGGCGCTCTCGG CCCGGCTGGCGGAGGAGGGCAGCGGGGACGCCGGTGGCCGCCGCCGCCCGCCAG TTGACCCCCGGCGATTGGCGCGCCAGCTGCTGCTGCTGCTTTGGCTGCTGGAGGCT CCGCTGCTGCTGGGGGTCCGGGCCCAGGCGGCGGGCCAGGGGCCAGGCCAGGGG CCCGGGCCGGGGCAGCAACCGCCGCCGCCGCCTCAGCAGCAACAGAGCGGGCAGC AGTACAACGGCGAGCGGGGCATCTCCGTCCCGGACCACGGCTATTGCCAGCCCATC TCCATCCCGCTGTGCACGGACATCGCGTACAACCAGACCATCATGCCCAACCTGCTG GGCCACACGAACCAGGAGGACGCGGGCCTGGAGGTGCACCAGTTCTACCCTCTAG TGAAAGTGCAGTGTTCCGCTGAGCTCAAGTTCTTCCTGTGCTCCATGTACGCGCCCG TGTGCACCGTGCTAGAGCAGGCGCTGCCGCCCTGCCGCTCCCTGTGCGAGCGCGC GCGCCAGGGCTGCGAGGCGCTCATGAACAAGTTCGGCTTCCAGTGGCCAGACACG CTCAAGTGTGAGAAGTTCCCGGTGCACGGCGCCGGCGAGCTGTGCGTGGGCCAGA ACACGTCCGACAAGGGCACCCCGACGCCCTCGCTGCTTCCAGAGTTCTGGACCAGC AACCCTCAGCACGGCGGCGGAGGGCACCGTGGCGGCTTCCCGGGGGGCGCCGGC GCGTCGGAGCGAGGCAAGTTCTCCTGCCCGCGCGCCCTCAAGGTGCCCTCCTACCT CAACTACCACTTCCTGGGGGAGAAGGACTGCGGCGCACCTTGTGAGCCGACCAAG GTGTATGGGCTCATGTACTTCGGGCCCGAGGAGCTGCGCTTCTCGCGCACCTGGAT TGGCATTTGGTCAGTGCTGTGCTGCGCCTCCACGCTCTTCACGGTGCTTACGTACCT GGTGGACATGCGGCGCTTCAGCTACCCGGAGCGGCCCATCATCTTCTTGTCCGGCT GTTACACGGCCGTGGCCGTGGCCTACATCGCCGGCTTCCTCCTGGAAGACCGAGTG GTGTGTAATGACAAGTTCGCCGAGGACGGGGCACGCACTGTGGCGCAGGGCACCA AGAAGGAGGGCTGCACCATCCTCTTCATGATGCTCTACTTCTTCAGCATGGCCAGCT CCATCTGGTGGGTGATCCTGTCGCTCACCTGGTTCCTGGCGGCTGGCATGAAGTGG GGCCACGAGGCCATCGAAGCCAACTCACAGTATTTTCACCTGGCCGCCTGGGCTGT GCCGGCCATCAAGACCATCACCATCCTGGCGCTGGGCCAGGTGGACGGCGATGTG CTGAGCGGAGTGTGCTTCGTGGGGCTTAACAACGTGGACGCGCTGCGTGGCTTCG TGCTGGCGCCCCTCTTCGTGTACCTGTTTATCGGCACGTCCTTTCTGCTGGCCGGCT TTGTGTCGCTCTTCCGCATCCGCACCATCATGAAGCACGATGGCACCAAGACCGAG AAGCTGGAGAAGCTCATGGTGCGCATTGGCGTCTTCAGCGTGCTGTACACTGTGCC |
| 2560 | GABRB1 | HSX2_00936262 | 272 | GATTATACACTCACCATGTATTTCCAGCAGTCTTGGAAAGACAAAAGGCTTTCTTAT TCTGGAATCCCACTGAACCTCACCCTAGACAATAGGGTAGCTGACCAACTCTGGGT ACCAGACACCTACTTTCTGAATGACAAGAAATCATTTGTGCATGGGGTCACAGTGA AAAATCGAATGATTCGACTGCATCCTGATGGAACAGTTCTCTATGGACTCCG |
| 2561 | GABRB2 | HSX2_00936319 | 273 | GTCCCCCCGTGGCTGTGGGGATGAACATTGACATTGCCAGCATCGATATGGTTTCT GAAGTCAATATG |
| 2561 | GABRB2 | HSX2_00936324 | 274 | GATTATACCTTGACAATGTACTTTCAACAAGCCTGGAGAGATAAGAGGCTGTCCTA TAATGTAATACCTTTAAACTTGACTCTGGACAACAGAGTGGCAGACCAGCTCTGGG TGCCTGATACCTATTTCCTGAACGATAAGAAGTCATTTGTGCACGGAGTGACTGTTA AGAACCGCATGATTCGCCTGCATCCTGATGGCACCGTCCTTTATGGACTCAG |
| 2561 | GABRB2 | HSX2_00936329 | 275 | AATCACAACCACAGCTGCCTGCATGATGGACCTAAGGAGGTACCCACTGGATGAAC AAAACTGCACCTTGGAAATTGAGAGCT |
| 2561 | GABRB2 | HSX2_00936349 | 276 | CACATCTGAGGCTGTGATGGGACTTGGAGACCCCAGAAGCACAATGCTAGCCTATG ATGCCTCCAGCATCCAGTATCGGAAAGCTGGGTTGCCCAGGCATAGTTTTGGCCGA AATGCTCTGGAACGACATGTGGCGCAAAAGAAAAGTCGCCTGAGGAGACGCGCCT CCCAACTGAAAATCACCATCCCTGACTTGACTGATGTGAATGCCATAGATCGGTGG TCCCGCATATTCTTCCCAGTGGTTTTTCCTTCTTCAACATCGTCTATTGGCTTTATTA TGTGAACTAAAACATGGCCTCCCACTGGAAGCAAGGACTAGATTCCTCCTCAAACC AGTTGTACAGCCTGATGTAGGACTTGGAAAACACATCAATCCAGGACAAAAGTGAC GCTAAAATACCTTAGTTGCTGGCCTATCCTGTGGTCCATTTCATACCATTTGGGTTG |
| 2561 | GABRB2 | HSX2_00936350 | 277 | GTAATGAATACACTAAGGTCCTTGTGGTTTTCCAGTTAAAACGCAAGTGATTTTTAC ACATGGTGGCTAGACAGCCTGAGTGCTCCATGTTGTCTGTTTAGTATGCAGCCTACT CAGAGGGGAATAATTTGGAAGATATTCTTAGAAGGCTCAACAGCATTGTCAGTCAT TTGGTCATAACCAGATACTCCTCATCTTCCTTTTTAAGGTCGGCATGCTGTTGAGAT GGCATTGTCCTTATGAAACATTATCTGTAATGCCATGCAAACACATTACTTGAAGAT GATCTATGATTTTCTTACTGAGATGATAATGGTGTCATGGGAAGATTATTCCCACTG GTTTCTAAAAGATTCTCTTGTGATTTGAGCAGGCTAGGGGAATCAAGACAGAGACT CAGGAAACCGATTTGGCCAGTCCCTTTGCTTGAGCACGGAGAATCAACTATGATGT CTTTCATTTTGGCATTTAGAGATGAGGAGACTTCTGGACAATATCATGCATGGTGG CCAAGAAAATTTTTGCTAAACGGCTATTTCCTAGGTGGTTAAAGTAAGAGTTAACA ACACAGAGCAGAGTAGAAAATCAGAAACGACGGTTGTAACAAAAAGCAAGCAAGC AAGAAAGCATGACCCTTATCAGCTCCACACAGCATCTGGTCTATACATACTGTGTCT AGTGGCAGAGGGTGAGTAAGAGAGATTGTTTTTCCAACATCACTAGCTTTTGACAG CATACCTAAAGAGGTTTTTATAGTTTAATCGAAGATAACCATGAAATACAAAAATTA GAGAAATGAGAATTGTCTTAGATATTCAGGTTAGATTCAGAGTCATTCAAATATAC AAATACAGAGCACAAACTAGCAAACAGATTGTTTTTCTCAGACGAAGTCAGCTGTC CCTGCAATTTTTGTCCCTATAATTTTATTTCTTAATTTAATAGAGTATTTCAAGAGAA GGATTTGTTCATCACTTTAAAGGATTCATGGTCCTCTCCAGCTTTTTTTTTGCTTCCA AAATTCTGATTATATTCTTAGGAGACTGGCAAAGTAAGGCTTTGGAGAGATTGAGT GAACTATATTTTCTTATATGCAATGAAATTCACCATTGTATTTTTCAAAGCAAATAAT ACTTCAAGGAGTATGAACACACAGAGAGTTAATTTGTGCATTCATTGGTTTGGGGC TTGATTTTCTTAAGAGTGCTTTCATTGTATATGTTGCAATCTGCCTAATCAAGGAAA ATATGAAAACTAGTGGAAAATAATAACAAAGCCACATTAACATGATAAATGTTATG AGCCTCAGTTGATATTGCAGAAAAATATATAACTGTTAATCAGGCAGGCATGATGT ATTTTTTTTCTATTTTTAAGTTATGCCATTGTGCTTCTTTCTACACTAATTGGACATAA GCATTCATATGTTGGACAAGAGGAGAATAAGCTAATATGTCTTCCTTCTATCAATAT AAGGCTCATATTTTGCTTTTCTTATAGAAAATAGTGAATTGCTAAATTTTCCTTAAAG TTGAATTTGATTGAACTTTTTCTTGGAGGATGACTTTGCTATTTCTATTTTGAAAACA AAGAAAGCTCTTCTTTTTAGAGTAATATCCCTAATTGTGCATAGCAGCCAGTATCCA CCTGTGAATAATGTATGTTTTCTTGATTCCTTGTTTCCTTCATGATTTAGCAGCTTGG AATATGATGGAGTACTATTGCCCTTGCATTCCAGCTGTCCAAGAACAACAACAAACC TTTATCCTTCAGCTTTCTGAATTTGCCCCAAAATGGAATGGTATTTTGTTTTTCACCA GTGTAGGAAACCTTGGCCATTATATTTTGATTACATAATCTTGAGCACCTCTGAGTA TCATCTTTGGGCAGAATCTGACCAAGATTCTTCTCACAGGCTCAGAGTAGAGATTCC TCATGTTTCTCCAGGACACACTTTCTCCTAGGCAATCATTTTGGGGCATCACACTAA |
| 2561 | GABRB2 | HSX2_02488302 | 278 | GCCTGGATGTCAACAAGATGGACCCCCATGAGAA |
| 2563 | GABRD | HSX2_00936493 | 279 | GAGTACACCATGACGGTGTTCCTGCACCAGAGCTGGCGGACAGCAGGCTCTCCTA CAACCACACCAACGAGACCCTGGGTCTGGACAGCCGCTTCGTGGACAAGCTGTGGC TGCCCGACACCTTCATCGTGAACGCCAAGTCGGCCTGGTTCCACGACGTGACGGTG GAGAACAAGCTCATCCGGCTGCAGCCCGACGGCGTGATCCTGTACAGCATCCG |
| 2569 | GABRR1 | HSX2_00936876 | 280 | AGAAAGCAGTTCCGCACCTCTGGCCTTACTCCTCTTGGAAATTGCTTTGGTCCATTT TTACTTCCTTTTATTCGACGCACCAGAAAATAAGACTTTTACCAACATTTTTACTGCA TTTGACGATGAACTAATTTAGACCGGCTAAAATAATTGTTCCACTGGGACACAGGA ATTCAACCTCAGTTCAGAAAATCCCTGACATCTGACGTAGGAGGATTTATAGGTTTA GTGGAAATTGCTTTCTCCTGCTCTCCAGATTGCATCCTGTGGGTTGATTTTTTTTTG CATGAGTAAACATCCTTCTAATAATGAACAGACCAATAATGTCTTAAGAGAGAAAA AGAACAATCTTTTCCTTTTTGCTGTTTCTGGAGAGAGCTGTTTGAATTTGG |
| 2581 | GALC | HSX2_00937669 | 281 | ATTTTACAAATATTGAAGACCCTGGCGAGCATCACTTCACGCTACGCCAAGTTCTCA ACCAGAGACCCATTACATGGGCTGCCGATGCATCCAACACAATCAGTATTATAGGA GACTACAACTG |
| 51809 | GALNT7 | HSX2_01783826 | 282 | AAGATGAGGCTGAAGATTGGGTTCATCTTACGCAGTTTGCTGGTGGTGGGAAGCTT CCTGGGGCTAGTGGTCCTCTGGTCTTCCCTGACCCCGCGGCCGGACGACCCAAGCC CGCTGAGCAGGATGAGG |
| 51809 | GALNT7 | HSX2_01783838 | 283 | AAACAAGCAATTCAAGCCAGCATTAAAGAGTTTGGATTTAACATGGTGGCAAGTGA CATGATCTCACTGGACCGCAGCGTCAATGACTTACGCCAAGAAGA |
| 51809 | GALNT7 | HSX2_01783857 | 284 | GTTTTGATATACCTTGATGCCCACTGTGAGGTGGCAGTTAACTGGTATGCACCACTT GTAGCTCCCATATCTAAGGACAG |
| 51809 | GALNT7 | HSX2_01783878 | 285 | AATTATGTTAGAGTTGTGGAGGTTTGGTGGGATGAATATAAAGACTACTTCTATGC TAGTCGTCCTGAATCGCAGGCATTACCATATGGGGATATATCGGAGCTGAAAAAAT TTCGAGAAGATCACAACTGCAAAAGTTTTAAGTGGTTCATGGAAGAAATAGCTTAT GATATCACCTCACACTACCCTTTGCCACCCAAAAATGTTGACTGGGGAGAA |
| 51809 | GALNT7 | HSX2_01783883 | 286 | ATCAGAGGCTTCGAAACTGCTTACTGCATTGATAGCATGGGAAAAACAAATGGAG GCTTTGTTGAACTAGGACCCTGCCACAGGATGGGAGGGAATCAG |
| 51809 | GALNT7 | HSX2_01783895 | 287 | AACCTGCACAGATTTACTCATATTCCTTCAGGAAAGTGTTTAGATCGCTCAGAGGTC CTGCATCAAGTATTCATCTCCAATTGTGACTCCAGTAAAACGACTCAAAAATGGGAA ATGAATAACATCCATAGTGTTTAGAGAGAA |
| 51809 | GALNT7 | HSX2_02553388 | 288 | CTGGGGAGGACAGATTCAAACCTGTGGTACCATGGCCTCATGTTGAAGGAGTAGA AGTGGACTTAGAGTCTATTAGAAGAATAAACAAGGCCAAAAATGAACAAGAGCAC CATGCTGGAGGAGATTCCCAGAAAGATATCATGCAGAGGCAGTATCTCACATTTAA GCCTCAGACATTCACCTACCATGATCCTGTGCTTCGCCCAGGGATCCTCGGTAACTT TGAACCCAAAGAACCTGAGCCTCCTGGAGTGGTTGGTGGCCCTGGAGAGAAAGCC AAGCCATTGGTTTTGGGACCAGAATTC |
| 168391 | GALNTL5 | HSX2_02580131 | 289 | TAAAGCCTGCTAGCTAAAAAAAATTTTACATCTCTCAGTTCATTCGGCACAGACCCC TGCCTCATTCAGCTGTGACTCTGCTTGGAAAATTCATCAGTTACAAAGCAGCCAATG CAATTATCTCAAGG |
| 2595 | GANC | HSX2_00939002 | 290 | GGTTCTTCAGTGGGTGAGTTATATCTTGATGATGGCCATTCATTCCAATACCTCCAC CAGAAGCAATTTTTGCACAGGAAGTTTTCATTCTGTTCCAGTGTTCTGATCAATAG |
| 2635 | GBP3 | HSX2_00941807 | 291 | ATGAGATACAAAAGCTACAGAAGACCCTGAAAAAAAAACCAAGAGATATATGTC GCATAAGCTAAAGATCTAAACAACAGAGCTTTTCTGTCATCCTAACCCAAGGCATAA CTGAAACAATTTTAGAATTTGGAACAAGTGTCACTATATTTGATAATAATTAGATCT TGCATCATAACACTAAAAGTTTACAAGAACATGCAGTTCAATGATCAAAATCATGTT TTTTCCTTAAAAAGATTGTAAATTGTGCAACAAAGATGCATTTACCTCTGTACCAAC AGAGGAGGGATCATGAGTTGCCACCACTCAGAAGTTTATTCTTCCAGACGACCAGT GGATACTGAGGAAAGTCTTAGGTAAAAATCTTGGGACATATTTGGGCACTGGTTTG GCCAAGTGTACAATGGGTCCCAATATCAGAAACAACCATCCTAGCTTCCTAGGGAA GACAGTGTACAGTTCTCCATTATATCAAGGCTACAAGGTCTATGAGCAATAATGTG ATTTCTGGACATTGCCCATGG |
| 2635 | GBP3 | HSX2_02501985 | 292 | TCCCAAAGAAAAAATGTTTTGTCTTCGATCTGCCCATTCACCGCAGGAAGCTTGCCC AGCTTGAGAAACTACAAGATGAAGAGCTGGACCCTGAATTTGTGCAACAAGTAGC AGACTTCTGTTCCTACATCTTTAGCAATTCCAAAACTAAAACTCTTTCAGGAGGCAT CAAGGTCAATGGGCCTC |
| 6843 | GM13651 | HSX2_01248411 | 293 | GAAGGTCTCCACTCTCCTCTGGGCTCCTCCTTGCCTAATGCAGGGGGTCACCGCTG GAGAAGAACCACCACTGTCCTCGATGTGTCCCAAGCCTGGAGCGAATCCGTCCTCT TGGCTCTCCCAGCCCTATCACAGGAATCATTCCTGGGTTTCTGTCCCTCTGAGGCTC ACCAGGTGTAGTTGGCCTTGTTCTTTGGGGGTATTAGCCCTTCTACTTTCTTTCTCAC CCACCCTGTACCCTCTTCTGTGTGTCTCTGCTATCCCCCTTTCCTCCCACCACCCATGT GCATGAGCAAATGTGCAACAAAACCCTGGGACTTTGCAGTCAAATGAAGCTGAGCT TCCCACATCCCGTTCTTCGGTTTGCCATGAGACGATGTGGGGTTTCCACTGTGTGTC TGTCATCACCTCTTTGTCTTTTTTCCTAACCCTGATCTCATACTTGTATAGAGTAATA ACAACAGTTGTACTTCCACCAAAGGCATGTGGCATATTTACCAATGTCATGTATTCT GAACAAAGGCAAAAAATACAAATTCCTACCATTAA |
| 6843 10810 | GM13651 Gm18480 | HSX2_02520727 HSX2_01528591 | 294 295 | ATGATGATCATGCTGGGAGCCATCTGTGCCATCATCGTGGTAGTTATTGTAA GACAGAGTGCCCAGCGGGTCACATGCATCGGACG |
| 9203 | GM6416 | HSX2_01391230 | 296 | CCAAACCCATGCGTATCAAAGAGGATATTCTCGCCTGCTCAGCTGCTGAGCTCAACT ACGGTCTGGCCCAGTTTGTGAGAGAAATCACTCGGCCCAATGGTGAACGATATGAA CCTGACAGTATCTACTATTTGTGTCTTGGCATCCAGCAG |
| 140688 | GM7682 | HSX2_02221814 | 297 | CCC |
| 84527 | GM9118 | HSX2_02102522 | 298 | CTGAGAGCCACAGTCAGGTCTGTCCTCAGGGGTC |
| 23015 | GOLGA8A | HSX2_01586004 | 299 | GAATAATCGGTGGAGATGGATTGTGAAATGGAGGTGCATAAATATCCAGTTTGCC AACCGAGCTAAGAAATGCTGGATAAAAGTAAAAG |
| 440270 | GOLGA8B | HSX2_02404961 | 300 | AGCTTGGAGCAAAGAAGATGATGTCTCACTCTGT |
| 6196 | GPR128 | HSX2_01198733 | 301 | CCTTTCAGACGGAAGGAAAGCTCTACCTGATCCTGGACTTCCTGCGGGGAGGGGA CCTCTTCACCCGGCTCTCCAAAGAG |
| 6196 | GPR128 | HSX2_01198797 | 302 | GTTGTTCATCGAGACCTGAAGCCGAGTAACATCCTGTACAGGGATGAGTCGGGGA GCCCAGAATCCATCCGAGTCTGCGACTTCGGCTTTGCCAAGCAGCTGCGCGCGGGG AACGGGCTGCTCATGACACCCTGCTACACGGCCAATTTCGTGGCCCCGGAG |
| 2859 | GPR35 | HSX2_00954161 | 303 | GGCTGGGGACTGGAGGGGAACGTGGAGCTCCTTCTGCCTCCTTTCCTGCCCCATGA CAGCAGGCAGATCCCAGGAGAGAAGAGCTCAGGAGATGGGAAGAGGATCTGTCC AGGGGTTAGACCTCAAGGGTGACTTGGAGTTCTTTACGGCACCCATGCTTTCTTTG AGGAGTTTTGTGTTTGTGGGTGTGGGGTCGGGGCTCACCTCCTCCCACATCCCTGC CCAGAGGTGGGCAGAGTGGGGGCAGTGCCTTGCTCCCCCTGCTCACTCTCTGCTGA CCTCCGGCTCCCTGTGCTGCCCCAG |
| 51704 | GPRC5B | HSX2_01779589 | 304 | GGAGGACGTGCAGCTGCCGCGGGCCTATATGGAGAACAAGGCCTTCTCCATGGAT GAACACAATGCAG |
| 2869 | GRK5 | HSX2_00954458 | 305 | CCCCGCGCTGTGTACTGTAAGGACGTGCTGGACATCGAGCAGTTCTCCACTGTGAA GGGCGTCAATCTGGACCACACAGACGACGACTTCTACTCCAAGTTCTCCACGGGCT CTGTGTCCATCCCATGGCAAAACGAG |
| 2869 | GRK5 | HSX2_00954468 | 306 | TCTACTGTCTCAGTTTACATTTTGTATATTTGTATTTAAATGAAAGTGAGACTTTGAG GGTGTATATTTTCTGTGCAGCCACTGTTAAGCCATGTGTTCCAAGGCATTTTAGCGG GGAGGGGGTTATCAAAAAAAAAAAAATGTGACTCAAGACTTCCAGAGCCTCAAAT GAGAAAATGTCTTTATTAAATGTAGAAAGTGATCCATA |
| 54103 | GSAP | HSX2_01789812 | 307 | GAAGTTTGTGTGTATGTTACAGCCCGAAGTGTGCCTCTTGGGGACAAATCACATAT TCAGTGTTTTACATTCATAAAG |
| 54103 | GSAP | HSX2_01789986 | 308 | CCTCCG |
| 373156 | GSTK1 | HSX2_02371777 | 309 | GCCTTTGGGCTGCCCATCACCGTGGCCCATGTGGATGGCCAAACCCACATGTTATTT GGCTCTGACCGGATGGAGCTGCTGGCGCACCTGCTGG |
| 84705 | GTPBP3 | HSX2_02572437 | 310 | AGGAGGGGGTCCTGGAGGCTCTGGCCCACGTGGA |
| 727936 | GXYLT2 | HSX2_02430028 | 311 | GAAGCAACTGCAGAGAGGCTGAGCATGAAGGTGTGTCTGTTCTGCATGGAAACCG AGGCGTCTACCATGACGATAAGCAACCAACGTTCAGAGCACTCTATGAAGCAATAC GGGAT |
| 727936 | GXYLT2 | HSX2_02430046 | 312 | GGAGACTGTGCACACTTTATGTGGACGAATCCCGCAAGTTTTCTGAAGCAAATTG AGAAAACAATGAAAAGGGCTTATGAGAAACACGTCATCATCCATGTTGGCCCCAAC CAGATGCACTGAATATTTTGTCTTGTTGCAAGTCAATTAGGTGTCTTGTGACCAAGG AAATACTAATCTCTAAGCTGCCTGGGTCTTTT |
| 727936 | GXYLT2 | HSX2_02430047 | 313 | TGTGTGAATATTTAATGGTGCTCCATGACTGTTGAGTTTTAAAAACCTCGTTAAATT TTGCCAAATCAGTTGCCCCCAAAAGGGAATATGCTTTTCCTTATTTTTTTTCTAAAA TGCTATTTATCTCTAAGGAA |
| 3014 | H2AFX | HSX2_00965768 | 314 | ACTGGCGGCAAGGCCCGCGCCAAGGCCAAGTCGCGCTCGTCGCGCGCCGGCCTCC AGTTCCCAGTGGGCCGTGTACACCGGCTGCTGCGGAAGGCCACTACGCCGAGCG CGTTGGCGCCGGCGCGCCAGTGTACCTGGCGGCAGTGCTGGAGTACCTCACCGCT GAGATCCTGGAGCTGGCGGGCAATGCGGCCCGCGACAACAAGAAGACGCGAATCA TCCCCCGCCACCTGCAGCTGGCCATCCGCAACGACGAGGAGCTCAACAAGCTGCTG GGCGGCGTGACGATCGCCCAGGGAGGCGTCCTGCCCAACATCCAGGCCGTGCTGC TGCCCAAGAAGACCAGCGCCACCGTGGGGCCGAAGGCGCCCTCGGGCGGCAAGA AGGCCACCCAGGCCTCCCAGGAGTACTAAGAGGGCCCGCGCCGCGGCCGGCCGCC AGGCCTCCCCATGCCACCACAAAGGCCCTTTTAAGGGCCACCACCGCCCTCATGGA AAGAGCTGAGCCGCTTCAGACTGCGGGGCAAGCGGGCCGCGGCTCCCTTCCCCTCC CCTCCCCTCGCCCGCCTTCGCCGCCCGGCCTCGAGTCCCCGCCCGCCCCCGCTCCCG TCCCGCACCGCCTGCCGCGTCGGCCTCGGGCCCTGCCCTGTCCGCCGTCCGCCCTCC GGTAGGGTTCGGGCCTTCCGGATGCGGCTTGGGCGCTCTTCGGGGACCTCCGTGG CGCGGAAGACCCGAGCCTGCCGGGGGGAGGCCGGCGGCGCCGCACCTGCCCGCC TCGGCGTTCGTGACTCAGCCGCCCCATCCCGAGTCGCTAAGGGGCTGCGGGGAGG CCGCAGCACCTTCTGGAAGACTTGGCCTTCCGCTCTGACGCAGGGCCGAGGTGGGC AGTCCAGGCCGAGAGGCCGGCGGCCCTGAAGGTGAGTGAGGCCCTCGGCAGCTG CAGCCGGGGTGTCTGGTACCCCCCCGGCGTGGTGCTTAGCCCAGGACTTTCAGACG CGGCCGCTGGCCGGGAGGCTTTGGTGGGAGAGACGCGATCGCCGATTTCGGTCTG GCGCCCCTTCTGCGGCCGGGACCCAGGCCTTTCACATCAGCTCTCCCTCCATCTTCA TTCATAGGTCTGCGCTGGGGCCGGGACGAAGCACTTGGTAACAGGCACATCTTCCT CCCGAGTGACTGCCTCCTAGGAGGACATTTAGGGGAGGGCAGAGGCCTGCAGTTT GGCTTCACGGCTGGCTATGTGGACAGCAAGAGTCGTTTTCGCGGAAGCCGACTGG CAGCCAGGCCTGTCGGGCCCCCCGACGCCGCCCCATTTCCCTTCCAGCAAACTCAAC TCGGCAATCCAAGCACCTAGATACCAGCACAAGTCGGTTAATCCCTGTCTGGACTG |
| 57493 | HEG1 | HSX2_01924755 | 315 | TTGGAGAACATCAGCTTGCCAGCAGCTCTGAGGTGCAAAATGGAAGTCCCATGTCT CAGACTGAGACTGTGTCTAGGTCAGTCGCACCCATGAGAGGTGGAGAGATCACTG CACACTGGCTCTTGACCAACAGCACAACATCTGCAGATGTGACAGGAAGCTCTGCT TCATATCCTGAAGGTGTGAATGCTTCAGTGTTGACCCAGTTCTCAGACTCTACTGTA CAGTCTGGAGGAAGTCACACAGCATTGGGAGATAGGAGTTATTCAGAGTCTTCATC TACATCTTCCTCGGAAAGCTTGAATTCATCAGCACCACGTGGAGAACGTTCGA |
| 57493 | HEG1 | HSX2_01924813 | 316 | ACAGTTCACGCCTCTAGGGAGTCCAACGCGGTGG |
| 57493 | HEG1 | HSX2 01924845 | 317 | CCCCGTATGCTGAATACCCCAAAAATCCTCGCTCACAAGAATGGGGCCGAGAAGCT ATTGAAATGCATGAGAATGGAAGTACCAAAAACCTCCTCCAGATGACGGATGTGTA CTACTCG |
| 57493 | HEG1 | HSX2_01924856 | 318 | CGGATGTGTACTACTCGCCTACAAGTGTAAGGAA |
| 9931 | HELZ | HSX2_01455269 | 319 | ATTTTTAGAACAAGGAATCTGTAGGTATGGTGCCCAGTGTACTTCAGCACATTCCCA GGAAGAACTAGCAGAATGGCAGAAAAGATATGCTTCACGGCTGATAAAATTGAAA CAGCAAAATGAGAATAAACAGCTCTCAGGCAGTTACATGGAAACCTTGATAGAAAA GTGGATGAATTCATTGTCTCCTGAGAAAGTG |
| 9931 | HELZ | HSX2_01455291 | 320 | GTTCAACCTTAAAGTGCAATTGCAGATTCTGGCAAGCTTCATGCTCACTGGTGTTTC TGGAGGTGCAAAGTATGCTCAGAATGGACAACTTTTTGGTCGCTTTAAGCTTACTG AAACACTTTCTGAAGATACTTTGGCTGGACGACTGGTGATGACCAAAGTCAATGCT GTTTATTTATTACCAGTCCCTAAACAGAAGTTAGTACAGACCCAGGGAACCAAAGA GAAGGTTTATGAAGCTACTATTGAAGAAAAAACAAAGGAATATATATTTTTAAGGC TATCTAGGGAATGCTGTGAAGAACTTAATCTTCGGCCTGACTGTGACACACAG |
| 9931 | HELZ | HSX2_01455342 | 321 | TTTGGGAACGGTTTATTGCCCTGTGTCATGAAAACAGTAGCCTACATGGAATCACTT TTGAACAGATCAAAGCCCAGTTAGAGGCTTTAGAACTAAAGAAGACATATGTGTTG AATCCGCTGGCACCTGAATTTATCCCCCGGGCTCTAAGACTGCAGCATTCAGGAAG TACCAACAAACAGCAGCAATCACCACCCAAG |
| 9931 | HELZ | HSX2_01455369 | 322 | TTGCTCATCGGCCACAGTCTCCTCCTGCAGAAGCTGTAATTCCGGAGCAGCAGCCC C |
| 9931 | HELZ | HSX2_02533027 | 323 | GAAATTTTGGGAACGGTTTATTGCCCTGTGTCATGAAAACAGTAGCCTACATGGAA TCACTTTTGAACAGATCAAAGCCCAGTTAGAGGCTTTAGAACTAAAGAAGACATAT GTGTTGAATCCGCTGGCACCTGAATTTATCCCCCGGGCTCTAAGACTGCAGCATTCA GGAAGTACCAACAAACAGCAGCAATCACCACCCAAG |
| 23493 | HEY2 | HSX2_01639712 | 324 | AGGCAGCTCGGTAACTGACATCAGCAACTTTTGAAAACTTCACACTTGTTACCATTT AGAAGTTTCCTGGAAAATATATGGACCGTACCATCCAGCAGTGCATCAGTATGTCT GAATTGGGGAAGTAAAATGCCCTGACTGAATTCTCTTGAGACTAGATGGGACATAC ATATATAGAGAGAGAGTGAGAGAGTCGTGTTTCGTAAGTGCCTGAGCTTAGGAAG TTTTCTTCTGGATATATAACATTGCACAAGGGAAGACGAGTGTGGAGGATAGGTTA AGAAAGGAAAGGGACAGAAGTCTTGCAATAGGCTGCAGACATTTTAATACCATGC CAGAGAAGAGTATTCTGCTGAAACCAACAGGTTTTACTGGTCAAAATGACTGCTGA AAATAATTTTCAAGTTGAAAGATCTAGTTTTATCTTAGTTTGCCTTCTTTGTACAGAC ATGCCAAGAGGTGACATTTAGCAGTGCATTGGTATAAGCAATTATTTCATCAGTTCT CAGATTAACAAGCATTTCTGCTCTGCCTGCAGGCCCCCAGGCACTTTTTTTTTGGA TGGCTCAAAATATGGTGCTGCTTTATATAAACCTTACATTTATATAGTGCACCTATG AGCAGTTGCCTACCATGTGTCCACCAGAGGCTATTTAATTCATGCCAACTTGAAAAC TCTCCAGTTTGTAGGAGTTTGGTTTAATTTATTCAGTTTCATTAGGACTATTTTTATA TATTTATCCTCTTCATTTTCTCCTAATGATGCAACATCTATTCTTGTCACCCTTTGGGA GAAGTTACATTTCTGGAGGTGATGAAGCAAGGAGGGAGCACTAGGAAGAGAAAA GCTACAATTTTTAAAGCTCTTTGTCAAGTTAGTGATTGCATTTGATCCCAAAACAAG ATGAATGTATGCAATGGGATGTACATAAGTTATTTTTGCCCATGCCTAAACTAGTGC TATGTAATGGGGTTGTGGTTTTGTTTTTTTCGATTTCGTTTAATGACAAAATAATCTC TTAATATGCTGAAATCAAGCACGTGAGAGTTTTTGTTTAAAAGATAAGAGACACAG CATGTATTATGCACTTCATTTCTCTACTGTGTGGAGAAAGCAATAAACATTATGAGA ATGTTAAACGTTATGCAAAATTATACTTTTAAATATTTGTTTTGAAATTACTGTACCT |
| 3098 | HK1 | HSX2_00971987 | 325 | GACTATGATGCCAACATCGTAGCTGTGGTGAATGACACAGTGGGCACCATGATGAC CTGTGGCTATGACGACCAGCACTGTGAAGTCGGCCTGATCATCG |
| 9455 | HOMER2 | HSX2_01407795 | 326 | AGAACAGCCCATCTTCACCACCCGAGCGCATGTCTTCCAGATTGACCCCAACACCAA GAAGAACTGGATGCCTGCGAGCAAGCAGGCGGTCACCGTTTCCTACTTCTATGATG TCACAAGGAACAGCTATCGGATCATCAGTGTGGACGGAGCCAAG |
| 3249 | HPN | HSX2_00981105 | 327 | CCACGGTCTGCAGCTGGGGGTGCAGGCTGTGGTCTACCACGGGGGCTATCTTCCCT TTCGGGACCCCAACAGCGAGGAGAACAGCAACGATATTGCCCTGGTCCACCTCTCC AGTCCCCTGCCCCTCACAG |
| 3249 | HPN | HSX2_00981117 | 328 | AAACCAGATCAAGCCCAAGATGTTCTGTGCTGGCTACCCCGAGGGTGGCATTGATG CCTGCCAG |
| 3249 | HPN | HSX2_02504427 | 329 | GCCAACAGGCCGGGGTACTCCAGGAGGCTCGAGTCCCCATAATCAGCAATGATGTC TGCAATGGCGCTGACTTCTATGGAAACCAGATCAAGCCCAAGATGTTCTGTGCTGG CTACCCCGAGGGTGGCATTGATGCCTGCCAG |
| 90161 | HS6ST2 | HSX2_02132501 | 330 | TTCAATTTTCTTTAGTTTTAATTGAAAATGTTTGCTTTTAAAACTGATAGGTATTGTT GGAAAGCAGGATGAAGCCTGAGCCAGTGGAAAAGCTTGTTACAGAAAAAACATTT TGTGTTATTGCTGTGGTGTGCATGATTTGCAAAGATTAAGTGCATTTTCTCTGTCTA TACTGATTATTGTATATAGAGGATGTTATAAATATACATATACATTTTTGCCATTATG TAAATCCCATGATTTCAACTGTAAACATCTGTCCATTGGTGTAGCTTTACAAACCATT CACTGATTTTGTGTAATTTAACAATAGATATGAAATAAAGTTTAAATTACA |
| 163702 | IFNLR1 | HSX2_02268031 | 331 | GCAGAGGACGAAGAGGAGGAGGATGAGGAGGACACAGAAGATGGCGTCAGCTTC CAGCCCTACATTGAACCACCTTCTTTCCTGGGGCAAGAGCACCAGGCTCCAGGGCA CTCGGAGGCTGGTGGGGTGGACTCAGGGAGGCCCAGGGCTCCTCTGGTCCCAAGC GAAGGCTCCTCTGCTTGGGATTCTTCAGACAGAAGCTGGGCCAGCACTGTGGACTC CTCCTGGGACAGGGCTGGGTCCTCTGGCTATTTGGCTGAGAAGGGGCCAGGCCAA GGGCCGGGTGGGGATGGGCACCAAGAATCTCTCCCACCACCTGAATTCTCCAAGG ACTCGGGTTTCCTGGAAGAGCTCCCAGAAGATAACCTCTCCTCCTGGGCCACCTGG GGCACCTTACCACCGGAGCCGAATCTGGTCCCTGGGGGACCCCCAGTTTCTCTTCA GACACTGACCTTCTGCTGGGAAAGCAGCCCTGAGGAGGAAGAGGAGGCGAGGGA ATCAGAAATTGAGGACAGCGATGCGGGCAGCTGGGGGGCTGAGAGCACCCAGAG GACCGAGGACAGGGGCCGGACATTGGGGCATTACATGGCCAGGTGAGCTGTCCCC CGACATCCCACCGAATCTGATGCTGCTGCTGCCTTTGCAAGGACTACTGGGCTTCCC AAGAAACTCAAGAGCCTCCGTACCTCCCCTGGGCGGCGGAGGGGCATTGCACTTCC GGGAAGTCCACCTAGCGGCTGTTTGCCTGTCGGGCTGAGCAACAAGATGCCCCTCC CTCCTGTGACCCGCCCTCTTTAGGCTGAGCTATAAGAGGGGTGGACACAGGGTGG GCTGAGGTCAGAGGTTGGTGGGGTGTCATCACCCCCATTGTCCCTAGGGTGACAG GCCAGGGGGAAAAATTATCCCCGGACAACATGAAACAGGTGAGGTCAGGTCACTG CGGACATCAAGGGCGGACACCACCAAGGGGCCCTCTGGAACTTGAGACCACTGGA GGCACACCTGCTATACCTCATGCCTTTCCCAGCAGCCACTGAACTCCCCCATCCCAG GGCTCAGCCTCCTGATTCATGGGTCCCCTAGTTAGGCCCAGATAAAAATCCAGTTG GCTGAGGGTTTTGGATGGGAAGGGAAGGGTGGCTGTCCTCAAATCCTGGTCTTTG GAGTCATGGCACTGTACGGTTTTAGTGTCAGACAGACCGGGGTTCAAATCCCAGCT CTGCTGTTCACTGGTTGTATGATCTTGGGGAAGACATCTTCCTTCTCTGCCTCGGCT TCCTCATCTGCAGCTACGCCTGGGTGTGGTGAGGGTTCTAGGGGATCTCAGATGTG TGTAGCACGGAGCCTGCTGTGTCCTGGGTGCTCTCTACGTGGTGGCCGGTAGAATT CTCCATCTATCCAGGCTCCAGGAGACCCCTGGGCATCTCCCACCTGTGGCCCCTAAA CCCAGAGTGACTGAGAGCACTTAACATTCAGCTTGTCTCATCCCCAGTCTACCTCCT TCCTTCTACCCTCACTGCCTCCCAGTCAGGAGAGTGAGCTCTCAGAAGCCAGAGCCC CACCCAAGGGGACCCTGGTCTCTCCGCCTTCACCTAGCAATGGGAACCCTGCTTCCC AGGGGAGGAACCAACTGCTCCACCTTCTAGGGACCCAGTTTGTTGGAGTAGGACA GTAACATGGCAGGAATCGGACTTCTGGGCCTGTAATCCCAGTTTGGATGGCACGTT AGACTCTTGGTTGACCGTTGTGGTCCTTAGAAGTCCCATTCTCCCTTCCAGTTATGA GAAACCAATGCCTTCTAGATTCAGGTGACTATCCTTACCTGGGGGTGCTGATGCAT CCTCAGTTAACCTACACCCACCTGAATATAGATGAGCGTAGCTGAGTTTTCACCCGT AGGACCGAAGTGTTTTGTGGTGGAGTATCTGAACAACCTTGGCTCTGTGGCCATTC |
| 3476 | IGBP1 | HSX2_00991063 | 332 | GCATCAACTTCTAACTCATCTCGCCAGGAGAGGCCTCCAGTGAAACCCTTCATTCTC ACTCGGAACATGGCTCAA |
| 3489 | IGFBP6 | HSX2_00992250 | 333 | GGCCCATGCCGTAGACATCTGGACTCAGTGCTGCAGCAACTCCAGACTGAGGTCTA CCGAGGGGCTCAAACACTCTACGTGCCCAATTGTGACCATCGAGGCTTCTA |
| 3489 | IGFBP6 | HSX2_02505340 | 334 | CCGAGGGGCTCAAACACTCTACGTGCCCAATTGTGACCATCGAGGCTTCTACCGGA AGCGGCAG |
| 285313 | IGSF10 | HSX2_02487762 | 335 | ATTTTGCCAACAAATAACAATAAACAAACATTGAAACGGTTCACTATAAAATAACAA ATGGCTAATGTACCTGAATTTTTCAGTAAAAAAATGAACTTCTAATACCAGTTGCCT AGTGTCCACCTCCTATCAATGTTACAAGCATGGCACTCAGAACAGAGACAATGGAA AATATTAAATCTGCAATCTTTATGATGTAAATTTACCATCCTGATGTATAAATATTTT GTGGTTTATAAATTTTTTTGCTAAAACCTACAGAAAATAAGCACTGAACTGTCTTAT GATTCACCATTTGTTCACACTGAAAATTTTCATTGATGGGGAATACAAAAATGTTTT AATAACTGCATTTAAAAATAAATGCAGGCTCACTTTTCCACATGTAAAAAGCAATGT AATACCTGCACATCATAAAATGTGAAAAGGGGGTTATTTTTGTCCAAATACACCCAT TAAAAAGGTTTTAAAAATACAA |
| 22807 | IKZF2 | HSX2_01567897 | 336 | CCCGACATTGTCACCTCCTCTTTGAGGGGTTAGAAGAAGCTGAGATCTCCCGACAG AGCTGGAAATG |
| 22807 | IKZF2 | HSX2_01567903 | 337 | CCTCCGTTTATCTTCAGCCCGACATTGTCACCTC |
| 22807 | IKZF2 | HSX2_01567916 | 338 | CATTGCACTTTGACTATGGAAACAGAGGCTATTGATGGCTATATAACGT |
| 22807 | IKZF2 | HSX2_01567953 | 339 | GTGAACGCCCCTTCCACTGTAACCAGTGTGGAGCTTCTTTACTCAGAAGGGCAAC CTTCTGAGACACATAAAGTTACACTCTGGAGAGAAGCCGTTCAAATGTCCTTTCTGT AGCTACGCCTGTAGAAGAAGGGACGCCCTCACAGGACACCTCAGGACCCATTCTG |
| 22807 | IKZF2 | HSX2_01567978 | 340 | TACCTCCTATGGAAGATTGTAAGGAACAAGAGCCTATTATGGACAACAATATTTCTC TGGTGCCTTTTGAGAGACCTGCTGTCATAGAGAAGCTCACGGGGAATATGGGAAA ACGTAAAAGCTCCACTCCACAAAAGTTTGTGG |
| 22807 | IKZF2 | HSX2_01568048 | 341 | GTAAACTGAAATGTGACGTCTGTGGCATGGTTTGCATTGGGCCCAATGTGCTTATG GTACATAAAAGGAGTCACACTG |
| 22807 | IKZF2 | HSX2_01568069 | 342 | TAAAAGGAGTCACACTGGTGAACGCCCCTTCCAC |
| 386653 | IL31 | HSX2_02379521 | 343 | GCCCTGACGCCCAGCCGCCAAACAACATCCACAGCCCAGCCATCCGGGCATATCTC AAGACAATCAGACAGCTAGACAACAAATCTGTTATTGATGAGATCATAGAGCACCT CGACAAACTCATATTTCAAGATGCACCAGAAACAAACATTTCTGTGCCAACAGACAC CCATGAATGTAAACGCTTCATCCTGACTATTTCTCAACAGTTTTCAGAGTGCATGGA CCTCGCACTAAAATCATTGACCTCTGGAGCCCAACAGGCCACCACTTAAGGCCATCT CTTCCTTTCGGAT |
| 26034 | IPCEF1 | HSX2_01676158 | 344 | TGCCAAGGGTGGCTGTATAAGAAAAAGGAAAAGGGAAGTTTCCTAAGCAACAAAT GGAAAAAGTTCTGGGTGATACTGAAGGGGTCGTCACTGTACTGGTATAGCAATCA AATG |
| 26034 | IPCEF1 | HSX2_01676239 | 345 | AATGTTACAGTGAAAGTGAACAGGAAGATCCAGAAATAGCTGCGGAGACACCACC CCCTCCTCACGCTTCCCAGACTCAGTCTTTG |
| 26034 | IPCEF1 | HSX2_01676280 | 346 | AAGAGACAAAAGTGTCTGAAGATGATGAAATGGAGAAGCTGTACAAATCATTAGA GCAAGCTAGTCTATCTCCTCTTGGGGACCGACGACCTTCGACTAAAAAGGAGTTGA GAAAATCCTTTGTTAAGCGGTGTAAAAATCCATCTATAAACGAGAAACTCCACAAA ATCCGAACATTGAATAGCACATTAAAG |
| 26034 | IPCEF1 | HSX2_01676301 | 347 | AGCCAGGAAATACAGAGAGTGGAAAGTCATGAACACCCTGCTGATCCAGGACATC TATCAGCAGCAGCGGGCTTCGCCTGCCCCTGATGACACTGATGACACCCCCCAGGA ACTCAAGAAATCACCTTCTTCTCCCTCTGTTGAAAATTCCATTTGAGACAAAGTCAG GGTTTTCTCCTCTT |
| 26034 | IPCEF1 | HSX2_01676305 | 348 | ATATTTTATCACAAGCAACTCTTCAAGATGTTGCAAAAGCTTACATTTTTCCTTAAAA GGAAAACTGAAACCCAGTCCTTCAAGCATCAGCTTCCCATCTAAAGATGCACGTTA GATGAAGATAATCACCGAATACACCAGGGCTCCTCATTTCAGTCGTAACGCATCCAT TGAGATTGGAATGATTGAGTTCAGTTGAAAGGCGTTTTTTGTGTTCCTACTGGGAG CTCACGGTGATGTGGGGCTCTAGGGGGGATGTGAGAAATGTCTCTTCTGTTCATTG CTCTCCAGAGAATGCTATGGACAAGAATCAAATTTTACCAGGAGTAATTGCCCATG GAAACATTCTGTTTTTCAAAAGAAGAATGTACATATTCCACTTGACTGGGAAGCTGT GTGGCCCAAATCACTGTTTGAGTGGAGGAATGTTTTCTAGAGCAGTGACTGGAAG GACAAACGTGGTGTTGCAGAACAAAAATTCCTTGCCTTTGGTATGGAATGGTGGTG CCTTTTCCATGTGATGAGAAATGCTTCATAGACAGTGGCATGTGGGAGAGCTGAAG GAGGAGGCGTGTTCCATATTCCACTCATTCAAAGGACAGCTTGGTGTTTTCACACTT TATGCAGGTTAGGGGATCTGATTAATGTTTCCTTTCCTTACAACAGCTAGAGAAAAC ATCTCATCACAATTGGAGACCTCTACTTCCTTTGAGCTTTCCTACTCAATGGTAGGA GATAGGAATATGTTCCGCAAAGAATGTCTACTGCTCTTGTCCTTGGCTGTGATCCCT ATACATAGTGTGAATTTATATCCAGGTTTCCCAGGATCAATATTGACAAGACGACTT TGGCATCTGAATTGTTTTAATATCTTTACTCAAAAAAACAAAAAAACAAAAACCCAT GGAAGCAACAAAAGAGTTAAACATGAGGGATTTATTTTTGTATGTTTATATGTCACT TGTGTGCTATGTGAATAGCTATGTTTGTTATTTAAATATATTTATAGAAGTTCAAATT ACTAGTGTTTAAAAGATGATATACTATTATATATTGTGCTTAGTATATATTTGATGA GTCTATACTTTTTGCTTGAGAAAAATAGAAATACTATTGATTCACTGGTGTTATGAT GTTGCACTGTGTCTGAGGAATGCTCAGGAAATCTTGTGGGAAAAAAATGGGGCAT GCATTCATAATTTTTCATTTTCTTCCCCACCCCAACCCCCATGAGCTCTTAAATTTAG ATTTCTATCTCTTGTAAGTCACTCTTTTAACCAGCAAAGGACTTTCATCTTAGATTAT CCTTTCTATTAAATAAACTTTAAGCTTTGTGCCATTTATGCCTGCAAACCTTAGACCA AAGGCAACACAACTAATGTCATCTTTTCATTAAAGACTAATAGAAATTTAGAAATCA TTGTGTTAAAGAGAGCTATAGAGAAACAATATGAAAGTCTTCCCTTACTAATCACCA TGAATATTTCTCACCATAGGAATAACCACAACCCACCAAGTACCCATGTTGGCCCTG CCTACCCGTAACAAATTGTTCACACAACCTATTTTGTTTTATAGCTTCAAAGTGTGGC TTGACATTCAAATGGAAGGAACCACCTGAATTATATCTGCAAACAAAATGGAAATA GTTGTTGAATAGTTCCCCAAGCTTTACTAAGAAGCCTTAATACTCTGTAAACAAACA AACAAACAAAAAACAACCTTAGCATTAAGAAATAAAAACAAAAAACAAGAAGCAA GTTGTACTGCTAGAAACTTCTCTAAGATAAGAGGCCAGGCCAGTCAGTTTGGCCCA GCAAACATGTGGGTACTTCTCCACCTAGGGAAGCCACTGAAGATGAGTTTGACCTG TGGTGACCTGACAGAGAGAACATTCAGATCCTCAGATTGCACCCTGATGCCCTGGT TTAGGAAAGCAGTGCTTTAACCTTTTGCCCCTGGAAATTACTGATTCTTTCTGTGATT |
| 3679 | ITGA7 | HSX2_01011260 | 349 | GAGTACTCAGCTGTGAAGTCCCTGGAAGTGATTGTCCGGGCCAACATCACAGTGAA GTCCTCCATAAAGAACTTGATGCTCCGAGATGCCTCCACAGTG |
| 3682 | ITGAE | HSX2_01011786 | 350 | TGTGGCTTTTTAAAAGAAAATATCAACAACTGAACTTGGAGAGCATCAGGAAGGC CCAGCTGAAATCAGAGAATCTGCTCGAAGAAGAGAATTAGGACCTGCTATCCACTG GGAGAGGCTATCAGCCAGTCCTGGGACTT |
| 3685 | ITGAV | HSX2_01012164 | 351 | AGCCTCAGACGCTGCGTGGAGCGGCGGAGCCGGAGGGAAGCAAAGGACCGTCTG CGCTGCTGTCCCCGCCCCGCGCGCTCTGCGCCCCTCGTCCCTGGCGGTCGCTCCGA AGCTCAGCCCTCTTGCCTGCCCCGGAGCTGTCCCGGG |
| 3710 | ITPR3 | HSX2_01015842 | 352 | CTGGGCACCTGCCCCACCAAGGAGGACAAGGAGGCCTTTGCCATCGTGTCAGTGCC CGTGTCTGAGATCCGAGACCTGGACTTTGCCAATGACGCCAGCTCCATGCTGGCCA GTGCCGTGGAGAAACTCAACGAGGGCTTCATCAGCCAGAATGACCGCAG |
| 3751 | KCND2 | HSX2_01017841 | 353 | CTTATGTGACTACAGCAATAATAAGCATCCCAACACCTCCAGTAACCACACCAGAAG GAGACGATAGGCCAGAATCCCCTGAGTACTCAGGAGGAAATATTGTCAGAGTTTCT GCTTTGTAAGACAATTGGAATAAGGTCTAAGAGAATTCGAGCCCTGGCTGTGAAAA GAATCTCAACATAGAAGAAAGAAGAAACAATAAATATTCTGCAGATTAATGCAGCA AAGAAAGAAGGTTGGTAGTGAAACACAAAGCTTCCAATCTTAAGGATGTGAATAA AACCACCAAATGGCATTTCTAGACAGTTTGACCTGTTATACAGAGTAATATTCTGTG GCCCTTTGACTTTGTGAATGAGCACAATGAAATGCCGCCTACTGA |
| 8645 | KCNK5 | HSX2_01350094 | 354 | ATCTACTTGGGGCTGGCCTGGCTGTCCCTTTTTGTCAACTGGAAGGTGAGCATGTTT GTGGAAGTCCACAAAGCCATTAAGAAGCGGCGGCGGCGACGGAAGGAGTCCTTTG AGAGCTCCCCACACTCCCGGAAGGCCCTGCAGGTGAAGGGGAGCACAGCCTCCAA GGACGTCAACATCTTCAGCTTTCTTTCCAAGAAGGAAGAGACCTACAACGACCTCAT CAAGCAGATCGGGAAGAAGGCCATGAAGACAAGCGGGGGTGGGGAGACGGGCCC GGGCCCAGGGCTGGGGCCTCAAGGCGGTGGGCTCCCAGCACTGCCCCCTTCCCTG GTGCCCCTGGTAGTCTACTCCAAGAACCGGGTGCCCACCTTGGAAGAGGTGTCACA GACACTGAGGAGCAAAGGCCACGTATCAAGGTCCCCAGATGAGGAGGCTGTGGCA CGGGCCCCTGAAGACAGCTCCCCTGCCCCCGAGGTGTTCATGAACCAGCTGGACCG CATCAGCGAGGAATGCGAGCCATGGGACGCCCAGGACTACCACCCACTCATCTTCC AGGACGCCAGCATCACCTTCGTGAACACGGAGGCTGGCCTCTCAGACGAGGAGAC CTCCAAGTCCTCGCTAGAGGACAACTTGGCAGGGGAGGAGAGCCCCCAGCAGGGG GCTGAAGCCAAGGCGCCCCTGAACATGGGCGAGTTCCCCTCCTCCTCCGAGTCCAC CTTCACCAGCACTGAGTCTGAGCTCTCTGTGCCTTACGAACAGCTGATGAATGAGT ACAACAAGGCTAACAGCCCCAAGGGCACATGAGGCAGGGCCGGCTCCCCACCCCA CCTTTGATGGCCTCTTCCCCCCTCACCCTAGGGTGTCCCGAGATGACCGGGACGCCT |
| 8909 | KCNN4 | HSX2_01371797 | 355 | TGGGATTCTTACCCCGATGTGCTGGCAATGCAGTTCAACTGGGACGGCTACTATAA GGAAGTGGGCTCTGCTTTCATCGGCAGCAGCCCTGAGTTTGAGTTTGCACTCTACT CCCTGTGCTTCATCGCCAGGCCAGGCAAAGT |
| 23081 | KDM4C | HSX2_02542306 | 356 | TTCCTACGGTCGCTGCCCGGCCTCCTTCCATGTCACTTGTGCCCATGCTGCTGGGGT ACTGATGGAGCCTGATGACTGGCCTTATGTGGTGAACATTACATGCTTTCGACATA AGGTCAACCCCAACGTG |
| 90231 | KIAA2013 | HSX2_02132945 | 357 | ATCCCAGTGTCTGAGTGAACTAACAGTCCTGCTTTCAGCCACCATTTGCACAAGACA CCCAGCACTGAAAGTCCCGCTGCCAGGAGCAAGGGATCCTTTGGAAGCACCCGCCC TTTGTGCCTTGTTGGGGGAAACCGGTGACGCAGAAGTGAGTGTGGATACACCAGA GTTTGCATTGGAAGGAATGAGTGTCACGTGGGGAGGGAAGGGGCCAGTGGACCT TTTGTAAGCTTTCCACTCAATAAAATGAACCTGTATGGCAA |
| 55220 | KLHDCBA | HSX2_01840918 | 358 | GGGGATGGCTGAAGATGGAACGATCGTTCTTCCTCAAGAAGCGGCGGGCAGATTT TGTGGCTGGCTCTCTGAGTGGACGGGTCATAGTGGCTGGGGGACTTG |
| 5655 | KLK10 | HSX2_02514669 | 359 | ACCAGCGGCAGACCACAGGCAGGGCAGAGGCACGTCTGGGTCCCCTCCCTCCTTCC TATCGGCGACTCCCAG |
| 5650 | KLK7 | HSX2_01154792 | 360 | ATCAAGGCCTCGAAGTCATTCCGCCACCCCGGCTACTCCACACAGACCCATGTTAAT GACCTCATGCTCGTGAAGCTCAATAGCCAGGCCAGGCTGTCATCCATGGTGAAGAA AGTCAGGCTGCCCTCCCGCTGCGAACCCCCTGGAACCACCTGTACTGTCTCCGGCT GGGGCACTACCACGAGCCCAGATG |
| 5650 | KLK7 | HSX2_01154804 | 361 | GGGGAACTTTCCCTTGCGGCCAACCCAATGACCCAGGAGTCTACACTCAAGTGTGC AAGTTCACCAAGTGGATAAATGACACCATGAAAAAGCATCGCTAACGCCACACTGA GTTAATTAACTGTGTGCTTCCAACAGAAAATGCACAGGAGTGAGGACGCCGATGAC CTATGAAGTCAAATTTGACTTTACCTTTCCTCAAAGATATATTTAAACCAACCTCATG CCCTGTTGATAAACCAATCAAATTGGTAAAGACCTAAAACCAAAACAAATAAAGAA ACACAAAACCCTCAGTGCTGGAGAAGAGTCAGTGAGACCAGCACTCTCAAACACTG GAA |
| 79875 | KRT8P30 | HSX2_02028559 | 362 | ATGAAAACTGCAAGGACAAGTACTACAACTGCAACGTGGTGGTCCAGGCAAGACT CTGTGTCTACAACTACTACAAGACCGCCTGCTGTGCCTCCTGCACCCGTGTGGCCAA CAGGCAGACGGGCTTCCTGGGGAGCAGATAACACTCCTGCACCCCCATCAGTAGG GCAGCATCACTGCCTTCCCGGGGGCTTCAGCAGTGCGCCTGGCTGGCTGCTGCTCC ACCACGGGCCCCCTGGCCCAGGCGCTGCCAACCAACTTAGTCACCACCCCTGCCTCC GGTGAATGCACCCCGTGGTACCCAGGGGCTTTTTACACAAGATGTTTGAAAGCCAC AGTCAGTCCTTTAAGCATCACCATGTACTGATGATCCCCTCCTTGGACCTGGCATCT GCTAATGGTGCCCTTTGAAAGTCAAGCAGTGGGAAGTACATGGAGCTCTCAGCCCT GCTCCCATCTGGCACCTTCAAGTCAGCAGATGGGCCACTGACTGAGCACTGCCCCG TCCCTGGTGCTACTGGTCTTTCTAAACTTAGCACCCTGGAGAGTCCAAGGAGGCAG CGCCCCCAACCCAGCGCCCCACTAAGCCTTGCTGACACGCGTGCATCCCTCTGTGAC CTCAGCCCAGATGTGCCTGTTTTCATTCTCAAAGACATTAGACTGTTTTCCTGCCCTA TGACACAGATAGCTCACATGAATATTGTGCTTTATTTAGCAGGTGTACTCACAGATA CTAGCTCCTTAGCAGCTCACAACATCCCAGAATGGGAGGCAGGGGGTGACTCATTA TCCCCATTTTACTGACAGGGAAACTGAGGCTCAACTTAAGTAATTGACCTGCCAGGT ATATTCACCCATCCAGTGGAAGAGCTGAGTCCCCGCCCCAGTCATCTACCAGTATCC AGCCTGGGGCCTGTACTTAGATGTGAAAGGTGCTGCTTCATTTCTGACCAAGAGAC TGAGAAGTTTCCCAGAATGCAAACAAAGCCCAGGCCCCTGAAATCTTTCCGGTCAA GCCTTTATCCCAGCACTCAGTTGTTTTGGATGTCTGTTCCTACTTGCCCTTACCCCCA AAGTTACAGATCCTAGTTACAGGACTCTGCCAGCTTTGTTAAACTGTCCGTGAGACA AGAAAGCCATTGGGGAAACCAGGTGATTGCCTGAAATTCTTACTCCGTTCCAAGTG CTGTTCCTCCCAGGAAATCAAAGGCCAGGGTCCTTATGGCCGTGGAGCCTTCCCGA CCACAGAGCCAACTTGTGAAGCACACAGCTCTGCAGCCTGGGCTCTGCCCTGCCTC AGCCGCCTCCCCCACGCTCTTCACCACGTTCCTGGAGAGTCCGGCCAACCTGTCCCA GCCGAAACACTGCTGTATTAGAAAAAGTCTCTTTCTGGTCTTTCTGGTTTTGTTTAT GAATTTCCCTCTGTGGCCACAAATTCCTCCCCTCCCCCATGACTCACAGTCCATATGG CCCACCCCCAGACTTGAGCACCAAGCTCTGCATTAATGCAGTTGGCCTGCGACAAG GAGCTGTGGACCCTTCCCCATCTCTTCCAATTCACTTTCCCCAACTATCCAGTTCCAG AGGCCGCAGGCCTGGAAGGATGCAGTGCATATTGAAAGGTGGACCCTCTGAAAAC AGTTAAGAGGAATATATGTATGTTTTACCCATTAAGAAAAAATGGCAAGCTAAACA AATGTTAAACTTACAGAAAATTTGTCTTATGGTCCTGAGCATATTTCCCTTTTAGAG CAAGCCTGGATTCTTAGCAAAGTGTTTCCCCCATTTGCTCTTTTAGCTGACAAATCT GCCACTGTGATGATGGTTTGCAGCTTTTGGAAGCAGTATGGCAACCTGGCCTGACA TGCTCTTTAGGCTTCCACTAACCTGGGGCTTTCAGAAATTCTATTTGGCCTTTCTGTG GGTAGCTTTCCAGCTTCTCTTCTAGGGAGCCCCAGGCATCATTTCCCAAAAGCATCC |
| 23355 | L3MBTL1 | HSX2_01628191 | 363 | GAGAGTGATGGGAGTGAGAACCTGTGAATCTAGATGTCTGTTCAGTGGTTCCAAG GGTGAAGTCTGCTGTATTGAGCCTCTGCATTCTAAGCCTGAGTTGAAAGATCATCCC ATCACACAGTTTTCATTATTGGCCATGGCATCCTTGACAAAA |
| 23355 | L3MBTL1 | HSX2_01628350 | 364 | AGAGTGGATCATCTCCTGAAACAAGATTGTCTTACAGAAGCGTTGGCTCTTGCGTG GTCTTTCCATGAAGGAAAAGCAAAAGCAGTAGTGG |
| 7462 | LAT2 | HSX2_01300472 | 365 | GAGCCAAGGCAAAGAGGGACCACTGTGCTCATGGACCCATCGCTGCCTTCCAAGG ACCATTTCCCAGAGCTACTCAACTTTTAAGCCCCTGCCATGGTTGCTCCTGGAAGGA GAACCAGCCACCCTGAG |
| 286256 | LCN12 | HSX2_02348587 | 366 | AGGCCAGCACTGTGACACATGGTCTTATGTGCTGATACCGGCAGCCCAGCCTGGGC AGTTCACTGTGGACCACGGTGTGG |
| 3977 | LIFR | HSX2_01034760 | 367 | GGAAAGCACCCTCTGGAACAGGCCGTGGTACTGATTATGAAGTTTGCATTGAAAAC AG |
| 200879 | LIPH | HSX2_02286232 | 368 | ATTTACATGATCGGAGTAAGTCTAGGAGCCCACATATCTGGGTTTGTTGGAGAGAT GTACGATGGATGGCTGGGGAGAATTACAG |
| 200879 | LIPH | HSX2_02286275 | 369 | GATTTCAGTATTTTAAATGTGACCACCAGAGGTCTGTATACCTGTACCTGTCTTCCCT GAGAGAGAGCTGCACCATCACTGCGTATCCCTGTGACTCCTACCAGGATTATAGGA ATGGCAAGTGTGTCAGCTGCGGCACGTCACAAAAAGAGTCCTGTCCCCTTCTGG |
| 200879 | LIPH | HSX2_02286297 | 370 | GCTATTATGCTGATAATTGGAAAGACCATCTAAGGGGGAAAGATCCTCCAATGACG AAGGCATTCTTTGACACAGCTGAGGAGAGCCCATTCTGCA |
| 200879 | LIPH | HSX2_02286330 | 371 | ACCTATTCTTTGCCCAGAGTTGCAGTTGTAACTGTTGCCAGGACACATGGCCATAAA TAATAGAAAGAAAGCTACAACCACAGGCTGTTTGAAAGCTTCACCTCACCTTTCTGC AAGGCAGAAAAAGTATGAAAAAAACCAAGGCTTTTTTCAGTAGCGTCCTATGGATG TCACATTGTACATCAAACAACCTTGTGATTATAAAACGATCCTGGGAAGGAGCCCCT AACTAGGGCAAGTCAGAAATAGCCAGGCTCGCAGCAGCGCAGCGCTGTGTCTGCT GTGTCCTGGGGCCTCCCTTGTTCCGACCTGTCAATTCTGCTGCCTGTCACGCGGGTG GTTCTGCCCATCGCGGCTGCGGGTCAAGCATCTTCAAGGGAAGGACGGACTGGAG GCCTCACCGTGGACTCAACTCTGCATTCTCCGTGCCACATTCCTCCAGTTCCCACAC GTAGAAGGGAACGAAACTGACGTCTACCTCATGGGGCTGCTGTGTGGGTTTGGGA GGCAAAAATCTATGAAGGGTTTTTTGAAATCCCATAGGTGCCACATCTATGAGATG TTTGATAAATGTGAATATGCTTTTACATTTGGGCTTATCTAATTTGCAATAAGAGAG CCTCTCTCTATCAACACCAGCTTCTCTCTCGGGCTGTTTGCTCAGGGAAGGCAAGAA AGCCACGTGCTGGCCCTCTGCCTTCTCTAAAGTGCTGTTGGAGCATGGAGGAGCTG GAGGAGATGGGGATGGACTGACAGCTAAGAGGGCGGCTGCTGGGACTAGATAGT GGATGAAGAAAGAAGGACGAGGAAGCCGTGGGGCAGCCTCTTCACATGGGGACA GGGGATGGAGCATGAGGCAAGGGAAGGAAAAGCAGAGCTTATTTTTCACCTAAGG TGGAGAAGGATCACTTTACAGGCAACGCTCATTTTAAGCAACCCTTAAGAAATGTTT |
| 200879 | LIPH | HSX2_02580660 | 372 | GCAGAAGGAGCTTCTCTTGATGACATTTACATGATCGGAGTAAGTCTAGGAGCCCA CATATCTGGGTTTGTTGGAGAGATGTACGATGGATGGCTGGGGAGAATTACAG |
| 4000 | LMNA | HSX2_02507428 | 373 | TGGGGGCAGCCTCTCCCCAGCCTCCCCGTGCCAAAAATCTTTTCATTAAAGAATGTT TTGGAACTTT |
| 55164 | LOC100289661 | HSX2_01835838 | 374 | AACATGTTAACTGCTCTTCTGGCACCAAGAAAATCCAGGACAGCAAAACCACTTGT GGAAG |
| 55164 | LOC100289661 | HSX2_02556484 | 375 | AACATGTTAACTGCTCTTCTGGCACCAAGAAAATCCAGGACAGCAAAACCACTTGT GGAAGAAATAG |
| 23211 | LOC100508859 | HSX2_01609614 | 376 | GAACTATGGAGGCCGCGCCCGGGACCCCCCGCCGCCGCCATCAGAGTCGCCGCC GCCGCCATCGCCGCCGCCGCCATCAACGCCTTCGCCTCCTCCGTGTTCCCCCGACGC CCGCCCGGCCACCCCGCACCTCCTCCACCACCGCCTCCCGCTCCCTGACGACAG |
| 1,01E+08 | LOC100509091 | HSX2_02468548 | 377 | CTCCACACACCTGGAGAACAACCTCATTGACCGGCGCCGCATCCCGCCCACTGCCTT CTCCTGCACCCGAGCCTATCACAGCGTGGTCCTCCAGCCCCAGCGGCGGGGGGAG GAGGGCTCCTAGTCCTGCCTGCCGCCTTCCTCCAGCACACACTCTGCTTCTCGAGGG GGTGGAGGGGCAGGCTGAGGTGTGCTTGGCCTTTGCACCACTGGGAGAAAGGTCT AGAAGACCCCACTGCTTCCAGTGCACCCACTTCCCATTGACTGGTGAGGCCGCTTCA CTTAGCAAATGGCCCAGGGGCGAAAGGTGGCACTGGTTTGTACTTGAACATAATAA ATGCAGCTTGGGCAGGCTCACCAGCCAGCCTCCCTCCCTCCAGGAGCTACGCTGCA TGTCCTGGC |
| 9697 | LOC101927254 | HSX2_01428829 | 378 | GTTCAGTGCCTGGGCTGCTGTTTTTGGGGTTACCCGCCTCTTCATCCTCACCCTTGC CGTGCTGGCCATTGGCTTTGGACTGGCTCGCATGGAAAACCAGGCATTTGATCCCG AGAAAGGGAACTTCAACACTTTGTTTTGCAG |
| 23248 | LOC101929500 | HSX2_01614852 | 379 | ACAAAGCCAACAGAAAATATCTCAAAGGCCTCTTCATGTACCCCAGTGCCTGTGACC ATGACAGCAACTCCACCTCTTCCAAAGCCTGTGAATACTTCTCTTTCCCCTTCCCCAG CATTGGCTTTGCCAAACCTGGCTAATGTGGATCTGGCAAAGATCAGTTCCATCCTTA GCAGTTTAACATCAGTCATGAAAAATACTG |
| 23248 | LOC101929500 | HSX2_01614881 | 380 | GGGTCAGTCCTGCATCAAGACCTTCTCCAGGAACGCCCACCAGCCCCAGCAACCTC ACCAGTGGCCTGAAAACACCTGCACCTGCCACGACAACATCTCACAACCCTCTGGC AAATATCCTCTCCAAGGTGGAGATCACCCCAGAGAGCATTCTGTCTGCACTTTCCAA AACCCAGACACAGTCAGCCCCTGCACTGCAAG |
| 23248 | LOC101929500 | HSX2_01614918 | 381 | CAGCAAGGCCTCAATTGGGCAAAGCCCAGGGCTCCCAAGCACTACTTTTAAACTAC CTTCCAACTCTTTGGGGTTTACAGCTACCCACAATACTAGCCCTGCTGCCCCACCTAC TGAAGTTACCATCTGCCAATCTTCAGAGGTCTCCAAGCCAAAGCTGGAGTCAGAGT CCACCTCCCCAAGCCTGGAAATGAAGATTCACAACTTCTTAAAAGGTAATCCTGGTT TCAGTGGCTTAAACTTAAACATCCCAATCCTGAGCAGTTTGGGGTCCAGCGCCCCAT CAGAGAGCCATCCCTCAGACTTCCAGCGTGGCCCTACTAGCACCTCAATCGACAAC ATTGATGGAACCCCTGTACGGGATGAACGGAGTGGGACACCCACCCAGGATGAGA TGATGGACAAGCCCACATCCAGCAGTGTAGATACTATGTCCCTGCTTTCTAAGATCA TTAGCCCTGGTTCCTCAACACCCAGCAGTACAAGATCACCACCCCCTGGGAGAGAT GAAAGCTACCCCCGAGAGCTCTCCAATTCTGTATCTACATATCGACCCTTTGGTCTG GGCAGTGAATCTCCCTATAAGCAGCCTTCTGATGGAATGGAGAGACCATCTTCCCT GATGGACTCTTCACAGGAAAAGTTCTACCCAGATACTTCTTTCCAAGAAGATGAGG ATTACCGAGATTTTGAGTATTCAGGGCCTCCACCCTCTGCCATGATGAACCTAGAGA AGAAACCAGCCAAATCTATCCTGAAATCAAGCAAGCTGTCTGATACCACCGAGTAC CAGCCAATTCTGTCCAGTTATAGCCACAGAGCCCAAGAATTTGGGGTAAAGTCTGC CTTCCCTCCATCTGTAAGGGCCCTCCTGGACTCTAGTGAGAACTGTGACCGTCTCTC ATCTTCCCCTGGGCTATTTGGTGCCTTCAGCGTAAGAGGGAATGAACCTGGGTCTG |
| 23248 | LOC101929500 | HSX2_01614922 | 382 | GTAAGAATGATTCATTTTCACCCCTGACTCCAACCACAATAGCTTGTCTCAATCTAC CACTGGGCATCTCAGTTTGCCACAGAAGCAGTACCCAGACTCTCCTCACCCAGTCCC ACATCGTTCCCTTTTCTCTCCGCAG |
| 23248 | LOC101929500 | HSX2_01614923 | 383 | GGCCTCCACCATTTCCACCACGTCGACGATTGAATTTAAGAATATGCTTAAAAACGC CTCACGTAAGCCCTCAGATGATAAGCATTTTGGCCAGGCTCCCAGCAAGGGCACTC CAAGTGATGGTGTCAGTCTCTCAAACCTCACCCAACCCAGCTTGACCGCCACTGATC AGCAGCAACAAGAAGAGCACTACCGCATAGAAACCCGCGTCTCCTCCTCCTGCTTA GACTTGCCTGATAGCACAGAAGAAAAGGGGGCCCCTATAGAAACCTTGGGTTATCA CAGTGCATCCAATAGGAGGATGTCAGGGGAGCCGATCCAGACCGTAGAGTCCATC CGAGTTCCTGGGAAGGGAAATAGAGGACATGGGCGTGAGGCTTCAAGGGTGGGT TGGTTTGATCTGAGCACATCAGGTAGCTCTTTTGACAATGGCCCTTCAAGTGCCTCT GAGTTGGCATCCCTTGGGGGTGGGGGCAGCGGAGGCCTCACTGGCTTTAAAACAG CACCATACAAGGAACGGGCACCTCAATTTCAGGAGAGTGTCGGCAGCTTTCGTTCC AACAGTTTCAACTCAACATTTGAGCATCATCTTCCCCCATCCCCCTTGGAACATGGG ACACCCTTCCAGAGAGAGCCAGTGGGGCCATCATCTGCCCCACCTGTCCCTCCTAA GGATCATGGTGGTATCTTCTCTCGAGATGCACCCACTCATCTACCCTCTGTGGATCT TTCGAACCCCTTCACAAAGGAGGCAGCCCTGGCCCATGCTGCCCCACCCCCTCCTCC TGGAGAGCACAGTGGAATTCCTTTCCCTACCCCACCTCCTCCTCCCCCTCCTGGGGA ACATAGCAGCAGTGGTGGGAGTGGTGTCCCCTTTTCTACTCCACCCCCTCCTCCACC CCCTGTTGACCACTCTGGAGTTGTACCCTTCCCAGCCCCACCACTGGCAGAGCACG GAGTGGCAGGGGCTGTGGCAGTATTTCCCAAGGACCATAGTTCCCTCCTTCAAGGG ACCCTGGCTGAGCATTTTGGGGTACTCCCAGGACCCAGGGACCACGGGGGCCCCA CCCAACGGGACCTCAACGGCCCTGGCCTTAGCCGTGTACGAGAGAGCCTCACCCTA CCCTCCCATTCTCTGGAACACCTGGGCCCACCCCATGGAGGAGGAGGTGGGGGAG GCAGCAACAGCAGCAGTGGCCCCCCCTTGGGTCCCTCACACAGAGACACCATCAGC CGGAGTGGTATAATCTTACGGAGTCCCCGGCCAGACTTTCGGCCTAGGGAACCTTT |
| 84561 | LOC101929724 | HSX2_02103940 | 384 | ATTTCTTCCTGAAGTCCAGGCTCCAAGAACAAGATGTCTGGAGAAGATCCACTTCTT TCTATACCCACATGTGCAACCCCTGGGTCTCCCTGTTGGGG |
| 54532 | LOC649605 | HSX2_02554222 | 385 | ATTGGAACTAGATGGAAAGATGTTGTCTCCAAATGCATTCGATGCCACTTTCAGCCA CTACTTTTGTTTTATGCAAACCCAGATGGCACAGCAGTTTCTACTGAGGATGCACTC AGGCAGGTCATCAGCTGGTCACATTACAAATCTGTTGCAGAAAATATGG |
| 285180 | LOC91119 | HSX2_02341096 | 386 | GTCACGCTGCTCCAGTTCCACCCAAACCCAGGGAAGGAGACCCAGAAAAAACAAA GATGCCCCAAAGAAG |
| 164832 | LONRF2 | HSX2_02269570 | 387 | GTAATGTGTGAAGTGCTGTTTTCAGCTACAGCAAATGTGCATGAAAATTTAACATCT TCCATCCAAAGCAGATTAAAGGCTCAGGGTCACAGCCACATGAATGCCCAGGCTCT GCTGGAAGAAGGTGATGCAGGGAGCTCGGAG |
| 164832 | LONRF2 | HSX2_02269581 | 388 | AATTCATCTGAGAAATCTGATATGCTTGGAAATACCAATTCTTCAGTTTTGTATTTTA TACTGGGTCTACACTTTGAAGAGGATAAAAAGGCGTTAGAAAGCATCCTTCCAACA GCACCCAGCGCTGGCTTAAAGAGACAGTTTCCGGATGACGTGGAGGATGCACCTG ACCTGAACGCCCCTGGAAAAATTCCCAAGAAAG |
| 164832 | LONRF2 | HSX2_02269589 | 389 | ATCTCTCACTTCAAAGGAGCCCAAACTCTGAGACAGAAGAAAGTCAGGGGCTCTCG CTTGATGTAACTGACTTTGAGTGTGCCCTCTGCATGAG |
| 164832 | LONRF2 | HSX2_02269599 | 390 | GAGCGCTGCCTTGACCACGCCCCACACTGTCCTTTGTGCAAAGACAAACTTTCGGA A |
| 164832 | LONRF2 | HSX2_02269630 | 391 | GCTTTCAGAGTATGGATGCATGCTGGAGATTAAGGACGTGAGAACGTTTCCTGATG GAAGTTCTGTTGTAGACGCGATTGGCATCAGTCGGTTCCGAGTGCTAAGCCACCGC CACAGAGATGGCTATAACACAGCGGACATTGAATATCTTGAAGATGAAAAG |
| 164832 | LONRF2 | HSX2_02269640 | 392 | GTGGAGGGTCCAGAGTATGAAGAACTTGCCGCTCTCCACGATTCTGTGCATCAACA GTCTGTTTCCTGGTTCGCGTCTCTCCAGGATCGCATGAAAGAACAAATTTTAAGTCA TTTTGGGGTAATGCCAGACAGAGAACCTGAGCCTCAG |
| 164832 | LONRF2 | HSX2_02269652 | 393 | CGTCCATTCATTGTGCTTCATCAAGTGCTTGTGGATGAGGTTCCAAAATGGGACGCT TGCCAAACATTGAGTCCTCCTCAAAAATGACAATTCTGTGTCTGGTGGGATCTGACC TTGTGTGAGGTTAGCCTGAAGTCTGAATGGAGCCCATAGTTGGAAAACAACCTAAG AAAATCTCTTAGAAGCAGGTGCTTGGGGAATGCAGTTCACTGACAGCACAGGACCC TGCAGATGGTTTACATGTGGTTTGGGTTTCACGAGAAAGAAGGATTCACTTCCCAG TCAGCATCTGGCTCTGCCAGATGGTAAAGGCGTGCTTTAGTGTGTAGACAATATGG GGGAACCACGTTTTTATCTGGAAGTGGATTTCTTAGAACACAGGCTAACCAAAACT ACGCTTAGGCTTTGCGTGTTGCTGTGAAGTTGTCTGTGAAATCGAATAATCACACCA TTGTTCAGTGCAGGAGCCCAAACTAGTCCTTACCCAAGAAGTAGTAGCCTCTGGAT AGAACTGTGTTTAATGTCCTGTTGTAGTCCCAGGTGTTGTAAATTGCATGTTGTAAT CAAACGAATGTCAAAACATAAGAAAGTATACCTTGGATATAGAAAAACCTGAGAAC AGTATCATTCACTTGAGGATATATATATATATATTTACACACAATAAAGTGAGTTAA AATTGTATATGCATTGGGATGTCAAACATAAAACCACCAAGTGCAAAGAATGCTTT GAAAGTAGAACCTTGTCTCATTGATCAGTGGTTACTAAGCATTTAGGAAACAGTCA TCTTTTTCTATTGGGATTTGCATTAGAAATTAGCCCCATCAGTAAATTTCAGTTTACT ATCCATTTATTAATATACAAAAACATTAAAAAATCTGCCATGCCATGGTAATCTATCT ATTCCAGGAAAATAGATCAGAATATATGAAGGGAGGATGGCCGGTATGGGATGTA AATATTCAGTAAGTTTTTCTCCAACACCAACCTAAAATTATCTTTATTTTTTTGAAAG AAAGTTTTACGGTGATCAGACCAGGTGTTATCTGTTAAAGGACAGTAGTATGACTT CCATCCTGTAATGCACGTTGGTGATATTGATTGGAATAACTGAATCTGAGATACTCA TATGTTCTCTCTGGCCCAACCAGCTGGTGCATTTGGTTGGCTTCCTCGTCTGTAAGG TGGACATTCTTAACCAGAGGGTCACGACCACATCACAGCTGCAAGTTTTGCTTTGTT CATTGAAGGTGATTTTTTAGTTTTAAAATATATATATCTCATAATATACAATGTTGAT GGTATATCAATCCATCAAGCATTTTATTAGAAAGACTTTATAATATTATTAATTTTTC TTTAGTTTTCAGTAGATAAAGTAGTTTCAGTAGATAAAGACTTTATAATATTATTAAT TTTTCTTTTAGTTTTCAGTCTCATCATGGGAGTTAGAAAATACCAGCATCACTTAATT GTAAAGAGAAAATGTGAAAGTATTTTCCAATCCAATGTTTCAAGTGCCTCACTTATT GTGAATCTCAAATATCATCTTGAGATGGTTTAATGGCAGTTTGGCCAAGTGATTTCT AAGAGATTTTATTAATTTATGCATTAACAATAGTATTCAACTTTCTTAGGCATTATAA CCTAACCCGATCATTTGATTGGAATAACTGAATCATTCATTGATTGGAATAAAATAA AGAAAACAAAATGCATCTCACATGCCAGAAGACCTTTACCAGAAGTGGTTCATGAA AATCAGTATGCATTGACCATTCTCTGCCTTTCCCTTTTGGAAAATTTTTTTGTACCTTC ATATCACAGCTGAATAGTTATCAATGCCATTTCCATTTGGAAGTATCTAAATAACCA CAATGTGATCACAGCTTCTCCACGAGCCTTCCTGAAGTAAAGCTGGTATGCTTGCTA TAATACTTGGAACCTGTTGCTTTCTCTTCCTCACTCTTTAAATGTCATAAGGTCACAC |
| 57121 | LPAR5 | HSX2_01910604 | 394 | AGCAACACGGAGCACAGGTCTCTGCTGCTGATGA |
| 4038 | LRP4 | HSX2_01040417 | 395 | CACCGCTCTGAGTACACACTGCTGCTTAACAACCTGGAGAATGCCATTGCCCTTGAT TTCCACCACCGCCGCGAGCTTGTCTTCTGGTCAGATGTCACCCTGGACCGGATCCTC CGTGCCAACCTCAACGGCAGCAACGTGGAGGAGGTTGTGTCTACTGGGCTGGAGA GCCCAG |
| 4038 | LRP4 | HSX2_01040427 | 396 | TACCATTTACTGGACAGACTGGGGCAACACCCCCCGTATTGAGGCCTCCAGCATGG ATGGCTCTGGACGCCGCATCATTGCCGATACCCATCTCTTCTGGCCCAATGGCCTCA CCATCGACTATGCCGGGCGCCGTATGTACTGGGTGGATGCTAAGCACCATGTCATC GAGAGGGCCAATCTGGATGGGAGTCACCGTAAGGCTGTCATTAGCCAGG |
| 4038 | LRP4 | HSX2_01040469 | 397 | TGTCTACACCATGTGCTATGGAGAATGGCGGCTGTAGCCACCTGTGTCTTAGGTCC CCAAATCCAAGCGGATTCAGCTGTACCTGCCCCACAGGCATCAACCTGCTGTCTGAT GGCAAGACCTGCTCACCAG |
| 4038 | LRP4 | HSX2_01040499 | 398 | CCTTCTGGCTTCTCCTGTGCCTGCCCCACTGGCATCCAGCTGAAGGGAGATGGGAA GACCTGTGATCCCTCTCCTGAGACCTACCTGCTCTTCTCCAGCCGTGGCTCCATCCG GCGTATCTCACTGGACACCAGTGACCACACCGATGTGCATGTCCCTGTTCCTGAGCT CAACAATGTCATCTCCCTGGACTATGACAGCGTGGATGGAAAGGTCTATTACACAG ATGTGTTCCTGGATGTTATCAG |
| 4038 | LRP4 | HSX2_01040509 | 399 | GTACCTCTTCTGGACAGACTGGGGCCACATTGCCAAGATCGAACGGGCAAACTTGG ATGGTTCTGAGCGGAAGGTCCTCATCAACACAGACCTGGGTTGGCCCAATGGCCTT ACCCTGGACTATGATACCCGCAG |
| 4038 | LRP4 | HSX2_01040514 | 400 | GATCTACTGGGTGGATGCGCATCTGGACCGGATCGAGAGTGCTGACCTCAATGGG AAACTGCGGCAGGTCTTGGTCAGCCATGTGTCCCACCCCTTTGCCCTCACACAG |
| 4038 | LRP4 | HSX2_01040519 | 401 | CAAGACAGGTGGATCTACTGGACAGACTGGCAGACCAAGTCAATCCAGCGTGTTG ACAAATACTCAGGCCGGAACAAGGAGACAGTGCTGGCAAATGTGGAAGGACTCAT GGATATCATCGTGGTTTCCCCTCAGCGGCAGACAG |
| 4038 | LRP4 | HSX2_01040529 | 402 | TGCCTGGCCTGGTACCACCAGCTCCTAGGGCTACTGGCATGAGTGAAAAGAGCCCA GTGCTACCCAACACACCACCTACCACCTTGTATTCTTCAACCACCCGGACCCGCACG TCTCTGGAGGAGGTGGAAGGAAG |
| 4038 | LRP4 | HSX2_01040534 | 403 | ATGCTCTGAAAGGGATGCCAGGCTGGGCCTCTGTGCACGTTCCAATGACGCTGTTC CTGCTGCTCCAG |
| 4038 | LRP4 | HSX2_01040539 | 404 | GGGAAGGACTTCATATCAGCTACGCCATTGGTGGACTCCTCAGTATTCTGCTGATTT TGGTGGTGATTGCAGCTTTGATGCTGTACAG |
| 4038 | LRP4 | HSX2_01040549 | 405 | GGAGGGCCTGACCATAACTACACCAAGGAGAAGATCAAGATCGTAGAGGGAATCT GCCTCCTGTCTGGGGATGATGCTGAGTGGGATGACCTCAAGCAACTGCGAAGCTCA CGGGGGGGCCTCCTCCGGGATCATGTATGCATGAAGACAGACACGGTGTCCATCC AGGCCAGCTCTGGCTCCCTGGATGACACAGAGACGGAGCAGCTGTTACAGGAAGA GCAGTCTGAGTGTAGCAGCGTCCATACTGCAGCCACTCCAGAAAGACGAGGCTCTC TGCCAGACACGGGCTGGAAACATGAACGCAAGCTCTCCTCAGAGAGCCAGGTCTA AATGCCCACATTCTCTTCCCTGCCTGCCTGTTCCTTCTCCTTTATGGACGTCTAGTCC TTGTGCTCGCTTACACCGCAGGCCCCGCTTCTGTGTGCTTGTCCTCCTCCTCCCA CCCCATAACTGTTCCTAAGCCTTCACCGGAGCTGTTTACCACGTGAGTCCATAACTA CCTGTGCACAAGAAATGATGGCACATCACGAGAATTTAGACCTGGATTTTACCATG AACCTCACATCTTGTACTCCATCCTGGGCCCCCTGAAACTGCTTATTCGTGATTCCTC ACCAGCGTAGAGCTCCACCTCCCCTTTCCCCAGTACCCTCAGTGCCTGCTTCTCAGT GCTGATGCAGCTGATGACCCAGGACTGCGCTCTGCCCCATCACAGCCAGCATGACT GCTTCTCTGAGAGAACTTGCCCATCAGGGGCTGGGACATGGGGGTGTGGGTAAAG ACAGGGATGAAGGATAGAGGCTGAGAGAAGAAGGAAGAATCAGCCCAGCAGGTA TGGGCATCTGGGAAACCTCCAGCCTCAAGTGTGTTAGTAACATGAAAAAGCTTTGG GGGGTAGTTGGATCTGGGTGTCTGGTCCATTGCTGGCAGTGGACATTATTCTTGCC CTAAGAGACACTGCCTTTTCAGCAGCAGATACTGGTGAGATGGGGGTGGCTCAGG CTGTTCTTCCTCCTCCTAGAATGTCTGGAGCTGTTTCTACATTCAGATAACTGGGTCC CCTATCACAAGGCTACTGGCTAATAGGAATTCCCTCCTGGTGCCACCACTGGCCAGT ACCTTTCCTAAGTCTTTGCTCAAATTAACCAGGTTGTGAGCCAGTGGCTTGAGTGAA TGTTAGGCCTTGGGGGCTGAGTCTCTGAAAAGTCTAAGAAGCTCTGCCTAGACCAA ATATGGTATACCTCCTGACCCCTCTCTCCCTCATGTCCTGGGATTCTGGGGAAGAGA CCTAGAAACAAGCTTTCAAAGAAAAACCAGAAGTTGTCATAAATGGTCAGAAAGAA CGATCAGGTTGGAGACTTGGGAAACCCAGGGCCTAAAGAGAAGTATCCATGAGGG TCAAACTTCCTGTTGAACTTCCTATGTTCTTTCTCAAGTGCTCAGGGATCTAAGTTAG TGGACAGCAAGCCTGTGGCTACGGGGTGGTGATGTTCCTCTTCCAGCTGTCCCCTC AGCTAAGGGGCTTAGTTTCCATGTGGGATGCCATCACTTGGTTCATGCTCATTCACA CAAAGGGCACGTGTCTCAGCCTGGTATCAGGGAAATTGAGACTTATTTTTGCCCTA AAACGTCTCCCTAGCTGTTCTTCGTGGGGTTTTTTTGTTTGTTTTTTTGCCTAATTTG CTTTTTCTGACCAAGCCTTGTGGCACCAGCAATCTCCAAAGTCCTGTGGTGGGAGG GCTGAATAAATAAAAATACAAAGAGGTGGGTAAGGAGTAGGAAGGTAGAGAGCA CCACTGATGAGGCCCTCCTAGCCCATGGCAGACCCAGACCTCTTCTCCCCCAGGAAT TAGAAGTGGCAGGAGAGAACAACAGGGGCTGGGAATGGAGGGGAGAATTTCTAG GGGAAGTTTCCTGAGTTGAAACTTCTCCTGTGGTTACTGGTATTGAGAAATCAGCT |
| 4038 | LRP4 | HSX2_01040550 | 406 | ACTATGTTCAGTATTTTAGGACTTTATGATAATTTAATATAAATTTAGCTTTTCTTAA TCA |
| 11216 | LRRC17 | HSX2_01559349 | 407 | ACAAGTCTTGCACGACACTATTGTCCTCCCTTACTTCATTCAATTCATGGAACTTCGG CGAATGGAGCATTTGGTGAAATTTTGGTTAGAGGCTGAAAGTTTTCATTCAACAAC TTGGTCGCGAATAAGAGCACACAGTCTAAACACAGTGAAGCAGAGCTCACTGGCT GAGCCTGTCTCTCCATCTAAAAAGCATGAAACTACAGCGTCTTTTTAACTGATTCTC TTGATAAGAGATTGGAGGATTCTGGCTCAGCACAGTTGTTTATGACTCATTCAGAA GGAATTGACCTGAATAATAGAACTAACAGCACTCAGAATCACTTGCTGCTTTCCCAG GAATGTGACAGTGCCCATTCTCTCCGTCTTGAAATGGCCAGAGCAGGAACTCACCA AGTTTCCATGGAAACCCAAGAATCTTCCTCTACACTTACAGTAGCCAGTAGAAATAG TCCCGCTTCTCCACTAAAAGAATTGTCAGGAAAACTAATGAAAA |
| 115353 | LRRC42 | HSX2_02172282 | 408 | TGACAGCACCAGTTCGAGTGATGAAAAGAGGCCTTGAGAATCTAACATTATTAGAC TTATCAT |
| 120892 | LRRK2 | HSX2_02185024 | 409 | TTCCCTGAGCAGCGGACGTTCATGCTGGGAGGGCGGCGGGTTGGAAGCAGGTGCC ACCATGGCTAGTGGCAGCTGTCAGGGGTGCGAAGAGGACGAGGAAACTCTGAAG AAGTTGATAGTCAGGCTGAACAATGTCCAGGAAGGAAAACAGATAGAAACGCTGG TCCAAATCCTGGAGGATCTGCTGGTGTTCACGTACTCCGAGCGCG |
| 120892 | LRRK2 | HSX2_02185048 | 410 | GTGGGTTGGTCACTTCTGTGCAAATTAATAGAAGTCTGTCCAGGTACAATGCAAAG CTTAATGGGACCCCAGGATGTTGGAAATGATTGGGAAGTCCTTGGTGTTCACCA |
| 120892 | LRRK2 | HSX2_02185064 | 411 | ATTGATTCTTAAAATGCTAACAGTTCATAATGCCAGTGTAAACTTGTCAGTGATTGG ACTGAAGACCTTAGATCTCCTCCTAACTTCAG |
| 120892 | LRRK2 | HSX2_02185088 | 412 | TCTCAGAGGAGCAACTGACTGAATTTGTTGAGAACAAAGATTATATGATATTGTTA AGTGCGTTAACAAATTTTAAAGATGAAGAGGAAATTGTGCTTCATGTGCTGCATTG TTTACATTCCCTAGCGATTCCTT |
| 120892 | LRRK2 | HSX2_02185098 | 413 | GCAATAATGTGGAAGTCCTCATGAGTGGCAATGTCAGGTGTTATAATATTGTGGTG GAAGCTATGAAAGCATTCCCTATGAGTGAAAGAATTCAAGAAGTGAGTTGCTGTTT GCTCCATAGGCTTACATTAG |
| 120892 | LRRK2 | HSX2_02185108 | 414 | TGCAGCAGTACCCAGAGAATGCAGCATTGCAGATCTCAGCGCTCAGCTGTTTGGCC CTCCTCA |
| 120892 | LRRK2 | HSX2_02185118 | 415 | CTGAGACTATTTCTTAAATCAAGATTTAGAGGAAAAGAATGAGAATCAAGAGAAT GATGATGAGGGGGAAGAAGATAAATTGTTTTGGCTGGAAGCCTGTTACAAAGCAT TAACGTGGCATAGAAAGAACAAGCACGTGCAG |
| 120892 | LRRK2 | HSX2_02185128 | 416 | GAGGCCGCATGCTGGGCACTAAATAATCTCCTTATGTACCAAAACAGTTTACATGA GAAGATTGGAGATGAAGATGGCCA |
| 120892 | LRRK2 | HSX2_02185137 | 417 | TTTCCCAGCTCATAGGGAAGTGATGCTCTCCATGCTGATGCATTCTTCATCAAAG |
| 120892 | LRRK2 | HSX2_02185139 | 418 | GAAGTTTTCCAGGCATCTGCGAATGCATTGTCAACTCTCTTAGAACAAAATG |
| 120892 | LRRK2 | HSX2_02185153 | 419 | TTAATTTCAGAAAAATACTGTTATCAAAAGGAATACACCTGAATGTTTTGGAGTTAA TGCAGAAGCATATACATTCTCCTGAAGTGGCTGAAAGTGGCTGTAAAATGCTAAAT CATCTTTTTGAAGGAAG |
| 120892 | LRRK2 | HSX2 02185176 | 420 | AAAAAACAGTGTTTCAAGAATGATATTCACAAACTGGTCCTAGCAGCTTTGAACAG G |
| 120892 | LRRK2 | HSX2_02185188 | 421 | TTCATTGGAAATCCTGGGATTCAGAAATGTGGATTAAAAGTAATTTCTTCTATTGTA CATTTTCCTGATGCATTAGAGATGTTATCCCTGGAAGGTGCTATGGATTCAGTGCTT CACACACTGCAGATGTATCCAGATGACCAAG |
| 120892 | LRRK2 | HSX2_02185200 | 422 | AAATTCAGTGTCTGGGTTTAAGTCTTATAGGATACTTGATTACAAAGAAGAATGTG TTCATAGGAACTGGACATCTGCTGGCAAAAATTCTGGTTTCCAGCTTATACCGATTT AAGGATGTTGCTGAAATACAGACTAAA |
| 120892 | LRRK2 | HSX2_02185226 | 423 | AATGTGATGCTAGAGAGAGCGTGTGATCAGAATAACAGCATCATGGTTGAATGCTT GCTTCTATTGGGAGCAGATGCCAATCAAGCAAAGGAGGGATCTTCTTTAATTTGTC AG |
| 120892 | LRRK2 | HSX2_02185236 | 424 | GTATGTGAGAAAGAGAGCAGTCCCAAATTGGTGGAACTCTTACTGAATAGTGGATC TCGTGAACAAGATGTACGAAAAGCGTTGACGATAAGCATTGGGAAAGGTGACAGC CAGATCATCAGCTTGCTCTTAAGGAGGCTGGCCCTGGATGTGGCCAACAATAGCAT TTGCCTTGGAGGATTTTGTATAGGAAAAGTTGAACCTTCTTGGCTTGGTCCTTTATT TCCAGATAAGACTTCTAATTTAAGGAAACAAACAA |
| 120892 | LRRK2 | HSX2 02185241 | 425 | GAAGAAGGAACAGCCTCAGGCAGCGATGGAAATTTTTCTGAAGATGTGCTGTCTAA ATTTGATGAATGGACCTTTATTCCTGACTCTTCTATGGACAGTGTGTTTGCTCAAAG TGATGACCTGGATAGTGAAG |
| 120892 | LRRK2 | HSX2_02185269 | 426 | GGTCATCAAAACTTCAATCCCATATGAGGCATTCAGACAGCATTTCTTCTCTGGCTT CTGAGAGAGAATATATTACATCACTAGACCTTTCAGCAAATGAACTAAGAGATATT GATGCCCTAAGCCAGAAATGCTGTATAAGTGTTCATTTGGAGCATCTTGAAAAGCT GGAGCTTCACCAGAATGCACTCACGAGCTTTCCACAACAGCTATGTGAA |
| 120892 | LRRK2 | HSX2_02185278 | 427 | CTTATTGTTGAAAATGAGTTGTATTGCTAATCTTGATGTCTCTCGAAATGACATTG GACCCTCAGTGGTTTTAGATCCTACAGTGAAATGTCCAACTCTGAAACAGTTTAACC TGTCATATAACCAGCTGTCTTTTGTACCTGAGAACCTCACTGATGTGGTAGAGAAAC TGGAGCAGCTCATTTTAGAAGG |
| 120892 | LRRK2 | HSX2_02185287 | 428 | AAATAAAATATCAGGGATATGCTCCCCCTTGAGACTGAAGGAACTGAAGATTTTAA ACCTTAGTAAGAACCACATTTCATCCCTATCAGAGAACTTTCTTGAGGCTTGTCCTA AAGTGGAGAGTTTCAGTGCCAGAATGAATTTTCTTG |
| 120892 | LRRK2 | HSX2_02185299 | 429 | CTGCTATGCCTTTCTTGCCTCCTTCTATGACAATCCTAAAATTATCTCAGAACAAATT TTCCTGTATTCCAGAAGCAATTTTAAATCTTCCACA |
| 120892 | LRRK2 | HSX2_02185363 | 430 | TTGCTGTCTATGACCTCAGCAAGGGACAGGCTGAAGTTGATGCCATGAAGCCTTGG CTCTTCAATATAAAG |
| 120892 | LRRK2 | HSX2_02185381 | 431 | GCTCGCGCTTCTTCTTCCCCTGTGATTCTCGTTGGCACACATTTGGATGTTTCTGATG AGAAGCAACGCAAAGCCTGCATGAGTAAAATCACCAAGGAACTCCTGAATAAGCG AGGGTTCCCTGCCATACGAGATTACCACTTTGTGAATGCCACCGAGGAATCTGATG CTTTGGCAAAACTTCGGAAAACCATCATAAACGAGAGCCTTAATTTCAAG |
| 120892 | LRRK2 | HSX2_02185395 | 432 | ATCCGAGATCAGCTTGTTGTTGGACAGCTGATTCCAGACTGCTATGTAGAACTTGA AAAAATCATTTTATCGGAGCGTAAAAATGTGCCAATTGAATTTCCCGTAATTGACCG GAAACGATTATTACAACTAGTGAGAGAAAATCAGCTGCAGTTAGATGAAAATGAGC TTCCTCACGCAGTTCACTTTCTAAATGAATCAG |
| 120892 | LRRK2 | HSX2_02185411 | 433 | GAGTCCTTCTTCATTTTCAAGACCCAGCACTGCAGTTAAGTGACTTGTACTTTGTGG AACCCAAGTGGCTTTGTAAAATCATGGCACAG |
| 120892 | LRRK2 | HSX2_02185433 | 434 | TTTGTCTGACCACAGGCCTGTGATAGAGCTTCCCCATTGTGAGAACTCTGAAATTAT CATCCGACTATATGAAATGCCTTATTTTCCAATGGGATTTTGGTCAAGATTAATCAA TCGATTACTTGAGATTTCACCTTACATGCTTTCAGGGAGAG |
| 120892 | LRRK2 | HSX2_02185443 | 435 | AACGAGCACTTCGCCCAAACAGAATGTATTGGCGACAAGGCATTTACTTAAATTGG TCTCCTGAAGCTTATTGTCTGGTAGGATCTGAAGTCTTAGACAATCATCCAGAGAGT TTCTTAAAAATTACAGTTCCTTCTTGTAGAAAAG |
| 120892 | LRRK2 | HSX2_02185455 | 436 | TTCCTGGGTTGCTGGAGATTGATATTTGTGGTGAAGGAGAAACTCTGTTGAAGAAA TGGGCATTATATAGTTTTAATGATGGTGAAGAACATCAAAAAATCTTACTTGATGAC TTGATGAAGAAAGCAGAGGAAG |
| 120892 | LRRK2 | HSX2_02185470 | 437 | GAGATCTCTTAGTAAATCCAGATCAACCAAGGCTCACCATTCCAATATCTCAGATTG CCCCTGACTTGATTTTGGCTGACCTGCCTAGAAATATTATGTTGAATAATGATGAGT TGGAATTTGAACAAGCTCCAGAGTTTCTCCTAG |
| 120892 | LRRK2 | HSX2_02185480 | 438 | GTGATGGCAGTTTTGGATCAGTTTACCGAGCAGCCTATGAAGGAGAAGAAGTGGC TGTGAAGATTTTTAATAAACATACATCACTCAGGCTGTTAAGACAA |
| 120892 | LRRK2 | HSX2_02185490 | 439 | GGGATTCGTCCCCGGATGTTGGTGATGGAGTTAGCCTCCAAGGGTTCCTTGGATCG CCTGCTTCAGCAGGACAAAGCCAGCCTCACTAGAACCCTACAGCACAGGATTGCAC TCCACGTAGCTGATGGTTTGAG |
| 120892 | LRRK2 | HSX2_02185500 | 440 | ATACCTCCACTCAGCCATGATTATATACCGAGACCTGAAACCCCACAATGTGCTGCT TTTCACACTGTATCCCAATGCTGCCATCATTGCAAAGATTGCTGACTACGGCATTGC TCAGTACTGCTGTAGAATGGGGATAAAAACATCAGAGGGCACACCAG |
| 120892 | LRRK2 | HSX2_02185515 | 441 | GTTTATTCATTTGGTTTACTACTCTATGACATTTTGACAACTGGAGGTAGAATAGTA GAGGGTTTGAAGTTTCCAAATGAGTTTGATGAATTAGAAATACAAGGAAAATTACC TG |
| 120892 | LRRK2 | HSX2_02185530 | 442 | ATCCAGTTAAAGAATATGGTTGTGCCCCATGGCCTATGGTTGAGAAATTAATTAAA CAGTGTTTGAAAGAAAATCCTCAAGAAAGGCCTACTTCTGCCCAG |
| 120892 | LRRK2 | HSX2_02185541 | 443 | GTCTTTGACATTTTGAATTCAGCTGAATTAGTCTGTCTGACGAGACGCATTTTATTA CCTAAAAACGTAATTGTTGAATGCATGGTTGCTACACATCACAACAGCAGGAATGC AAGCATTTGGCTGGGCTGTGGGCACACCGACAGAGGACAGCTCTCATTTCTTGACT TAAATACTGAAGGATACACTTCTGAG |
| 120892 | LRRK2 | HSX2_02185551 | 444 | GAAAGCTGGATTGTGTCTGGGACACAGTCTGGTACTCTCCTGGTCATCAATACCGA AGATGGGAAAAAGAGACATACCCTAGAAAAGATGACTGATTCTGTCACTTGTTTGT ATTGCAATTCCTTTTCCAAGCAAAG |
| 120892 | LRRK2 | HSX2_02185560 | 445 | CAAACAAAAAAATTTTCTTTTGGTTGGAACCGCTGATGGCAAGTTAGCAATTTTTGA AGATAAGACTGTTAAG |
| 120892 | LRRK2 | HSX2_02185571 | 446 | ATGTGTTTGAGTGAATCCACAAATTCAACGGAAAGAAATGTAATGTGGGGAGGAT GTGGCACAAAGATTTTCTCCTTTTCTAATGATTTCACCATTCAGAAACTCATTGAGAC AAGAACAAGCCAACT |
| 120892 | LRRK2 | HSX2_02185581 | 447 | GTTTTCTTATGCAGCTTTCAGTGATTCCAACATCATAACAGTGGTGGTAGACACTGC TCTCTATATTGCTAAGCAAAATAGCCCTGTTGTGGAAGTGTGGGATAAGAAAACTG AAAAACTCTGTGGACTAATAGACTGCGTGCACTTTTTAAG |
| 120892 | LRRK2 | HSX2_02185595 | 448 | GGAGGTAATGGTAAAAGAAAACAAGGAATCAAAACACAAAATGTCTTATTCTGGG AGAGTGAAAACCCTCTGCCTTCAGAAGAACACTGCTCTTTGGATAGGAACTGGAGG AGGCCATATTTTACTCCTGGATCTTTCAACTCGTCGACTTATACGTGTAATTTACAAC TTTTGTAATTCGGTCAGAGTCATGATGACAGCACAGCTAG |
| 120892 | LRRK2 | HSX2_02185609 | 449 | GAAGCCTTAAAAATGTCATGCTGGTATTGGGCTACAACCGGAAAAATACTGAAGGT ACACAAAAGCAGAAAG |
| 120892 | LRRK2 | HSX2_02185619 | 450 | AGATACAATCTTGCTTGACCGTTTGGGACATCAATCTTCCACATGAAGTGCAAAATT TAGAAAAACACATTGAAGTGAGAAAAGAATTAGCTGAAAAAATGAGACGAACATC TGTTGAGTAAGAGAGAAATAGGAATTGTCTTTGGATAGGAAAATTATTCTCTCCTCT TGTAAATATTTATTTTAAAAATGTTCACATGGAAAGGGTACTCACA |
| 120892 | LRRK2 | HSX2_02185621 | 451 | TAAAAATACTTACCAGTAAATGTGTATTTTAAAGAACTATTTAAAACACAATGTTAT ATTTCTTATAAATACCAGTTACTTTCGTTCATTAATTAATGAAAATAAATCTGTGAAG TACCTAATTTAAGTACTCATACTAAAATTTATAAGGCCGATAATTTTTTGTTTTCTTG TCTGTAATGGAGGTAAACTTTATTTTAAATTCTGTGCTTAAGACAGGACTATTGCTT GTCGATTTTTCTAGAAATCTGCACGGTATAATGAAAATATTAAGACAGTTTCCCATG TAATGTATTCCTTCTTAGATTGCATCGAAATGCACTATCATATATGCTTGTAAATATT CAAATGAATTTGCACTAATAAAGTCCTTTGTTGGTATGTGAATTCTCTTTGTTGCTGT TGCAAACAGTGCATCTTACACAACTTCACTCAATTCAAAAGAAAACTCCATTAAAAG TACTAATGAAAAAACATGACATACTGTCAAAGTCCTCATATCTAGGAAAGACACAG AAACTCTCTTTGTCACAGAAACTCTCTGTGTCTTTCCTAGACATAATAGAGTTGTTTT TCAACTCTATGTTTGAATGTGGATACCCTGAATTTTGTATAATTAGTGTAAATACAG TGTTCAGTCCTTCAAGTGATATTTTTATTTTTTTATTCATACCACTAGCTACTTGTTTT CTAATCTGCTTCATTCTAATGCTTATATTCATCTTTTCCCTAAATTTGTGATGCTGCA GATCCTACATCATTCAGATAGAAACCTTTTTTTTTTTCAGAATTATAGAATTCCACAG CTCCTACCAAGACCATGAGGATAAATATCTAACACTTTTCAGTTGCTGAAGGAGAA AGGAGCTTTAGTTATGATGGATAAAAATATCTGCCACCCTAGGCTTCCAAATTATAC TTAAATTGTTTACATAGCTTACCACAATAGGAGTATCAGGGCCAAATACCTATGTAA TAATTTGAGGTCATTTCTGCTTTAGGAAAAGTACTTTCGGTAAATTCTTTGGCCCTG ACCAGTATTCATTATTTCAGATAATTCCCTGTGATAGGACAACTAGTACATTTAATAT TCTCAGAACTTATGGCATTTTACTATGTGAAAACTTTAAATTTATTTATATTAAGGGT AATCAAATTCTTAAAGATGAAAGATTTTCTGTATTTTAAAGGAAGCTATGCTTTAAC TTGTTATGTAATTAACAAAAAAATCATATATAATAGAGCTCTTTGTTCCAGTGTTATC |
| 120892 | LRRK2 | HSX2_02576451 | 452 | TTTCCCAGCTCATAGGGAAGTGATGCTCTCCATGCTGATGCATTCTTCATCAAAGGA AGTTTTCCAGGCATCTGCGAATGCATTGTCAACTCTCTTAGAACAAAATG |
| 4050 | LTB | HSX2_01041650 | 453 | GGGTTCCCTCCTGCTAGCTGTGGCAGGAGCCACTTCTCTGGTGACCTTGTTGCTGG CGGTGCCTATCACTGTCCTGGCTGTGCTGGCCTTAGTGCCCCAGGATCAGGGAGGA CTG |
| 23499 | MACF1 | HSX2_01641182 | 454 | GATGAAAAGGAGCAGCTTATACAGTCCAAGAGTTCCGTTGCCAGTCTCGTTGGGAG ATCAAAAACCATCGTTCAGCTAAAACCACGCAGTCCAGACCATGTGTTAAAGAACA CCATTTCTGTCAAGGCTGTCTGTGACTACAGGCAGATCGAG |
| 23499 | MACF1 | HSX2_01641195 | 455 | ATTACTATTTGCAAAAATGATGAATGTGTGCTAGAAGATAATTCTCAGCGGACCAA ATGGAAAGTGATCAGCCCCACAGGGAACGAGGCAATGGTGCCGTCAGTCTGCTTC CTCATCCCCCCACCCAATAAGGATGCCATTGAGATGGCCAGCAG |
| 23499 | MACF1 | HSX2_01641210 | 456 | CCAAAAGCCTTATCTCTTGGAACTATCTGCGTAAAGACCTTGACCTTGTACAGACCT GGAACCTAGAAAAG |
| 9223 | MAGI1 | HSX2_01392893 | 457 | CTATGTCACCTTCACCTGCGTCGGGATTGAGCAAGGGTGAGAGAGAGAGAGAAAT CAATTCCACGAATTTTGGAGAATGTCCGA |
| 4124 | MAN2A1 | HSX2_01046549 | 458 | ATGTTGGATGTTTACAGTCTAATTTCTTTTGACAATCCAGATGGTGGAGTTTGGAAG CAAGGATTTGACATTACTTATGAATCTAATGAATGGGACACTGAACCCCTTCAAGTC TTTGTGGTGCCTCATTCCCATAACGACCCAG |
| 149175 | MANEAL | HSX2_02487371 | 459 | ATCAGTACAGCATCCAGGTGGCCTTCCACATCCA |
| 4128 | MAOA | HSX2_01047014 | 460 | ATTCCAACTGATGCACCCTGGGAGGCTCAACATGCTGACAAATGGGACAAAATGAC CATGAAAGAGCTCATTGACAAAATCTGCTGGACAAA |
| 4128 | MAOA | HSX2_01047060 | 461 | AGTGGAGCGGCTACATGGAAGGGGCAGTTGAGGCTGGAGAACGAGCAGCTAGGG AG |
| 4128 | MAOA | HSX2_01047065 | 462 | AATCAAAG |
| 4128 | MAOA | HSX2_02507973 | 463 | AT |
| 4133 | MAP2 | HSX2_01047357 | 464 | ATTAAAAG |
| 4133 | MAP2 | HSX2_01047371 | 465 | AAAGGCACCTCACTGGACCTCAGCACCGCTAACAGAGGCATCTGCACACTCACATC CACCTGAGATTAAGGATCAAGGCGGAGCAGGGGAAGGACTTGTCCGAAGCGCCAA TGGATTCCCATACAGGGAGGATGAAGAGGGTGCCTTTGGAGAGCATGGGTCACAG GGCACCTATTCAAATACCAAAGAGAATGGGATCAACGGAGAGCTGACCTCAGCTG ACAGAGAAACAGCAG |
| 4133 | MAP2 | HSX2_01047388 | 466 | AGTCTCT |
| 4133 | MAP2 | HSX2_01047403 | 467 | GGTCAGAGCCAATTCGCAGAGCAGGGAAGAGTGGTACCTCAACACCCACTACCCCT GGGTCTACTGCCATCACTCCTGGCACCCCACCAAGTTATTCTTCAC |
| 4133 | MAP2 | HSX2_01047404 | 468 | ACCCCCAAGTCTGCCATCTTGGTGCCGAGTGAGAAGAAGGTCGCCATCATACGTAC TCCTCCAAAATCTCCTGCGACTCCCAAGCAGCTTCGGCTTATTAACCAACCACTGCC AGACCTGAAGAATGTCAAATCCAAAATCGGATCAACAGACAACATCAAATACCAGC CTAAAGGGGGGCAG |
| 4133 | MAP2 | HSX2_01047415 | 469 | GTACAAATTGTTACCAAGAAAATAGACCTAAGCCATGTGACATCCAAATGTGGCTC TCTGAAGAACATCCGCCACAGGCCAG |
| 4133 | MAP2 | HSX2_01047421 | 470 | GCGGACGTGTGAAAATTGAGAGTGTAAAACTAGATTTCAAAGAAAAGGCCCAAGC TAAAGTTGGTTCTCTTGATAATGCTCATCATGTACCTGGAGGTGGTAATGTCAAG |
| 4133 | MAP2 | HSX2_01047427 | 471 | ATTGACAGCCAAAAGTTGAACTTCAGAGAGCATGCTAAAGCCCGTGTGGACCATGG GGCTGAGATCATTACACAGTCCCCAGGCAGATCCAGCGTGGCATCACCCCGACGAC TCAGCAATGTCTCCTCGTCTGGAAGCATCAACCTGCTCGAATCTCCTCAGCTTGCCA CTTTGGCTGAGGATGTCACTGCTGCACTCGCTAAGCAGGGCTTGTGAATATTTCTCA TTTAGCATTGAAATAATAATATTTAGGCATGAGCTCTTGGCAGGAGTGGGCTCTGA |
| 4133 | MAP2 | HSX2_02508006 | 472 | CAGCTGAAGAAACAGCTAATCTGCCTCCTTCTCCACCCCCATCACCTGCCTCAGAAC AGACTGTCACAGTGGAGGAAG |
| 4133 | MAP2 | HSX2_02508011 | 473 | GGGTCTACTGCCATCACTCCTGGCACCCCACCAAGTTATTCTTCACGCACACCAGGC ACTCCTGGAACCCCTAGCTATCCCAGGACCCCTCACACACCAGGAACCCCCAAGTCT GCCATCTTGGTGCCGAGTGAGAAGAAGGTCGCCATCATACGTACTCCTCCAAAATC TCCTGCGACTCCCAAGCAGCTTCGGCTTATTAACCAACCACTGCCAGACCTGAAGAA TGTCAAATCCAAAATCGGATCAACAGACAACATCAAATACCAGCCTAAAGGGGGGC AG |
| 6441 | MAP4K5 | HSX2_01215290 | 474 | TTTGTAAAACCATTTACGGAGGCACAGCTGCTGTGCACACAGGCTGGTGGACAGTT GGCCTCTCCACGCTCTGCCGCTGAGAATGCCGCCTTGCAACAGCTGGTCGTAGCTA AGAACGAGGCTGCTTTCCTGAGCATGACTGATTCCAAGACAGAGGGCAAGTTCACC TACCCCACAGGAGAGTCCCTGGTCTATTCCAACTGGGCCCCAGGGGAGCCCAACGA TGATGGCGGGTCAGAGGACTGTGTGGAGATCTTCACCAATGGCAAGTGGAATGAC AGGGCTTGTGGAGAAAAGCGTCTTGTGGTCTGCGAGTTCTGAGCCAACTGGGGTG GGTGGGGCAGTGCTTGGCCCAGGAGTTTGGCCAGAAGTCAAGGCTTAGACCCTCA TGCTGCCAATATCCTAATAAAAAGGTGACCAT |
| 79143 | MBOAT7 | HSX2_02566141 | 475 | CGTGGGAATCATGACAGCCCGACGTGCCCTCCCT |
| 64769 | MEAF6 | HSX2_01981938 | 476 | TTGATCTGAAGTTAAACAAAAAACCACGA |
| 64769 | MEAF6 | HSX2_01981953 | 477 | AATAAAAACCGGCACAGGATTGATCTGAAGTTAA |
| 64769 | MEAF6 | HSX2_02564579 | 478 | GATTGATCTGAAGTTAAACAAAAAACCACGAGCT |
| 9969 | MED13 | HSX2_01458570 | 479 | GAGGAAGTATTCTGCTTCATCAGGTGGTCTATGCGAAGAAGCGACAGCTGCTAAA GTGGCATCCTGGGATTTTGTTGAAGCCACACAAAGAACAAATTGCAGTTGTTTGAG |
| 9969 | MED13 | HSX2_01458575 | 480 | GTCAGCAACAACAAATACTTCCTAAGCACAAGACCAATGAGAAGCAAGAAAAGAG TGAAAAGCCACAGAAACGCCCCTTGACTCCTTTTCACCATCGTGTGTCTGTTAGTGA TGATGTTGGCATGGACGCAGATTCAGCCAGCCAAAGACTTGTGATCTCTGCTCCAG ACAGTCAAGTGAGATTTTCAAATATCCGAACTAATGATGTAGCAAAGACTCCTCAG ATGCATGGCACCGAAATGGCAAATTCACCTCAACCACCCCCACTTAGTCCTCACCCT TGTGATGTGGTTGATGAAGGAGTGACTAAAACACCTTCAACTCCTCAGAGTCAACA TTTTTATCAAATGCCAACACCAGATCCCTTGGTTCCTTCTAAACCAATGGAAGATAG GATAGACAGTTTGTCCCAGTCTTTCCCACCTCAATATCAGGAAGCTGTAGAACCTAC AGTATATGTTGGTACAGCAGTAAACTTGGAAGAAGATGAAGCCAATATAGCCTGG AAGTATTACAAGTTCCCAAAGAAAAAAGATGTAGAGTTTTTACCACCTCAACTTCCA AGTGATAAATTCAAGGATGATCCAGTTGGACCTTTTGGACAGGAAAGTGTAACATC |
| 9969 | MED13 | HSX2_01458679 | 481 | GCCTCTTTGCACCTCCACGTGCCTTCAGTGCAATCTGACGAGCTGCTTCACAGTAAA CACTCCCACCCACTTGACTCAAATCAGACTTCAGATGTCCTCAG |
| 9969 | MED13 | HSX2_01458684 | 482 | GGACAGACGCTCATGTCTCCCAATTCATTTTGTGGTGCTGAATCAGTTATATAACTT TATTATGAATATGCTGTGATCTTCATTTGATGGAACTGTGCAAGAAAAGAACAAGG AAAAATGGATGTTTCGCTGCAGGAT |
| 4233 | MET | HSX2_01055592 | 483 | TACCTTGGTGCAGAGGAGCAATGGGGAGTGTAAAGAGGCACTAGCAAAGTCCGAG ATGAATGTGAATATGAAGTATCAGCTTCCCAACTTCACCGCGGAAACACCCATCCA GAATGTCATTCTACATGAGCATCACATTTTCCTTGGTGCCACTAACTACATTTATGTT TTAAATGAGGAAGACCTTCAGAAGGTTGCTGAGTACAAGACTGGGCCTGTGCTGG AACACCCAGATTGTTTCCCATGTCAGGACTGCAGCAGCAAAGCCAATTTATCAGGA GGTGTTTGGAAAGATAACATCAACATGGCTCTAGTTGTCGACACCTACTATGATGA TCAACTCATTAGCTGTGGCAGCGTCAACAGAGGGACCTGCCAGCGACATGTCTTTC CCCACAATCATACTGCTGACATACAGTCGGAGGTTCACTGCATATTCTCCCCACAGA TAGAAGAGCCCAGCCAGTGTCCTGACTGTGTGGTGAGCGCCCTGGGAGCCAAAGT CCTTTCATCTGTAAAGGACCGGTTCATCAACTTCTTTGTAGGCAATACCATAAATTCT TCTTATTTCCCAGATCATCCATTGCATTCGATATCAGTGAGAAGGCTAAAGGAAACG AAAGATGGTTTTATGTTTTTGACGGACCAGTCCTACATTGATGTTTTACCTGAGTTC AGAGATTCTTACCCCATTAAGTATGTCCATGCCTTTGAAAGCAACAATTTTATTTACT TCTTGACGGTCCAAAGGGAAACTCTAGATGCTCAGACTTTTCACACAAGAATAATC AGGTTCTGTTCCATAAACTCTGGATTGCATTCCTACATGGAAATGCCTCTGGAGTGT ATTCTCACAGAAAAGAGAAAAAAGAGATCCACAAAGAAGGAAGTGTTTAATATACT TCAGGCTGCGTATGTCAGCAAGCCTGGGGCCCAGCTTGCTAGACAAATAGGAGCC AGCCTGAATGATGACATTCTTTTCGGGGTGTTCGCACAAAGCAAGCCAGATTCTGC CGAACCAATGGATCGATCTGCCATGTGTGCATTCCCTATCAAATATGTCAACGACTT |
| 4233 | MET | HSX2_01055597 | 484 | GTTTACCACAGCTTTGCAGCGCGTTGACTTATTCATGGGTCAATTCAGCGAAGTCCT CTTAACATCTATATCCACCTTCATTAAAGGAGACCTCACCATAGCTAATCTTGGGAC ATCAGAGGGTCGCTTCATGCAG |
| 4233 | MET | HSX2_01055603 | 485 | GGTTTCTCGATCAGGACCATCAACCCCTCATGTGAATTTTCTCCTGGACTCCCATCC AGTGTCTCCAGAAGTGATTGTGGAGCATACATTAAACCAAAATGGCTACACACTGG TTATCACTGGGAAGAAG |
| 4233 | MET | HSX2_01055610 | 486 | TGTGGCTGGTGCCACGACAAATGTGTGCGATCGGAGGAATGCCTGAGCGGACAT GGACTCAACAGATCTGTCTGCCTGCAATCTACAAG |
| 4233 | MET | HSX2_01055633 | 487 | TGTGTCAAACAGTATTCTTGAATGTTATACCCCAGCCCAAACCATTTCAACTGAGTT TGCTGTTAAATTGAAAATTGACTTAGCCAACCGAGAGACAAGCATCTTCAGTTACC GTGAAGATCCCATTGTCTATGAAATTCATCCAACCAAATCTTTTATTAG |
| 4233 | MET | HSX2_01055665 | 488 | CAGACATGTCCCCCATCCTAACTAGTGGGGACTCTGATATATCCAGTCCATTACTGC AAAATACTGTCCACATTGACCTCAGTGCTCTAAATCCAGAGCTGGTCCAGGCAGTG CAGCATGTAGTGATTGGGCCCAGTAGCCTGATTGTGCATTTCAATGAAGTCATAGG AAGAG |
| 4233 | MET | HSX2_01055670 | 489 | GGCATTTTGGTTGTGTATATCATGGGACTTTGTTGGACAATGATGGCAAGAAAATT CACTGTGCTGTGAAATCCTTGAACA |
| 4233 | MET | HSX2_01055680 | 490 | AATCCAACTGTAAAAGATCTTATTGGCTTTGGTCTTCAAGTAGCCAAAGGCATGAA ATATCTTGCAAGCAAAAAGTTTGTCCACAGAGACTTGGCTGCAAGAAACTGTAT |
| 4233 | MET | HSX2_01055701 | 491 | TGAACTGGTGTCCCGGATATCAGCGATCTTCTCTACTTTCATTGGGGAGCACTATGT CCATGTGAACGCTACTTATGTGAACGTAAAATGTGTCGCTCCGTATCCTTCTCTGTT GTCATCAGAAGATAACGCTGATGATGAGGTGGACACACGACCAGCCTCCTTCTGGG AGACATCATAGTGCTAGTACTATGTCAAAGCAACAGTCCACACTTTGTCCAATGGTT TTTTCACTGCCTGACCTTTAAAAGGCCATCGATATTCTTTGCTCTTGCCAAAATTGCA CTATTATAGGACTTGTATTGTTATTTAAATTACTGGATTCTAAGGAATTTCTTATCTG ACAGAGCATCAGAACCAGAGGCTTGGTCCCACAGGCCACGGACCAATGGCCTGCA GCCGTGACAACACTCCTGTCATATTGGAGT |
| 4233 | MET | HSX2_01055719 | 492 | ACCAAATCTTTTATTAGTGGTGGGAGCACAATAA |
| 4233 | MET | HSX2_02508602 | 493 | TGTTTACCTTGGTGCAGAGGAGCAATGGGGAGTGTAAAGAGGCACTAGCAAAGTC CGAGATGAATGTGAATATGAAGTATCAGCTTCCCAACTTCACCGCGGAAACACCCA TCCAGAATGTCATTCTACATGAGCATCACATTTTCCTTGGTGCCACTAACTACATTTA TGTTTTAAATGAGGAAGACCTTCAGAAGGTTGCTGAGTACAAGACTGGGCCTGTGC TGGAACACCCAGATTGTTTCCCATGTCAGGACTGCAGCAGCAAAGCCAATTTATCA GGAGGTGTTTGGAAAGATAACATCAACATGGCTCTAGTTGTCGACACCTACTATGA TGATCAACTCATTAGCTGTGGCAGCGTCAACAGAGGGACCTGCCAGCGACATGTCT TTCCCCACAATCATACTGCTGACATACAGTCGGAGGTTCACTGCATATTCTCCCCAC AGATAGAAGAGCCCAGCCAGTGTCCTGACTGTGTGGTGAGCGCCCTGGGAGCCAA AGTCCTTTCATCTGTAAAGGACCGGTTCATCAACTTCTTTGTAGGCAATACCATAAA TTCTTCTTATTTCCCAGATCATCCATTGCATTCGATATCAGTGAGAAGGCTAAAGGA AACGAAAGATGGTTTTATGTTTTTGACGGACCAGTCCTACATTGATGTTTTACCTGA GTTCAGAGATTCTTACCCCATTAAGTATGTCCATGCCTTTGAAAGCAACAATTTTATT TACTTCTTGACGGTCCAAAGGGAAACTCTAGATGCTCAGACTTTTCACACAAGAATA ATCAGGTTCTGTTCCATAAACTCTGGATTGCATTCCTACATGGAAATGCCTCTGGAG TGTATTCTCACAGAAAAGAGAAAAAAGAGATCCACAAAGAAGGAAGTGTTTAATAT ACTTCAGGCTGCGTATGTCAGCAAGCCTGGGGCCCAGCTTGCTAGACAAATAGGA GCCAGCCTGAATGATGACATTCTTTTCGGGGTGTTCGCACAAAGCAAGCCAGATTC TGCCGAACCAATGGATCGATCTGCCATGTGTGCATTCCCTATCAAATATGTCAACGA |
| 196410 | METTL7B | HSX2_02278211 | 494 | CATACACCCACTCAACTCTGGCAAATAGGAAATTGTCAAGTAGGAGACAAGGAGCA AAGTCCTATCACAGCGGGAGGGGACGCCAGCGCCTGCAGAGGCTGAGCAGGGAA AAAGCCAGTGCCCCAGCGGAAGCACAGCTCAGAGCTGGTCTGCCATGGACATCCT GGTCCCACTCCTGCAGCTGCTGGTGCTGCTTCTTACCCTGCCCCTGCACCTCATGGC TCTGCTGGGCTGCTGGCAGCCCCTGTGCAAAAGCTACTTCCCCTACCTGATGGCCGT GCTGACTCCCAAGAGCAACCGCAAGATGGAGAGCAAGAAACGGGAGCTCTTCAGC CAGATAAAGGGGCTTACAGGAGCCTCCGGGAAAGTGGCCCTACTGGAGCTGGGCT GCGGAACCGGAGCCAACTTTCAGTTCTACCCACCGGGCTGCAGGGTCACCTGCCTA GACCCAAATCCCCACTTTGAGAAGTTCCTGACAAAGAGCATGGCTGAGAACAGGCA CCTCCAATATGAGCGGTTTGTGGTGGCTCCTGGAGAGGACATGAGACAGCTGGCT GATGGCTCCATGGATGTGGTGGTCTGCACTCTGGTGCTGTGCTCTGTGCAGAGCCC AAGGAAGGTCCTGCAGGAGGTCCGGAGAGTACTGAGACCG |
| 4240 | MFGE8 | 4240.004.1-D | 495 | CTGGCAGCAGTAAGATCTTCCCTGGCAA |
| 4240 | MFGE8 | 4240.004.1-F | 496 | GTGGGTAACAGGTGTGGTGACGCAGGGTGCCAGCCGCTTGGCCAGTCATGAGTAC CTGAAGGCCTTCAAGGTGGCCTACAGCCTTAATGGACACGAATTCGATTTCATCCAT GATGTTAATAAAAAACACAAGGAGTTTGTGGGTAACTGGAACAAAAACGCGGTGC ATGTCAACCTGTTTGAGACCCCTGTGGAGGCTCAGTACGTGAGATTGTACCCCACG AGCTGCCACACGGCCTGCACTCTGCGCTTTGAGCTACTGGGCTGTGAGCTGAACGG ATGCGCCAATCCCCTGGGCCTGAAGAATAACAGCATCCCTGACAAGCAGATCACGG CCTCCAGCAGCTACAAGACCTGGGGCTTGCATCTCTTCAGCTGGAACCCCTCCTATG CACGGCTGGACAAGCAGGGCAACTTCAACGCCTGGGTTGCGGGGAGCTACGGT |
| 4240 | MFGE8 | HSX2_01056073 | 497 | GGGCTTGCATCTCTTCAGCTGGAACCCCTCCTATGCACGGCTGGACAAGCAGGGCA ACTTCAACGCCTGGGTTGCGGGGAGCTACGGTAACGATCAGTGGCTGCAG |
| 4240 | MFGE8 | HSX2_01056081 | 498 | CCAGGGGGCCCGTAACTTTGGCTCTGTCCAGTTTGTGGCATCCTA |
| 4240 | MFGE8 | HSX2_01056085 | 499 | GTGACAGGCATCATCACCCAGGGGGCCCGTAACT |
| 4240 | MFGE8 | HSX2_01056092 | 500 | TTCCCTGGCAACTGGGACAACCACTCCCACAAGAAGAACTTGTTTGAGACGCCCAT CCTGGCTCGCTATGTGCGCATCCTGCCTGTAGCCTGGCACAACCGCATCGCCCTGC GCCTGGAGCTGCTGGGCTGTTAGTGGCCAC |
| 4240 | MFGE8 | HSX2_01056094 | 501 | CCACCCCCAGGTCTTCCTGCTTTCCATGGGCCCGCTGCCTCTTGGCTTCTCAGCCCCT TTAAATCACCATAGGGCTGGGGACTGGGGAAGGGGAGG |
| 4240 | MFGE8 | HSX2_01056095 | 502 | ACCTCTCACGGGCCCTGCCCCAGCCCCTAAGCCCCGTCCCCTAACCCCCAGTCCTCA CTGTCCTGTTTTCTTAGGCACTGAGGGATCTGAGTAGGTCTGGGATGGACAGGAAA GGGCAAAGTAGGGCGTGTGGTTTCCCTGCCCCTGTCCGGACCGCCGATCCCAGGT GCGTGTGTCTCTGTCTCTCCTAGCCCCTCTCTCACACATCACATTCCCATGGTGGCCT CAAGAAAGGCCCGGAAGCGCCAGGCTGGAGATAACAGCCTCTTGCCCGTCGGCCC TGCGTCGGCCCTGGGGTACCATGTGGCCACAACTGCTGTGGCCCCCTGTCCCCAAG ACACTTCCCCTTGTCTCCCTGGTTGCCTCTCTTGCCCCTTGTCCTGAAGCCCAGCGAC ACAGAAGGGGGTGGGGCGGGTCTATGGGGAGAAAGGGAGCGAGGTCAGAGGAG GGCATGGGTTGGCAGG |
| 4240 | MFGE8 | HSX2_01056096 | 503 | GTGGGCGTTTGGGGCCCTCTATGCTGGCTTTTCACCCCAGAGGACACAGGCAGCTT CCAAAATATATTTATCTTCTTCACGGGAA |
| 4240 | MFGE8 | HSX2_01056103 | 504 | GGACTGGCAGCAGTAAGATCTTCCCTGGCAACTG |
| 4240 | MFGE8 | HSX2_02508672 | 505 | CATCCTGGCTCGCTATGTGCGCATCCTGCCTGTAGCCTGGCACAACCGCATCGCCCT GCGCCTGGAGCTGCTGGGCTGTTAGTGGCCACCTGCCACCCCCAGGTCTTCCTGCT TTCCATGGGCCCGCTGCCTCTTGGCTTCTCAGCCCCTTTAAATCACCATAGGGCTGG GGACTGGGGAAGGGGAGGGTGTTCAGAGGCAGCACCACCACACAGTCACCCCTCC CTCCCTCTTTCCCACCCTCCACCTCTCACGGGCCCTGCCCCAGCCCCTAAGCCCCGTC CCCTAACCCCCAGTCCTCACTGTCCTGTTTCTTAGGCACTGAGGGATCTGAGTAGG TCTGGGATGGACAGGAAAGGGCAAAGTAGGGCGTGTGGTTTCCCTGCCCCTGTCC GGACCGCCGATCCCAGGTGCGTGTGTCTCTGTCTCTCCTAGCCCCTCTCTCACACAT CACATTCCCATGGTGGCCTCAAGAAAGGCCCGGAAGCGCCAGGCTGGAGATAACA GCCTCTTGCCCGTCGGCCCTGCGTCGGCCCTGGGGTACCATGTGGCCACAACTGCT GTGGCCCCCTGTCCCCAAGACACTTCCCCTTGTCTCCCTGGTTGCCTCTCTTGCCCCT TGTCCTGAAGCCCAGCGACACAGAAGGGGGTGGGGCGGGTCTATGGGGAGAAAG GGAGCGAGGTCAGAGGAGGGCATGGGTTGGCAGGGTGGGCGTTTGGGGCCCTCT ATGCTGGCTTTTCACCCCAGAGGACACAGGCAGCTTCCAAAATATATTTATCTTCTT |
| 4257 | MGST1 | HSX2_01057030 | 506 | GTTTTTGCCAATCCAGAAGACTGTGTAGCATTTGGCAAAGGAGAAAATGCCAAGAA GTATCTTCGAACAGATGACAGAGTAGAACGTGTAC |
| 4281 | MID1 | HSX2_01057790 | 507 | ACTGTAAAGGATCAGAAAGTCATTGGCAGAGAGAAGCAGCAGTATTACCCTAGGA ATAGGAGAAAGTGGGTCAGCAACTCCCTACAGGCAAGACTGATGGGGAGAAGATG CATTTCAAGACGCGAAAGAAAACTGACTCTAGATGTAGAGAAG |
| 54471 | MIEF1 | HSX2_01794393 | 508 | GTGGTCATGTATTCCTGGTGAAGACACCATCATGAATGTCCCTGGCTTCTTCCTGGT GCGTCGTGAGAATCCAGAGTACTTTCCTCGTGGGAGCAGTTACTGGGACCGCTGTG TAGTAGGGGGCTACCTCTCTCCAAAGACAGTCGCAGATACATTTGAGAAGGTAGTG GCTGGCTCCATCAATTGGCCAGCCATAGGGTCCCTCTTGGACTATGTGATCCGCCC GGCCCCACCCCCAGAAG |
| 10962 | MLLT11 | HSX2_01539260 | 509 | CCTGACTGGGGACACCTGAGAGTAGTATAGTAGTGCAAAATGGAAGACTGATTTTT GACTCTATTATAATCAGCTTCAGAGATTCCTTAAACCTTCCTAATTTCCTGCTCCAGG GCAGTAAACACAAATATTTCTTCAAGGGGTGATGAAAACCTCGGAAGTTTTAATTT GAGGTTATCTGCTACGAAACAGTATTTCTAAAAGGCTAAAGTGATAAGTCTCTTGCT TTTTTTTGATCCTGCTCTTATATTCTTTTTTTCCTCAGAGAAATCAGGAGGGTAGTT AGAGGTATAAAACAGGAGGAAATATTATGGAAAATGAAAATAGGGAAAATAATTG AATCATTTTAGAAGTAGCTAATTTCTTTTCTCAAAAGAGTGTCCCTTCTTCACACCTA CTCACTTTACAACTTTGCTCCTAACTGTGGGTTGAAAACTCTAGCTAAAGAAAGTTA TCAAATCTTAACATGCATTCCTACTATTATGATAGTTTTTAAGGTTTCAATTCAATCT TCTGAACGGCATAAGTCCTATTTTAGCCTTACCTCCTGCATTTGCAATACGTAATACT GATCAGTGGGCACAGTTCTTCAGCTACATTGAGACCCTGAAATGAACAATTATATTC TGACTCGACATCTTGTCCCCAATCCTTCCAAAAATATTGATGGTGATTTGTGCTACC |
| 4301 | MLLT4 | HSX2_01059895 | 510 | AGTAGAGTTAAGCCCAGATGGTTCTGACTCTAGA |
| 4301 | MLLT4 | HSX2_01059916 | 511 | CTGAAGATTCATTTTTGTCTGCCATTATAAATTATACTAATAGCTCTACAGTCCACTT TAAGTTGTCCCCTACATATGTATTATATATGGCATGCCGGTATGTATTGTCCAACCA GTACAGACCTGACATCAGCCCTACAGAGCGCACACATAAAGTCATTGCAGTCGTCA ACAAGATGGTGAGCATGATGGAGGGTGTCATCCAG |
| 4301 | MLLT4 | HSX2_01060018 | 512 | GACCAAACTCTTACCCAGGATCTACTGGAGCAGCTGTTGGAGCCCATGACGCCTGT CGGGATGCAAAAGAGAAGCGCTCTAAAAG |
| 4301 | MLLT4 | HSX2_01060025 | 513 | GAGAAGCGCTCTAAAAGCCAAGATGCAGATTCAC |
| 4301 | MLLT4 | HSX2_02508915 | 514 | AGGTGCTGCAGATGATGGATGGACGTCTAGCTGC |
| 84954 | MPND | HSX2_02118356 | 515 | ATCTCCTTGGCCAGCAGGACGCCCAAGGACCAGAGCCTGTGTCACGTCCTGGAACA GGTGTGCGGCGTCCTCAAGCAGGGGAGCTGAGCCTTCCAGGGCAGGGTGGGCTCC AGTTGTCTTGAGGGTCCGGATGGGCTCAGGTAATAAAGAAACGGAAGCAGCA |
| 10205 | MPZL2 | HSX2_01479681 | 516 | CTCTTTGGCCTATAGCAGCTGTGGAAATTTATACCTCCCGGGTGCTGGAGGCTGTT AATGGGACAGATGCTCGGTTAAAATGCACTTTCTCCAGCTTTGCCCCTGTGGGTGA TGCTCTAACAGTGACCTGGAATTTTCGTCCTCTAGACGGGGGACCTGAGCAGTTT |
| 10205 | MPZL2 | HSX2_01479685 | 517 | GTATTCTACTACCACATAGATCCCTTCCAACCCATGAGTGGCGGTTTAAGGACCG GGTGTCTTGGGATGGGAATCCTGAGCGGTACGATGCCTCCATCCTTCTCTGGAAAC TGCAGTTCGACGACAATGGGACATACACCTGCCAGGTGAAGAACCCACCTGATGTT GATGGGGTGATAGGGGAGATCCGGCTCAGCGTCGTGCACACTG |
| 10205 | MPZL2 | HSX2_01479690 | 518 | TACGCTTCTCTGAGATCCACTTCCTGGCTCTGGCCATTGGCTCTGCCTGTGCACTG |
| 10205 | MPZL2 | HSX2_01479691 | 519 | ATGATCATAATAGTAATTGTAGTGGTCCTCTTCCAGCATTACCGGAAAAAGCGATG GGCCGAAAGAGCTCATAAAGTGGTGGAGATAAAATC |
| 10205 | MPZL2 | HSX2_01479697 | 520 | AAAAGAAGAGGAAAGGCTCAACCAAGAGAAAAAGGTCTCTGTTTATTTAGAAGAC ACAGACTAACAATTTTAGATG |
| 10205 | MPZL2 | HSX2_01479701 | 521 | ACTTTTCTTTGGCTTTTCCAGTTGTGACCCGTTTTCCAACCAGTTCTGCAGCATATTA GATTCTAGACAAGCAACACCCCTCTGGAGCCAGCACAGTGCTCCTCCATATCACCA GTCATACACAGCCTCATTATTAAGGTCTTATTTAATTTCAGAGTGTAAATTTTTTCAA GTGCTCATTAGGTTTTATAAACAAGAAGCTACATTTTTGCCCTTAAGACACTACTTA CAGTGTTATGACTTGTATACACATATATTGGTATCAAAAGGGATAAAAGCCAATTTG TCTGTTACATTTCCTTTCACGTATTTCTTTTAGCAGCACTTCTGCTACTAAAGTTAAT GTGTTTACTCTCTTTCCTTCCCACATTCTCAATTAAAAGGTGAGCTAAGCCTCCTCGG TGTTTCTGATTAACAGTAAATCCTAAATTCAAACTGTTAAATGACATTTTTATTTTTA TGTCTCTCCTTAACTATGAGACACATCTTGTTTTACTGAATTTCTTTCAATATTCCAG GTGATAGATTTTTGTTGTTTTGTTAATTAATCCAAGATTTACAATAGCACAACGCTA AATCACACAGTAACTACAAAAGGTTACATAGATATGAAAAGATTGGCAGAGGCCAT TGCAGGATGAATCACTTGTCACTTTTCTTCTGTGCTGGGAAAAATAATCAACAATGT GGGTCTTTCATGAGCAGTGACGGATAGTTTAGCTTACTATGTTTCCCCCCCAATTCA ATGATCTATAACAACAGAGCAAAGTCTATGCTCATTTGCAGACTGGAATCATTAAGT AATTTAATAAAAAAATTGTGAAACAGCATATTACAAGTTTGAAAATTCAGGGCTGG TGAAAAAAATCAACTCTAAATGATGATAATTTTGTACAGTTTTATATAAAACTCTGA GAACTAGAAGAAATTATTAACTTTTTTTCTTTTTTAATTCTAATTCACTTGTTTATTTT GGGGGAGGAAGACTTTGGTATGGAGCAAAGAAATACCAAAACTACTTTAAATGGA ATAAAACCAACTTTATTCTTTTTTTCCCCCATACTGGTAGATAAAGCAAACTTTATAA GTGGGCTATTGAAAGAAAAGTTACAAGCTTAAGATACAGAAGCATTTGTTCAAAGG ATAGAAAGCATCTAAAAGTTTAGGCTCAAGATCAATCTTTACAGATTGATATTTTCA GTTTTTAATCGACTGGACTGCAGATGTTTTTTCTTTTAACAAACTGGAATTTTCAAAC AGATTATCTGTATTTAAATGTATAGACCTTGATATTTTTCCAATACTATTTTTTAAAA AATTGTATGATTTACATATGAACCTCAGTTCTGAAATTCATTACATATCTGTCTCATT CTGCCTTTTATACTGTCTAAAAAAGCAAAGTTTTAAAGTGCAATTTTAAAACTGTAA ATTACATCTGAAGGCTATATATCCTTTAATCACATTTTATATTTTTTCTTCACAATTCT AACCTTTGAAAATATTATAACTGGATATTTCTTCAAACAGATGTCCTGGATGATGGT CCATAAGAATAATGAAGAAGTAGTTAAAAATGTATGGACAGTTTTTCCGGCAAAAT |
| 10205 | MPZL2 | HSX2_01479703 | 522 | ACTAACAATTTTAGATGGAAGCTGAGATGATTTC |
| 10205 | MPZL2 | HSX2_01479705 | 523 | TCTAATGAGGACTCCCAGATCATCACTTTATGGCTGTTAGGTGTTTCCCATATGAAA TTAGAGGAGCTGGGTCAGGGAGACAAAAGTCTTCTATTAGTCTTATGGATAGCTCC TCCTTGAGTGTATTTTGTGCAAAAGATTAAGAAGCTGGACTCTACTGCCATTAAAGC TGAGAGAATCCTAAGGTTATTTGTGGCTTCGGGGTTATATTTATTACTACTACTACT AATAAATATTCAACAAGTAAATAAATCTTTTTTAAATCAA |
| 10205 | MPZL2 | HSX2_01479706 | 524 | ACTAACAATTTTAGATGGTAAGGTTCACAAATAG |
| 10205 | MPZL2 | HSX2_02534823 | 525 | TACGCTTCTCTGAGATCCACTTCCTGGCTCTGGCCATTGGCTCTGCCTGTGCACTGA TGATCATAATAGTAATTGTAGTGGTCCTCTTCCAGCATTACCGGAAAAAGCGATGG GCCGAAAGAGCTCATAAAGTGGTGGAGATAAAATC |
| 9902 | MRC2 | HSX2_01451912 | 526 | CCAGGAGGAGCAGCACCCGAGCCCTGGAGTGGCCCAGTACCCTTCCAAGAGGCCA CAGTCCCAGCCAGGACAAAGTATGCGGCCCATCCTGGTGCGACAGCGTGGGACAA TGTGAACATGGACTCGAAGACATGGCCCTTTCTCTGTAGTTGATTTTTTAAATGTGC CATTATTGTTTTTAA |
| 4524 | MTHFR | HSX2_01066737 | 527 | CCCCGTCCCGCACCATCATCCAGTACATCCACGACAACTACTTCCTGGTCAACCTGG TGGACAATGACTTCCCACTGGACAACTGCCTCTGGCAGGTGGTGGAAGACACATTG GAGCTTCTCAACAG |
| 9617 | MTRF1 | HSX2_01419835 | 528 | AGCAGGAGGGCAGCATGTTAATAAAACTGATAGTGCCGTCAGACTTGTCCACATCC CCACAG |
| 54587 | MXRA8 | HSX2_02554391 | 529 | GAAGGATGTTAACTTGGCGGAGTTCGCTGTGGCTGCAGGGGACCAGATGCTTTAC AGGAGTGAGGACATCCAGCTAG |
| 23077 | MYCBP2 | HSX2_01593794 | 530 | GCAGCAACCATACGGCAGTACTTTTAATGGATGGACAGGTCTTCACATTTGGAAGT TTTTCT |
| 4628 | MYH10 | HSX2_01073103 | 531 | TGAAACACTCCATAAGAACAATGCCCTTAAAGTTGTGCGAGAGCTACAAGCCCAAA TTGCTGAACTTCAGGAAGACTTTGAATCCGAGAAGGCTTCACGGAACAAGGCCGAA AAGCAGAAAAGGGACTTGAGTGAGGAACTGGAAGCTCTGAAAACAGAGCTGGAG GACACGCTGGACACCACGGCAGCCCAGCAGGAACTACG |
| 4628 | MYH10 | HSX2_01073190 | 532 | GGCAAGCTACGGGATTCCTTCCTGAAAGATCAACTGTGTCTTAAGGCTCTCCAGCCT ATGCATACTGTATCCTGCTTCAGACTTAGGTACAATTGCTCCCCTTTTTATATATAGA CACACACAGGACACATATATTAAACAGATTGTTTCATCATTGCATCTATTTTCCATAT AGTCATCAAGAGACCATTTTATAAAACATGGTAAGACCCTTTTTAAAACAAACTCCA GGCCCTTGGTTGCGGGTCGCTGGGTTATTGGGGCAGCGCCGTGGTCGTCACTCAG TCGCTCTGCA |
| 140469 | MY03B | HSX2_02220753 | 533 | GTGTTTCAGCTCAACTCACCAGTACACGTCTGCGGAGAAACACATCTGTTGGCACCC CGTTCTGGATGGCCCCTGAG |
| 4646 | MYO6 | HSX2_01075718 | 534 | GGTACCTCTGAAAGTGGAGCAAGCAAACAATGCTCGTGATGCCCTGGCAAAGACA GTGTATAGCCATCTTTTTGATCATGTGGTAAACAGAGTAAATCAGTGTTTTCCTTTT GAAACATCATCCTATTTTATTGGAGTCCTAGATATTGCTGGTTTTG |
| 4664 | NAB1 | HSX2_01077841 | 535 | TTGTGGTGGATGGGGAGCTGAGCAGACTTTACCCCAGTGAGGCAAAGTCCCACTC ATCAG |
| 4665 | NAB2 | HSX2_01077903 | 536 | GGTGGAGGGTCGCAGGAGCAGCGTGAAAGTGGAGGCTGAGGCCAGCCGGCAGTG AGGGTTGGACTGGTGTCTTCAGACCCAGGACCTCAGACTTCTGGCTCACACAGACC CCCACGCTCTCCATCCCCGGAATCTAGTCACAACCCTGGATCCTTCCTCTGCCCTTCT CCTGCCTCCCCACCTGCTCCATGGGCATAAGACTGTGGGGCTTCAAGCAATAACAA GCAGAGGCCTGGAGAGAGGACACAAGGAGGGTGCGTGGTGCCCTCACCCCTGCCC AGAGCGAGGGGGCAAGGACTCTGCCTCCAGGGCATCTGGGGTTTTCCCCTCCCTCA CACAACACACTCCCATTCTCTTTAGGTTTGCACCAGTGGTGTGAGCAGTTGGACTCA GTTTGGACAAGGGGGAAAGGGGGACTTCCCTGGGAAGGTCCAGCTAAAAGTGGC AACATTTGCCCCCAGAATTGGGGGCCTGGGAACACTGGACCTGCTCCTTCTCCCCTC TCCTTCCCCGTTTTTGTGCTTCTGGTTTGTTTCTTTAATTAATTTAACAAGTGCTGCA GTTTGCCCTCCCATTCCCATCTATCCCCCAAGTCCTTTGCAATTTCTTCCCTGCCCTAC ATAGGGGCGGTGGGTGTGGGATCCCTTCACTGGCCCCCTCGGGAGGCCTGGGTTG GACTCAGGGTCTCCTCCAGCTGGGGGCTGGACCGCAGCACCTATCTGAGCAGTTAG AGCGTCTTTCTTTTCAGATTGTGTACAGTAGATTATTTATTTTGTTATTTTGGAATAA |
| 89797 | NAV2 | HSX2_02128541 | 537 | GAATTGCGCCAGTACCTGTCCAACCTTGCTGACCAGTGCAACAGTGAGAACAATGC TGTGGACATGCCCCTCGTCATCATCCTGGACAACCTACACCACGTGAGCTCTCTGGG AGAGATCTTCAATGGGCTGCTCAACTGCAAGTACCACAAATG |
| 26960 | NBEA | HSX2_01695002 | 538 | CTGGCAAAGAAGGGAAATTTCAAACTTCGAATATTTGATGTTCCTTAATACTATTGC AG |
| 26960 | NBEA | HSX2_01695190 | 539 | AATCGCCACTGGGCTCGTCACGACAGCCTCCTTCAGCTCCAACCCGGCTAATTAATC CCCACTCATGGATATAGGCACACAAACTAAAG |
| 29781 | NCAPH2 | HSX2_01722501 | 540 | AAGCGCAAGAGGAAGGGCGCTGCCAAGCTGCAGG |
| 23154 | NCDN | HSX2_01602288 | 541 | CT |
| 400746 | NCMAP | HSX2_02394908 | 542 | CTTCACAATGAGCTTCTTCTGGTCAGGTCGACAGAGACATCTTTGACGCAATCTCTG ATGCTTCCAGCAATCCTCAACCTTGTCTGCCCTGCCCTACCCCAACTGTGTCCACATC CCTGCCGCCACCCCACCAAAAAGCTGCAGAACATTCTTTTGTCATCTGATGAGGTAG AGCTATGTTGGGAATCCACCAATGTGGGCTTGGCTTTCCCCCACACTGTAGTTAGAC AGATAGACAGATAGCCCAGGAGCCAGGTGTCAGGGAGCACTGCTGAGAGTATCAC AATAGGATCTGTCACGGGGTTCATATCAGATGAAGCGCCGTATCCACTGCTTCACA GAGCAAAACATTCAATCCCATAACCAGGCACAGGGGAACTAACTTGGACTAACTAA CCAGAAAACCTTGTTAACGTATAACTTGTTCCAGTACTACATCTCTGCCTGCTGGCT CATGACAATTGCTCAGCACATTTTCCCCTCTTGAAGAAAGGTTGCAAGAAGAACTA AATTATCCTCAAAAGATTTCTGCTTCATTAGTAAAGAGTCAGTGATGGAATAGGGT GACTCTGCAGAATAGTGGCCTCTAGGGTAGGAGCTTGTTGTGTTGTCCGTGGGCCT GGAATGATCCTGGTGGCTGATCAGGGTCCTTCTCCCACTCTGGGCTGTATCAACCCT GACGGTCTTGGTCTTTGGCTCCCCTTTATCTGGATTCTGAGCACGCTGACTGTCCTG TTAATGCCTTCCCTCCAAGGACCAGTATTTGGAGATTAATTAGATTACAACTCTATCT ATGTTACCTTTGTCCTTCCTGGTCACCTTGCAGATTCAAGACATGTTCAAAGCAACA CATTCACAACCCATTTCTATTCTATAGCAACCTCGTCTGTGACTCCTTAGCCTGGAGA ACAATCTACCAAGAAGAGAAAGTATCTGGAATTAAGAAGTCCTACCATCCAAGCCC TACTTCCTGGTTGTGTGGCCTTGGAAAAGTGACTCAACCTCTTTATATTCAGTTTCCT AACCATGAAGTGGAAATGATAACACCTGCCTCATTGGGGCACTATAACAAGTGAAG GACTTAGGAAAACATCTGGAGTATAGCGCCTGGCACCCAGGAGATGCTTAATAAAT |
| 8202 | NCOA3 | HSX2_01324111 | 543 | CTCCTTTTCCCAGCAGCAGTTTGCCCACCAGGGGAATCCTGCAGTGTATAGTATGGT GCACATGAATGGCAGCAGTGGTCACATGGGACAGATGAACATGAACCCCATGCCC ATGTCTGGCATGCCTATGGGTCCTGATCAG |
| 25915 | NDUFAF3 | HSX2_01668012 | 544 | GGTCCGGTTCGGCCTGGCAGGTGTTCGGGACGCG |
| 4753 | NELL2 | HSX2_01086049 | 545 | GGTATAATGCAAGATGTCCAATTACTTGTCATGCCCCAGGGATTTATTGCTCAGTGC CCAGATCTTAATCGCA |
| 25825 | NFAM1 | HSX2_01660260 | 546 | ATTTCCCCATCCACAAATGCGCTGGTGATCGGTGCCACGGTGATGGAGGGCTTCTA CGTCATCTTCGACAGAGCCCAGAAGAGGGTGGGCTTCGCAGCGAGCCCCTGTGCA G |
| 4781 | NFIB | 4781.006.2-B | 547 | CCTGGAACTCTAGCACGCCGAGTGAACTTGAATCTTTGGCTATTTAAGGAGGACTG GGTTTGTTGTGAAGTTGCGGTGATCCAGCGCAGAGCCCCGTCCTGATTGATCGCAT CGCGGGGCTCAGATGACTGTAAAATGAATAGATGAAATTCTTGCTTCTCGAAGATT TTCTTGGGCATCTCCCGGAAAGTGCGTTTTAAGGCGAAGTCATGATGTATTCT |
| 4781 | NFIB | HSX2_01087767 | 548 | TTTGGCTATTTAAGGAGGACTGGGTTTGTTGTGA |
| 57185 | NIPAL3 | HSX2_01915059 | 549 | AAAAAAAAAAGTACTAGGTGCAAATAACATTATGAAGTGGTATTTAATTAAAAATC CATGCAGTGACAGCTGA |
| 79159 | NOL12 | HSX2_02004046 | 550 | AACATCCCCGACGGGCCCAGGACTCCAAAAAGCCCCCAAGGGCCCCTCGTACCAGC AAGGCCCAGCGCCGCCGTCTCACAGGCAAAGCACGGCACAGCGGGGAGTGAGACC GAGAACGAAGCGGTGCCCCAGTCTAGGCTGCGGGGACCTGTCCTTGCTCAGCTTG GCTGTCCCTGTAGCCCAGCCTGCACCTAG |
| 7064 | NOP16 | HSX2_01266163 | 551 | GAACCAACATGCCTGCAACCTTCGGCCATCTGGCAG |
| 7064 | NOP16 | HSX2_02521832 | 552 | GAACCAACATGCCTGCAACCTTCGGCCATCTGGCAGGTGGCTACGACGCCCAGTAC TACGGGTACCTGTGGAGCGAGGTGTATTCCATGGACATGTTCCACACGCGCTTCAA GCAGGAGGGTGTCCTGAACAGCAAG |
| 124056 | NOXO1 | HSX2_02190737 | 553 | CGGTCAGGCCAGCCTGGATGCACCACTGTTGGGA |
| 10577 | NPC2 | HSX2_01512515 | 554 | GTTCTGTGGATGGAGTTATAAAGGAAGTGAATGTGAGCCCATGCCCCACCCAACCC TGCCAGCTGAGCAAAGGACAGTCTTACAGCGTCAATGTCACCTTCACCA |
| 10577 | NPC2 | HSX2_02536964 | 555 | GTTCTGTGGATGGAGTTATAAAGGAAGTGAATGTGAGCCCATGCCCCACCCAACCC TGCCAGCTGAGCAAAGGACAGTCTTACAGCGTCAATGTCACCTTCACCAGCA |
| 10577 | NPC2 | HSX2_02536987 | 556 | GTTTCTCATCTCTAAGTGCCTCATTGAGTTCGGTGCATCTGGCCAATGAGTCTGCTG AGACTCTTGACAGCACCTCCAGCTCTGCTGCTTCAACAACAGTGACTTGCTCTCCAA TGGTATCCAGTGATTCGTTGAAGAGGAGGTGCTCTGTAGCAGAAACTGAGCTCCG GGTGGCTGGTTCTCAGTGGTTGTCTCATGTCTCTTTTTCTGTCTTAGGTGGTTTCATT AAATGCAGCACTTGGTTAGCAGATGTTTAATTTTTTTTTAACAACATTAACTTGTG GCCTCTTTCTACACCTGGAAATTTACTCTTGAATAAATAAAAACTCGTTTGTCTTGTC |
| 56654 | NPDC1 | HSX2_01896007 | 557 | TTGATGCTGTGTGCTTTTGGCTGGGCCTCGGGCTCCAGGCCCTGGGACCCCTTGCC AGGGAGACCCCCGAACCTTTGTGCCAGGACACCTCCTGGTCCCCTGCACCTCTCCTG TTCGGTTTAGACCCCCAAACTGGAGGGGGCATGGAGAACCGTAGAGCGCAGGAAC GGGTGGGTAATTCTAGAGACAAAAGCCAATTAAAGTCCATTTCAGACCTGCGGCTT CTG |
| 1728 | NQO1 | HSX2_00881265 | 558 | GCAGAAGAGCACTGATCGTACTGGCTCACTCAG |
| 1728 | NQO1 | HSX2_02498359 | 559 | AGTAAGAAGGCAGTGCTTTCCATCACCACTGGTGGCAGTGGCTCCATGTACTCTCT GCAAGGGATCCACGGGGACATGAATGTCATTCTCTGGCCAATTCAG |
| 4897 | NRCAM | HSX2_01095706 | 560 | TCTTTAGTACTTCCCTGCAGACCCCCAATTGGATTACCACCACCTATAATATTTTGGA TGGATAATT |
| 4897 | NRCAM | HSX2_01095731 | 561 | CGGCTCCATACTGGATCACAGCCCCTCAAAATCTTGTGCTGTCCCCAGGAGAGGAT GGGACCTTGATCTGCAGAGCTAATGGCAACCCCAAACCCAGAATTAGCTGGTTAAC AAATGGAGTCCCAATAGAAA |
| 4897 | NRCAM | HSX2_01095756 | 562 | GGACCTACACGTGTGTGGCCAACACCACTCTGGACAGCGTCTCCGCCAGCGCTGTG CTTAGCGTTGTTG |
| 4897 | NRCAM | HSX2_01095795 | 563 | GGAATGGGATCCACCGAGCCACCCGAATGGCATTTTGACAGAGTACACCTTAAAGT ATCAGCCAA |
| 4897 | NRCAM | HSX2_01095798 | 564 | TAATACTCCAGAAGGAGTCCCCAGTGCTCCCTCG |
| 4897 | NRCAM | HSX2_01095800 | 565 | ACACGGTGGACTTTAAAAAATTTAAATTTCAGCACTCGATATAAGTTTTATTTCTAT GCACAAACATCAGCAGGATCAGGAAGTCAAATTACAGAGGAAGCAGTAACAACTG TGGATGAAG |
| 4897 | NRCAM | HSX2_01095803 | 566 | CTTAAAGTATCAGCCAATTAACAGCACACATGAA |
| 4897 | NRCAM | HSX2_01095810 | 567 | TTAAAGAAAAGGAAGATGCCCATGCTGACCCTGAAATCCAGCCTATGAAGGAAGAT GATGGGACATTTGGAGAATACAG |
| 4897 | NRCAM | HSX2_01095844 | 568 | GGAAGTACCTCTTGATCTGGTACAGCCTCCAACC |
| 4907 | NT5E | HSX2_01096741 | 569 | ATTAACAACAACCTGAGACACACGGATGAAATGTTCTGGAACCACGTATCCATGTG CATTTTAAATGGAGGTGGTATCCGGTCGCCCATTGATGAACGCAACAATG |
| 4915 | NTRK2 | HSX2_02510945 | 570 | GCGTCAGCCCTCACGTCACTTCGCCAGCAGTAGCAGAGGCGGCGGCGGCGGCTCC CGGAATTGGGTTGGAGCAGGAGCCTCGCTGGCTGCTTCGCTCGCGCTCTACGCGCT CAGTCCCCGGCGGTAGCAGGAGCCTGGACCCAGGC |
| 4926 | NUMA1 | HSX2_02511046 | 571 | CTTTCTCTCAGGTTCTCCAGCTTCTCCCATGGGTGATATCCTGCAGACCCCACAGTTC CAGATGAGACGGCTGAAGAAGCAGCTTGCTGATGAGAGAAGTAATAGGGATGAG CTGGAGCTGGAGCTAGCTGAGAACCGCAAGCTCCTCACCGAGAAGG |
| 9688 | NUP93 | HSX2_01427660 | 572 | GGATGAGAAAGATAGTCAAGGAGAAAACATGTTTCTGCGCTGTGTGAGTGAGCTT GTGATTGAAAGCCGAGAG |
| 91775 | NXPE3 | HSX2_02574394 | 573 | AGGAAGCGGGGGGCTAGCGGCCTGGCGCTGCGGCGACCGACCGGGGCGTCAGGA TCCCTGGCCCCCGGAGAGCGAAGGGCGGGCGGGTCCCGGAG |
| 4967 | OGDH | HSX2_01100520 | 574 | CCCTGGGGATTTTGGATGCTGATCTGGACTCCTCCGTGCCCGCTGACATTATCTCAT CCACAGACAAACTT |
| 283208 | P4HA3 | HSX2_02325767 | 575 | TGGCCAACAAGTGGATACATGAGTATGGACAGGAATTCCGCAGACCCTGCAGCTCC AGCCCTGAAGACTGAACTGTTGGCAGAGAGAAGCTGGTGGAGTCCTGTGGCTTTC CAGAGAAGCCAGGAGCCAAAAGCTG |
| 5053 | PAH | HSX2_01105450 | 576 | GAGACTCCATAAAGGCATATGGTGCTGGGCTCCTGTCATCCTTTGGTGAATTACAG |
| 5066 | PAM | HSX2_01106278 | 577 | ACAGCCTTTAATTGCTGGCATGTACCTTATGATGTCTGTTGACACTGTTATCCCAGC AGGAGAAAAAG |
| 5087 | PBX1 | HSX2_01107803 | 578 | GAGCAGAAGAGGAAGAGCCGGGGGCTGCCGTAGCCTTTGGAGATGGACGAGCAG CCCAGGCTGATGCATTCCCATGCTGGGGTCGGGATGGCCGGACACCCCGGCCTGTC CCAGCACTTGCAGGATGGGGCCGGAGGGACCGAGGGGGAGGGCGGGAG |
| 5087 | PBX1 | HSX2_01107842 | 579 | CCAACGTGCAATCACAGGTGGATACCCTTCGCCA |
| 5090 | PBX3 | HSX2_01107983 | 580 | GAAGCAGGACATCGGCGACATCCTCCACCAGATCATGACCATCACCGACCAGAGCT TGGACGAGGCGCAAGCAAA |
| 5090 | PBX3 | HSX2_01107986 | 581 | GCGGACGGCGACGGCAGGAAGCAGGACATCGGCG |
| 5090 | PBX3 | HSX2_01107988 | 582 | GAAACATGCCCTGAACTGTCACAGAATGAAACCAGCGCTCTTCAGCGTCCTGTGTG AGATCAAAGAGAAAACAG |
| 5090 | PBX3 | HSX2_01107991 | 583 | GACGAGGCGCAAGCAAAGAAACATGCCCTGAACT |
| 5090 | PBX3 | HSX2_01107992 | 584 | GTCTCAGCATCAGAGGAGCCCAGGAGGAGGACCCTCCCGATCCCCAGCTAATGAG ACTGGACAATATGCTTTTGGCAGAAGGGGTTTCAGGTCCTGAGAAAG |
| 5090 | PBX3 | HSX2_01107994 | 585 | GCCAAATTGACCCAGATCAGACAAATCTATCACACAGAACTGGAGAAATATGAACA G |
| 5090 | PBX3 | HSX2_01107996 | 586 | GGCGGCAGCAGCTGCAGCCGCGGCAGCCTCTGGA |
| 5090 | PBX3 | HSX2_01107998 | 587 | GATCAAAGAGAAAACAGGTCTCAGCATCAGAGGA |
| 5090 | PBX3 | HSX2_01108000 | 588 | ACGTCCCATTTCTCCAAAAGAGATTGAAAGAATGGTGGGCATCATCCATCGAAAAT TTA |
| 5090 | PBX3 | HSX2_01108001 | 589 | GTTCCATTCAGATGCAGCTCAAACAAAGCACTTGTGAAGCAGTTATGATTTTAAGAT CAAGGTTCCTTGATGCCAG |
| 5090 | PBX3 | HSX2_01108005 | 590 | TGGAGAAATATGAACAGGCATGTAATGAATTTAC |
| 5090 | PBX3 | HSX2_01108007 | 591 | ACGGAAAAGGCGTAACTTCAGTAAACAGGCCACAGAAATCTTGAATGAATATTTTT ACTCACACCTCAGCAACCCCTACCCCAGTGAAGAAGCCAAAGAGGAGCTGGCCAAG AAATGCAGCATCACAGTGTCACAG |
| 5090 | PBX3 | HSX2_01108012 | 592 | GTATCCAATTGGTTTGGCAACAAACGAATCAGGTACAAGAAGAACATTGGCAAGTT TCAGGAAGAAGCCAACCTCTATGCTGCAAAGACGGCCGTGACAGCTGCACACGCA GTAGCAGCAGCTGTGCAGAACAACCAGACCAATTCGCCCACCACACCAAA |
| 5090 | PBX3 | HSX2_01108019 | 593 | AGTCGGAGCCAATGTGCAATCACAGGTTCTTCTGGTTCTTTAACCTCCCAAATTCT GGGGACATGTTCATGAACATGCAGAGTCTGAATGGGGATTCTTACCAAGGGTCCC |
| 5090 | PBX3 | HSX2_01108031 | 594 | AGGCCCAGGAAGTGTGCACTCGGATACCTCTAACTAATCTCTGGCCACACTTTTCCC TGAGCTACATGCCTTGATAAGTGCATTCAGAGCAATAGGAGGAAAAGGAAAGCGT TTTTGTAGCCCACCATCTACAGCTTTACTGTAAAACCTTGTCTTATTCGAGAACTTGG TAAATCTGTTTTTTAAGGAATCATAATCATTTGTATTTATACTTAAAAACACACAATG TTAAAAAAAATAAAGCACTTTATCCAATTAGGCCAAGATTTAACATTGTTGACAGTC CTGTAGCTATTTTATCATAATTTATTATCAATATTTTACATTAATGGTTTCACAGTTG CCAATTACTTGGCCTTAAGGGTAAAAAGTACAATATACACTAAACCTCAACC |
| 5090 | PBX3 | HSX2_01108032 | 595 | ACTGACTTGGATTGTCTTTCTTTCATATCACTAATGGAGTTGGAATAAAGAGCTGTT TGCCTATCCCTGTTAATGATGGTTGTGTTTAAGAATCTTCCTCGTCACGTTTGTGTTC AGATCTCTTATGTTATAATTAGATCAGAGACTGGTAGCATCGTTTCTCTCTCTGAAA GCACCAGTGCCCAGAGTCTGCTCGGTAATAAAATTATGGATCCAGATTGTTCTGAG AGACGAAGATACTTGCTGCTGATAGAGGTGAAAACGAGATTGATCCGTCTGGGGT TTTACGGTGTGCACTGGGTGCTGCACAGACTTGTCAAGGTTTGCTACGTCCTCTGG GCATCTGCAAAAGGCCCTGCTCTCTGGAGTGTTGTATATAGTGTAGCAAAAGAGTA TTTATACATCCCACCAATCAAAACACAGCTTTATTACCTCATGCGAACTCATACAAAC CAATAGAATTTCAACATGTTCTGTAGCTTAGAGTGCTCACTTACTACCTCTGAACAA TACTCACGCTGTAGTTTGTCTCTTTCTTATCTTTTTGCATCTTGTAATTAACTCTTTGT TTCCCTTCATAAAATGTAATGTACATTGTAATCTTTTAAAAGAAAAATCAGGGTTGC ACTTGCAACTTTTAAAAAACCGAGTGTGGAAACATTGGGTCTTAATTCAACACAGG ATCGGTAAAACTGTTGTAAATACTGAGAAACATTTTGAATGTTCTTCATCTTATTACT |
| 5090 | PBX3 | HSX2_02511701 | 596 | CCGCCGCCGCCGCCGCCGCCTCAGCCTTCGCCTCAGCCGCCGCCCGCTCCCGCCCG CGCGCGGCGGGATGGACGATCAATCCAGGATGCTGCAGACTCTGGCCGGGGTGAA CCTGGCTGGCCACTCGGTGCAGGGGGGCATGGCCCTGCCGCCTCCCCCGCACGGC CACGAAGGGGCGGACGGCGACGGCAGGAAGCAGGACATCGGCGACATCCTCCAC CAGATCATGACCATCACCGACCAGAGCTTGGACGAGGCGCAAGCAAA |
| 5090 | PBX3 | HSX2_02511702 | 597 | ACTGGACAATATGCTTTTGGCAGAAGGGGTTTCAGGTCCTGAGAAAGGTGGGGGA TCGGCGGCAGCAGCTGCAGCCGCGGCAGCCTCTGGAGGTTCTTCAGATAACTCTAT TGAACACTCAGATTACAGAGCCAAATTGACCCAGATCAGACAAATCTATCACACAG AACTGGAGAAATATGAACAG |
| 5090 | PBX3 | HSX2_02511706 | 598 | ACGTCCCATTTCTCCAAAAGAGATTGAAAGAATGGTGGGCATCATCCATCGAAAAT TTAGTTCCATTCAGATGCAGCTCAAACAAAGCACTTGTGAAGCAGTTATGATTTTAA GATCAAGGTTCCTTGATGCCAG |
| 5090 | PBX3 | HSX2_02511710 | 599 | GTATCCAATTGGTTTGGCAACAAACGAATCAGGTACAAGAAGAACATTGGCAAGTT TCAGGAAGAAGCCAACCTCTATGCTGCAAAGACGGCCGTGACAGCTGCACACGCA GTAGCAGCAGCTGTGCAGAACAACCAGACCAATTCGCCCACCACACCAAATTCCG |
| 5090 | PBX3 | HSX2_02511713 | 600 | AGGCCCAGGAAGTGTGCACTCGGATACCTCTAACTAATCTCTGGCCACACTTTTCCC TGAGCTACATGCCTTGATAAGTGCATTCAGAGCAATAGGAGGAAAAGGAAAGCGT TTTTGTAGCCCACCATCTACAGCTTTACTGTAAAACCTTGTCTTATTCGAGAACTTGG TAAATCTGTTTTTTAAGGAATCATAATCATTTGTATTTATACTTAAAAACACACAATG TTAAAAAAAATAAAGCACTTTATCCAATTAGGCCAAGATTTAACATTGTTGACAGTC CTGTAGCTATTTTATCATAATTTATTATCAATATTTTACATTAATGGTTTCACAGTTG CCAATTACTTGGCCTTAAGGGTAAAAAGTACAATATACACTAAACCTCAACCGTTAA AGCAGATGCAAAAATTCACCTCACCTAAATTGAACTTCTTGCATATTTCCATTACTG ACTTGGATTGTCTTTCTTTCATATCACTAATGGAGTTGGAATAAAGAGCTGTTTGCC TATCCCTGTTAATGATGGTTGTGTTTAAGAATCTTCCTCGTCACGTTTGTGTTCAGAT CTCTTATGTTATAATTAGATCAGAGACTGGTAGCATCGTTTCTCTCTCTGAAAGCAC CAGTGCCCAGAGTCTGCTCGGTAATAAAATTATGGATCCAGATTGTTCTGAGAGAC GAAGATACTTGCTGCTGATAGAGGTGAAAACGAGATTGATCCGTCTGGGGTTTTAC GGTGTGCACTGGGTGCTGCACAGACTTGTCAAGGTTTGCTACGTCCTCTGGGCATC TGCAAAAGGCCCTGCTCTCTGGAGTGTTGTATATAGTGTAGCAAAAGAGTATTTAT ACATCCCACCAATCAAAACACAGCTTTATTACCTCATGCGAACTCATACAAACCAAT AGAATTTCAACATGTTCTGTAGCTTAGAGTGCTCACTTACTACCTCTGAACAATACT CACGCTGTAGTTTGTCTCTTTCTTATCTTTTTGCATCTTGTAATTAACTCTTTGTTTCC CTTCATAAAATGTAATGTACATTGTAATCTTTTAAAAGAAAAATCAGGGTTGCACTT GCAACTTTTAAAAAACCGAGTGTGGAAACATTGGGTCTTAATTCAACACAGGATCG |
| 80714 | PBX4 | HSX2_02053487 | 601 | CGAGCGACGTCCTGCAGCAGATCATGGCCATCACCGACCAGAGCCTGGACGAGGC ACAGGCCAG |
| 80003 | PCNXL2 | HSX2_02036459 | 602 | CATGTGAGTCAGGATCTGGAAGCCTCGTCATGTTCTTCAACACAAGGAAAATTTAA CCGAGAGCAGTTTTACAAATTTATCATTTTCCCTGGCAAGTGGATTAAAGTCTGGTA TGATCGACTGACCTTGCTGGCATTACTTGATCG |
| 80003 | PCNXL2 | HSX2_02036493 | 603 | GGGCCAAAGCCAGGCACCCTCCCAGTTACGTTGTGTATGGCCTGAAGCTCTTCTCTC CAGTGTTTCTACAATCAGCTAGGGACTACTTAATAG |
| 80003 | PCNXL2 | HSX2_02036518 | 604 | GAACCGTGGAGCATGCAACACATCCCGGCACTGTTTTCGGCCTTCTGTGGCCTCTTG GTCGCCCTTTCTTACCATCTGAGCCGTCAGAGCAGTGACCCATCTGTACTCAT |
| 80003 | PCNXL2 | HSX2_02036531 | 605 | ACGGATGTCTTAAAATGGGATCTCATCGTCTGCGCAGTGGTTGCTGTCCTCTCATTT GCAGTCAGCGCCAGCACTGTATTCCTGTCATTGCGA |
| 80003 | PCNXL2 | HSX2_02036542 | 606 | ATGTTGCCCATTTAATGTGGTTCGAAAGACTCTATGTTTGGCTTCAGTGTTTTGAAA AATACATCTTGTACCCAGCGCTAATTTTGAATGCCCTCACTATTGATGCATTTTTAAT AAGCAATCACCGGAGACTTGGTACCCA |
| 80003 | PCNXL2 | HSX2_02036681 | 607 | CCGATGTCTGGCTGAGGCTGTGGCGGACACTCTCGGGGTTGTCTGCAGGAGAGCA AGCCAGGAGGACATGGGCCTGGACGACACGGCCTCGCAGCAAAGTGTGTCAGACG AGCAGTGACGGGCGTGCGGCCGGGCGGGGAGGCTGGCTCCCCCACACCTCCCACC TGCATTGCTCTCCCTCGTGCTCCCCAAATCACCACAACCAACCAATACCGCGATCCA TGAGGGACTCCTCCTGTGGAAAAGGAGAGCTGTTCCAGAACACAGAACTGATCTCA GGTTTTTG |
| 27344 | PCSK1N | HSX2_01710668 | 608 | CCGCGCCGCCTCCGCCGTGCCGCCGACCACGATGTGGGCTCTGAGCTGCCCCCTGA GGGCGTGCTGGGGGCGCTGCTGCGTGTGAAACGCCTAGAGACCCCGGCGCCCCAG GTGCCTGCACGCCGCCTCTTGCCACCCTGAGCACTGCCCGGATCCCGTGCACCCTG GGACCCAGAAGTGCCCCCGCCATCCCGCCACCAGGACTGCTCCCCGCCAGCACGTC CAGAGCAACTTACCCCGGCCAGCCAGCCCTCTCACCCGAGGATCCCTACCCCCTGG CCCCACAATAAACATGATCTGAAGCA |
| 5125 | PCSK5 | HSX2_01110707 | 609 | GGGCAGGAGCTGATGGGTGCATTAACTGCACAGAGGGCTACTTCATGGAGGATGG GAGATGCGTGCAGAGCTGTAGTATCAGCTATTACTTTGACCACTCTTCAGAGAATG GATACAAATCCTGCAAAAA |
| 8654 | PDE5A | HSX2_01350859 | 610 | TACCAGAGAGCCTCCGAGGCGCGCGGTGCCAACCATGGAGCGGGCCGGCCCCAGC TTCGGGCAGCAGCGACAGCAGCAGCAGCCCCAGCAGCAGAAGCAGCAGCAGAGG GATCAGGACTCGGTCGAAGCATGGCTGGACGATCACTGGGACTTTACCTTCTCATA CTTTGTTAGAAAAGCCACCAG |
| 8654 | PDE5A | HSX2_01350876 | 611 | GACTTTGCTGCTTATTTGGCATTTTGTGGTATTGTTCTTCATAATGCTCAGCTCTATG AGACTTCACTGCTG GAGAACAAGAGAAATCAG |
| 8654 | PDE5A | HSX2_01350881 | 612 | GTGCTGCTTGACCTTGCTAGTTTAATTTTGAAGAACAACAATCATTAGAAGTAATT TTGAAGAAAATAGCTGCCACTATTATCTCTTTCATGCAAGTGCAGAAATGCACCATT TTCATAGTGGATGAAGATTGCTCC |
| 8654 | PDE5A | HSX2_01350921 | 613 | AACAAGCTGACTGACCTGGAGATACTTGCATTGCTGATTGCTGCACTAAGCCACGA TTTGGATCACCGTGGTGTGAATAACTCTTACATACAGCG |
| 8654 | PDE5A | HSX2_01350941 | 614 | ACAACGG |
| 8654 | PDE5A | HSX2_01350956 | 615 | CAGGCAGAAATGGCAGGCCCTTGCAGAACAGCAGGAGAAGATGCTGATTAATGGG GAAAGCGGCCAGGCCAAGCGGAACTGAGTGGCCTATTTCATGCAGAGTTGAAGTT TACAGAGATGGTGTGTTCTGCAATATGCCTAGTTTCTTACACACTGTCTGTATAGTG TCTGTATTTGGTATATACTTTGCCACTGCTGTATTTTTATTTTTGCACAACTTTTGAG AGTATAGCATGAATGTTTTTAGAGGACTATTACATATTTTTTGTATATTTGTTTTATG CTACTGAACTGAAAGGATCAACAACATCCACTGTTAGCACATTGATAAAAGCATTGT TTGTGATATTTCGTGTACTGCAAAGTGTATGCAGTATTCTTGCACTGAGGTTTTTTT GCTTGGGGATTATTTAAATAATTGGTTTTTGTGTTTTCTGAATTACCATTTTTTCAA GAATGTTTGGAATCTTTCCTTTTTCAAAAGTAGGTTAGGAGCAAATTATCATACATT CTGTGACATTTAAAGCCTTTATAGGATAGTGAAAAATGCTGGCTGAGTGGATTTTA AGAGAAATAATTGTATTTGTTAACAGTGTCTTTTTTTAAAAAGTTAAGGCACTCTGA AACAAATGGAAAGTCCTATGAAACTGTATTGTAAAGAAAACATTATTTAATTGATAT GCTGTTTTGTGAGAGAACAGGCAAGACAGAACTTTGTCACTTCAGTGCAGTACATT TTTCTGAAAGCTACCCATAAAATCACTTTCATCTCACCTACCTGATGCAAAGCAGGT GAAACCTTAGGAGATGATCCAGTCACTGACTTGATTGAGGGATAAGTGTGATTTAG AAATGGAATGGCCTTGGATGTCTATCAGTGAAGAAAAATGTTCTGTTAGAAGATCT CTCTAAGAGTTTTTTTCCTTCTGAGCTTCCTTTTCAAAATAAAAGTGACAATTGTAGC ATTGACTTGAAGTGAGACATGGTTATAGATAAGAGAGTACAAAATGACTCTTTTTC CTGTCAATTGAAATTTAAAGAAAAGTTTTAATTATATAAATAGCAAAGGGCTATTGC CAATACTAGGGTCAAAAATGAATTTGAGGGAACAGTGGGTAAGAAACTTTATGCCT GAATAACATTTAGCAGTATTGTGATTGAAAAATTGCCATATTTTGATGTATAGGACA AGTCAACTGAGATCCAGAGAATCCTGGATGTGAATGCTAAACACTGGCCCTTAACT |
| 8654 | PDE5A | HSX2_01350958 | 616 | ATATACTTACTAAAAGCCTGTGTAAACATGTCTTAATGAATGTTGTTGAAAGCAATG TAAATAGTTGAAAATATAAATTTATATTACAGTTTAAGAAAACCTTATGAGGCATCA CTAGCCACTGTTAATATCTATTTGTATTCTTATACCTTTTCAATATATTTGAACAAATA TAGTTTCTGGCACTATTTTTATACTAGGAAAAGGAGTTACTATGTATATTATGCTTA GCTTTTAAGGCATTTTAAATAACCATGAATGTTGATTTCATTACTTTCCTTTCCTCCA TCACGAGAGTCATTTCAGATGACTCTTTCATGACAAAATCACTTTAAAGGAACACTT ACCTCCGATTCCTGTATAAAGTCATGAGATGGTCAAGGTGGTTTTCCATTGTGCAAA TTCTTCACCTGTCAGTGGTTTCCTCATTTTGCCATGCTTTGTAAAAATAAAAAGAATG ATCAAGTAGGTATGAA |
| 5162 | PDHB | HSX2_02512202 | 617 | AAGGTGGTCAGTCCCTGGTGTAGCTGCCCAGCAC |
| 5164 | PDK2 | HSX2_02512156 | 618 | GAGCGCTGCCCGCGCGGGGACCACAACCAAAGTCGCGGCCGCCGCAGCCATGCGC TGGGTGTGGGCGCTGCTGAAGAATGCGTCCCTGGCAGGGGCGCCCAAGTACATAG AGCACTTCAGCAAGTTCTCCCCGTCCCCGCTGTCCATGAAGCAGTTTCTGGACTTCG |
| 5166 | PDK4 | HSX2_01115087 | 619 | GTATATACAGAGCCTGATGGATTTGGTGGAATTCCATGAGAAAAGCCCAGATGACC AGAAAGCATTATCAGA |
| 5166 | PDK4 | HSX2_01115092 | 620 | GGCACAAGGAATCATAGAGTATAAAGATGCCTGTACAGTTGACCCAGTCACCAATC AAAATCTTCAATATTTCTTGGATCGATTTTACATGAACCGTATTTCTACTCGGATGCT GATGAACCAGCACA |
| 5166 | PDK4 | HSX2_01115102 | 621 | ATGCCTTTGAGTGTTCAAGGATGCTCTGTGATCAGTATTATTTATCATCTCCAGAAT TAAAGCTTACACAAGTGAATG |
| 5166 | PDK4 | HSX2_01115112 | 622 | AATGCAATGCGGGCAACAGTTGAACACCAGGAAAATCAGCCTTCCCTTACACCAAT AGAGGTTATTGTTGTCTTGGGAAAAGAAGACCTTACCATTAAG |
| 5166 | PDK4 | HSX2_01115117 | 623 | ATTTCAGACAGAGGAGGTGGTGTTCCCCTGAGAATTATTGACCGCCTCTTTAGTTAT ACATACTCCACTGCACCAACGCCTGTGATGGATAATTCCCGGAATGCTCCTTTG |
| 5166 | PDK4 | HSX2_01115122 | 624 | GCTGGTTTTGGTTACGGCTTGCCAATTTCTCGTCTGTATGCAAAGTACTTTCAAGGA GATCTGAATCTCTACTCTTTATCAGGATATGGAACAGATGCTATCATCTACTTAAAG |
| 5166 | PDK4 | HSX2_01115127 | 625 | GCTTTGTCTTCTGAGTCTATAGAAAAACTTCCAGTTTTTAACAAGTCAGCCTTCAAAC ATTATCAGATGAGCTCTGAGGCTGATGACTGGTGTATCCCAAGCAGGGAACCAAAG AACCTGGCAAAAGAAGTGGCCATGTGAAGAGGGACACTCAGGACACTTTACGGGA TCAAAGTGGGTCTACACCAGTGCTGCTTCCTGAATGTTTGTGTGTGAACCCTTGTTT CCTCCAAAACAAACGACAGCAACGAAAACTCCTTAATCAGAACACTGATCCAATGA GGAATGGAGCTTGTTTCTGTGACCCAGGAGAACTTAGTGCAAGACTACAGGAGTTA ACAGATGGCCAGCTCCTTATTTTTAATGTAGAATAACTCCTGAGTTTATATCAAATC CTGAAGAAATAAGCCTCAGTTTTCCATCTGTTTTTGATAAGAATAAGAAAGGGAGT GAGTGTGAAGATGGTGGTTAGCAGTTTCACTAAGACTGATATTTTAGGCCTCTTGT TCACATCAAAAGATATTGGTGTCAGAATACCAGCATTTTCCTGCCATGCAAAGGATT AAAACTTAGTTTACACTATGTGGTTACAAATATATGTCAATGTACATTTTGAACATA TTTATGTGCTATGGAAGGAAATGCTGGTGACTAAAATAAGGTTTACTCTGAAAGAG GAGGAATTTTATTCAAAGCATTCAAACATTTTATTCAAGTGTTTCAAAATTCAAAGC ATTGTATTCAAAGTTGCAGTGAAGGCATCAACTTATGTAAAAACTCAGAAGGAAGG CTCCTCTGATAAAAACACAGCTCCTTTATTATGCTGCTTTTCTTGTTCACTTTACACA CTAAGTAAACACTTATTGTCAGGTGCCTAGTCTTGAGTGAATTGTTAGATGTGCACT GAACTCGGGATGTTGGGGATTGGAGAGAGAGAATTGCCAAAGTAACAGCAAAAAT ATCTCTTACTTTGCTTTGTTTATAAATAAATTAGTAGATTGGAAAAACTAGTGTTAG GGAAAGAAATCACATGTTCAGAGCCTAATTCAGTAGGAAGGGCTTTTCTCTACCCT GAAATGAAGGTAATCCAAAGGCATCCATTTTCTAGGCTTAAAAGATATATTTTTGAT ATATTTAATTATATTCTCTACACTCCAGCATTAATATGTCTGTTTAAAAATTACTAATT CTCAAATGGCTCAAGAACATTAGAATTTAAGTACCTTTTAGAGTAATTATTTTAAGC AAATAGCCTGGACGTAAGAGATTCTCATGCCAGCATGCTTTCATTTGTCAGTTGTTG TGACTGAGAGATAATGAATGACACCTGAAATGCATATGGTATTTTTGGGAGAGTTA AGGTATAATTTGAAGGTTGGCAGACCAGTTGCGCTGATTACTCTTAGAGAAGAAGA AATGGAAAAATGAAAGAAGGCAGGAAGGAAAGAAAGGATATAGGAAGAGAGGG AAGCAGAAGGCAGGCATTTTTCTATTTCCCCACAAATTATTTCAAAAAAAATCTGT ATTTTCTGGGATATGTCATTGGCAAGAGGAAGAACTGGTGTTTTGAAAGCAGTATG GATTCTTTAAATGCCTCTCACTCTTACAAGATAGTAGGCTTTGAGATAATAAACTTA CCCGTGTCAATTAACATTTAAACTGGCATATAGAAAAAAAGGAGGATTTTTCTGCAT TGTAAAATAATCAGTATGGTTTATATGTTGAATTTGACATTTGTGTGTAATTTCATG GTGGCCTAGTGTTGTGGTGCTTCTGGTAATGGTAATAGAAGCTCAACTATTTTTTTG TGGATTTCAGTTTTTATCATCAGAAGTCCTAGACAGTGACATTTCTTAATGGTGGGA GTCCAGCTCATGCATTTCTGATTATACAAAACAGTTTGCAGTAGGTTATTTGTCATTT CAGTTTTTTACTGAAATTTGAGCTAAACATTTTTACATGTAAATACTTGTATTTACCA |
| 5255 | PHKA1 | HSX2_01122215 | 626 | ATGTTTCGACTTCGAATTGGTCTGATCATACAAGTTATGGCAACAGAACTGGCCCAC TCCCTTCGATGCTCAG |
| 7262 | PHLDA2 | HSX2_01281663 | 627 | GCAGAAGAACTTCCCGTGCGCGCGGATCCTCGCTCCGTTGCACGGGCGCCTTAAGT TATTGGACTATCTAATATCTATGTATTTATTTCGCTGGTTCTTTGTAGTCACATATTTT ATAGTCTTAATATCTTGTTTTTGCATCACTGTGCCCATTGCAAATAAATCACTTGGCC AGTTTGCTTTTCTACCA |
| 7262 | PHLDA2 | HSX2_02522700 | 628 | CCTGCAGCAGAAGAACTTCCCGTGCGCGCGGATCCTCGCTCCGTTGCACGGGCGCC TTAAGTTATTGGACTATCTAATATCTATGTATTTATTTCGCTGGTTCTTTGTAGTCAC ATATTTTATAGTCTTAATATCTTGTTTTTGCATCACTGTGCCCATTGCAAATAAATCA CTTGGCCAGTTTGCTTTTCTACCA |
| 55650 | PIGV | HSX2_02557993 | 629 | GGAGGAGCTGAGGTTCGGTTCGGTCCCCGCCGGG |
| 93035 | PKHD1L1 | HSX2_02151899 | 630 | AGTCCCAGGAGCCGAGCTCCAGCACTAGAGCCAGCTGCGAGCGGAGGGCACCAAC TCCGCAGAACTGGCTTTTCAATGGGACACCTGTGGCTCCTGGGTATTTGGGGCCTC TGTGGGCTGCTCCTGTGTGCCGCGGATCCCAGCACAG |
| 93035 | PKHD1L1 | HSX2_02151902 | 631 | ATGGCTCTCAAATAATCCCCAAAGTCACAGAAATAATACCTAAATATGGCAGTATAA ATGGAGCAACAAGGCTGACTATAAGAGGGGAAG |
| 93035 | PKHD1L1 | HSX2_02151911 | 632 | AGCAATGCCGGAAGATTCCTACACTGTTAGAGTCAGTGTGGACGGGGTTCCTGTTA CGGAAAATAACACCTGCAAAGGTCACATCAACAGCTGGGAATGTACCTTCAAC |
| 93035 | PKHD1L1 | HSX2_02151919 | 633 | G |
| 93035 | PKHD1L1 | HSX2_02151939 | 634 | AGTTTACATTGGAGGAATGCCCTGTGAGCTTCTCATACCACAATCTGATAATTT |
| 93035 | PKHD1L1 | HSX2_02151983 | 635 | AGGTCACCATGATTTTCCCTTCACAAGGAAGCATTCGAGGTGGCACCACGCTGACA ATAAGTGGGCGTTTCTTTGATCAGACAGATTTCCCCGTCAGAGTTCTAGTTGGAG |
| 93035 | PKHD1L1 | HSX2_02151994 | 636 | GTGAACCTTGTGATATTTTGAATGTCACAGAAAATAGTATATGTTGCAAGACACCCC CCAAACCTCATATTCTCAAAACTGTATATCCAG |
| 93035 | PKHD1L1 | HSX2_02152006 | 637 | GAGGGAGAGGCCTGAAGCTTGAGGTGTGGAATAATAGCCGTCCAATACGTTTGGA AGAGATACTGGAATACAATGAAAAAACGCCTGGGTACATGGGTGCCAGTTGGGTA GATTCAGCTTCCTATATTTGGCTCATGGAACAAGACACATTTGTTGCACGCTTTAGT GGATTTTTGGTGGCTCCAGATTCTGATGTTTATAGATTCTACATCAAGGGTGATGAC CGTTATGCTATTTATTTTAGCCAGACTGGACTTCCAGAAGATAAG |
| 93035 | PKHD1L1 | HSX2_02152050 | 638 | GTTATAACATTGGAAAACTGGGAAACAACTAATGCAATTAATGAGGTTCAGAAGAT CAAGGTAACCAGCCCATGTGTGGAAGCTAATTCATGTTCACTTTACCAATATAGATT AATCTATAATATGGAAAAAACTG |
| 93035 | PKHD1L1 | HSX2_02152077 | 639 | GAGACTTTGATCTGCTTGGTTATGAAGTAGTTGAAGGGAATAATGTCACACTGGAT ATTACAGAACAAACCAAAGGAAAACCCAACTTGGAGACATTCACACTGAATTGGGA TGGGATCGCTTCTAAGCCACTCACTCTATGGTCATCAGAAGCTGAA |
| 93035 | PKHD1L1 | HSX2_02152101 | 640 | GAACATATTAACAGAGGGCAGAAGACAGCTGAAACCGATGCTTACTGTGGTCGTTA TTCCCTGAAAAACCCAGCTGTTCTTTTGACTCAGCAGATGTTAAACCAAACAGACG ACCATATGGAGATATTTTATTGTTTCCTTATAATCAG |
| 93035 | PKHD1L1 | HSX2_02152113 | 641 | CAAGACAATAGCAAGATTACTAGAAGCACTGATACACAGTTTACATACAACTTTGCT TATGGAAACAA |
| 93035 | PKHD1L1 | HSX2_02152180 | 642 | AAAACTGAGGGTACAGTTGATATTTCAGTTACTACCAATGGATTTCAAGCCACAGC AAGGGATGCTTTTAGTTATAATTGTTTACAGACACCAATTATAACTGATTTTAGTCC AAAAGTACGAACAATACTAG |
| 93035 | PKHD1L1 | HSX2_02152186 | 643 | GAGAAGTTAATTTAACAATTAAGGGCTATAATTTTGGAAATGAACTCACACAAAAC ATGGCGGTGTATGTTGGAGGAAAAACCTGCCAGATTCTTCACTGGAACTTCACAGA TATTAGATGCCTTTTGCCCAAGTTGTCTCCTGGAAAACATGATATCTATGTAGAAGT CAGAAACTGGGGTTTTGCATCAACAAG |
| 93035 | PKHD1L1 | HSX2_02152208 | 644 | GTTCATTTTCTTACCAATTTACTTCTCCTGGAATCCATTATTATAGCAGCGGGTATGT TGATGAGGCTCACTCCATTTTTCTCCAAGGAGTCATTAATGTTTTACCAGCTGAAAC CAGACACATTCCCTTGCACCTGTTTGTGGGTCGCTCTGAAGCCACATATGCTTATG |
| 93035 | PKHD1L1 | HSX2_02152220 | 645 | ATGTCATATTGACCCTCAAAACTCAATGGATGTTGGTATCAGGGAAACTGTCACTTT GACTGTCTACAACCTGGGCACTGCTATCAATACGTTGTCCAATGAATTTGATAGGC GATTTGTACTTTTGCCAAACATTGACCTGGTGTTGCCAAATGCAGGATCAACTACAG GAATGACAAGCGTGACCATAAAAGGCTCTGGATTTGCCGTTTCTTCTGCAGGTGTA AAAGTCCTTATGGGTCATTTCCCATGTAAAGTTCTATCAGTGAATTATACGGCCATT GAATGTGAAACATCCCCTGCTGCCCAACAGCTTGTGGATGTAGATCTTCTAATACAT GGAGTGCCTGCCCAGTGCCAGGGAAACTGCACCTTTTCATACTTAGAAAGCATCAC TCCTTACATAACAGGAGTCTTCCCAAACTCTGTCATAGGATCTGTAAAAGTTCTTATT GAAGGAGAAGGTTTGGGGACTGTTTTGGAGGACATTGCTGTTTTCATTGGAAATCA ACAGTTCAGAGCAATAGAGGTTAATGAAAACAACATCACTGCTCTTGTGACTCCTCT CCCAGTTGGACATCATTCTGTTAGTGTTGTGGTGGGAAGTAAAGGCTTGGCTCTGG GAAACCTGACTGTCAGCAGCCCCCCAGTAGCATCTCTATCACCAACTTCTGGAAGCA TTGGTGGTGGAACTACACTGGTGATCACAGGAAATGGCTTCTATCCAGGCAACACT ACAGTCACTATTGGGGATGAACCTTGTCAAATTATTTCCATCAACCCCAATGAAGTC TACTGCCGCACTCCCGCTGGGACCACTGGAATGGTCGATGTTAAAATCTTTGTTAAT ACAATTGCTTATCCACCTTTGCTTTTTACATATGCCCTGGAGGATACTCCATTTCTCA |
| 93035 | PKHD1L1 | HSX2_02152254 | 646 | TGAAAATATGGAGGATGTTCATATCACCATAGCTGAAGCCAAATGTGATGTTGAGT ATTCCAACAAGACACACATCATCTGCATGACAGATGCCCATACTCTATCAGGGTGG GCTCCAGTTTGTGTCCACATCAGAGGTGTCGGCATGGCCAAACTG |
| 93035 | PKHD1L1 | HSX2_02152259 | 647 | GATAATGCTGACTTTCTTTATGTTGATGCCTGGTCCTCCAATTTCTCATGGGGGGA AAATCTCCCCCAGAAGAAGGATCTCTTGTTGTTATTACAAAAGGACAGACCATTCTG CTGGATCAAAGCACCCCTATTTTGAAAATGTTGCTTATTCAGG |
| 93035 | PKHD1L1 | HSX2_02152274 | 648 | ATTGGAACAGAGACATCCCCATTCCAACACAAGGCTGTCATTACCTTGCATGGTCAC CTGCGATCTCCTGAGCTCCCTGTCTATGGTGCCAAAACACTGGCTGTGCGGGAGGG AATCCTGGATCTGCACG |
| 93035 | PKHD1L1 | HSX2_02152281 | 649 | GTGTGCCTGTTCCTGTGACCTGGACTCGCTTGGCTCATACTGCAAAGGCAGGGGAA AGAATTTTAATTTTACAAGAAGCAGTAACATGGAAACCAGGAGATAACATTGTAAT TGCAAGCACAGGACACAG |
| 93035 | PKHD1L1 | HSX2_02152287 | 650 | TAAACATAACACTAAGTAACCCACTAAATTACACACACTTAGGAATTACGGTCACAC TCCCTGATGGAACTCTGTTTGAAGCAAGAGCAGAAGTTGGAATTCTTACAAGAAAT ATTTTAATAAGAGGATCTGATAATGTTGAGTGGAATAACAAAATTCCTGCATGTCCT GATGGATTTGACACAG |
| 93035 | PKHD1L1 | HSX2_02152293 | 651 | GAGAATTTGCTACACAGACCTGTCTCCAAGGAAAGTTTGGAGAAGAAATAGGAAG TGACCAATTTGGAGGCTGCGTTATGTTTCATGCTCCTGTACCTGGTGCTAACATGGT AACTGGGAGAATAGAATATGTAGAG |
| 93035 | PKHD1L1 | HSX2_02152298 | 652 | GCTTGGAGACTTACAGTTTAAATCTTATGTAAGAGGCTGTGCAATTCACCAGGCCT ATAACAGAGCTGTTACTATTCATAACACACACCATCTTCTGGTTGAGAGGAATATTA TATATGATATTAAGGGAGGAGCATTTTTTATAGAAGATGGTATTGAACATGGCAAT ATCCTCCAGTATAACTTGGCAGTATTTGTACAGCAAAGTACCAGTCTTCTGAATGAT GATGTGACCCCGGCTGCATTTTGGGTCACCAACCCGAACAATACCATACGACACAA TGCTGTTGCTGGTGGCACTCACTTTGGCTTTTGGTACCGGATGAACAACCACCCTGA TGGGCCATCCTATGACAGAAACATTTGTCAAAAAAGAGTTCCCCTTGGCGAATTTTT TAACAATACTGTCCATTCTCAAGGTTGGTTTGGAATGTGGATCTTTGAGGAATATTT CCCCATGCAAACGGGATCTTGTACATCTACAGTGCCTGCACCTGCAATATTTAACTC ACTTACTACTTGGAATTGTCAAAAAGGAGCTGAATGGGTCAATGGAGGTGCCCTTC AGTTCCATAACTTTGTGATGGTGAATAACTATGAGGCTGGAATTGAGACTAAGAGG ATCCTGGCTCCTTATGTTGGAGGGTGGGGTGAAACCAATGGAGCGGTGATTAAAA ATGCCAAAATAGTCGGCCATCTTGATGAACTGGGAATGGGGTCTGCATTTTGCACA GCAAAAGGCCTGGTTCTCCCATTTAGTGAAGGCTTGACTGTCTCTTCTGTGCACTTT ATGAACTTTGACCGTCCCAACTGTGTAGCTTTGGGAGTGACATCCATCTCTGGAGTT TGTAATGACAGATGTGGGGGTTGGAGTGCAAAGTTTGTTGACGTCCAGTATTCTCA |
| 93035 | PKHD1L1 | HSX2_02152308 | 653 | CTCAGGAAGCTGAGTGGAGCATTGGGTTCCCTGGATCAGTCTGTGATGCTTCAGTC AGCTTTCACCGTTTAGCGTTCAACCAGCCTTCTCCAGTATCTCTGCTTGAAAAGGAT GTGGTTCTTTCAGACTCTTTTG |
| 93035 | PKHD1L1 | HSX2_02152318 | 654 | GCACAAGCATTATTCCATTTCAGAAGAAACGACTGACTCATATGTCTGGATGGATG GCTCTGATTCCAAATGCAAATCACATTAACTGGTATTTTAAAGGTGTGGATCACATA ACCAACATTTCATATACATCGACATTCTATGGATTCAAG |
| 93035 | PKHD1L1 | HSX2_02152328 | 655 | GAAGAAGACTATGTAATTATATCACATAACTTCACTCAAAATCCTGACATGTTTAAT ATTATTGATATGAGGAATGGTTCCTCAAATCCATTGAATTGGAATACTAGCAAGAAT GGGGACTGGCACCTTGAAGCAAACACTAGTACTCTATATTACTTGG |
| 93035 | PKHD1L1 | HSX2_02152339 | 656 | GATGTGAAAGACGTTGTTATTAATTTCCAAGCTTACTGTTGTATTCTCCAGGATTGC TTTCCTGTACATCCGCCATCAAGAAAACCAATTCCCAAGAAGCGACCAGCCACATAT AA |
| 93035 | PKHD1L1 | HSX2_02152370 | 657 | GGAGGTAGATTAATCGGTGGCTGGGAAGATAACCCTTTTAAAGGAGACTTAAAGA TTGTTCTTAGAGGAAATCATACTACACAAGACTGGGCTCTTCCAGAAGGACCAAAT CAAGGGGCAAAGGTCTTAG |
| 93035 | PKHD1L1 | HSX2_02152379 | 658 | CTCTCAGAAACTGCATTTGCAGGTTCCAAAGTCCTGTCTCTGATGGATGCTGTGGAT TGGCAG |
| 93035 | PKHD1L1 | HSX2_02152389 | 659 | GAGGGAGAAGAGATTGTGATAACAACCACAAGCTACGATTTCCACCAGACAGAAA CAAGAAGTATCGTTAAAATCCTGCATGATCATAAAATTCTCATTCTTAATGATAGCC TTTCCTATACTCACTTTG |
| 93035 | PKHD1L1 | HSX2_02152399 | 660 | GGGATACTGAGTAGGAACATCAAAATAGTTGGTGAAGATTACCCCGGTTGGTCTG AGGACTCTTTTGGAGCACGCGTACTGGTTGGCTCATTCACTGAAAATATGATGACA TTTAAAG |
| 93035 | PKHD1L1 | HSX2_02152410 | 661 | GAAATGCAAGAATAAGTAATGTGGAATTTTATCACAGTGGTCAAGAAGGCTTCAGG GATAGCACAGATCCAAGATATGCTGTAACGTTTCTTAACCTAGGACAG |
| 93035 | PKHD1L1 | HSX2_02152474 | 662 | AGCTCATTAATTGTTGGAAGTAGCCCTGGGTTTAATTGCTCTGATGTCCTAACTAAT GATGATCCTAATATTGAACTCACTGCTGCTCATCGGAGTCCTAGATCTCCATCAG |
| 93035 | PKHD1L1 | HSX2_02152481 | 663 | CGAAAGCCCCATGCAGGAATCATGAGTTACAATGCCATCAGTGGCCTTTTGGACAT CTCAG |
| 93035 | PKHD1L1 | HSX2_02152497 | 664 | GAATTATTAGAGATTCAACCTGTAAGTACCTTCCAGAGTGGCAGAGCTATCAGTGC TTTGGGATGGAATATGCAATGATGGTTATTGAAAGTCTGGATCCTGACACAGAAAC TCGAAGACTTTCCCCAGTGGCTATAATGGGCAACGGTTATGTTGATCTTATTAATG |
| 93035 | PKHD1L1 | HSX2_02152524 | 665 | GTGACTGCCCAGCCAGTTGAAAGGTCTGCATTTCCTGTTCATCACGTGGCCTTCGTG TCCTCACTCTTAGTGATCACTCAGCCGGTGGCAGCACAGCCAGGACAGCCATTTCCT CAGCAGCCTTCGGTAAAGGCAACAGATTCTGAC |
| 93035 | PKHD1L1 | HSX2_02152529 | 666 | GGTAACTGTGTATCAGTTGGAATTACTGCACTAACTTTGAGGGCCATACTCAAGGA CTCCAATAATAACCAAGTCAATGGCCTTAGTGGAAATACAACAATTCCGTTTAGCAG CTGTTGGGCCAACTACACAGACCTTACTCCCCTTAGAACAG |
| 93035 | PKHD1L1 | HSX2_02152541 | 667 | TAGCTACTTTGTTGGGCAATAGGCAAAAGTCTATAGCATTTTCATGAAAATATACTA AAAATATTTTATGATATATAAAATGTACTAATTAGCTTTAAACACTAAAATCAGATT TCTTCAAAATATAAATTTGTTTTGATTCTTTATATTTATATGTTTTTATTTCATTTCAAT AAACTTCCAGAAATTTGTCATTTGGAAGTAGATATGACACCTCTAAGTTATTGTACC AACAAATCATAGAATCCTTTAAATAATGGAAAGAGCTTGTCTAGTTCCGACTTAAGC TCTTTACATCAGATTGGAAGCATTTTAAGTATTCTTTTTAATACTTGAGGGGGGGGG TTCTTATTTTCTCTATCCATTTACAAGGTTTTGATTTCCAATAATCTAAGCCATTCTAG TTTCTAAGGATTTTGGAGAGACGAGATTGTCTTCACACAATCTGAGATGACAATCTC ATCTCAGATTTTTCACACTGAGTTCCTGTCTTTCAGATTCACAGTAATCCCAGGAATT CTAGACTGTCCTGTATTTCTGGATCTGCCCAACTCTGGCCAATATTGGGAAAAGTTT TCTTGTCCTTGGAATGAACTTTTGGAACAAGTCCAAACTCCTTCTCTGCCTACCCCTC CTTCCCACTCAAAACTGCCACTACTAAGAGCAGGACTCCCCTCAACCCCGGGCATGA GTTGAGGGTTGAGGGGTTCCCTTAACTGGTGGATGCTTATTCACTGTTTACTGGAT GAATGAAGTATAAAGTGTGGGAAGTCAACAACAGAACAGAACTTATTTTCAAGAA GATAAATTAGAGAATGCAAAAAAGCTACAGACTAAGGTAGCTAAGTTAACCGAACT CTCTAACAGTATTGAAAATAGCAATTCTTTACTCAGAAAATTCTAAAGGGAATACTT AATTTGACAGAACTCCTAATAAAGACATTGTAGCCAGATCCAGAGCCTTCCAGCAA GTGACACTCAGCAAACATTTGGGACAGCTGAGAGAATTCAACAAAGCCCATACCGT TGTCTCTTGTATAACATCTCCTAGTACATTCACCTCAGATCATTGTTAGTCTTACTTA |
| 5315 | PKM | HSX2_01127816 | 668 | CGGCGGCCCGCAGCGGGGCTCCTTGCACAGCAGC |
| 22925 | PLA2R1 | HSX2_01578093 | 669 | AGGTAAATTTTGAGCCATTTGTTGAAGATCACTGTGGAACATTTAGTTCATTTATGC CAAGTGCCTGGAGGAGTCGGGATTGTGAGTCCACCTTGCCATATATATGTAAAAAA TATCTAAACCACATTGATCATGAAATAGTTG |
| 22925 | PLA2R1 | HSX2_01578104 | 670 | AAAAAGATGCGTGGAAATATTATGCTACCCACTGTGAGCCTGGCTGGAATCCCTAC AATCGTAATTGCTACAAACTTCAGAAAGAAGAAAAGACCTGGCATGAGGCTCTGCG TTCTTGTCAGGCTGATAACAGTGCATTAATAGACATAACCTCATTAGCAGAGGTGG AGTTTCTTGTAACCCTCCTTGGAGATG |
| 22925 | PLA2R1 | HSX2_01578263 | 671 | ATATTCACACTGCAGAGGCGCTGCCAGAAAAAG |
| 22925 | PLA2R1 | HSX2_01578389 | 672 | TTGGGGGAATTTTAAAACTAATCTGGTATCTATTCAGACATTTACCTGCACTCGTAC CATTAAGAAAGACAGAAAGAAGCAAAAAAAAATTAATCTTGTATATGAGAGGAAA AGGAAAGGGCTTCTGAGAGGATTCTTAGTTGTTTCTTTTGAATTCACTTTTAATAGC AGGATTTGGAAAATACTAATTTCTGTGCTTAAGGGTCACAGGTTCTGGGCTCTCAA CTGATATTTAAGGTGACATTCATTTTTATTAGGTCTAACATCTCAAGCTAAAGGAGA AAGAAAAATACCTTCTTTTAAATGGCAAAGACCTTCATTAGCAGCACACTTTTATAA ACAACCATATAGTTAAAATGTGGCCTTAAACTTTCAATTACTAAATGATGATTAAGT TGGATATTTTAAAATGTCTTATACATAAGCTTAAAGTAATATATTGAAACTTTAACA GTTGTACTGTTAAAACTCATGGACTTTCTGAAAAACTAAATATATAAAATAATATGT AACACTCTTAATAACTGGAAGATATAAAATGTAACTTGGATTTAAAGGAGAAGAGC TAAAGATCTGAATTATAGTCTCATTAGAACCAGACAGATGTTGTTGAGAAGTACTA AATAAATTAGTAATCTAGATATCTTTATTATGTAAATGAGTTTAGGTGTTCTATTTAA |
| 57664 | PLEKHA4 | HSX2_01939952 | 673 | TCCGGTCCTGCCACAAGACGAGGGGCATGGCCTCTGCGAGTCACTCTGCTACAAT CCAGCTTCTAATCCGCCCAAAAGCGGCAGCCAATCGGAGCGCGAGGACGTGGTCT GGAGGTACCGCCGGAGATCTGGGACCACTCAGGGCATCAGGGGGCGTGGTCTGG TCCCCATTGCGGGCCCGGGAGGGGAATGGTTTCTATGGCCAAAGTTTGGTTTTCTC AACACTGTCTAAATTTGGATTAAAACTTTGAACTTTTA |
| 57664 | PLEKHA4 | HSX2_02562176 | 674 | GACCCCACGCGCCAGGAGACCCCTCCCCCCAGATCTCCCCCGGTGGCTAATTCGGG TTCCACGGGGTTCTCTCGCCGAGGGAGTGGGCGTGGAGGAGGTCCCACCCCCTGG GGGCCCGCGTGGGATGCCGGGATCGCCCCTCCGGTCCTGCCACAAGACGAGGGGG CATGGCCTCTGCGAGTCACTCTGCTACAATCCAGCTTCTAATCCGCCCAAAAGCGGC AGCCAATCGGAGCGCGAGGACGTGGTCTGGAGGTACCGCCGGAGATCTGGGACC ACTCAGGGCATCAGGGGGCGTGGTCTGGTCCCCATTGCGGGCCCGGGAGGGGAAT GGTTTCTATGGCCAAAGTTTGGTTTTCTCAACACTGTCTAAATTTGGATTAAAACTTT GAACTTTTA |
| 5362 | PLXNA2 | HSX2_01133693 | 675 | TGGCCACACACCCCTGACCATCACAGGCTTCAACCTGGATGTCATTCAGGAGCCAA GGATCCGAGTCAAATTCAATGGCAAAGAATCTGTCAAT |
| 55558 | PLXNA3 | HSX2_01855156 | 676 | TGGAAGCACTGCCATCACTGTGAGTGGGACCCACCTGCTGACGGTCCAGGAGCCCC GGGTCCGTGCCAAGTACCGCGGCATTGAGACCACCAAT |
| 5365 | PLXNB3 | HSX2_02513200 | 677 | AACAGGAGCCTGGGCTGCCTGTGGTGTGCTGACGGCCAGCCTGCCTGTCGCTATG GGCCCTTGTGCCCGCCGGGGGCTGTGGAGCTGCTGTGTCCTGCGCCCAGCATTGAT GCA |
| 5371 | PML | HSX2_01134444 | 678 | CCCT |
| 10585 | POMT1 | HSX2_01513339 | 679 | AAGTACATGGGTGTGTTCACGTACGTGCTCGTGCTGGGTGTTGCAGCTGTCCATGC CTGGCACCTGCTTGGAGACCAGACTTTGTCCAAT |
| 10585 | POMT1 | HSX2_02537077 | 680 | CATCAAGTACATGGGTGTGTTCACGTACGTGCTCGTGCTGGGTGTTGCAGCTGTCC ATGCCTGGCACCTGCTTGGAGACCAGACTTTGTCCAAT |
| 64091 | POPDC2 | HSX2_02563563 | 681 | CGGGCCAGGTTGTCCAGGTCACTCTGACTGCTGA |
| 25833 | POU2F3 | HSX2_01660779 | 682 | TAACGTCATCCTGTTCCCCTGGGAACAACAGCAGGCCTTCATCTCCTGGCTCAGGAC TCCACGCCAGCAGCCCCACTGCATCTCAAAATAACTCCAAAGCAGCAGTGAACTCC GCCTCCAGTTTTAACTCTTCAGG |
| 25833 | POU2F3 | HSX2_01660796 | 683 | ATCTTGGTACCGATGGAATCATTCCACCTACCTCCACTGAGACCAAAAAGTTTCTCC TACTCCAGCTGGCCCTGTATTCCCCTGG |
| 5504 | PPP1R2 | HSX2_01142158 | 684 | GGAAGAATCAAATCAAGGATCTACTCCAAGTGAC |
| 129285 | PPP1R21 | HSX2_ 02204481 | 685 | TGCACAGAGCCGCAGGGCTGAGACCACGTCCATG |
| 5567 | PRKACB | HSX2_01146811 | 686 | CAGCTGGCTATCCCCCATTCTTTGCAGACCAACCAATTCAGATTTATGAAAAGATTG TTTCTGGAAAG |
| 222171 | PRR15 | HSX2_02304267 | 687 | GGGGTGGAGTGAACGGCCGGAGACCACGTGGAGAAAGGGGCCGCTTTGGCCCTT CCATCTGGGTGCCGGGAGCCCCTAGGCCCTCCGGCCATGGCCGACAGCGGCGATG CTGGCAGCTCCGGCCCCTGGTGGAAATCGCTCACCAACAGCAGAAAGAAAAGCAA GGAAGCCGCAGTGGGGGTGCCGCCTCCCGCCCAGCCCGCTCCCGGGGAGCCCACG CCACCTGCGCCGCCCAGCCCGGACTGGACCAGCAGCTCCCGGGAGAACCAGCACC CCAATCTCCTCGGGGGCGCCGGCGAGCCCCCCAAACCAGACAAGTTATACGGGGA CAAATCCGGCAGCAGCCGCCGCAATTTGAAGATCTCGCGCTCCGGCCGCTTTAAGG AGAAGAGGAAAGTGCGCGCCACGCTGCTCCCGGAGGCGGGCAGGTCCCCGGAGG AGGCAGGCTTTCCTGGTGACCCCCACGAGGACAAGCAGTAGCCCCAATAGCCTGCG CGCTCCAGGACTGCCTACCCAGCACTACCCCAAACCCCCAGTTCCAAACCCGAGACT TCAGGCCCGCCCCCTTACGCGTTGTCTCATTCCACCAAATTCAGAATATTTACACAAT GCCTTCATGATTTTATTTTTCTGGAAATTGAAGTGTCAATTGGGTTCTCAATATTTCA TGACTCCAAGGATGCATTAAATATTTATTTGTGGTAAGAGAAGATACCTGCCGCGG AGGAGGGTGGCATAATTATTTTTTTTTCAAAAAGCTATTCTGGGGCCCTGGCCCCCT GGCTCCTGTCACAGCTCAGCTTGAACTCTGTAGCCTCTCAAATGAAGAAGGTGGCT GTTATTTAGGACTCTGTGGAAAGCAAATCACAGTGCTGTTTTTAATGCCTGAGAAAT GCACTTTAGGGGTAGACTTATACCTTAAGTGAAGGAGTGGGGGAGGAAGAAGCAT TCTTAAGTAGAAGCATTTTAAAATAAGGCAGGATTGCTTTTAATGTTAAACTACAAA |
| 11098 | PRSS23 | HSX2_01548939 | 688 | CTTCATGTGGACCCCAGTGTCATAAGGGAACTCCA |
| 23362 | PSD3 | HSX2_01629459 | 689 | GATCAGCGAACAAGAGGAGCACGTTAAGGGGGAAGATGAAGACATCCTTGGGCCT GGATATACGGAGGACTCCACCGACGTGTACAGCTCCCAGTTTGAAACCATTTTGGA CAACACTTCTTTATACTACAGTGCAGAGTCCCTGGAGACATTATACTCAGAGCCTGA TAGCTATTTTAGCTTTGAAATGCCCCTCACTCCAATGATACAACAGCGCATTAAAGA AGGTGGTCAGTTCTTGGAGAGGACATCAGGGGGAGGACATCAGGATATCCTG |
| 23362 | PSD3 | HSX2_01629461 | 690 | GCTGAATGATGCCAGCGACTCCATCTACACGAAAGGCACCCCGGAGATTGCTTTCT GGGGAAG |
| 23362 | PSD3 | HSX2_01629472 | 691 | GAGAGAGACAGGGAAAGGATCAGCGAACAAGAGG |
| 23362 | PSD3 | HSX2_01629478 | 692 | CAATGCTGGGGTGAAAACAACACGGCTAGAAGCTCATTCTGAAATGGGGAGCACT GAAATTTTGGAAAAGGAGACCCCAGAAAATCTCAGTAATGGTACCAGCAGCAATGT GGAAGCAGCCAAAAGGTTGGCCAAACGCCTTTATCAGCTGGACAGATTCAAAAGA TCAGATGTTGCAAAACACCTTGGCAAGAA |
| 23362 | PSD3 | HSX2_01629528 | 693 | AC |
| 23362 | PSD3 | HSX2_01629587 | 694 | AGATGATGAAGAGAAAAAAAAGTCTCCCTCAGAAAGTACTGAGGAGAAAGCTAAC GGAACACATCCAAAGACCATCAGTCGTATTGGAAGTACTACTAACCCATTTTTGGAC ATTCCTCATGATCCAAATGCTGCTGTGTACAAAAGTGGATTCTTGGCTCGGAAAATT CATGCAGATATGGATGGAAAGAAGA |
| 23362 | PSD3 | HSX2_01629635 | 695 | GAGGAGCAACTGAAGTCACATGAAAGTAAGCTGAAGCAGATCACCACCGAGCTGG CCGAGCACCGCTCATATCCCCCCGACAAGAAGGTCAAAGCCAAGGACGTCGATGA GTACAAACTGAAAGACCACTATCTGGAGTTTGAG |
| 23362 | PSD3 | HSX2_01629644 | 696 | GAGTAACGATGAAAGCGAGGCTGCAGGACTGAAGAAGTCGCACTCGAGTCCTTCG CTGAACCCGGATACTTCTCCAATCACTGCCAAAGTCAAGCGTAACGTGTCAGAGAG GAAGGATCACCGACCTGAAACACCAAGCATTAAGCAAAAAGTTACTTAGAGTCCAT CTGCGGCCAGGAAGTGCTGGTCATGGAG |
| 23362 | PSD3 | HSX2_01629645 | 697 | CAAAATAGGGTTTTCAAGATCTTTCTGGTAATCCGTGAATATATTTAAAAAAAAAA AGTCTGTGACAAAACGGTGCATTAGTAATTTTTCTATTGTATATTTTGTTAGTTTC TGTACAGATTGTCTTTGCTCTTGATTTCTTTTGCTTTGATGATTTTGCAACTTGATA GCTAATGCACCTTTTCTGTGAGGAGGAGGGGATCGTGATTTCAGAATGAATTATGT ATCCCTTCTCTTTTGGTTTTCTCTTGTTTGCAGTCTGCTCAGTTGTTTTATGTATTCTC ATATCAACTGTTAAACTTTTTTTAAGGTTAAAGAATTTAATCCATTGTGAAACACTT AACTGGACAAACTGTAGTTTTAGTAAATTCTAGCTGGAGTTAATATACGCCTTTATA TGTGAAATCTTGCCCAGTCACAGAGGTAGAATTGAGCACTCACAGATGCTCCAGTA AGAATCACAGTGCTGGGAATCTAGTTGCTCCAATATGAGGCAGCTTCATGTGCAGC TTAGCACTTGTTGTTGAGATCGGACCCTGCTGGAAGCAGGGAAAAGAAGCGTGAA GATCGTAGGATTGAGAACTTAGGGAAGCACATTAGCTTGCTTGAAGTGCTGATTCC ATTTCAGCCAAGCAAGGGAAAGAGGAAGTGGAGTCATTTTGCCTTTGAAGGCTGA GGAAAGATTGATACCCAGTTAATTTTGTTTGCTAAAGGATGGGGGCAATAATCGGC CCTTGAGGAGCTGCAGCAGTAGGCATGTGCTCAGTCTGCAGGAATTGTTACCTCAC TCCCACAGGGTCTAGACTAGAAATCCATCATCTCTATCGTTGATATCCTTCCATCCA GGAATAGATTTTTCTTACTCTACATATGTGTGTGTGCGTGCGTGTGTGTGCGTGTGT GGGCATGGGGTTGTGTCCTGGTTGTGATATTGAGGTCTTCCTTCCTAACAAATTAAT ACTAAAATGAAACAGCTTTTCTTGTGTCCTTAAGACAAAATAAGGAAGGAAAACGT AGCTGCAGTTGTCCACGATGGATATTGGTTCTTTAAAATATATCTGAAAGTAGTAGT CAGAATGAATTATGGTTGGAAAACTGAGGAATCTTCTGGTTGCAGGTGCAAAGTG ACTTTGTTTATTCTTGTCTCAGTCTCCTTGATAGCCACTTCACTCTGCTACTACTCAAC TTTCTCCTAAAAATACTTCATCTATTTTCAGTCCTTTCTTTCTGTCTACTCAAAATGGT TCTATTAACTTTGCAGTCATGAGCTTGTTCCAGTTACAGTCCCTTTGAAGTTCAGGG TGATAAACAGAATATTCTTCTGTAGAGGAAGAGAAAGGAGTGAAAGTTTAGCCCAC TGAGACCTAGAGCTTTGTGATTTCCTAACCTTGAAACTCTGTAATCCCTAAAGTTAA AATCTCCGCAAGTGGCACAACTTCAGAACTAATAGTATCACTTTGATTTTTCTTTTTC CTCCCTTAGAAAGTTTCTCTAGTTCTATAGTTTATTTGTTGAAGGTACTATGACCAAA GAATCAGCTGCTCTACAGGAATAGCATGGTTCCAGTGAATTAGAGAAAACCTGCTG TAAAGCCATGGTAGTGTCTAAGTGGTATGTTATTATGATGTACTAGCATTTATTTAC AGAATTATTTATTAACGTTTACTTCCTTCCCCTCTGTAAATGTCCATGACTATTGCCC AGAGAAGGCTTACCCCTCTCTAGGGTTGCAGTTGCTTTCTTTGTAATAAGTATTTTG CCACACCTGTAAAAAAAAAAACCTCACTTTTAACTCTCTGCCTTGTTTGGGTAAAGG CAGTAACTAAGTTTATGTTTCAGAACTGCAAAACAAACAGGATAGTTACCAATATG GCCCATGTATCAGATTGATTTTTGTAGCCTCTCACTGAATCCAACATATCCACAAGC AAGTTATCTGTCTTTCTACCTGATAATCTAAATTATCAGGATATTTGTTTCTGCCTA |
| 5725 | PTBP1 | HSX2_02515245 | 698 | GCCTTTCCGTTCCGAACGTCCACGGCGCCCTGGCCCCCCTGGCCATCCCCTCGGCGG CGGCGGCAGCTGCGGCGGCAGGTCGGATCGCCATCCCGGGCCTGGCGGGGGCAG GAAATTCTGTATTGCTGGTCAGCAACCTCAACCCAGAG |
| 5747 | PTK2 | HSX2_01163391 | 699 | TTGCCATCAATACCAAAGTTGGCCAACAGCGAAA |
| 5786 | PTPRA | HSX2_01166131 | 700 | ATGACCACTCTAGAGTCCACCTGACACCGGTTGAAGGGGTTCCAGATTCTGATTAC ATCAATGCTTCATTCATCAAC |
| 5793 | PTPRG | HSX2_01167541 | 701 | ACAGCGACAAAGACTTGAAAGCCACCATTAGCCA |
| 25797 | QPCT | HSX2_01658589 | 702 | GAGGCTTTTCTTCACTGGTCTCCTCAAGATTCTCTCTATGGGTCTCGACACTTAGCT GCAAAGATGGCATCGACCCCGCACCCACCTGGAGCGAGAGGCACCAGCCAACTGC ATGGCATG |
| 5873 | RAB27A | HSX2_01173704 | 703 | GAGGAAGCCATAGCACTCGCAGAGAAATATGG |
| 5873 | RAB27A | HSX2_01173714 | 704 | GAAATATTTCAGAGCCAAAAGTGCCTTATACAACCTTAGCCTATTATAGTAAATCAT TCAAGGATTCAGAATTTTGCAGTCACAGAAGAGTGTATTTATTATGTAGAATGAAT GAGGGTACTGTCACCTGCCTTAATGTAGGTAGGCCCAGAGTCTTACATTTAAGATC TTACATGCAGTTATAAAACCGCCACAGTCTTCAATCCAGATTTGAAGACTCATGCCA TAGGTGACATTCTAAAATACCATTAAAGCCACTTAAATGTTAAATAAGAATATACAT GCACATCAGCTCAATGTCTTTGAGTATTAATTTTATGTAAGCATTCTATTTAACATGA ATATAGGACAAATCATGGCTATATCTATAGACCTTGGATAAACTGGATTGACCAATT ATACACTCACGGTGACTTTTTTATTGGTGGGAAGGGGATTGGGGTGGGGCAGGCT GGCTTAATGTAATATGAGCAACCAAAGTGGGACTTCTGTCTCCCCGCTATATTCCCA TTGCTCTGAATGGTTGATTGAAGGGTCAGGGAACTAGATTTTATGGCTTTAGTTCA CTGTGATTGTACATTTATACTTGGCCTATGTGCTGGCCGCACCTGAACATAGCTGG |
| 7033 | RAB37 | HSX2_01262852 | 705 | CTGCAAACCAGTGTGCCGTGCCAGCCAAGGACAGGGTGGACTGCGGCTACCCCCA TGTCACCCCCAAGGAGTGCAACAACCGGGGCTGCTGCTTTGACTCCAGGATCCCTG GAGTGCCTTGGTGTTTCAAGCCCCTGCAGGAAGCAG |
| 5887 | RAD23B | HSX2_01174939 | 706 | GGAATCCCTGGAGATAGAGAAAGTCAGGCTGTGGTTGACCCCCCTCAAGCAGCTA GTACTGGGGCTCCTCAGTCTTCAGCAGTGGCTGCAGCTGCAGCAACTACGACAGCA ACAACTACAACAACAAGTTCTGGAG |
| 5887 | RAD23B | HSX2_02516158 | 707 | CGAGAGCAAGTAATTGCAGCCCTGAGAGCCAGTTTCAACAACCCTGACAGAGCAGT GGAGTATCTTTTAATG |
| 26953 | RANBP6 | HSX2_01694312 | 708 | GGTTTATCCAAATCTGCCTGCTGATGTTCAGAGAGATGTCAAGATTGAACTGATTCT GGCTGTTAAGTTAGAAACACATGCTAGCATGAGGAAAAAACTTTGTGATATTTTTG CAGTGCTGGCCAGGAATTTGATAGATGAGGATGGCACTAACCACTGGCCGGAAGG TCTGAAGTTTCTTATTGATTCAATCTACTCCAAAAATGTGGTTCTATGGGAAGTTGC ACTTCACGTTTTCTGGCACTTTCCTGGGATTTTTGGGACCCAAGAGCGGCATGATTT GGATATCATCAAACGGTTGTTGGACCAGTGTATTCAAGATCAAGAACATCCAGCAA TCAGGACATTATCCGCTAGAGCTGCAGCTGCATTTGTACTTGCTAATGAGAATAATA TTGCTCTTTTCAAAGACTTTGCAGACTTGCTTCCTGGAATCTTACAGGCTGTGAATG ACTCATGCTACCAGGATGATGATTCAGTGCTAGAATCCCTTGTTGAGATTGCAGAT ACCGTACCTAAGTACTTGGGTCCTTATTTAGAAGATACTCTACAGTTGAGTTTGAAG TTATGTGGAGACTCTAGGCTTAGTAATCTGCAGCGCCAGCTGGCCCTCGAAGTTAT AGTGACCTTGTCTGAAACTGCCACTCCGATGTTGAAAAAACATACAAATATTATTGC ACAGGCAGTTCCTCATATATTAGCAATGATGGTTGATCTACAAGATGATGAGGACT GGGTAAATGCTGATGAAATGGAAGAAGATGATTTTGACAGCAATGCAGTTGCTGC GGAGAGTGCACTAGACAGACTGGCTTGTGGGCTTGGTGGAAAAGTTGTTTTACCA ATGACCAAGGAGCATATCATGCAGATGCTTCAGAGCCCTGACTGGAAGTATCGACA TGCTGGATTAATGGCCTTATCTGCCATTGGAGAAGGATGCCATCAACAAATGGAAT |
| 51195 | RAPGEFL1 | HSX2_01752559 | 709 | TCCGCCGGATAGACTCCTGTCTGCAGACCCGGGTGGCCTTCCGGGGCTCTGATGAG A |
| 5921 | RASA1 | HSX2_01177981 | 710 | CCAGTAGAAGATAGAAGGCGTGTACGAGCTATTCTACCTTACACAAAAGTACCAGA CACTGATGAAATAAG |
| 158158 | RASEF | HSX2_02259119 | 711 | AGAAGAGCAAGTTAGTACCTTGTACCAAAACATCAACCTTGTGGAGCCAAGATTAA TTCAGCCATATGAACATGTTATAAAGAACTTCATCCGTGAGATCAGACTTCAAAGCA CAGAAATGGAAAATTTGGCCATTGCGGTGAAGAG |
| 158158 | RASEF | HSX2_02259164 | 712 | TCAGAGTGAGTTAGATGCTTTGAAAAGTGATTATGCTGATCAGAGTCTGAATACTG AAAG |
| 158158 | RASEF | HSX2_02259175 | 713 | GGATCTGGAAATAATCCGAGCATACACAGAAGATCGAAATAGTCTTGAGAGGCAA ATTGAAATACTCCA |
| 158158 | RASEF | HSX2_02259183 | 714 | AACAGCTAACCGGAAGCTACATGACAGTAATGATGGCCTTAGAAGTGCCCTTGAAA ACAGTTATAGCAAGTTCAACAGATCTTTG |
| 1000 | RAVER1 | HSX2_00831170 | 715 | GGGATGTTGAGGTACAGAATCGTGTCTCAGGCTCCAAGCACCCCTTCACCCAACAT GTTTACAATCAACAATGAGACTGGTGACATCATCACAGTGGCAGCTGGACTTGATC GAGAA |
| 1000 | RAVER1 | HSX2_00831175 | 716 | AAAGTGCAACAGTATACGTTAATAATTCAAGCTACAGACATGGAAGGCAATCCCAC ATATGGCCTTTCAAACACAGCCACGGCCGTCATCACAGTGACAGATGTCAATGACA ATCCTCCAGAGTTTACTGCCATGACG |
| 1000 | RAVER1 | HSX2_00831180 | 717 | GACCGATAAGGATCAACCCCATACACCAGCCTGGAACGCAGTGTACAGAATCAGTG GCGGAGATCCTACTGGACGGTTCGCCATCCAGACCGACCCAAACAGCAACGACGG GTTAGTCACCGTGGTCAAA |
| 1000 | RAVER1 | HSX2_00831185 | 718 | GTGCCATTAGCCAAGGGAATTCAGCACCCCCCTCAGTCAACTGCAACCGTGTCTGTT ACAGTTATTGACGTAAATGAAAACCCTTATTTTGCCCCCAATCCTAAGATCATTCGC CAAGAAGAAGGGCTTCATGCCGGTACCATGTTGACAACATTCACTGCTCAGGACCC AGATCGATATATGCAGCAAAATATTAG |
| 1000 | RAVER1 | HSX2_00831190 | 719 | ATACACTAAATTATCTGATCCTGCCAATTGGCTAAAAATAGATCCTGTGAATGGACA AATAACTACAATTGCTGTTTTGGACCGAGAATCACCAAATGTGAAAAACAATATATA TAATGCTACTTTCCTTGCTTCTGACAATG |
| 1000 | RAVER1 | HSX2_00831195 | 720 | ACAATGCCCCTCAAGTGTTACCTCAAGAGGCAGAGACTTGCGAAACTCCAGACCCC AATTCAATTAATATTACAGCACTTGATTATGACATTGATCCAAATGCTGGACCATTT GCTTTTGATCTTCCTTTATCTCCAGTGACTATTAAGAGAAATTGGACCATCACTCGG CTTAATG |
| 1000 | RAVER1 | HSX2_00831200 | 721 | TTCCCATCATAATCACAGATTCGGGTAATCCTCCCAAATCAAATATTTCCATCCTGCG TGTGAAGGTTTGCCAGTGTGACTCCAACGGGGACTGCACAGATGTGGACAGGATT GTGGGTGCGGGGCTTGGCACCGGTGCCATCATTGCCATCCTGCTCTGCATCATCAT CCTGCTTA |
| 1000 | RAVER1 | HSX2_00831205 | 722 | TCCTTGTGCTGATGTTTGTGGTATGGATGAAACGCCGGGATAAAGAACGCCAGGCC AAACAACTTTTAATTGATCCAGAAGATGATGTAAGAGATAATATTTT |
| 1000 | RAVER1 | HSX2_00831219 | 723 | GTTTGACTATGAAGGCAGTGGCTCCACTGCTGGGTCCTTGAGCTCCCTTAATTCCTC AAGTAGTGGTGGTGAGCAGGACTATGATTACCTGAACGACTGGGGGCCACGGTTC AAGAAACTTGCTGACATGTATGGTGGAGGTGATGACTGAACTTCAGGGTGAACTT GGTTTTTGGACAAGTACAAACAATTTCAACTGATATTCCCAAAAAGCATTCAGAAGC TAGGCTTTAACTTTGTAGTCTACTAGCACAGTGCTTGCTGGAGGCTTTGGCATAGG CTGCAAACCAATTTGGGCTCAGAGGGAATATCAGTGATCCATACTGTTTGGAAAAA CACTGAGCTCAGTTACACTTGAATTTTACAGTACAGAAGCACTGGGATTTTATGTGC CTTTTTGTACCTTTTTCAGATTGGAATTAGTTTTCTGTTTAAGGCTTTAATGGTACTG ATTTCTGAAACGATAAGTAAAAGACAAAATATTTTGTGGTGGGAGCAGTAAGTTAA ACCATGATATGCTTCAACACGCTTTTGTTACATTGCATTTGCTTTTATTAAAATACAA AATTAAACAAACAAAAAAACTCATGGAGCGATTTTATTATCTTGGGGGATGAGACC ATGAGATTGGAAAATGTACATTACTTCTAGTTTTAGACTTTAGTTTGTTTTTTTTTT TTCACTAAAATCTTAAAACTTACTCAGCTGGTTGCAAATAAAGGGAGTTTTCATATC ACCAATTTGTAGCAAAATTGAATTTTTTCATAAACTAGAATGTTAGACACATTTTGG TCTTAATCCATGTACACTTTTTTATTTCTGTATTTTTCCACTTCACTGTAAAAATAGTA TGTGTACATAATGTTTTATTGGCATAGTCTATGGAGAAGTGCAGAAACTTCAGAAC ATGTGTATGTATTATTTGGACTATGGATTCAGGTTTTTTGCATGTTTATATCTTTCGT TATGGATAAAGTATTTACAAAACAGTGACATTTGATTCAATTGTTGAGCTGTAGTTA GAATACTCAATTTTTAATTTTTTTAATTTTTTTATTTTTTATTTTCTTTTTGGTTTGGGG AGGGAGAAAAGTTCTTAGCACAAATGTTTTACATAATTTGTACCAAAAAAAAAAAA AAAGGAAAGGAAAGAAAGGGGTGGCCTGACACTGGTGGCACTACTAAGTGTGTGT TTTTTTAAAAAAAAAAATGGAAAAAAAAAAAGCTTTTAAACTGGAGAGACTTCTGACA |
| 1000 | RAVER1 | HSX2_02495389 | 724 | GAGTCAGCAGAAGTTGAAGAAATAGTGTTCCCAAGACAATTCAGTAAGCACAGTG GCCACCTACAAAGGCAGAAGAGAGACTGGGTCATCCCTCCAATCAACTTGCCAGAA AACTCCAGGGGACCTTTTCCTCAAGAGCTTGTCAGG |
| 1000 | RAVER1 | HSX2_02495398 | 725 | TCCTTGTGCTGATGTTTGTGGTATGGATGAAACGCCGGGATAAAGAACGCCAGGCC AAACAACTTTTAATTGATCCAGAAGATGATGTAAGAGATAATATTTTAAAATATGAT GAAGAAGGTGGAGGAGAAGAAGACCAG |
| 11123 | RCAN3 | HSX2_01550572 | 726 | TGTCTGTGAAAGTGAAACTGAAGAGGAAGAAGAGACAAAAACCCCAAACAGAAA ATTGCCCAGACGAGGCGCCCCGACCCTCCGACCGCAGCGTTGAATGAGCCCCAGAC CTTTGATTGCGCGCTGTGAGGCCCTTGGTTGTGGTGCGAGGCGGCTGCCCTGGTG GGCTCTGGCCATGGCGCTCTGTGCCTGCGGCCGATGCGTTGCTGCGAACAGCATAG GTGAGACTCTGCCGAGTGAGGTATAGGTCTTCTCACCACGCCTGTACTGCAGACAC GGTCGTGTAGAGTAGCAGCTGATTTGACCTGTCCCAGATTTTAAGTGATATTCCAA AAGGGACTTTACATTAAAGGAGAAGCCCCCAAGATGTGGCCACCCTTAACCATTTT TAAATAGTAACAAATTAGGAAAACAGCTCCCCTCCCCTCCAGCCATGTAAGTCCTCC TGATTCTGTATCACATGAGACACCAAAAACTGGAAATGTAGTCACACCCAGTACAG TACCATTTTTATGAATTTAAAAATAGTCTTATAATTTTAATTGTTTGTGGGTATTTTTC TACTGGTATAAATAGCTTCTAATTAATAAATCGATCAAAGGTTGTTACTCAGTATAC ATGAAATTTTTTGCAGGAAAAGAAACCAGAAATACAGAATAATTCATAGAAATCAT GGTTCTCAAGTATTTGTTCAATTAGCTTTGGGGAAGAAATGCCAAATCCACTTTGAG GATGAGTCTTACGACACAGATCCACCCACATGTTGCTCTTATCTGGAATTTGGGACA |
| 5981 | RFC1 | HSX2_01182722 | 727 | CAAAGTTTGGTAAATTTTCCGGCAAAGATGATGGCTCTAGTTTTAAAGCAGCGTTG CTGTCAGGCCCTCCTGGTGTTGGCAAAACCACCACAGCTTCCCTGGTGTGTCAG |
| 84220 | RGPD5 | HSX2_02090776 | 728 | AGCCTCCATTATGGCATGCTGAATTTACCAAAGAAGAATTGGTTCAGAAGCTCCGTT CCACCACAAAAAGTGCAGATCACTTAAACGGCCTGCTTCGGGAAATAGAGGCAACC AATGCAGTCCTTATGGAGCAAATTAAG |
| 58480 | RHOU | HSX2_01949078 | 729 | GATGAATTTGACAAGCTGAGGCCTCTCTGCTACACCAACACAGACATCTTCCTGCTC TGCTTCAGTGTCGTGAGCCCCTCATCCTTCCAGAACGTCAGTGAGAAATGGGTGCC GGAGATTCGATGCCACTGTCCCAAAGCCCCCATCATCCTAGTTGGAACGCAGTCGG ATCTCAGAGAAGATGTCAAAGTCCTCATTGAGTTGGACAAATGCAAAGAAAAGCCA GTGCCTGAAGAGGCGGCTAAGCTGTGCGCCGAGGAAATCAAAGCCGCCTCCTACA TCGAGTGTTCAGCCTTGACTCAAAAAAACCTCAAAGAGGTCTTTGATGCAGCCATC GTCGCTGGCATTCAATACTCGGACACTCAGCAACAGCCAAAGAAGTCTAAAAGCAG GACTCCAGATAAAATGAAAAACCTCTCCAAGTCCTGGTGGAAGAAGTACTGCTGTT TCGTATGATGCTGGCAAGACACCCAGAAAGGCTATTTTCAGATGAAATCGATATTA GAAGCTATATTAGCTGAAACAACTCCTTTTACTGCGTAGAACCTATATCGAGAGTGT GTGTATATGTATTATAGGAGGAGCTCTCAATTTTATGTATTCTTTCTGCCTTTAATTT TCTTGTTTGTTTGAGCTTAGGGATGAGATACTTATGCAAGATATTTTTGAAGTAAAT TAAACATTTTTCACATCTCTGGAAATTTAGAGTTCTAGACCTCTGGTTAATTTATATC TAATATGAAGAAGACACCTCTAATCTGGATGTTAAGAATGAAGTTCTGCTACATTAT AATGTACAGAAGAGCAAAAGGGAGGAACACTATGGTTAACCCTCTCTTGATTAAGG GCTACTTAATGCACAGTGCATTATGTACACAGGTCAACCATGGTAACAATAGTTCTT AGCTTTGAAACTCCATGCAAACCATGCCTTTTTTTAAGGAGCAAAAATCTGAGAAA AAAAGTGAGAGACCTCTGCCTACAAAACCTCAAACCAGTCACTTTTGTCAATTGCTA ATACCCAGTTACTTATGATTTAAAAACAACCAACAGAAAACATCCCACTGACTGTAT GGCACTCTGTAGTCAAAAAAGGAAACTTCCTTATTGGGACTTTTCTTTCTTAGTCCA GTTGTGTTGACACATATGAACACAGACAAAGTGCTATGCGGAGGAAAGCAAGTGT TGGTCAGTAGTTTCATGTTTTAGGGAGTGGTTCCTGTGGAGATCAGAAAGTGACAT TTGCTTTCGGTACTGTAATACGTGCACCAAACTGCCTCAATCCTAGGTAACGAGGG CAACAGGGAGCACCTGTCTGGATTGTTTTTAAACCTCCATACTCAAGCTGTCTCTTC GGCAGGGAGGTGAATACTCTTGAAAGGCCAACAGCAAGTGTTTGTGGACACAAC ACAGATAATTTTTTCTTAAGTCGGCCAAGATGTACTTCTCTGTGTGCACACCCATGC ACACTCATGCACACAGATACATAGGTCTGTATGGCTGTATTTGCTGTTGATTCAGAC TTTCACACCATTAATGGGGAAAAGCGTGGCCACAAAAACAGATGCTAGGAAGCTTG GCTTCCTCTTCTTGTTGACCCTTTTTTGAACCAACATCTTTTTTATTATATTCAGAGTA TGTTTTTAAGTGTATCTTAATATATACATTTTTTAGGACATCTTAAATCTAAACAAAA AATAAAATGAACATCTCTTGAAACCTGTTAAAACAACCAGTTAAAGCCACAGATGG CTTTCAGGGCAGTAGCAGCAGAGGCCAGTGGACTCTGAGGACTCCTGAGGGGCGG GGCGTGTAGCCAGCCAGGTGCATGCCGGGACCATGGCCCCCATACTTGGCTGCTTC CTGTGACAGTGAAATACATCCTTCAAGGTGGCAGCTGTTAGGGCTGAATCTTCTGG AGAAAAAGGTGCCATCTCAGGAGAATAGCTTTTACTCTGGTAGGAATGCTTCCGAG |
| 63891 | RNF123 | HSX2_01961187 | 730 | CCCACCAGTGAGGAGGACCTCTGCCCCATCTGCTATGCCCACCCCATCTCTGCTGTG TTCCAGCCCTGTGGCCACAAGTCCTGCAA |
| 81847 | RNF146 | HSX2_02066510 | 731 | GCCGCAACGTACTCCGGGTCGGCCTTGCGCTCGG |
| 6569 | RNF149 | HSX2_01226407 | 732 | GTCACCAACACCATCGTGGCCCTGATGCAGGCGGGGGACAGGACTGACTTCCGGC G |
| 6258 | RXRG | HSX2_01203661 | 733 | CCCCGGGCTGCTAGGAAGCGATGACCACTCTTGTTAGCCCAAGTTGAAGAAAGCCG GGCTGTGCCTGGGAGCCGAGAGAGGCGGTAATATTTAGAAGCTGCACAGGAGAG GAACATGAACTGACGAGTAAACATGTATGGAAATTATTCTCACTTCATGAAGTTTCC CGCAGGCTATGGAG |
| 6258 | RXRG | HSX2_01203664 | 734 | GGAAGCCAATGGACAGCCACCCCAGCTACACAGATACCCCAGTGAGTGCCCCACG GACTCTGAGTGCAGTGGGGACCCCCCTCAATGCCCTGGGCTCTCCATATCGAGTCA TCACCTCTGCCATGGGCCCACCCTCAGGAGCACTTGCAGCGCCTCCAGGAATCAAC TTGGTTGCCCCACCCAGCTCTCAG |
| 6258 | RXRG | HSX2_01203667 | 735 | TCCCGCAGGCTATGGAGGCTCCCCTGGCCACACT |
| 6258 | RXRG | HSX2_01203684 | 736 | AAGCGTATTCCCCACTTCTCTGACCTCACCTTGGAGGACCAGGTCATTTTGCTTCGG GCAG |
| 6258 | RXRG | HSX2_01203703 | 737 | TGTACTCTTTAACCCAGATGCCAAGGGCCTGTCC |
| 6258 | RXRG | HSX2_02517790 | 738 | GTTGAATGGGCCAAGCGTATTCCCCACTTCTCTGACCTCACCTTGGAGGACCAGGT CATTTTGCTTCGGGCAG |
| 6284 | S100A13 | HSX2_01205966 | 739 | CCTGACAAAGGTCAGCTAGCCCCTTGAGGACATCAGCTTTGGCCTCAGGGTCCTAA TGGCAGCAGAACCACTGACAGAGCTAGAGGAGTCCATTGAGACCGTGGTCACCAC CTTCTTCACCTTTGCAAGGCAGGAGGGCCGGAAGGATAGCCTCAGCGTCAACGAGT TCAAAGAGCTGGTTACCCAGCAGTTGCCCCATCTG |
| 6284 | S100A13 | HSX2_01205987 | 740 | CGCTGCTTATAAACCTCAGCCCTGAGGCTCCAGCTCACTCTACCCCATCTCCTTGCC GG |
| 6284 | S100A13 | HSX2_01205990 | 741 | CCCCATCTCCTTGCCGGGTCAGCCCTGACAAAGG |
| 6284 | S100A13 | HSX2_02517894 | 742 | CCTGACAAAGGTCAGCTAGCCCCTTGAGGACATCAGCTTTGGCCTCAGGGTCCTAA TGGCAGCAGAACCACTGACAGAGCTAGAGGAGTCCATTGAGACCGTGGTCACCAC CTTCTTCACCTTTGCAAGGCAGGAGGGCCGGAAGGATAGCCTCAGCGTCAACGAGT TCAAAGAGCTGGTTACCCAGCAGTTGCCCCATCTGCTCAAG |
| 6299 | SALL1 | HSX2_01206855 | 743 | TTATTCTGCCCCAGCTGATGTTTGAGCCAGC |
| 6299 | SALL1 | HSX2_01206862 | 744 | CAGATCAAGTGGACTGCAGCGACCTTTCAGAACACAACGGACTTGACAGGGAAGA GTCCATGGAGGTGGAGGCCCCGGTTGCTAACAAAAGCGGCAGCGGCACTTCCA |
| 6299 | SALL1 | HSX2_01206865 | 745 | TGACACAGTTAACAAAACAGATCAAGTGGACTGC |
| 6297 | SALL2 | HSX2_01206829 | 746 | AATTCCTGCCCCATCTGCCAGAAGAAGTTCACCA |
| 401474 | SAMD12 | HSX2_02398686 | 747 | CATCATCTGAGGGTTCTCCATAAACACGGTTAGCCTTCCAAGCTGCTGTCCTGCCAG TTGATATGAACATGGCCTGTTCCTCACAGAAAGACTAACCCATGGCACTGCCAGAA GATATCCTTGGATCGTGGACAGATTAACTGGATTAACTGAAAAAGCACCCATTCA GTCAATATTATGGGTTTTGGAAAAATCTTGCTTTGCTGTGGATTTAAAATGAGGTGC ATAATATAATGCCAACTCATTCCAAGTGGTCATTGGCAGACCATTAGCTGGAAAATT GCTTCCAGTTTTCATCACTGTGCATAACTCAAGTTCCCTTTGGGGAGCTGTCTGTGC CCTTCACTAGAAGTCCTGTTTTAGCATGGCAACTACTGGATTATTTTATTATGTGTGC AGATGGCATGATGTTTTAGAAAAAAAAAATTACAGCAATTCTGCAATCTCAGTGGA AGTGTTCACAATATTATGCCCTGGTAGGAATGTAAACACGTCTCGATTATGTTAGTT ATGATGTCTAAAGGTATACTGTCCAACTCTTAAGCACTTTATATGAGAACACTACAC CAAGATGAATCAGCACATTCAGCACATGTGAGAAATCAAGTATTTTCTTTTTATTTTT ATAAATCTTAGTGGTTTTTTTTTTTCCGGCTCAGACTGTCCAAGACAAACTCCACTAC TCGCATATCCCCAGTTGTTTGTGGTAGGTTGATGTAAATATGGCCACAATACTGTTA GACTTCACCTCCAGATACTTTATTGAACAACTGGTTTACTTCTAGCACCAAAGGGAA GAAGAAGGGGGTCCAGTGATGGTGATATCTTAGCTTGCTTCTTTGTTCACAGGCAC TATTGACCACATGAACATACAGGTACTCAAATACAGGAGGCTTCTATTTGGCTCCTC CAAGGGAGGAGTTCCTCCAAGGGAAAAAAGGGGATGGTATTCTTTTAATCAAAGA CCATGGTGGATGGATGGTAAGGGCAACAGAATGCAATTCTTTTGTGTGGTCTGACG TTAACACTTCGGAAAAAATTCTAAGTCAAGTTGAAATCGCCATTCTCTCAAGAGAGA AACATCAAGAGAGAAACATCAAGTGACCCCACTATGTGCCTAGAGAAGGAAAAGG GTCAAGTCCTATACTAGGTTCCTTACTTAGGGTAGAACCTACTAGTCCCTTGTTAAT CCTTTCTGGGATTATACCTGTGACTAATTTTTTTTTTTTTAACTATGTCTTACGACAAA GCACAGTCCTGGGAAACCAGTCAACTTAATTATTGCTTGTCTTTGAGCATTAGAGAC ATGTTCATATTGACTTGCTACATAACACTGTAGCATGTTTTTTCCTGAAAATATCAAG TTGGTTGAATGGAAAGCTCAGCAAAGTATGTTCTGTGTGCCCACTGTCCCAGCCTG GGACTGACGGGCATCAGAGGCTTTGCTTTAAAATATGGGTAACCAGTTCTGGAAGA CTTCCAACGCAAGCAAGCTTGGTGTTTTAGGGTGTCTCCAGATACATAAGCATTGTC TGCCTCCGCTCAGGGTCTCAGGAAATACACAGCCCATTGGAAACCACCCACGCCTG AGATTTGGAAACCTGACGGAAAGGGAGGAAGGTCCAAATGAGTCAGCAGCAGGA TGACTCACCACAAGCCTGGCAGGCTGCAGAGCCAGAGAAGGGGGCTTCCAACAAG GAACTCAGAGAAGTTGCAGTCACCACCAAGATCTGGCCCTTCGGCAGGTCAGCACC AGGACTGCCAGGCCCTCTGCAGACCGTAATTCTCTTCACATCTATAATTAGGAGGCC AAACTCCAGCTGCCAACACCCTTGGACACAATATTCCAGTCTCCACTGCCCATCTCA TGTGGTTCAGTTCTAATAGCAGGTCTTCTTCTTCCTTTCATTCCTTCCTTTATTCAGCA AGCATTTGTTGAGCATCTACTATGTGCCAAGCATTGTGCTTGGGCAACAACAGTCC |
| 6447 | SCG5 | HSX2_01215772 | 748 | TGTCCCCCATTTTTCAGATGAGGATAAGGATCCAGAGTAAAGAGAAGATGCTAGAC GAAAACCCACATTACCTGTTAGGCCTCAGCATGGCTTATGTGCACGTGTAAATGGA GTCCCTGTGAATGACAGCATGTTTCTTACATAGATAATTATGGATACAAAGCAGCTG TATGTAGATAGTGTATTGTCTTCACACCGATGATTCTGCTTTTTGCTAAATTAGAATA AGAGCTTTTTTGTTTCTTGGGTTTTTAAAATGTGAATCTGCAATGATCATAAAAATT AAAATGTGAATGTCAACAATAAAAAGCAAGACTATGAAAGGCTCAGATTTCTTGCA GTTTAAAATGGTGTCTGAGGTTGTACTATTTTGGCCAAGTCTGTAGAAAGCTGTCAT TTGATTTTGATTATGTAGTTCATCCAGCCCTTGGGCATTGTTATACACCAGTAAAGA AGGCTGTACTCAAGAGGAGGAGCTGACACATTTCACTTGGCTGCGTCTTAATAAAC |
| 51540 | SCLY | HSX2_01771392 | 749 | TCCGGCTCAGCGTGGGCCGCAGCACCACCAGGGCCGAGGTGGACCTCGTCGTGCA GGACCTGAAGCAGGCCGTGGCGCAGCTGGAGGACCAGGCCTAGCACTGGGGCCG CCTTCCCCACCCCGCTTCTGGGAAGCCCGTGGCAGGGCACAGGGTTGTCCCTCCAG TTCCCTCCTGAGGGCTGTGCCAGGATGACTGTCTCATGCCCCCTCTGCATTTTGTCC TGGAGTGCCAGCGAGTGTGCACCCCCAGTTTCCTTCCCTGGACCCCTGCAGAGCTC ACAGGGCCCAGGACACCAACGCCGCATAGGACTGCCCACATGGGACCGCCCACAT AGGACCGCCCACATAGGACCGCCCACATGGGACCGCCCACATGGGACCGCCCACA TGGGACCGCCCACATAGAACCGTCCTCCAGTGGTGAAGCGGAAACACTTAGCTTTA TCCACCCTCCCCACTGGGAACTGGGCACGCCTGTTGTGAGTGCCCTTTCCTGGAAG GTGTTTTTATCTGGAAGATAGAATCCAAGTATTTATAACTCATTGTCAGCCAAATGC TATCATGAACGTAGGAAACTTGATTTTTTTGTTTTGATCATGGCCTCTACATGCACCT TTCCAGAGTGGTCTCTTCTCAGGCCCTTTTTAGTCCCTTTCCAAAGCTGCCCCCACCA CCCTGCTCCTCTGGCCTCAGTGCACAGTGGCCCCCAGCCTCGGCCAGGCCTGCTCTG CTCAGCGCCCACCGCCCACCACCCCTCCTGCTTCCCCGAGCCTGACCCTGTTCCGCC CACTGGCAATCAGGGCTGCGCACTTCCCTGTCCACGGTCCCCAGGCCTTCCTGTCTT GTCCCTTTTGATCATTATTAACTCAGGGTTTCAGCTCCAACCTCGCTGAGTTGGTGC AGCTCCAGGTCATTCCTGGGGTGGGAATCGGATCATCCCTGACTCAGCTTTTACCTT AATTTTATTTGCAGAGGATTCTTTTCTCAAAATGCTCTGGCATTTGGACACACATCA CATGTCGATATTTGCATAGGAGTCATTTTCAGTGGAATAACATTTTTAATGTGTGGT |
| 6385 | SDC4 | HSX2_01211518 | 750 | ACTCGCCGCAGCCTGCGCGCCTTCTCCAGTCCGCGGTGCCATGGCCCCCGCCCGTCT GTTCGCGCTGCTGCTGTTCTTCGTAGGCGGAGTCGCCGAGTCG |
| 6385 | SDC4 | HSX2_02518216 | 751 | ATCCGAGAGACTGAGGTCATCGACCCCCAGGACCTCCTAGAAGGCCGATACTTCTC CGGAGCCCTACCAGACGATGAGGATGTAGTGGGGCCCGGGCAGGAATCTGATGAC TTTGAGCTGTCTGGCTCTGGAGATCTGG |
| 6385 | SDC4 | HSX2_02518220 | 752 | GTGCCTCTAGATAACCATATCCCTGAGAGGGCAGGGTCTGGGAGCCAAGTCCCCAC CGAACCCAAGAAACTAGAGGAGAATGAGGTTATCCCCAAGAGAATCTCACCCGTTG AAGAGAGTGAGGATGTGTCCAACAAGGTGTCAATGTCCAGCACTGTGCAGGGCAG CAACATCTTTGAGAGAACGGAGGTCCTGGCAG |
| 89866 | SEC16B | HSX2_02129253 | 753 | CTGGGAAGGGGCTTGTGGAGGCAGCTCACTTCTGCTATCTCATGGCTCACGTGCCC TTTGGCCACTACACCGTGAAGACAGACCATCTGGTCTTGCTGGGCAGCAG |
| 728492 | SERF1B | HSX2_02432910 | 754 | CATATCAGATGGACAGATGTATCATTTTGGGGTCCACCATTCAACCCACTACAAGG AGTTTTTTAAACAAAAATAGGAAA |
| 26297 | SERGEF | HSX2_01689967 | 755 | GTGCCCTGGGCAGACTCTTCCATTGTTTTCACAGCAAAGGAACCAAGCAGAGTGA CAG |
| 26297 | SERGEF | HSX2_01689996 | 756 | GTGTATGTTTGGGGAAGCAACAAGCATGGCAACTGGCTAATGAGGCTGCTTTCCT TCCTGTGCCCCAGAAAATAGAAGCACATTGTTTCCAGAATGAAAAGGTCACTGCCA TCTGGAGTGGATGGACACACCTGGTTGCTCAGACAG |
| 5270 | SERPINE2 | HSX2_01123425 | 757 | ATTGTGACAGTGGCTAACGCCGTGTTTGTTAAGAATGCCTCTGAAATTGAAGTGCC TTTTGTTACAAGGAACAAAGATGTGTTCCAGTGTGAGGTCCGGAATGTGAACTTTG AGGATCCAGCCTCTGCCTGTGATTCCATCAATGCATGGGTTAAAAATGAAACCAGG G |
| 5270 | SERPINE2 | HSX2_01123431 | 758 | ATATGATTGACAATCTGCTGTCCCCAGATCTTATTGATGGTGTGCTCACCAGACTGG TCCTCGTCAACGCAGTGTATTTCAAGGGTCTGTGGAAATCACGGTTCCAACCCGAG AACACAAAGAAACGCACTTTCGTGGCAGCCGACGGGAAATCCTATCAAGTGCCAAT GCTGGCCCAGCTCTCCGTGTTCCGGTGTG |
| 26470 | SEZ6L2 | HSX2_01691108 | 759 | TGACCCAGACCACAGATCCATCACGGCAGCTGGAAGGGGGGAACCTGGCCCTGGC CATCCTGCTGCCTCTAGGCTTGGTCATTGTCCTCGGCAGTGGCGTTTACATCTAC |
| 26470 | SEZ6L2 | HSX2_02547311 | 760 | CATCCTGCTGCCTCTAGGCTTGGTCATTGTCCTCGGCAGTGGCGTTTACATCTACTA CACCAA |
| 253970 | SFTA3 | HSX2_02311072 | 761 | GTTCTTTTTCTTCAGGATCAGGCACAGAGACTGACTGAATGGCTCCAATTATCAGGA TTTGAAAACCCAGTATCAGAATCTACCACTTTGT |
| 253970 | SFTA3 | HSX2_02311083 | 762 | TGCAATATTTGGAACACCAATGGAGGTCTACGTCAACACAGAATTTATACAGCAGC TGGTGCTAGTCAGAGCTAATGACAGAATTTCAGTTTAATAAAAAGACCCCCAACTG AGCACACCATCTTGAAAAAAGTATACTTATCAAACAGCTTTCAATCAGTTCAAGAGA GACACCTTAATTGGGGAGAGGAAGAATTGCAGAGTAGTTTGTAATCATGCCAATTC CAGATCAATAACTGCATGTCTGTTCTTTGGTAGAAATAGCTTTTGCTTTATATTAAGT AATCACATATATATTCTCTCTATTTGGATAAGGAAACCTTCGCTTTATTTGACAATGT ATAATGATATACTCTTCTAATTCACCTCTGTGTCTTCACAATAAACATGAGTAAAATT TAGACAAGTGATGGTAAAGGTCAATATAATTATTTATTTTTAAAATAAATTTTGTAT CTAACAGGAAAGCAGTTCTTATGAAATTTTTATATTTTCAAAAATTGTTTTGTTCAAA TAAAATTTTATGAGTAAAGTTAA |
| 166929 | SGMS2 | HSX2_02271509 | 763 | GCCACTGTGTAAATAGAACTGCCTAATGTTGAGAGTGGTTTTTAGCATTAGGTTTA GCAAGGGGGAGATCCGTGGGTTGTGCGTCAGCTTTGGGTGAATTTTGTTTCTACCC TGTCACGGGGAAAGTTCGGGTTGAGTCCAGGAGTGCACACTGCTGCTGCCACCCA ATGCGCTACATATCACTTTTTTTGTTTTGTTTTGTTTTGTTTTTAAAAGATCATTTTA TCTTAAAAAGGAAAGCTGATCCAAGTAAACACGAAAGTATTTGACACACCCCACAG ATTTTACATGTGTGTAAATGTTTCACTTTAAAATCTCTATGACAGATACACAGGAAA CATGAGATGGTTTCTGCTAATGAGTGGCCCTTGAGTACACACTTAGATGCTGTCTGC CCTGTAAATTTGGATCTGGTGCCCCAGGGCAGTCAACTCTTCTAGCACAGGCTGAA AACACGTGTGTGTCAACTGAGGTTCACACCACTTGGGGAATGAGCCTGTTTTCTTTC CAGGTCAGGCCCTGGTGTGAGATCAATTTTAGGGCTCCTAATTGGAGCACAGAAAT GTATTTGGTCAAATTTCATTGAAGGTGATTTCTTCCTTTTTCTGTTGTACTTTATGGA AAAACCAAGATGGAACCTGAAAGTTAATGTATCCTTGCTTTTAGCAAGAGACTCAG TTTCTTATAACTAGTCCTAAGGACATATGCCGCAATTGAGTAATTTTACTTTTACCCT ATGAAAATAACATGGTGCTGCACTATAATATACTCTCGGAATCAGTGTGTTAGTTAC AGCTATACAGTGAAGGGGGATAACTGTTTCTAAATTTCTTTAAGGTGATGCCAAAA AATGGATTAAAAAATCTGATCAGATTTAATGTTACCAGATTTAGTGCATTATTTAAT GCTATTGGATCTTTTGGATAATTCTTGGCTTAATTTCTTAGCTACTTGAAGGTTAATT TGCAAGACTTTTAAAACCTTAGAAAAGTTTTAAGGTTGCAAAGTTATCAACACTGG GGCAGAGGGTGGAGAGGCCAATGCGGGTAGAAGGAGGCAGTTATGTTTATATTG AAGGTGAAATTTTTCTTTCATTTAGAATGGAAAACATCCCCAAATGTATCATTATAA ACTAGTCAGCCTTGACTACACAAAATTGACCTTTAAGTTGCTTGAGAAAAACACAAT GCAAATCGTTCAGAAGGGTCAACATCCTTTGGTGCTAAAATCTTGTGTATGTTTTCA GAATGGCTTTTTCTGTATGTTATAGAATAATCACTAAAGGAAAGGTAGTTGAATTTA AAGTCATGAAGCAAGACTCTTTAATTCAGTTATTTTAAACAAGAATTAAAACCCCAA ACATCCTTGGCAGGCTTTGAAGCACACAGAATTTTCTAGTATTTCTTATTAAATACA CCAATAATAAACATATACTTAGTTTACCTGATGAGTTTAGACATAATTCTATATAAT GTTCGCTTATATTCATTTATTAACTAGCAACACTTGTGCAAGAACAAGGTATTCTGC TTGAAAACACTGATTTTTAAGTTCCTGTTGCCCGATTGTAAAGAAAATCATCTGACA CAGAAGCCTGGATTTTGCTCTCCTAACACTGGTGTTTTCCTTGAGCCTCTAATTCAA ATAAAACAAACTGATACACCTTACAGCCTTATTCATCTGTGTCTGGATAACTCACTTT TCATCTATTGAATAAGAAAAAGTGTTTAAACTTAATAAAATAAAAAATTGTGCACCA ATTACTACAGATCCTTGAAACAGTAAGCCTTGGATTTTAGTCAAGTAATCCAGTTTT TTTTTAATTTAAAAAAAACCATCACCTTTTAAAGAACTTTAAAAACACCTGTGATGCC AATGTGGACATTGCCGCTGCCATGTCTTTACACGCATGTGTCGTATAGCTCTGTCAT CGAGTTGAGGAAGTCCATGGTTTGCAAATAAGGGTGGGGGAATCAAAATTTCAAG |
| 6449 | SGTA | HSX2_02518607 | 764 | GAGATGCCGCAGGACCTGAGGAGCCCCGCGCGAACCCCGCCTTCCGAGGAGGACT CAGCAGAGGCAGAGCGCCTCAAAACCGAAG |
| 57477 | SHROOM4 | HSX2 01923419 | 765 | GAGCTGAAGGAGCACGTGGACCGCCGGGAGAAGTTGGTGTTTGGCATGGTCTCCC GCTACCTGCCTCAGGACCAGCTCCAAGATTACCAGCACTTTGTCAAGATGAAATCTG CTCTCATCATTGAACAGCGAGAGCTGGAGGAGAAGATCAAGCTCGGGGAAGAGCA ACTCAAATGTCTCAGGGAGAGTCTACTCCTGGGGCCCAGCAATTTCTAATTCTACCA GCACTCTGCCACAGCATCCCTGCCCAGCCATGTGGGAAGTGCTTTCAATCTTCTTTG TTAGCAGTTTCTCAGCAAGTAGATAGCAATTAGCAGTTTGTTCCAGCCCTCTACCCT GGATGTCTCTCACTACCCCTTCCCTAGCAGTGGTCCTAACCAGCTAGGAGACCCTGG GGAAGCCACAAGCTTCTACCCAAGGGAGCTGCAGCAAGGTGTGATCTTAGAACCA CACTCTCCTTCCCACAGTTGCCAAGGGCAAGTACTTGCTGCACAGAGAACCAAGGA AGTGCCTTCATTCTGCTTTGTACTAGGACACCAAAGACATCAAGTACTCATCACCCA CCCATATCATCAACAGCCTCTAAAGGCTCAGAGGGAATCTGCCTTGCAGCTCTACTC TGCCCCAGGGCTTGTGGCCAGCCATTTCTCACAGAGAGCTGGCTGCCTTGAGGGCA TTCACCTGGCACCAGTTTCAGGGCCTCACCCAAGCTTTGCAGGGGAAAGCACAGAG GGAGGAATTACACTGAAAAAAATGCAAGCAAAGGTTGAGTACCCCCAGGTGCCCC TTAGGAAGGAACCAGGTTTAAATAGGCTCTACCCTTACCTTTCCCAGCAGCAAGTTC AGGGGAAGAGGCCTACTCTTAGCCCTGGCTAGTGTGACCCTCTTCCTGTCCTAAGA CTTTGGTCCTACCACCTCTTGTTTCATCTTTCCTTTACATTGCTGGGGGTTACCGCAG |
| 57477 | SHROOM4 | HSX2_01923422 | 766 | GCGAGAAGAGGGAAGTTGGAGAGGGAAAAGTTAGCCCAGAAGGAAAGCATTTTC TGCAGATCAGCCTGAATCCACCGTGGCTAG |
| 8170 | SLC14A2 | HSX2_01323136 | 767 | ATGTTCATCAACAATCCTCTCAGCGGCCTCATCATCTTCATAGGGCTGCTGATCCAG AATCCCTGGTGGACAATCACTGGGGGCCTGGGGACAGTGGTCTCGACCTTAACAG CTCTCGCCTTGGGCCAAGACAG |
| 8170 | SLC14A2 | HSX2_01323147 | 768 | CCACAGGCCACTACAACCTCTTCTTCCCCACAACACTGGTAGAGCCTGTGTCTTCAG TGCCCAATATCACCTGGACAGAGATGGAAATGCCCCTG |
| 8170 | SLC14A2 | HSX2_01323168 | 769 | CTGACGACAAACAACCCAGCCATCTTCAGACTCCCACTCAGCAAAGTCACCTACCCC GAGGCCAACCGCATCTACTACCTGACAGTGAAAAGCGGTGAAGAAGAGAAGGCCC CCAGCG |
| 8170 | SLC14A2 | HSX2_01323213 | 770 | CACTGTTTGCTGCCTACCTGGGTGCTGCCCTGGCTAACATGTTATCTGTG |
| 8170 | SLC14A2 | HSX2_01323231 | 771 | ACAGGACCTGAATGACCGATTGTTTCTTGCTTAC |
| 9194 | SLC16A7 | HSX2_01390568 | 772 | ATAGGAAGCGACCCATGGCAAATGGATTGGCCATGGCAGGAAGTCCTGTTTTCTTA AGTTCATTGGCTCCTTTCAATCAGTACCTTTTTAATACTTTTGGCTGGAAAGGAAGC TTCCTGATTTTGGGAAGTCTACTTTTGAATGCCTGTGTGGCTGGTTCCCTCATGAGA CCCCTTGGACCCAATCAAACCACTTCTAAGTCTAAAAATAAGACTGGCAAAACAGA AGATGATTCAAGCCCAAAGAAAATCAAAACGAAGAAATCAACTTGGGAAAAAGTT AATAAGTATTTAGATTTCTCCCTTTTTAAGCATAGAGGATTTCTGATATATCTGTCTG GAAATGTCATTATGTTCCTAGGTTTTTTGCCCCCATTATATTCTTGGCTCCATATGC TAAAGACCAAGGAATTGATGAGTACTCGGCAGCTTTTCTGCTATCTGTTATGGCTTT CGTTGATATGTTTGCTAGGCCTTCTGTAGGATTAATTGCAAACTCCAAATATATTCG ACCTCGAATTCAGTACTTCTTCAGTTTTGCAATCATGTTCAATGGAGTGTGTCACCTC TTGTGCCCACTGGCACAGGACTACACAAGCCTGGTATTATATGCTGTATTTTTTGGC CTTGGATTTGGGAGTGTTAGCAGTGTTCTCTTTGAAACTCTCATGGACCTCGTGGGT GCACCAAGATTTTCCAGTGCCGTCGGACTTGTCACAATTGTGGAGTGTGGCCCAGT |
| 92014 | SLC25A51 | HSX2_02146193 | 773 | AGCTCATGAAAAGAGGCCACCAATACTAACATCTTCAAAACAAGATATATCACCTCA TATTACAAATGTTGGTGAGATGAAGCATTACTTGTGTGGCTGCTGTGCAGCCTTCA ACAATGTCGCAATCACATTTCCCATTCAGAAGGTCCTCTTTCGACAACAGCTGTATG GCATCAAAACCCGGGATGCAATACTTCAGTTGAGAAGGGATGGATTTCGAAATTTG TATCGTGGAATCCTTCCCCCATTGATGCAGAAGACAACTACGCTTGCACTTATGTTT GGTCTGTATGAGGATTTATCCTGCCTTCTCCACAAGCATGTCAGTGCTCCAGAGTTT GCAACCAGTGGCGTGGCGGCAGTGCTTGCAGGGACAACAGAAGCAATTTTCACTC CACTGGAAAGAGTTCAGACATTGCTTCAAGACCACAAGCATCATGACAAATTTACC AACACTTACCAGGCTTTCAAGGCACTGAAATGTCATGGAATTGGAGAGTATTATCG AGGCTTGGTGCCCATTCTTTTCCGGAATGGACTCAGCAATGTCTTGTTTTTCGGCCT TCGAGGTCCCATTAAGGAGCATCTGCCTACCGCAACGACTCACAGTGCTCATCTGG TCAATGATTTTATCTGTGGAGGTCTATTGGGTGCCATGTTGGGATTCTTGTTTTTC CAATTAATGTTGTAAAAACTCGCATACAGTCTCAGATTGGTGGGGAATTTCAGTCTT TCCCCAAGGTTTTCCAAAAAATCTGGCTGGAACGGGACAGAAAACTGATAAATCTT TTCAGAGGTGCCCATCTGAATTACCATCGGTCCCTCATCTCTTGGGGCATAATCAAT GCAACTTATGAGTTCTTGTTAAAGGTTATATGAAAAAACCATCAGTTAAGTGCCATT |
| 28965 | SLC27A6 | HSX2_01713935 | 774 | ATTTGCTTGGAACGGTAGAAGAAATCCTTCCAAGCCTCTCAGAAAATATCAGTGTTT GGGGGATGAAAGATTCTGTTCCACAAGGTGTAATTTCACTCAAAGAAAAACTGAGC ACCTCACCTGATGAGCCCGTGCCACGCAGCCACCATGTTGTCTCACTCCTCAAGTCT ACTTGTCTTTACATTTTTACCTCTGGAACAACAG |
| 28965 | SLC27A6 | HSX2_01713976 | 775 | AGAGAAGGAGAAAAGGATCATAAGGTGCGTTTGGCAATTGGAAATGGCATACGGA GTGATGTATGGAGAGAATTTTTAGACAGATTTGGAAATATAAAGGTGTGTGAACTT TATGCAGCTACCGAATCAAGCATATCTTTCATGAACTACACTGGGAGAATTGGAGC AATTGGGAGAACAAATTTGTTTTACAAA |
| 28965 | SLC27A6 | HSX2_01714019 | 776 | GAGAACCTGGACTTCTCATTTCTCGAGTGAATGCAAAAAATCCCTTCTTTGGCTATG CTGGGCCTTATAAGCACACAAAAGACAAATTGCTTTGTGATGTTTTTAAGAAGGGA GATGTTTACCTTAATACTGGAGACTTAATAGTCCAGGATCAGGACAATTTCCTTTAT TTTTGGGACCGTACTGGAGACACTTTCAG |
| 28965 | SLC27A6 | HSX2_01714061 | 777 | ATTTGGAAAAAGTTTATGAACAAGTTGTAACATTTCTACCAGCTTATGCTTGTCCAC GATTTTTAAGAATTCAG |
| 28965 | SLC27A6 | HSX2_01714088 | 778 | CTTTCATATGCTTTCTTAGGAAGAGTGAGAGGGGGTATATGATTCTTATGAAAT GGGGAAAGGGAGCTAACATTAATTATGCATGTACTATATTTCCTTAATATGAGAGA TAATTTTTTAATTGCATAAGAATTTTAATTTCTTTTAATTGATATAAACAGTAGTTGA TTATTCTTTTTATCTATTTGGAGATTCAGTGCATAACTAAGTATTTTCCTTAATACTA AAGATTTTAAATAATAAATAGTGGCTAGCGGTTTGGACAATCACTAAAAATGTACTT TCTAATAAGTAAAATTTCTAATTTTGAATAAAAGATTAAATTTTACTGAAATATTTTA |
| 2542 | SLC37A4 | HSX2_00934722 | 779 | GTAGGGCCTGCTCTTTTCTACCATGGCAGCCC |
| 55652 | SLC48A1 | HSX2_01863202 | 780 | GTATATGATGGGCCTCCCCATGTGGATCCTTAGTGCTGTGGCAGAGCCCTTGTTATT GTGCTGGGATTTTCCCTCCAGCTCCCGGCCGGAAGCTGGGCTCACGTGGGAGCTCA GTGCCCTCCTGCTACAGATCTGTCTCTTCCTTACAATG |
| 9353 | SLIT2 | HSX2_01400643 | 781 | GTCACCAGTCATTTATGGCTCCTTCTTGTAGTGTTTTGCACTGCCCTGCCGCCTGTAC CTGTAGCAACAATATCGTAGACTGTCGTGGGAAAGGTCTCACTGAGATCCCCACAA ATCTTCCAGAGACCATCACAGAAAT |
| 9353 | SLIT2 | HSX2_01400648 | 782 | ACGTTTGGAACAGAACACAATCAAAGTCATCCCTCCTGGAGCTTTCTCACCATATAA AAAGCTTAGACGAAT |
| 9122 | SMAP2 | HSX2_02529005 | 783 | AAGACCCATCCATATCAAAAGTGAGAACAATTCTGGTATTAAAGATAAAGGCAGCA GTTTGTCTGCACATGGTCCAGAGGCACATGCAACAGAAACACACTGCCATGAGACA GAAGAGTCTACCATCAAGGACAGTACTACGCAGAAGGCTGGACTACCTAGCAAAA ATTTAACAGTCTCACAAAATCAAAGTGAAGAGTTCTACAATGGGCCTAACAGGAAC AGACTGTTATTAAAGAGTGATGAAGAAAGTGATAAGGTTATTTCGTGGAGCTGCAA ACAACTGTTTGACATTTCTCTCTTTAGAAATCCTTTCTTCTACATATTTACTTGGTCTT TTCTCCTCAGTCAGTTAGCATACTTCATCCCTACCTTTCACCTGGTAGCCAGAGCCAA AACACTGGGGATTGACATCATGGATGCCTCTTACCTTGTTTCTGTAGCAG |
| 113444 | SMIM12 | HSX2_02575493 | 784 | ATGAGGCCATGAATGAGGTCGGGTCAGCCCAGGC |
| 57154 | SMURF1 | HSX2_01912910 | 785 | AACAAAGAACAACAGTCCAGGGCCAAGTTTACTTTTTGCATACACAGACTGGAGTT AGCACGTGGCACGACCCCAGGATACCAAG |
| 83891 | SNX25 | HSX2_02570708 | 786 | CTTGCCCAGCTGGCGTACAGAGAGCAAATGAATGAGCATCACAAGAGAGCCTACA CCTATGCCCCCTCTTACGAGGACTTCATCAAGCTCATTAACAGCAACTCTGATGTGG AGTTCTTGAAGCAACTAAG |
| 134548 | SOWAHA | HSX2_02214232 | 787 | GGCCGCCGCAGCAGAAGCCCTGTATGCTGCCGGTGCGCTGCGTCCCGGCCCCCGCC ACGCTCCGCCTCCGGGCGGAGGAGCCCGGCCTGCGCCGGCAGCTGTCGGAGGAGC CGAGCCCGCGGAGCTCCCCTCTGCTGCTGAGGCGGCTCTCGGTGGAAGAGTCCGG TCTGGGCCTCGGCCTGGGCCCGGGCCGCTCCCCGCACCTGAGGCGCCTGTCGCGC GCCGGCCCGCGTCTGCTGAGCCCTGACGCCGAGGAGTTGCCCGCCGCGCCGCCGC CGTCCGCGGTGCCCCTGGAGCCGTCCGAGCACGAGTGGCTCGTGCGGACTGCCGG GGGCCGCTGGACCCACCAGCTGCACGGGCTGCTGCTGCGCGACCGCGGCTTGGCG GCCAAGCGCGACTTCATGTCTGGCTTCACGGCCCTGCATTGGGCCGCCAAGAGCGG CGACGGCGAGATGGCGCTGCAGCTGGTGGAGGTCGCGCGGCGCAGTGGCGCACC AGTCGACGTGAACGCACGCTCCCACGGCGGCTACACGCCGCTGCACCTGGCTGCAC TGCACGGCCACGAGGACGCTGCCGTGCTGCTGGTGGTGCGTCTGGGTGCCCAGGT GCACGTGCGTGATCACAGCGGGCGTCGCGCCTACCAGTACCTGCGGCCCGGCTCCT CGTACGCGCTGCGCCGCCTTCTTGGCGATCCAGGCCTGCGAGGCACCACGGAGCCA GATGCGACCGGTGGTGGAAGTGGCAGTCTTGCTGCCAGGCGCCCCGTACAGGTGG CCGCCACCATCCTCAGTTCCACCACCAGTGCATTTCTGGGCGTCCTGGCTGACGACC TCATGCTCCAGGACCTGGCCCGAGGCTTGAAGAAGTCGAGCTCCTTCAGCAAGTTC TTGAGCGCCTCGCCCATGGCTCCACGTAAAAAGACAAAGATCCGCGGTGGTCTGCC AGCCTTCTCAGAAATCTCTCGTCGACCTACTCCGGGGCCTTTAGCTGGTCTAGTGCC |
| 56848 | SPHK2 | HSX2_01898098 | 788 | GTCAGGGCTAAGGCCATGGCCCCGCCCCCACCGCCACTGGCTGCCAGCACCCCGCT CCTCCATGGCGAGTTT |
| 23514 | SPIDR | HSX2_01645611 | 789 | CTGTAAATTTGCAGATGGGGAAACTGAAGTCATCAAGGTTAAATAACTTGCTGAAT TCCCACCACCAGTGGATCACAAAGCTGTGTGACACTGGACATGACTTCCCAAAAGA ACCCCATCTTGCCATCCTATGATCTGTAGATGAAAGTAGGGTTTGCTGATGGCCCTG AGAAAATCTTAACAAACTGGATGAGGCTCAAAGACTTTTCCAGGGATATGGCTCCA GAACTGTGACCTTCCCTTTATGGGTCCAGCATGACATCGTCCTGCTGCTCCATATGG CATCAAT |
| 6695 | SPOCK1 | HSX2_01236568 | 790 | AAGAGGAGCAGGAAACCTCAGGGGATTTTGGCAGTGGTGGGTCCGTGGTCCTGCT GGATGACCTAGAATATGAACGGGAGCTGGGACCAAAGGACAAAGAGGGGAAGCT GAGGGTGCACACCCGAGCCGTGACAGAGGATGATGAGGATGAGGATGATGACAA AGAGGATGAGGTCGGGTACATATGGTAGTGCCCACAAGAAAGAGGACACAAGTTT TGCACAAAATTGCAAGTCACTTCCTATTCCTGCATTTGTATCTAAGACTCCAAGGCA CCAAGGTCTCTTCTCCATTGTTGCTCTCTATACCCGACCTAAGGTTTGGAAGACAAC TGCTTGTTCCCAGAGGATTCTGATTTTGCATATGTTTGTATGGAGAAAGGGTGTT GTGTTTTTTTTTTGTTGTTGTTTATTTTTTGGATAGGGAAGTCATTGGCTTAATTAGA GCCTCCTTCCTTTCTGTGAGATTTTTCCAACAAGCATGTGATTTACGTGGAATTCTGA CAGTGCAGGGAGCCCCAACCCTCTTAAATGTCAAAGACCCTTTTTGATTACCCACAC TGGTGGTTATTACAGCATGGTTCCCAGCCTTACAGTGTCTAAGTGCTTCTCTTGTGT CCTGTAGATGTTGTGAAAAAGAAAAAAACAAAAAATACACCACACTGTACTTTTTCC CCCTGCCCCCGTTACTGCCGGTGATTATTATTAAAAATTAGTTTTTTTCACATCATTA TATCTGGCTTCCTATAAACAACAGCCTTAATTCAGTCAAGACTCCCTTTGGGGAATT CATTTTATTAAAAATTGGTGTCTGGATACTTCCCTGTACATGCATAAATATGCATGC ATGTACAGAAAGACTGTATGTGTGTGCCTTGCACACACACCCATACCTCTCAGAAA AAGTGTTTGGGTATCTTAAAAACTCGAAAAACAATGATAAATTTCTCAGCTTGTCCA GACCTGGAACAAAATTTCTGGAATAAGAAATTTGTATTAAAGTCCTTTTTTGCACTA ACAGTTGGCTCTTGTAGCCTGCAGGCTGAGGAAGTCTCTTCTCTGTGCATCAGCAG AGTTACTGAAAGCCTCTGATTGAGAAAAAACCTCCGTCTGCCTAAATCACTTTTCTC GCAGAAGCCATGCGACTCCCACACGACACGGGCAGCTTCACAAGCCATCTCTTTCA TTTCTGCTTGAAGCCCCTTGGCTGCAGCAATCCTGTCTGCCATAGGTTTCTTCCTTCC TTACCTACTCAAGGGCTTTTTCTAAGGCATGCACACATATCTCCTGTTCTCTGAGAG TACCATGGTGTTCCTTAAAAGAAGAAAATTTCTAATTCTGAACTCAATGTTTTGCTTT TACTCCCTTTCTACTGACAAATCATGATAAGGGCACAAAAGCTGTACAGATTTTTTT TTTTAACCACTCAATCCCAAATGGAGGCCTACAAAGAACATCGTAATAACACATGG AAGCAAACCCTGGGTTTTTAAGAGCAAATTCTGTCCCCCCCTCACTCCCCCAAGTGA CAAGATACTAATGAAGAAAGTTCTTCACCATAGTGTTTGTTTTAACTAAACTCATTG GAGTCTAGTTCCAAATTTGGTAGGGTCATCATCTCTACATTCCTTAGGATTTCTCTCC CTATCAAGCTGGCCCAGATACAAGTACCAAACAGTAGTCTCTGAAGTTCCCCCATTT CCTTCAGTACCAGTCTATAAGCTACTGTCCGCCACTGATTTTCATTTATCAGGGTGTC CTAATCAGAATCAGCCACCCAAGCAAGCCTCTCTGGCCCACATATCTATCTCTTGCC TTCCCCCATGAACTTCAGCCTGTCCACACAAAAGCCACATAAACTCAAGCAAGAAAT ATGTTCAGCCAAAACATGATTATAGTGGCAGCTGACCAATCCCCCATCCCAAATTGA CCATTTAGATGTACCAACTCACCTTAAATTAGCATGTTCCAATCCAGTCGGCATTGC |
| 56928 | SPPL2B | HSX2_02560330 | 791 | CCTGGCGCTCTTCTGCGTGGCCGTCAGCGTGGTGTGGGGCGTCTTCCGCAACGAGG ACCA |
| 6709 | SPTAN1 | HSX2_01237303 | 792 | CAACAAGTGGCCCCCACGGATGATGAGACTGGGAAGGAGCTGGTCTTGGCTCTCT ACGACTATCAGGAGAAGAGTCCCCGAGAGGTCACCATGAAGAAGGGAGATATCCT TACCTTACTCAACAGCACCA |
| 6709 | SPTAN1 | HSX2_01237310 | 793 | GATTGGTGGAAAGTGGAAGTGAACGATCGTCAGGGTTTTGTGCCGGCTGCGTACG TGAAGAAATTGGACCCCGCCCAGTCAGCCTCCCGGGAGAATCTCCTGGAGGAGCA AGGCAGCATAGCACTGCGGCAGGAGCAGATTGACAATCA |
| 6709 | SPTAN1 | HSX2_01237518 | 794 | GAGCAGATTGACAATCAGACACGCATAACTAAGG |
| 6715 | SRD5A1 | HSX2_01238416 | 795 | GCGCGCAATCGTCAGACGAACTCAGTGTACGGCCGCCACGCGCTGCCCAGCCACA GGCTCCGAGTGCCGGCGCGGGCCGCCTGGGTGGTGCAG |
| 23635 | SSBP2 | HSX2_01650778 | 796 | AATTCTCCCAATAATATGAGCCTGAGTAATCAACCGGGCACTCCAAGGGATGATGG CGAAATGGGGGGAAATTTCTTAAATCCTTTTCAGAGTGAGAGT |
| 81849 | ST6GALNAC5 | HSX2_02066372 | 797 | TGTACAATGTACCTCTCCCATGAGCGAGGACGCAAGGGCAGTCATCACCGCTTTAT CACAGAGAAACGAGTCTTTAAGAACTGGGCACGGACATTCAATATTCACTTTTTCA ACCAGACTGGAAACCAGAATCACTTGCTATAAATCATCCTGAGAATAAACCTGTGTT CTAAGGAATGAGCATGCCAGACTGTAATCCCAGGTATTCACTGCATCAGACACCGA GACACTGAACTTCCTGAGCCACCAGACAGGAAAGGGTAGCAGAAAACAGCTTCACT CCTCAGGAAGTACCATGGACAGACGCCTACCAGGGGTGACAAAGCAGTGCAGTTG GATTGTAAGGAAAAATTCCGGAATTAATGCATCCTAATGAATGTTGTCCCCTTCAAT GGTGTTACCTTAGGAGCTGAACATTCAATTCAGTTACACCACTATGACTAAAAACAG TTTGGATCTCTTAGTATTGCCTTTGAAACTGCAACATAAGCAACTCAACAATATTAG TTGCATTCCTTTATAGACATACCATGTCAAAGACGTTTTTCTATCAAGTTGTATTCTT TCCTGTTCTATAACCTTTGTCATCTGTTAGACTCTGTATGTGTGATTTGTAAAAAGCA GGCTGAAACTATGGACATGATTTCTGAAGAGCACATCTCCACTGACTTTCATAAAGC AAATGTCCAATATTTATTTATTGAGAGTTTTTTAGTGCAATCTGGGCCAGTATTTTTA TAGATTATGATTATGTGGTAATTTATCCTTCCTAACTCTTTAATCCTGAATGATGGTT GGAAATGGCCTAGAATTAGGTTACTCTGTTCACAATGCTCATTGTTAGCATGCAATT GGTATTTGACTTGGAAGTGTTGTGTTGTATTTTTTGAACCCCTAGGCTTCAGGAAAA CTGCTCTTTTGTAAAAAGAATAGCGATGACATTTTCTAATGTGCAGAAATGTTCCAA |
| 90627 | STARD13 | HSX2_02136138 | 798 | AAATTGAGGCAAAAGAAGCATGTGACTGGCTCCGTGCTGCCGGGTTCCCGCAATAC GCTCAGTTATATGAGG |
| 202374 | STK32A | HSX2_02290257 | 799 | TCAACTTTGACCACTTTGAAATTTTGCGAGCCATTGGGAAAGGCAGTTTTGGGAAG |
| 202374 | STK32A | HSX2_02290318 | 800 | CACCTGAGATGTTCAGCTCCAGAAAAGGAGCAGGCTATTCCTTTGCTGTTGACTGG TGGTCCCTGGGAGTGACGGCATATGAACTGCTGAGAGGCCGG |
| 202374 | STK32A | HSX2_02290333 | 801 | ACGACTGTTGTAACTTACCCTTCTGCCTGGTCACAGGAAATGGTGTCACTTCTTAAA AAG |
| 202374 | STK32A | HSX2_02290361 | 802 | TGTCACTTCTTAAAAAGCTACTCGAACCTAATCC |
| 202374 | STK32A | HSX2_02580836 | 803 | AAAGGCAGGCTGAATTGTGATCCTACCTTTGAACTTGAGGAAATGATTTTGGAGTC CAAACCTCTACATAAGAAAAAAAAGCGTCTGGCAAAGAAGGAGAAGGATATGAGG AAATGCGATTCTTCTCAG |
| 6812 | STXBP1 | HSX2_01245920 | 804 | TTGTGGAAGATATCAATAAGCGCAGAGAGCCGCTCCCCAGCCTGGAGGCTGTGTAT CTCATCACTCCATCCGAGAAG |
| 6812 | STXBP1 | HSX2_01245932 | 805 | CTTGTCCAGATGCCCTGTTTAATGAACTGGTAAAATCCCGAGCAGCCAAAGTCATCA AAACTCTGACGGAAATCAATATTGCATTTCTCCCGTATGAATCCCAG |
| 6812 | STXBP1 | HSX2_01245999 | 806 | CCTGAATGAGATGCGCTGCGCCTACGAGGTGACCCAGGCCAACGGAAAGTGGGAG GTGCTGATAG |
| 6812 | STXBP1 | HSX2_02488589 | 807 | GTGGGAGGTGCTGATAGGATCCACACACATCCTC |
| 6812 | STXBP1 | HSX2_02520590 | 808 | GCATCACGGAGGAAAACCTGAACAAACTGATCCAGCACGCCCAGATACCCCCGGA GGATAGTGAGATCATCACCAACATGGCTCACCTCGGCGTGCCCATCGTCACCGAT |
| 6812 | STXBP1 | HSX2_02520602 | 809 | CGCCCGCTATGGGCACTGGCATAAGAACAAGGCCCCAGGCGAGTACCGCAGTGGC CCCCGCCTCATCATTTTCATCCTTGGGGGTGTGAGCCTGAATGAGATGCGCTGCGC CTACGAGGTGACCCAGGCCAACGGAAAGTGGGAGGTGCTGATAG |
| 125206 | SUMO3 | HSX2_02194590 | 810 | CTTTTGACCAGATCGGTGGTTACGGGCAGCTGGAGGCAGCCTACGCCCAGGCCATT CCCTCCAGGACCATTGCCAACACCACCTGCCACCTGCCACGTACAGACGCCATGCAC ATGTTTCGAGACCCCCACACAGGGGACCTGCCGTGGACCGGGATGACCTTTGGCCT GACCATCATGGCCACCTGGTACTGGTGCACCGACCAG |
| 23345 | SYNE1 | HSX2_01626487 | 811 | TAACATCATGCAGGCAGCTGTGGTACAATATGAA |
| 10454 | TAB1 | HSX2_01499454 | 812 | GCCTTCGATGTGGTGGAGAGGAGCTTCCTGGAGTCCATTGACGACGCCTTGGCTGA GAAGGCAAGCCTCCAGTCGCAATTGCCAGAG |
| 9882 | TBC1D4 | HSX2_01449478 | 813 | TGATGGAGAAGGGAGAAAAAGGACCTCATCTACCTGCAGCAATGAGTCCCTAAGT GTGGGAGGAACCTCTGTCACTCCTCGCCGGATCTCCTGGCGGCAGCGCATTTTCCT CAGGGTTGCTTCTCCCATGAACAAATCTCCCTCAGCAATGCAACAGCAAG |
| 8557 | TCAP | HSX2_01344131 | 814 | GACACCCAGAGACATGAGACCTACCACCAGCAGGGGCAGTGCCAGGTGCTGGTGC AGCGCTCGCCCTGGCTGATGATGCGGATGGGCATCCTCGGCCGTGGGCTGCAGGA GTACCAGCTGCCCTACCAGCGGGTACTGCCGCTGCCCATCTTCACC |
| 6920 | TCEA3 | HSX2_01254327 | 815 | ACTCCCCTGGACCCCCAAAAGGAGAAAAGGAGAGGAAAGAGAAAAGGCAAAGA AGAAGGAAAAAGGGCTTGAGTGTTCAGACTGGAAGCCAGAAGCAGGCCTTTCTCC ACCAAGGAAAAAACGAGAAGACCCCAAAACCAG |
| 6920 | TCEA3 | HSX2_01254358 | 816 | GAAATGGCCAGTGATGAACTGAGGGAGTTGAGGAATGCCATGACCCAGGAGGCCA TCCGTGAGCACCAGATGGCCAAGACTGGCGGCACCACCACTGACCTCTTCCAGTGC AGCAAATGCAAGAAGAAGAACTGCACCTATAACCAG |
| 10178 | TENM1 | HSX2_01477556 | 817 | TGCTTGCATTAAAGGACTTCCTCATCCTTTTTTCATGAAACTGAGCTTGCTTAATCA GAGATGGAGCAAACTGACTGCAAACCCTACCAGCCTCTACCAAAAGTCAAGCATGA AATGGATCTAGCTTACACCAGTTCTTCTGATGAGAGTGAAGATGGAAGAAAACCAA GACAGTCATACAACTCCAGGGAGACCCTGCACGAGTATAACCAGGAGCTGAGGAT GAATTACAATAGCCAGAGTAGAAAGAGGAAAGAAGTAGAAAAATCTACTCAAG |
| 10178 | TENM1 | HSX2_01477563 | 818 | GTTTCAAATTCTCTCCTGTTTGTTGTGACATGGAGGCTCAAGCTGGGTCTACTCAAG |
| 10178 | TENM1 | HSX2_01477568 | 819 | CGCCTCCTCATGCCTGCACCTGTGCCAGGAAGCCACCCCCTGCAGCGGACTCTCTTC AGAGGAGATCAATGACTACCCGCAGCCAGCCCAGCCCAGCTGCTCCAGCTCCCCCA ACCAGCACGCAGGATTCAGTCCATCTGCATAACAGCTGGGTCCTGAACAGCAACAT ACCATTGGAGACCAG |
| 10178 | TENM1 | HSX2_01477573 | 820 | GAACTACCCTCTGACATCCAATACCGTGTACTCGCCCCCTCCCAGGCCTCTTCCTCG AAGCACCTTTTCCCGACCTGCCTTTACCTTTAACAAACCTTACAGGTGCTGCAACTG GAAGTGCACAGCATTGAGCGCCACTGCAATCACAGTGACTTTGGCCTTGTTACTAG CCTATGTGATTG |
| 10178 | TENM1 | HSX2_01477585 | 821 | TGTTTCAGAAGGGACGGGCGATAGACACTGGAGAAGTTGACATTGGTGCACAGGT CATGCAGACCATTCCACCTGGTTTATTCTGGCGTTTCCAGATTACTATCCACCATCCA ATATATCTGAAGTTCAATATTTCTTTAGCCAAGGACTCTCTGCTGGGAATTTATGGC AGAAGAAACATTCCACCTACACATACTCAG |
| 10178 | TENM1 | HSX2_01477615 | 822 | CTCACTACTTAGATGCTGTCCGAG |
| 10178 | TENM1 | HSX2_01477625 | 823 | ATGGTTTAACCGACTGTGTGGATCCTGACTGTTGTCAACAAAGCAACTGTTATATAA GTCCTCTCTGCCAGGGCTCACCAGATCCTCTTGACCTCATTCAGCAAAGCCAAACTC TCTTCTCTCAGCACACTTCAAGACTTTTTATGATCGAATCAAATTCCTCATTGGCAA GGACAGTACTCATGTCATTCCTCCTGAGGTGTCATTTGACAGCAG |
| 10178 | TENM1 | HSX2_01477630 | 824 | GGAGTGAATGTCAGTTTCTTGCACCACAGTGATTATGGGTTTACCATCAGCCGGCA AGATGGAAG |
| 10178 | TENM1 | HSX2_01477635 | 825 | TTCCTGCCTGAGAAGAGAACACTCTGGTTGCCTTGGAATCAGTTTATTGTGGTAGA GAAAGTCACCATGCAGAGAGTTGTATCAGACCCGCCATCCTGCGATATCTCCAACTT TATCAGCCCAAACCCTATTGTGCTTCCTTCACCGCTCACATCATTTGGAGGGTCCTG TCCAGAGAGGGGAACTATTGTTCCTGAGCTGCAG |
| 10178 | TENM1 | HSX2_01477640 | 826 | GTTGTACAGGAGGAAATTCCCATTCCCTCCAGCTTTGTGAGGCTGAGTTACCTGAG CAGCCGCACCCCTGGGTATAAAACCCTGCTACGGATCCTTCTGACACATTCAACGAT TCCTGTAGGCATGATAAAAGTACACCTCACAGTAGCTGTGGAAGGGCGACTCACAC AGAAGTGGTTTCCCGCCGCAATTAATCTTGTCTACACATTTGCTTGGAACAAGACCG ACATCTATGGACAGAAGGTTTGGGGCCTGGCAGAGGCTTTGG |
| 10178 | TENM1 | HSX2_01477645 | 827 | TATCTGTGGATATGAATATGAAACGTGCCCTGACTTTATTCTCTGGGAGCAAAGG ACAGTCGTTTTACAAGGTTTTGAGATGGATGCTTCTAACCTAGGAGGCTGGTCTTT GAATAAGCATCACATTTTGAATCCTCAAAGTG |
| 10178 | TENM1 | HSX2_01477650 | 828 | ATATCAACCATAATGGGTAATGGACACCAAAGGAGTGTAGCCTGCACCAACTGCAA TGGCCCAGCCCACAACAACAAACTCTTTGCTCCTGTCGCCTTAGCTTCTGGCCCTGA TGGCAGTGTGTATGTTGGCGACTTCAATTTTGTAAGGAGAATATTTCCCTCGGGAA ACTCCGTTAGTATTTTGGAATTAAG |
| 10178 | TENM1 | HSX2_01477665 | 829 | GCATCACAGTTGATAGGCATGGATTTATTTACTTTGTGGATGGGACTATGATTCGCA AAATTGATGAGAATGCTGTGATCACAACTGTAATCGGCTCAAATGGTCTGACTTCC ACACAACCACTGAGCTGTGACTCAGGAATGGACATCACTCAG |
| 10178 | TENM1 | HSX2_01477670 | 830 | TGTCTTGGATAACAACATTGTGCTGCAAATTTCTGAGAACAGGCGTGTTCGGATCA TCGCAGGACGCCCCATTCACTGCCAGGTGCCAGGCATCGATCATTTCCTGGTCAGC AAGGTAGCAATTCACTCCACTCTAGAGTCAGCGAGGGCCATCAGTGTCTCCCACAG CGGGCTGCTCTTCATAGCTGAAACAGACGAGAGGAAAGTAAACCGCATTCAGCAA GTAACCACCAATGGGGAGATCTACATCATCGCTGGTGCCCCCACTGACTGTGACTG CAAAATTGATCCAAACTGTGACTGTTTTTCAG |
| 10178 | TENM1 | HSX2_01477675 | 831 | TCGCCTGATGGAACCCTCTATGTGGCAGACCTCGGAAATGTTCGAATTCGTACCATC AGCAGGAACCAAGCCCACCTGAATGACATGAACATTTATGAGATTGCTTCACCCGC TGATCAGGAACTGTACCAGTTCACTGTAAATGGAACCCACCTACACACCCTGAACTT GATAACAAGGGACTATGTTTATAACTTCACCTACAATTCTGAAGGTGACTTGGGCG CGATTACCAGCAGCAATGGCAATTCAGTGCACATTCGCCGTGATGCAGGCGGAATG CCGCTATGGCTTGTGGTGCCTGGCGGACAAGTATACTGGCTGACTATAAGCAGCAA TGGAGTCCTGAAAAGAGTGTCAGCCCAAGGCTATAATCTGGCCTTAATGACCTATC CAGGAAACACAGGGCTTCTGGCTACCAAAAGTAACGAAAATGGATGGACAACCGT |
| 10178 | TENM1 | HSX2_01477685 | 832 | CCAGCGGGATGGAGATCGGCCTCAGCTCAGAGCCCCACATCCTGGCAGGGGCAGT CAACCCTACCCTGGGCAAATGCAACATCTCATTGCCCGGAGAGCACAATGCAAACC TCATCGAGTGGCGGCAGAGGAAGGAGCAAAACAAAGGCAATGTTTCGGCTTTTGA AAGGAGGCTGAGG |
| 10178 | TENM1 | HSX2_01477690 | 833 | ATCTATGATGACCATCGAAAATTCACCCTTCGAATTCTTTATGACCAGACTGGGCGA CCCATTCTGTGGTCTCCTGTAAGCAGATATAATGAAGTGAACATCACATATTCACCT TCGGGATTGGTGACGTTTATTCAAAGAGGAACGTGGAATGAAAAAATGGAATATG ACCAGAGTGGGAAAATTATTTCAAGAACTTGGGCTGATGGGAAAATTTGGAGCTAT ACCTACTTAGAAAAA |
| 10178 | TENM1 | HSX2_01477695 | 834 | GATTGTCTGCTCTCAGTCACCATGCCTAGCATGGTGCGTCACAGCTTACAAACCATG CTTTCAGTGGGCTACTACCGGAACATCTACACCCCACCGGACAGTAGCACTTCTTTT ATCCAAGACTATAGTCGAGATGGCCGATTGCTACAGACCCTGCATCTGGGGACAGG GCGCAGAGTCTTATACAAGTACACCAAGCAAGCAAGGCTTTCTGAGGTTCTCTATG ATACCACTCAGGTCACATTAACATATGAAGAGTCTTCTGGAGTGATTAAGACAATA CACCTGATGCATGACGGATTCATCTGCACAATCAGATACAGGCAAACAG |
| 10178 | TENM1 | HSX2_01477700 | 835 | CGGTTCGACTACAGCTACAACAATTTCCGAGTCACAAGCATGCAAGCTGTAATCAA TGAAACCCCTTTGCCTATAGATCTTTACCGATATGTTGATGTCTCTGGCAGAACAGA GCAGTTTGGAAAATTCAGTGTAATTAATTACGATTTAAATCAGGTCATAACTACTAC AGTGATGAAACACACCAAAATCTTCAGTGCCAATGGACAAGTCATTGAAGTCCAAT ATGAAATCCTAAAGGCAATTGCCTACTGGATGACCATTCAATATGATAATGTGGGC CGCATGGTAATATGTGACATAAGGGTAGGAGTAGATGCCAATATAACAAGGTACTT CTATGAATACGATGCTGATGGGCAACTTCAGACTGTTTCTGTAAATGACAAAACCC AGTGGCGTTATAGTTACGATCTGAATGGAAACATCAACCTCTTAAGCCATGGGAAG AGTGCTCGTCTTACTCCTCTCCGATATGACCTCCGAGACCGCATCACCAGATTAGGA GAAATTCAGTATAAAATGGATGAAGATGGCTTTCTGAGGCAGAGGGGAAATGATA TTTTTGAATATAATTCTAATGGCCTGCTGCAGAAAGCCTACAATAAGGCTTCTGGCT GGACTGTGCAGTATTACTATGATGGGCTTGGGCGACGTGTCGCGAGTAAGTCCAG CCTAGGGCAGCACCTTCAGTTCTTTTATGCAGACCTTACCAACCCCATAAGAGTTAC TCATTTGTACAACCACACAAGCTCGGAGATTACATCTCTGTATTATGATCTCCAAGG TCACCTTATTGCCATGGAGTTAAGCAGTGGTGAAGAATATTATGTAGCCTGTGATA ATACAGGTACCCCACTAGCTGTGTTCAGCAGCCGAGGTCAGGTCATAAAGGAGATA CTATACACACCTTATGGCGATATCTATCATGACACTTACCCTGACTTTCAGGTCATTA TTGGTTTTCATGGAGGACTCTATGATTTCCTTACTAAATTAGTGCACCTGGGGCAAA GGGATTATGATGTTGTTGCTGGCAGATGGACAACGCCTAATCATCACATATGGAAA |
| 10178 | TENM1 | HSX2_01477705 | 836 | ACATCAGAAGTTGGTTGGAGCTATTTGGTTTCCAATTACACAATGTACTACCTGGAT TTCCCAAACCTGAATTAGAAAATTTAGAATTAACTTACGAGCTTCTACGGCTTCAGA CAAAAACTCAAGAGTGGGATCCTGGAAAG |
| 10178 | TENM1 | HSX2_01477710 | 837 | GACCAACTACCTATGACTCCCCGATACAATGATGGACGGTGCCTTGAAGGAGGGAA GCAACCAAGGTTTGCTGCTGTCCCTTCTGTTTTTGGGAAAGGTATAAAATTTGCCAT CAAGGATGGCATAGTAACAGCTGATATTATAGGAGTAGCCAATGAAGATAGCAGG CGGCTTGCTGCCATTCTCAATAATGCCCATTACCTGGAAAACCTACATTTTACCATA GAGGGGAGGGACACTCACTACTTCATTAAGCTTGGGTCTCTGGAGGAAGACCTGG TGCTCATCGGTAACACTGGGGGGAGGCGGATTCTGGAGAATGGTGTCAATGTCAC TGTGTCCCAGATGACTTCTGTGTTGAATGGGAGGACTAGACGGTTTGCAGATATTC AGCTCCAGCATGGAGCCCTGTGCTTCAACATCCGGTATGGGACAACTGTCGAAGAG GAAAAGAATCACGTGTTGGAGATTGCCAGACAGCGCGCAGTGGCCCAGGCCTGGA CTAAGGAACAAAGAAGGCTGCAAGAGGGGGAAGAGGGGATTAGGGCATGGACAG AAGGGGAAAAGCAGCAGCTTTTGAGCACTGGGCGGGTACAAGGTTACGATGGGTA TTTTGTTTTGTCTGTTGAGCAGTATTTAGAACTTTCTGACAGTGCCAATAATATTCAC TTTATGAGACAGAGCGAAATAGGCAGGAGGTAACAAAAATATCTCTGCCTTTGCGT |
| 10178 | TENM1 | HSX2_01477711 | 838 | AGACTGCCTGTTTTTAAAACATAAAATGGTTTATTGTATTGGTTTTCTAGATCAGAA CTCTGTATATGTAAATATGGAGGAAAAACATATCCAACTGCCTTTCAATGTGACGG AAGATGGTATTTTAATATTGTTTGTTTAAACTCTTTAAGAAATGACAGAGATTTTTA GTTCTTGTGTGGCAGTATTCAAAATAACACAAGTAGAACTCAAACAGCTAAAAACA GTTTTCAGAAAGCACCACTTTCAATTTGCCGAGCCATGCATATGTTCCAATATCCAG AAAGAACCCAAGGTTCTCTATCTCTATTGTGAGAAGCAGTTTCATCCTTAACTGTTG GCAGAACTTACGGGCTATTTGAATAGGTGGTGCAATAGTATCTGAAACTTGCCTTT CGAAAGACTGCCAGCCCTTTGACGTTTTCCAGATCTGTTATAGGAAACTTAAAAACA GGTGTAAAATGTCTTCAGCCACCATCTCCTAGAGTGAGGACCCAATTGCCCTTCCTT CTTGATTATTCCTCCTTGCTTGTTAAAGTAAATGCCATATTGTTGTGCTGTGTTTTGG CGTGTGGTGGCTGGGTTCTGTCTACCATGCTTCCCTGTGGGTGTGGTAACCAGACT GTATAGCCGCTATTTGCTCGTGTGTACATGATACCAAAGCAGCTGGCCAGCGTGAC CTCTCTCACACGACCTGTTTTGACTCAATTTTTTACTAAAAGTTGTTCAGCTGTATTG GTATCATGTAAACATAGCTTTTATTAACCTGGGTAGGAATTTCTCATTTATATATAG GATGTGTTTTGGTCATAGTTTCACATTAGTGATTCAGTATCTATACACTGACCCAAT GGTTTTGTGCACATGAACGGTAATTTACTTAAAAGTATGATTCTGGTACAAAAACAA ACAAAGGCTTTAGCAGGCATACGTGTCTGGGATGCCGATACATACATTAACTACTA CTGCAGAAATTCATAAGAGCCAAAACCTTAAAAAAATAGACCTGGTACTTAAGTGA AAGTACTAAAGGGAAGACCAGACCAAACATCACAGCAGTTGCTGCCACATTGTTTC AGCCCACTTAGATTTATCTTTCAAATGTACAATTCTGTATTGAACATCTCCCAGCCAT CTTCAGGAAATCGAATCAAGTAAATCCTTTCCAACCGAAAACATTTCAACTAACTAT AGAGAGGCAGACTCATTTTTACTAAAATAATTTATACAGTTAGTTATTTTCGTTCTCC GTACTTACCCATTTATCTTTATTTAATCGTCTCTACTGCCTAGGAAAATAACTATTTT CCAGGACGGGTTATTTGTTCTGCGATCATTTAAAATTTGGAGAAAGGTCAGGATTA GTGTTAATATCAGCTGCAGTTTCTCAATCTCTAGGAATCCTGCAGTAAAACAAGCCC CTTGGTGAGCTGGAAGATTTGTGCCCAGTGACAAAGAGATAGTTTGTAAAATGCTG TGTAATTGTAAGTTACCACAAATGAAAATACATGACAGCACAATGTGGCCCGTAGA AAATTCCCCTGAGCCAGCTTCTGCACTTTCATCACCGAATCTGAACATTTGCTATGTC TGAAGGCAAATTTATGATGGAATGTTAGTTTGGATTCTTTCCAGATGCTACCTAAAT GCAGTGTGGGGTCATTGCCTTGCTTTGCGATGACAGTTTCTTTGAAAATATGCAAA GTCATAAGCTCATGTTAAGGTTTTTCAAGAGTCTGCCTCCTACTACACAAAGGAAAG CAAGGGAAAGGAAATGACCCTGGCAAACAGTAGGGAAGGGTGTATTCAAACATTT CATTTTCAAAACCTTCGGGTTAGAATACCACTTACACATGTATTCTGAGAGACAGAA TTCATGAGGAACTCATCTCTCTTTATAACTGGAAACACACCAGCTTGATATATTGCT AATCCATACTAAAATCATATTATTGGGTTTTTCTGAATCAGGCCTGTATTAATGGT |
| 29842 | TFCP2L1 | HSX2_01723004 | 839 | ACTGCCCAAGAGAAGGAGAAATACCAGCCGTCCTATGAAACCACCATCCTCACAGA G |
| 7039 | TGFA | HSX2_01263636 | 840 | AAGAAAGGCTTCTTCAGGACAGCACTGCCAGAGATGCCTGGGTGTGCCACAGACCT TCCTACTTGGCCTGTAATCACCTGTGCAGCCTTTTGTGGGCCTTCAAAACTCTGTCA AGAACTCCGTCTGCTTGGGGTTATTCAGTGTGACCTAGAGAAGAAATCAGCGGACC ACGATTTCAAGACTTGTTAAAAAAGAACTGCAAAGAGACGGACTCCTGTTCACCTA GGTGAGGTGTGTGCAGCAGTTGGTGTCTGAGTCCACATGTGTGCAGTTGTCTTCTG CCAGCCATGGATTCCAGGCTATATATTTCTTTTTAATGGGCCACCTCCCCACAACAG AATTCTGCCCAACACAGGAGATTTCTATAGTTATTGTTTTCTGTCATTTGCCTACTGG GGAAGAAAGTGAAGGAGGGGAAACTGTTTAATATCACATGAAGACCCTAGCTTTA AGAGAAGCTGTATCCTCTAACCACGAGACCCTCAACCAGCCCAACATCTTCCATGGA CACATGACATTGAAGACCATCCCAAGCTATCGCCACCCTTGGAGATGATGTCTTATT TATTAGATGGATAATGGTTTTATTTTTAATCTCTTAAGTCAATGTAAAAAGTATAAA |
| 7048 | TGFBR2 | HSX2_01264514 | 841 | CCTACCATGACTTTATTCTGGAAGATGCTGCTTCTCCAAAGTGCATTATGAAGGAAA AAAAAAAGCCTGGTGAGACTTTCTTCATGTGTTCCTGTAGCTCTGATGAGTGCAAT GACAACATCATCTTCTCAGAAG |
| 7069 | THRSP | HSX2_01266503 | 842 | AGCCAATGATGAGAGCAGAAAGGCCTAGACCTGCAGCCAGAAGTGAAGGCGGCTC AGTTCTCCGGGATGCTTCTCTACCTCCTGAGCACCAATTCCTGGATTCCAGTCACTG GCTCACCTTTAGAATGTCTGTTGCTATTCACTGCTCCCCTCGCTCCTCTTAACAGCTT GGGGAGGTGACCAGTGGTTCAGGAGGGACTAGACAATTACCTGTCCAGTGTGGTA TGGTAGGAAGAGTGTAGGTGTTGGCACGTGACCAAAATTCACATCCCTCCTCATGG CAGTCATTCAGTATGTGTACTTGTACAAGTTATTTAACCCATTGGAGCCTAAATTCC CTCATCTATAAAATGGGGATAATATTATCTACCTCACAAGCTTATGAAAACTAAACA TGATGAATCAAAAGCACTTGGCATGTGAGGGCTATTAAAATAGCCTGATTTTTTTT TCTCCCCCTCTCCCCAATGTATTTGCTCTGGCCCTTGCTTTTTACCCTCCAGAGCTAA GAGGTAGCAGAGTCTCTTGGGATGAGTGATTCACCCTCTTACTTGGCGACCACTGA TGAGATCAACAACAGGTGAACTATAAACCTATTATTTATTGCAGAACTAATAAAAAA |
| 25976 | TIPARP | HSX2_01671479 | 843 | ACCGAACCTGAGCCAGACTGTGTAGTGCAGCCTCCCTCTCCTCCTGATGACTTTTCA TGCCAAATGAGACTCTCTGAGAAGATCACTCCATTGAAGACTTGTTTTAAGAAAAA GGATCAGAAAAGATTGGGAACTGGAACCCTGAGGTCTTTGAGGCCAATATTAAAC ACTCTTCTAGAATCTGGCTCACTTGATGGGGTTTTTAGATCTAGGAACCAGAGTACA GATGAGAACAGCTTACATGAACCTATGATGAAGAAAGCCATGGAAATCAATTCATC ATGCCCACCAGCAGAAAATAATATGTCTGTTCTGATTCCTGATAGGACAAATGTTG GGGACCAGATACCGGAAGCCCATCCTTCCACTGAAGCTCCAGAACGAGTGGTTCCA ATCCAAGATCACAGCTTTCCATCAGAAACCCTCAGTGGGACGGTGGCAGATTCCAC ACCAGCTCACTTCCAGACTGATCTTTTGCACCCAGTTTCAAGTGATGTTCCTACTAGT CCTGACTGCTTAGACAAAGTCATAGATTATGTTCCAGGCATTTTCCAAGAAAACAGT TTTACAATCCAATACATTCTGGACACCAGTGATAAGCTGAGTACTGAGCTCTTTCAG GACAAAAGTGAAGAGGCTTCCCTTGACCTCGTGTTTGAGCTGGTGAACCAGTTGCA GTACCACACTCACCAAGAGAACGGAATTGAAATTTGCATGGACTTTCTGCAAGGCA CTTGTATTTATGGCAGGGATTGTTTGAAGCACCACACTGTCTTGCCATATCATTGGC AGATCAAAAGGACAACTACTCAAAAGTGGCAGAGTGTATTCAATGATTCTCAGGAG |
| 25976 | TIPARP | HSX2_01671483 | 844 | AGGACAAGAATTTTGGGCAGATTTGAATGCCATGAACGTGTATGAAACAACTGAAT TTGACCAACTACGAAGGCTGTCCACACCACCCTCTAGCAATGTCAACTCTATTTACC ACACAGTCTGGAAATTCTTCTGTAGGGACCACTTTGGATGGAGAGAGTATCCCGAG |
| 25976 | TIPARP | HSX2_01671512 | 845 | AGACATTTATTTCATGGAACATCCCAGGATGTGGTAGATGGAATCTGCAAACACAA CTTTGACCCTCGAGTCTGTGGAAAGCATGCTACAATGTTTGGACAAGGCAGTTATTT TGCAAAGAAGGCAAGCTACTCTCATAACTTTTCTAAGAAGTCCTCCAAAGGAGTCC ACTTCATGTTTCTGGCCAAAGTGCTGACGGGCAGATACACAATGGGCAGTCATGGC ATGAGAAGGCCCCCGCCAGTCAATCCTGGCAGTGTCACCAGTGACCTTTATGACTC TTGTGTGGATAATTTCTTTGAGCCTCAGATTTTTGTCATTTTTAATGATGACCAGAGT TACCCTTATTTTGTTATCCAATATGAAGAAGTCAGTAACACTG |
| 9414 | TJP2 | HSX2_01405349 | 846 | AAAACGCACAAGCCAGACCCTGGCACGCCCCAGCACACGAG |
| 7088 | TLE1 | HSX2_01268117 | 847 | CAAAAATCGCCTGCTAAAGAAGGATGCTTCTAGCAGTCCAGCTTCCACGGCCTCCTC G |
| 7088 | TLE1 | HSX2_01268172 | 848 | GTCACTGGCTCGGGGACAAGAAGGCTACAGTCTATGAAGTCATCTACTGAAAACA TTATGTGGTTTAACGTTTATAGTTGAATTGGGCCAAAATGTTTCGAATTTATAGAAA TAGAAAAGTTGTAACTTTAAAAGAGAAAAAAAATTACAAACACCTGTTTCCAAACCT TGACAGAAAACTACTTTGAGTCTACAAAGAGGAGGCGACAAGTCCATCAGCAGAA AGTCACCTGTCTACATAGACCAAATGGAGCACCAAGGCCAAGCGGACAGAGGGGC CATGGGTTGTAGGATTGAGGAACGGAATCTGCCGACTCACATGACAGCCCATTCTT TCTTTCTGGGTGATCTGGGGATCACGCCTTGCCCAAGTGTGAGATTACCTTTCTGTT CCTTGCAGTTCACCTCACTTTCCGTCCTTTGTAGAGCAGTGGTGTCTCCAATGAACTT GTTTCCTGGTTTTGCATCTTGTGAAATGTTTTTTTGTATTTTTGTTGAAGGTTAAACA TTTGTATAAATTGTAAATATATTTGGTTTATTACAGTAAAGGCTTTAGTACCAATAA |
| 83660 | TLN2 | HSX2_02072578 | 849 | CCTGGAAGCAAGATGGTGTCCTCTGCCAAAGCCGCAGTGCCCACCGTGAGTGACCA GGCCGCAGCCATGCAGCTGAGCCAGTGTGCCAAGAACCTGGCCACCAGCTTGGCG GAGCTGCGTACCGCCTCGCAGAAG |
| 25963 | TMEM87A | HSX2_01670934 | 850 | TGATGGAGAACCTTGTGACCTGTCTTTGAATATAACCTGGTATCTGAAAAGCGCTG ATTGTTACAATGAAATCTATAACTTCAAG |
| 25963 | TMEM87A | HSX2_01670939 | 851 | CACTATCTTCCTGAAGTTTGATGGAGAACCTTGT |
| 25963 | TMEM87A | HSX2_01670944 | 852 | GAAGTAGAGTTGTATTTGGAAAAACTTAAGGAAAAAAGAGGCTTGTCTGGGAAAT ATCAAACATCATCAAAATTGTTCCAGAACTGCAGTGAACTCTTTAAAACACAG |
| 25963 | TMEM87A | HSX2_01670955 | 853 | ACCTTTTCTGGAGATTTTATGCATCGACTGCCTCTTTTAGGAGAAAAAC |
| 25963 | TMEM87A | HSX2_01671067 | 854 | TTGGATTGGTGCTGTCATCTTCCTGGGAATGCTT |
| 25963 | TMEM87A | HSX2_01671111 | 855 | GGATATGGCATCGTCAAGCCACGCCTTGGAGTCA |
| 25963 | TMEM87A | HSX2_01671149 | 856 | GCCTTGTGCTGGTGGATATTTATTAGCCTGACTC |
| 25963 | TMEM87A | HSX2_01671180 | 857 | TAGTGACATGTCAGTCGGACTGGCGGGAGCTGTG |
| 25963 | TMEM87A | HSX2_01671193 | 858 | GCCTTTTCACCATTGTCTGAGGAAGAGGAGGAGGATGAACAAAAGGAGCCTATGC TGAAAGAAAGCTTTG |
| 25963 | TMEM87A | HSX2_01671207 | 859 | TCTGCAAACAACCAGAGGTTTGCCTTTTCACCAT |
| 25963 | TMEM87A | HSX2_01671265 | 860 | TTGCAGTTAAAGATGGCTACCATCAGGGAAGAGATCAGCATCTGTGTCAGTCTTCT GTACGGCTCCATGGGATTAAAGGAAGCAATGACATCCTGATCTGTTCCTTGATCTTT GGGCATTGGAGTTGGCGAGAGGTGTCAGAACAAAGAGAACATCTTACTGAAAACA AGTTCATAAGATGAGAAAAATCTACGAGCTTCTTATTTACAACACTGCTGCCCCCTT TCCTCCCAGACTCTGACATGGATGTTCATGCAACTTAAGTGTGTTGTTCCTGAACTT TCTGTAATGTTTCATTTTTTAAATCTGACAAACTAAAAAGTTTAACGTCTTCTAAAAG ATTGTCATCAACACCATAATATGTAATCTCCAGGAGCAACTGCCTGTAATTTTTATTT ATTTAGGGAGTTACATAGGTGATGGGGGAAATTGTTAACTACCTTTCATTTTCCTGG GAAGTCAAGGTTACATCTTGCAGAGGTTGTTTTGAGAAAAAAGGGCCCTTCTGAGT TAAGGAGCCATAGTTCTATCAATGATCAA |
| 25963 | TMEM87A | HSX2_02546143 | 861 | TTGATGGAGAACCTTGTGACCTGTCTTTGAATATAACCTGGTATCTGAAAAGCGCT GATTGTTACAATGAAATCTATAACTTCAAG |
| 25963 | TMEM87A | HSX2_02546146 | 862 | GGAAATATCAAACATCATCAAAATTGTTCCAGAACTGCAGTGAACTCTTTAAAACAC AG |
| 25963 | TMEM87A | HSX2_02546153 | 863 | ACCTTTTCTGGAGATTTTATGCATCGACTGCCTCTTTTAGGAGAAAAACAGGAG |
| 25963 | TMEM87A | HSX2_02546163 | 864 | TGATT |
| 26022 | TMEM98 | HSX2_01675662 | 865 | AACTGGACGATGTCGTTATCACCAACCCCCACATTGAGGCCATTCTGGAGAATGAA GACTGGATCGAAGAT |
| 26022 | TMEM98 | HSX2_01675678 | 866 | GGGTCTCATGTCCCACTGCATTGCCATCTTGAAG |
| 26022 | TMEM98 | HSX2_02546441 | 867 | GATGAAGACTTCAGCCAGTGTCAGCGACATCATTGTGGTGGCCAAGCGGATCAGC CCCAG |
| 51330 | TNFRSF12A | HSX2_01760100 | 868 | GCGCTGCAGCACCTCCTGCCCCCTTCCGGCTGCTTTGGCCCATCCTTGGGGGCGCTC TGAGCCTGACCTTCGTGCTGGGGCTGCTTTCTGGCTTTTTGGTCTGGAGACGATGC CGCAGGAGAGAGAAGTTCACCA |
| 23043 | TNIK | HSX2_01589734 | 869 | GGGCTGAGTCACCTGCACCAGCATAAAGTGATTCATCGAGATATTAAAGGGCAAAA TGTCTTGCTGACTGAAAATGCAGAAGTTAAACTAG |
| 23043 | TNIK | HSX2_01589742 | 870 | TGGACTTTGGAGTCAGTGCTCAGCTTGATCGAACAGTGGCAGGAGGAATACTTTC ATTGGAACTCCCTACTGGATGGCACCAGAAGTTATTGCCTGTGATGAAAACCCAGA TGCCACATATGATTTCAAG |
| 23043 | TNIK | HSX2_01589753 | 871 | CCACATATGATTTCAAGAGTGACTTGTGGTCTTT |
| 23043 | TNIK | HSX2_01589756 | 872 | CTCTCTGTGACATGCACCCCATGAGAGCTCTCTTCCTCATCCCCCGGAACCCAGCGC CTCGGCTGAAGTCTAAGAAGTG |
| 23043 | TNIK | HSX2_01589779 | 873 | CTCCATCCTGAATCTGCCAGGGGAGTCGACGCTGCGGAGGGACTTTCTGAGGCTGC AGCTGGCCAACAAGGAGCGTTCTGAGGCCCTACGGAGGCAGCAGCTGGAGCAGCA GCAGCGGGAGAATGAGGAGCACAAGCGGCAGCTGCTGGCCGAGCGTCAGAAGCG CATCGAGGAGCAGAAAGAGCAGAGGCGGCGGCTGGAGGAG |
| 23043 | TNIK | HSX2_01589799 | 874 | GAATATAAGCGCAAACAATTGGAAGAACAGAGACAAGCAGAAAGACTGCAGAGG CAGCTAAAGCAAGAAAGAGACTACTTAGTTTCCCTTCAGCATCAGCGGCAGGAGCA GAGGCCTGTGGAGAAGAAGCCACTGTACCATTACAAAGAAGGAATGAGTCCTAGT GAGAAGCCAGCATGGGCCAAGGAG |
| 23043 | TNIK | HSX2_01589809 | 875 | GTAGAAGAACGGTCAAGGCTCAACCGGCAAAGTTCCCCTGCCATGCCTCACAAGGT TGCCAACAGGATATCTGACCCCAACCTGCCCCCAAGGTCGGAGTCCTTCAGCATTA GTGGAGTTCAGCCTGCTCGAACACCCCCCATGCTCAGACCAGTCGATCCCCAG |
| 23043 | TNIK | HSX2_01589851 | 876 | CCAACAGTAAGTCAGAAGGATCACCTGTGCTCCCCCATGAGCCTGCGAAGGTGAAA CCAGAAGAATCCAGGGACATTACCCGGCCCAGTCGACCAGCT |
| 23043 | TNIK | HSX2_01589906 | 877 | ACGTCTGGAGAGAAGAAGCGATCTGGCCACAGTGACAGCAATGGCTTTGCTGGCC ACATCAACCTCCCTGACCTGGTGCAGCAGAGCCATTCTCCAGCTGGAACCCCGACT GAGGGACTGGGGCGCGTCTCAACCCATTCCCAGGAGATGGACTCTGGGACTGAA |
| 23043 | TNIK | HSX2_01589937 | 878 | AAGATTTCGGTGGTAAATGTAAACCCAACCAACATTCGGCCTCATAGCGACACACC AGAAATCAGAAAATACAAGAAACGATTCAACTCAGAAATACTTTGTGCAGCTCTGT GGG |
| 23043 | TNIK | HSX2_01590045 | 879 | CCTACATTCATTCCAATCAGATAATGGGCTGGGGCGAGAAAGCTATTGAGATCCGG TCAGTGGAAACAGGACATTTGGATGGAGTATTTATGCATAAGCGAGCTCAAAGGTT AAAGTTTCTATGTGAAAGAAATGATAAG |
| 7159 | TP53BP2 | HSX2_01274309 | 880 | GCCTCGGATGCCCTCAAGGCCTGAATTGCTGGTGAAGCCAGCCCTGCCGGATGGTT CCTTGGTCATTCAGGCTTCAGAGGGGCCGATGAAAATACAGACACTGCCCAACATG AGATCTGGGGCTGCTTCACAAACTAAAG |
| 7159 | TP53BP2 | HSX2_01274325 | 881 | GATTAATTTGCCTTATTTGGACAAACTAATCAGCCACCTTCAGACATTAAGCCAGA CGGAAGTTCTCAGCAGTTGTCAACAGTTGTTCCGTCCATGGGAACTAAACCAAAAC CAGCAGGGCAGCAGCCGAGAGTGCTGCTATCTCCCAGCATACCTTCGGTTGGCCAA GACCAGACCCTTTCTCCAGGTTCTAAGCAAGAAAGTCCACCTGCTGCTGCCGTCCG GCCCTTTACTCCCCAGCCTTCCAAAGACACCTTACTTCCACCCTTCAGAAAACCCCAG ACCGTGGCAGCAAGTTCAATATATTCCATGTATACGCAACAGCAGGCGCCAGGAAA AAACTTCCAGCAGGCTGTGCAGAGCGCGTTGACCAAGACTCATACCAGAGGGCCA CACTTTTCAAGTG |
| 7164 | TPD52L1 | HSX2_01274704 | 882 | TACTCCATTCGCCATTCCATAAGTATGCCTGCTATGAG |
| 7164 | TPD52L1 | HSX2_02522322 | 883 | TTACTCCATTCGCCATTCCATAAGTATGCCTGCTATGAG |
| 55521 | TRIM36 | HSX2_01854056 | 884 | TACCCACCCATTGATTCTCCCTTGCCAACATAGTATCTGTCATAAATGTGTAAAAGA ACTCCTGCTGACTCTCGATGATTCATTCAACGATGTGGGATCAGACAACTCCAATCA AAGCAGTCCTCGACTTCGGCTCCCCTCCCCTAGTATGGATAAAATTGACCGAATTAA CAGACCAG |
| 286144 | TRIQK | HSX2_02489942 | 885 | GCCTTGGCAGAGCTGAGCAGGCATTTTGGAGATC |
| 8848 | TSC22D1 | HSX2_01365731 | 886 | GGTGTTTTTGGCTGCAATTGCATGAAATCCCAATGGTGTAGACCAGTGGCGATGGA TCTAGGAGTTTACCAACTGAGACATTTTTCAATTTCTTTCTTGTCATCCTTGCTGGGG ACTGAAAACGCTTCTGTGAGACTTGATAATAG |
| 1E+08 | TSTD1 | HSX2_02584821 | 887 | TCTTCGACGTGCGCTCTCGCGAGGAGGCGGCAGCTGGGACCATCCCAGGGGCGCT CAACATCCCGG |
| 7991 | TUSC3 | HSX2_01317834 | 888 | CGGGTTTTCAGACCACCCAACTACTCTGGTACCATTGCTTTGGCCCTGTTAGTGTCG CTTGTTGGAGGTTTGCTTTATTTGAGAAGGAACAACTTGGAGTTCATCTATAACAAG ACTGGTTGGGCCATGGTGTCTCTG |
| 7991 | TUSC3 | HSX2_01317841 | 889 | CTATGACTTCTGGCCAGATGTGGAACCATATCCGTGGACCTCCATATGCTCATAAGA ACCCACACAATGGACAAGTG |
| 7991 | TUSC3 | HSX2_01317865 | 890 | TTCGTTCCAAGTACCACGGCTATCCTTATA |
| 7991 | TUSC3 | HSX2_01317884 | 891 | TAACCTCAG |
| 7991 | TUSC3 | HSX2_02525011 | 892 | ATTCGGGTTTTCAGACCACCCAACTACTCTGGTACCATTGCTTTGGCCCTGTTAGTG TCGCTTGTTGGAGGTTTGCTTTATTTGAGAAGGAACAACTTGGAGTTCATCTATAAC AAGACTGGTTGGGCCATGGTGTCTCTG |
| 7991 | TUSC3 | HSX2_02525012 | 893 | TGTATAGTCTTTGCTATGACTTCTGGCCAGATGTGGAACCATATCCGTGGACCTCCA TATGCTCATAAGAACCCACACAATGGACAAGTG |
| 7991 | TUSC3 | HSX2_02525016 | 894 | CTTTTTAATTAAATGAAGCCAAGTGGATTTGCATAAAGTGAATGTTTACCATGAAG ATAAACTGTTCCTGACTTTATACTATTTTGAATTCATTCATTTCATTGTGATCAGCTA GCTTATTCTTGTGTACTTTTTTTAAACTGTGGGTTTCCTAGTAAATTTAATTTACAG AAATCAATGGTAGCATTTAGTAATCTACAAAGGAAATATCAAAGTGTTTTTCAAGCC TGTTATATTCAGTGTGTGCCACAGGATTGAAATAAATGACAATGTAATTATGAA |
| 51377 | UCHL5 | HSX2_01762885 | 895 | GCTCATTAAAGGATTCGGTTGCCGAGGAGCCCAA |
| 51377 | UCHL5 | HSX2_01762887 | 896 | GCCAGTTCATGGGTTAATTTTTCTTTTCAAGTGGCAGCCAGGAG |
| 51377 | UCHL5 | HSX2_01762911 | 897 | CACCAGGATGTCCATTTAGGCGAGACATTATCAGAGTTTAAAGAATTTTCACAAAG TTTTGATGCAGCT |
| 51377 | UCHL5 | HSX2_01762923 | 898 | CGCCAG |
| 51377 | UCHL5 | HSX2_01762946 | 899 | GTGCATGCAATCAAGATGATTGGATCAGTGCAGTAAGGCCTGTCATAGAAAAAAG GATACAAAA |
| 51377 | UCHL5 | HSX2_01762958 | 900 | GTACAGTGAAGGTGAAATTCGATTTAATTTAATGGCCATTGTGTCTGACAGAAAAA TGATATATGAGCAGAAGATAGCAGAGTTACAAAGACAACTTGCAGAG |
| 51377 | UCHL5 | HSX2_01763188 | 901 | GCCGCCGGGGATTCCAGGTTGCCGAGGAGCCCAA |
| 51377 | UCHL5 | HSX2_02551898 | 902 | GCCAGTTCATGGGTTAATTTTTCTTTTCAAGTGGCAGCCAGGAGAAGAACCAGCAG GCTCTGTGGTTCAGGACTCCCGACTTGACACGATATTTTTGCTAAGCAG |
| 55813 | UTP6 | HSX2_02559089 | 903 | GAGAATCCTGATTATTCTGAAGAAATCCTTAAGGGCGAGTTGGCATGGATCATCTA CAAAAATTCTGTAAGCATAATTAAAG |
| 4013 | VWA5A | HSX2_01038194 | 904 | GATAAAATGCCAATCAGGTTTTCGAAAGGCCTTA |
| 4013 | VWA5A | HSX2_02507460 | 905 | GATAAAATGCCAATCAGCACCATTCACCGCCTTG |
| 55339 | WDR33 | HSX2_01849878 | 906 | GGCCAGGTCATGAACATTTTCGTGATACTCCCCGCCCTGATCATCCCCCTCACGACG GTCATTCCCCAGCCAGCAGAGAACGCTCCTCTTCTCTCCAAGGCATGGACATGGCAT CCCTACCTCCCCGAAAGCGCCCCTGGCATGATGGCCCAGGCACTTCTGAGCACAGA GAGATGGAGGCCCCAGGAGGCCCTTCTGAAGACCGAGGAGGCAAAG |
| 9213 | XPR1 | HSX2_01392082 | 907 | GGAGAGAAGCGCAGCGCCGCGCCGCGCCGGGGCCCATGTGGGGAGGAGTCGGAG TCGCTGTTGCCGCCGCCGCCTGTAGCTGCTGGACCCGAGTGGGAGTGAGGGGGAA ACGGCAGGATGAAGTTCGCCGAGCACCTCTCCGCGCACATCACTCCCGAGTGGAG GAAGCAATACATCCAGTATGAG |
| 9213 | XPR1 | HSX2_01392085 | 908 | GCTTTCAAGGATATGCTGTATTCAGCTCAGGACCAGGCACCTTCTGTGGAAG |
| 9213 | XPR1 | HSX2_01392094 | 909 | AGAAGCTCGCAGAGGCTCAGCGCAGGTTTGCTACACTTCAGAATGAGCTTCAGTCA TCACTGGATGCACAGAAAGAAAGCACTGGTGTTACTACGCTGCGACAACGCAGAA AGCCAGTCTTCCACTTGTCCCATGAGGAACGTGTCCAACATAGAAATATTAAAGAC CTTAAACTGGCCTTCAGTGAGTTCTACCTCAGTCTAATCCTGCTGCAGAACTATCAG |
| 9213 | XPR1 | HSX2_01392124 | 910 | GATCAGCTGAACAGCCTGTCAGTGATACTGATGGACCTGGAATATATGATCTGCTT CTACAGTTTGGAGCTCAAATGGGATGAAAGTAAGGGCCTGTTGCCAAATAATTCAG AAG |
| 9213 | XPR1 | HSX2_01392129 | 911 | CCTGCTTGGCTTCGCTTCATCCAGTGCCTGCGCCGATATCGAGACACAAAAGGGC CTTTCCTCATTTAGTTAATGCTGGCAAATACTCCACAACTTTCTTCATGGTGACGTTT GCAGCCCTTTACAGCACTCACAAAG |
| 9213 | XPR1 | HSX2_01392144 | 912 | GCGATTTGTGTGGAACTTCTTCCGCCTGGAGAATGAACATCTGAATAACTGTGGTG AATTCCGTGCTGTGCGGGACATCTCTGTGGCCCCCCTGAACGCAGATGATCAGACT CTCCTAGAACAGATGATGGACCAGGATGATGGGGTACGAAACCGCCAGAAGAATC GGTCATGGAAGTACAACCAGAGCATATCCCTGCGCCGGCCTCGCCTCGCTTCTCA |
| 65986 | ZBTB10 | HSX2_01994861 | 913 | GGGGCGAGCCTGGGCGACGGCAGCGGGGCGGAAGGCGGCAGCTGCAGCAGCAG CAGGCGGTCGGGCGGCGATGGCGGGGACGAAGTGGAGGGCAGCGGTGTGGGAG CTGGCGAAGGAGAGACTGTCCAGCACTTCCCGCTCGCGCGGCCCAAGTCTCTAATG CAGAAGCTCCAATGCTCCTTCCAGACCTCCTGGCTCAAGGACTTTCCCTGGCTGCGC TATTCCAAGGATACTGGTCTTATGTCTTGCGGCTGGTGCCAAAAGACCCCTGCAGA TGGGGGAAGCGTGGACCTTCCCCCAGTGGGGCATGATGAGCTTTCGCGAGGGACC CGCAACTACAAGAAAACCCTCCTCCTGAGGCACCACGTCTCTACCGAGCACAAACTC CACGAAGCCAACGCCCAG |
| 340595 | ZCCHC16 | HSX2_02359509 | 914 | CTTTGGCTGAAGTCCAAATGACATTCCTATAATCTGCTTATATTCTCCTCCTAGAGTT TGGACCATGCTTCACCTATTGACTACGATCTACCTTCAGCATAAATCAACCTTCCTG GAACCACAGCTCTGCCACCCTTCCATAGAGCTATATGTTTTGTTTCCATCTAAAGACT CATGCTTTCCCAACAAACAGAACCTCCTGGCTATTTACAAACCAGATTGAATGGGCA TTTGAATACTGCTTGGAACTGTTGGTCCCTTTTTCTTTGCAGGCTAGCCCATCAGGC AGGTGTATGCTTTTAGCCAAGCCCACCAATTCCAACTTATTCCTCTCTTCTCCAACAA CCCTACTTGAGGTTCCAGCATGTCTTATAGCCAAGTCAATATGCTCTAGGATAAGGA TTTCTGAAAGGTATTTAGTGAGAGTTGGGGATGGTGTGTCATAATTACTGTATCTG TAGCATGTCCTTGCTGCCTTGGACAAGGGACCTGGCCAAATAAATCTCCACTGAAG ACTGACAGCCAGACCCTGCAGTAGTTTCTAACTCTAGAAGCCTCAATGTAGTCACCA TCTAGGATTACTACTCATTGCTATTGAGCTCTACCTTTATGGACCACCTCTGAAGTCC TTCATCTAATTGAGGATCTTAAAAAACAATGAATGGCAAAAAACTTCCTACACTATC TCATCTGAAAAATCCTGGGGCACATACATTAAAACCATTTGTGCACAAAACTCAGG AAGTCGGGATTTTATTGCTTATGTTTTACTCTTGGTACACACCAGCTTTGTGTAGATT CCTGATGCTTCTGAACAAAAGTAGTGTTATGCTAGAAAGGCAAAACTCCCTAAAAT GTTAAAACTGTTCAGCAATGATCACTGCTGGTAATTACCAGCCCTCTGTTCCCTGCT TCTTGTTTCTGATGTATGTCCCCAAAATGGATATCCTGGAGAAATATCAGTAATCAC ATATTCCCTAGCAACCATACCATTGGCCTTTTCCATGAAGAAGAGGCAACATTGAGT GGCGGACAACACTTTTTGGCATCTCTCCAATTCAGCTGTTAGCATCTCCTAGTGTCT TCTTCACAGCTAACAGCTTTGGCTACTTGCTCATGACACTCCGTCTCTGATCACAGA GCAAATCCACTGTCTCCATTGGTTTCCAGTACTAGTACTCAATTCTACTCAATCCCAC CTGATTCCAGGAGACATAATGGAGAAGTGCACGAAATCATCATCTACCATGCAGGT AGAGCCTTCCTTTCTTCAGGCAGAGAATCTGATTCTGCGGCTTCAAATGCAGCATCC AACCACGGAGAACACTGCTAAAAGGGGCCAAGTCATGCCTGCCCTGGCCACCACA GTGATGCCTGTACCATACTCACTTGAGCATCTCACCCAGTTTCATGGTGACCCTGCC AATTGCTCAGAGTTCCTCACTCAGGTGACTACCTACTTGACAGCTCTCCAAATCTCT AATCCTGCAAATGATGCCCAGATCAAACTCTTTTTTGATTACCTATCTCAGCAGTTA GAAAGTTGTGGGATCATATCTGGGCCTGACAAGAGTACCTTACTGAAGCAATATGA GAATCTTATTCTTGAGTTCCAGCAGTCATTTGGTAAACCCACAAAACAGGAAATCAA TCCTCTGATGAATGCTAAGTTTGACAAAGGAGACAACTCCTCTCAGCAGGACCCTG CTACTTTCCACCTCCTCGCTCAAAATCTGATCTGTAATGAAACCAATCAGAGTGGTC AGTTCGAAAAGGCACTAGCTGATCCCAACCAGGATGAAGAGAGTGTCACTGATAT GATGGACAATCTTCCAGACCTGATCACTCAGTGCATTCAGTTGGACAAGAAACATA GTGACAGGCCAGAGCTCCTACAGTCAGAGACCCAGCTCCCATTGTTGGCTTCCTTG ATCCAACACCAAGCCCTCTTTAGCCCCACAGATCCACCACCCAAGAAAGGGCCTATA |
| 51364 | ZMYND10 | HSX2_01761846 | 915 | GAGGTGTGTGAGTCAGCAGAAGACACTGTCTTGGACTTGGTAGACTATTGCCACCG CAAACTGACCCTGCTGGTGGCCCAGAGTGGCTGTGGTGGCCCCCCTGAGGGGGAG GGATCCCAGGACAGCAACCCCATGCAG |
| 155054 | ZNF425 | HSX2_02256272 | 916 | CCTGGAGAAGTGGCAGAAGCAAATGTATAAGCAAGAGATGAAGACCAATTACGAG ACCCTTGATTCCCTGG |
| 57474 | ZNF490 | HSX2_01923238 | 917 | GCTGGATCCTGGCCAGAGGAATATCTACAGAGATGTGATGCGGGCAACCTTCAAG AACCTGGCCTGTATAG |
| 23140 | ZZEF1 | HSX2_01600756 | 918 | GTGCAGCAGAAGGAAAGTGGCTTAAAATATAAATCTTTTCTGGACTTCGCGGGTCT TGATTTGCAGATCTTCTGGAATTTTTACAGTAAATTAAAGCAAAA |

## Claims

1. A method for the diagnosis of thyroid cancer in a mammal, comprising measuring an expression product of at least two genes selected from CITED1, TENM1, CCM2, SCLY and IGSF10, or measuring at least two distinct domains of at least one expression product of a gene selected among CITED1, TENM1, CCM2, SCLY and IGSF10, in a sample from the mammal and diagnosing said thyroid cancer by comparing the measured value to a reference value.

2. The method of claim 1, for determining whether said mammal has or is likely to have a malignant thyroid lesion or nodule.

3. The method of claim 1 or 2, which comprises measuring an expression product of at least the CITED1 and TENM1 genes.

4. The method of claim 1 or 2, which comprises measuring an expression product of at least the CITED 1, TENM1, CCM2, SCLY and IGSF10 genes.

5. The method of anyone of the preceding claims, wherein measuring an expression product comprises determining the presence, absence or amount of said expression product.

6. The method of anyone of the preceding claims, which comprises determining the presence, absence or amount of at least two distinct domains of an expression product of a CITED1 gene.

7. The method of anyone of the preceding claims, which comprises determining the presence, absence or amount of a sequence encoded by exon4 of the CITED1 gene.

8. The method of anyone of the preceding claims, which comprises determining the presence, absence or amount of at least one sequence encoded by exon4, exon3 and/or exon (-2) of the CITED1 gene.

9. The method of anyone of the preceding claims, which comprises determining the presence, absence or amount of at least two distinct domains of an expression product of the TENM1 gene.

10. The method of anyone of the preceding claims, which comprises determining the presence, absence or amount of at least one sequence encoded by exon1, 3, 13, 15, 17, 18, 19, 25, or 32 of the TENM1 gene, preferably by exon1, 19 or 25.

11. The method of anyone of the preceding claims, which further comprises measuring an expression product of the FRMD3 gene.

12. The method of anyone of the preceding claims, which further comprises measuring an expression product of at least one gene listed in Table A, preferably of each gene listed in Table A.

13. The method of anyone of the preceding claims, which further comprises measuring an expression product of at least one gene listed in Table B, preferably of each gene listed in Table B.

14. The method of anyone of the preceding claims comprising:
a. providing a biological sample from the subject having a thyroid condition;
b. performing a cytological exam of the sample to classify the thyroid condition in a category according to the Bethesda classification, and
c. for any sample of indeterminate cytology, diagnosing thyroid cancer by measuring in said biological sample an expression product as defined in anyone of the preceding claims.

15. The method of any one of the preceding claims, wherein the gene products in the sample are measured simultaneously or sequentially.

16. The method of any one of the preceding claims, wherein each gene product is measured by selective hybridization with specific nucleic acid probes and/or by selective amplification with specific nucleic acid primers.

17. The method of any one of the preceding claims, wherein the measured value is compared to a reference value of a control benign thyroid condition and to a reference value of a control malignant thyroid condition, wherein, when the measured value is closer to the reference value of the control benign thyroid condition, then the tested thyroid condition is benign, and when the measured value is closer to the reference value of the control malignant thyroid condition, then the tested thyroid condition is malignant.

18. A method for determining in vitro or ex vivo the benign or malignant state of a thyroid condition in a subject, wherein said method comprises determining in a biological sample from the subject the variation of expression of at least two genes selected from CITED1, TENM1, CCM2, SCLY and IGSF10 or of at least two distinct domains of at least one expression product of a gene selected among CITED1, TENM1, CCM2, SCLY and IGSF10.

19. The method of claim 18, wherein an increased expression as compared to a control non-malignant thyroid tissue is indicative of a malignant status.

20. The method of any one of the preceding claims, wherein the sample is a thyroid tissue biopsy or a fine needle thyroid aspirate.

21. The method of any one of the preceding claims, wherein the mammal is a human subject.

22. A kit comprising a solid support comprising at least two capture agents attached thereto, wherein the capture agents each binds a distinct gene product or domain as defined in any one of claims 1 to 13.

23. A product comprising a support on which are immobilised at least two distinct nucleic acid probes, each probe comprising a sequence complementary to and specific of a target nucleic acid selected from anyone of the sequences listed as SEQ ID NOs: 1-918, or the complementary strand thereof.

24. A product of claim 23, comprising at least two probes selected from anyone of the sequences listed as SEQ ID NO: 919 to 968.

25. Use of a product or kit according to any one of claims 22 to 24, for *in vitro* or *ex vivo* diagnosis of thyroid cancer in a subject.

26. Use of a set of at least two probes, each probe comprising a sequence complementary to and specific of a distinct target nucleic acid selected from anyone of the sequences listed as SEQ ID NOs: 1-918, or the complementary strand thereof, for *in vitro* or *ex vivo* diagnosis of thyroid cancer in a subject.

27. Use of a set of at least two pairs of primers, each pair or primers comprising a sense or antisense nucleic acid primer complementary to and specific of a target nucleic acid selected from SEQ ID NOs: 1-918, or the complementary strand thereof, for *in vitro* or *ex vivo* diagnosis of thyroid cancer in a subject.
